# EUROPEAN PATENT APPLICATION

(11) **EP 1 321 518 A1**
(43) Date of publication of application: **25.06.2003**
(21) Application number: 02258507.9
(22) Date of filing: 10.12.2002
(51) Int. Cl.: C12N 9/12

(54) **Modified MEK1 and MEK2, crystal of a peptide: ligand: cofactor complex containing such modified MEK1 or MEK2, and methods of use thereof**

(30) Priority: 21.12.2001 US 341882 P
(71) Applicant: WARNER-LAMBERT COMPANY, Morris Plains, New Jersey 07950 (US)
(72) Inventor: Chen, Huifen, Pfizer Global R&D, Ann Arbor, Michigan 48105 (US); Delaney, Amy Marie, Pfizer Global R&D, Ann Arbor, Michigan 48105 (US); Dudley, David Thomas, Pfizer Global R&D, Ann Arbor, Michigan 48105 (US); Hasemann, Charles Albert, Jr., Pfizer Global R&D, Ann Arbor, Michigan 48105 (US); Kuffa,Peter, Pfizer Global R&D, Ann Arbor, Michigan 48105 (US); McConnell, Patrick Charles, Pfizer Global R&D, Ann Arbor, Michigan 48105 (US); Ohren, Jeffrey Francis, Pfizer Global R&D, Ann Arbor, Michigan 48105 (US); Pavlovsky, Alexander Gregory, Pfizer Global R&D, Ann Arbor, Michigan 48105 (US); Tecle, Haile, Pfizer Global R&D, Ann Arbor, Michigan 48105 (US); Whitehead, Christopher Emil, Pfizer Global R&D, Ann Arbor, Michigan 48105 (US); Yan, Chunhong, Pfizer Global R&D, Ann Arbor, Michigan 48105 (US); Zhang, Erli, Pfizer Global R&D, Ann Arbor, Michigan 48105 (US)
(74) Representative: England, Peter Michael

(57) **Abstract**

Modified MEK1 peptides and modified MEK2 peptides, polynucleotides encoding those peptides, and methods for purifying the peptides and crystallizing them as peptide: cofactor: ligand complexes have been discovered. The three-dimensional structures of MEK1 peptide and MEK2 peptide, including the cofactor-and ligand-binding pockets, and uses of this information, for example, in molecular replacement and the modification, design and screening of compounds that may associate with MEK1, MEK2, or peptides structurally related thereto, have also been discovered.

SEQUENCE LISTING

<110> Warner-Lambert Company

<120> MODIFIED MEK1 AND MEK2, CRYSTAL OF A PEPTIDE: LIGAND: COFACTOR
COMPLEX CONTAINING SUCH MODIFIED MEK1 AND MEK2, AND METHODS OF USE
THEREOF

<130> PC20519A

<150> US 60/341,882

<151> 2001-12-21

<160> 19

<170> PatentIn version 3.1

<210> 1

<211> 2222

<212> DNA

<213> Homo sapiens

<400> 1
attcggcacg agggaggaag cgagaggtgc tgccctcccc ccggagttgg aagcgcgtta 60

cccgggtcca aaatgcccaa gaagaagccg acgcccatcc agctgaaccc ggcccccgac 120

ggctctgcag ttaacgggac cagctctgcg gagaccaact tggaggcctt gcagaagaag 180

ctggaggagc tagagcttga tgagcagcag cgaaagcgcc ttgaggcctt tcttacccag 240

aagcagaagg tgggagaact gaaggatgac gactttgaga agatcagtga gctgggggct 300

ggcaatggcg gtgtggtgtt caaggtctcc cacaagcctt ctggcctggt catggccaga 360

aagctaattc atctggagat caaacccgca atccggaacc agatcataag ggagctgcag 420

gttctgcatg agtgcaactc tccgtacatc gtgggcttct atggtgcgtt ctacagcgat 480

ggcgagatca gtatctgcat ggagcacatg gatggaggtt ctctggatca agtcctgaag 540

aaagctggaa gaattcctga acaaatttta ggaaaagtta gcattgctgt aataaaaggc 600

ctgacatatc tgagggagaa gcacaagatc atgcacagag atgtcaagcc ctccaacatc 660

ctagtcaact cccgtgggga gatcaagctc tgtgactttg gggtcagcgg gcagctcatc 720

gactccatgg ccaactcctt cgtgggcaca aggtcctaca tgtcgccaga aagactccag 780

gggactcatt actctgtgca gtcagacatc tggagcatgg gactgtctct ggtagagatg 840

gcggttggga ggtatcccat ccctcctcca gatgccaagg agctggagct gatgtttggg 900

tgccaggtgg aaggagatgc ggctgagacc ccacccaggc caaggacccc cgggaggccc 960
cttagctcat acggaatgga cagccgacct cccatggcaa tttttgagtt gttggattac 1020
atagtcaacg agcctcctcc aaaactgccc agtggagtgt tcagtctgga atttcaagat 1080
tttgtgaata aatgcttaat aaaaaacccc gcagagagag cagatttgaa gcaactcatg 1140
gttcatgctt ttatcaagag atctgatgct gaggaagtgg attttgcagg ttggctctgc 1200
tccaccatcg gccttaacca gcccagcaca ccaacccatg ctgctggcgt ctaagtgttt 1260
gggaagcaac aaagagcgag tcccctgccc ggtggtttgc catgtcgctt ttgggcctcc 1320
ttcccatgcc tgtctctgtt cagatgtgca tttcacctgt gacaaaggat gaagaacaca 1380
gcatgtgcca agattctact cttgtcattt ttaatattac tgtctttatt cttattacta 1440
ttattgttcc cctaagtgga ttggctttgt gcttggggct atttgtgtgt atgctgatga 1500
tcaaaacctg tgccaggctg aattacagtg aaatttttgg tgaatgtggg tagtcattct 1560
tacaattgca ctgctgttcc tgctccatga ctggctgtct gcctgtattt tcggactttg 1620
acatttgaca tttggtggac tttatcttgc tgggcatact ttctctctag gagggagcct 1680
tgtgagatcc ttcacaggca gtgcatgtga agcatgcttt gctgctatga aaatgagcat 1740
cagagagtgt acatcatgtt attttattat tattatttgc ttttcatgta gaactcagca 1800
gttgacatcc aaatctagcc agagcccttc actgccatga tagctggggc ttcaccagtc 1860
tgtctactgt ggtgatctgt agacttctgg ttgtatttct atatttattt tcagtatact 1920
gtgtgggata cttagtggta tgtctcttta agttttgatt aatgtttctt aaatggaatt 1980
atttgaatgt cacaaattga tcaagatatt aaaatgtcgg atttatcttt ccccatatcc 2040
aagtaccaat gctgttgtaa acaacgtgta tagtgcctaa aattgtatga aaatcctttt 2100
aaccatttta acctagatgt ttaacaaatc taatctctta ttctaataaa tatactatga 2160
aataaaaaaa aaaggagaaa gctaaaaaaa aaaaaaaaaa aaaaaaaaaa aaaaaaaaaa 2220
aa 2222

<210> 2
<211> 393
<212> PRT
<213> Homo sapiens

<400> 2

<210>  3

<211>  1768

<212>  DNA

<213>  Homo sapiens

<400>  3
   ggcacgaggc ccctgcctct cggactcggg ctgcggcgtc agccttcttc gggcctcggc      60
   agcggtagcg gctcgctcgc ctcagcccca gcgcccctcg gctaccctcg gcccaggccc     120
   gcagcgccgc ccgccctcgg ccgccccgac gccggcctgg gccgcggccg cagccccggg     180
   ctcgcgtagg cgccgaccgc tcccggcccg ccccctatgg gccccggcta gaggcgccgc     240
   cgccgccggc ccgcggagcc ccgatgctgg cccggaggaa gccggtgctg ccggcgctca 300
   ccatcaaccc taccatcgcc gagggcccat cccctaccag cgagggcgcc tccgaggcaa 360
   acctggtgga cctgcagaag aagctggagg agctggaact tgacgagcag cagaagaagc 420
   ggctggaagc ctttctcacc cagaaagcca aggtcggcga actcaaagac gatgacttcg 480
   aaaggatctc agagctgggc gcgggcaacg gcggggtggt caccaaagtc cagcacagac 540
   cctcgggcct catcatggcc aggaagctga tccaccttga gatcaagccg gccatccgga 600
   accagatcat ccgcgagctg caggtcctgc acgaatgcaa ctcgccgtac atcgtgggct 660
   tctacggggc cttctacagt gacggggaga tcagcatttg catggaacac atggacggcg 720
   gctccctgga ccaggtgctg aaagaggcca agaggattcc cgaggagatc ctggggaaag 780
   tcagcatcgc ggttctccgg ggcttggcgt acctccgaga gaagcaccag atcatgcacc 840
   gagatgtgaa gccctccaac atcctcgtga actctagagg ggagatcaag ctgtgtgact 900
   tcggggtgag cggccagctc atagactcca tggccaactc cttcgtgggc acgcgctcct 960
   acatggctcc ggagcggttg cagggcacac attactcggt gcagtcggac atctggagca 1020
   tgggcctgtc cctggtggag ctggccgtcg gaaggtaccc catccccccg cccgacgcca 1080
   aagagctgga ggccatcttt ggccggcccg tggtcgacgg ggaagaagga gagcctcaca 1140
   gcatctcgcc tcggccgagg ccccccgggc gccccgtcag cggtcacggg atggatagcc 1200
   ggcctgccat ggccatcttt gaactcctgg actatattgt gaacgagcca cctcctaagc 1260
   tgcccaacgg tgtgttcacc cccgacttcc aggagtttgt caataaatgc ctcatcaaga 1320
   acccagcgga gcgggcggac ctgaagatgc tcacaaacca caccttcatc aagcggtccg 1380
   aggtggaaga agtggatttt gccggctggt tgtgtaaaac cctgcggctg aaccagcccg 1440
   gcacacccac gcgcaccgcc gtgtgacagt ggccgggctc cctgcgtccc gctggtgacc 1500
   tgcccaccgt ccctgtccat gccccgccct tccagctgag gacaggctgg cgcctccacc 1560
   caccctcctg cctcacccct gcggagagca ccgtggcggg gcgacagcgc atgcaggaac 1620
   gggggtctcc tctcctgccc gtcctggccg gggtgcctct ggggacgggc gacgctgctg 1680
   tgtgtggtct cagaggctct gcttccttag gttacaaaac aaaacaggga gagaaaaagc 1740
   aaaaaaaaaa aaaaaaaaaa aaaaaaaa 1768

<210> 4

<211> 400

<212> PRT

<213> Homo sapiens

<400>  4

<210>  5

<211>  33

<212>  DNA

<213>  Artificial Sequence

<220>

<223>  Probe

<400>  5
catatgccga aaaagaagcc gaccccgatc cag                                33

<210>  6

<211>  25

<212>  DNA

<213>  Artificial Sequence



<220>

<223>  Probe

<400>  6
aagcttgacg ccagcagcat gggtt                                          25


<210>  7

<211>  29

<212>  DNA

<213>  Artificial Sequence



<220>

<223>  Primer

<400>  7
cttgcatatg gaggcctttc ttacccaga                                      29


<210>  8

<211>  29

<212>  DNA

<213>  Artificial Sequence



<220>

<223>  Primer

<400>  8
cttgaagctt cacctggcac ccaaacatc                                      29


<210>  9

<211>  30

<212>  DNA

<213>  Artificial Sequence

<220>

<223>  Primer

<400>  9
cttgcatatg gaactgaagg atgacgactt                                        30

<210>  10

<211>  29

<212>  DNA

<213>  Artificial Sequence

<220>

<223>  Primer

<400>  10
cttgcatatg cttgatgagc agcagcgaa                                         29

<210>  11

<211>  33

<212>  DNA

<213>  Artificial Sequence

<220>

<223>  Primer

<400>  11
catatgaagc cggtgctgcc ggcgctcacc atc                                    33

<210>  12

<211>  27

<212>  DNA

<213>  Artificial Sequence

<220>

<223>  Primer

<400>  12
aagcttggcc actgtcacac ggcggtg                                          27

<210>  13

<211>  24

<212>  DNA

<213>  Artificial Sequence

<220>

<223>  Primer

<400>  13
atgcttgacg agcagcagaa gaag                                             24

<210>  14

<211>  27

<212>  DNA

<213>  Artificial Sequence

<220>

<223>  Primer

<400>  14
atggaagcct ttctcaccca gaaagcc                                          27

<210>  15

<211>  24

<212>  DNA

<213>  Artificial Sequence

<220>

<223>  Primer

<400>  15
atggcctttc tcacccagaa agcc                                             24

<210>  16

<211>  23

<212>  DNA

<213>  Artificial Sequence

<220>

<223>  Primer

<400>  16
atgacccaga aagccaaggt tgg                                              23

<210>  17

<211>  27

<212>  DNA

<213>  Artificial Sequence

<220>

<223>  Primer

<400>  17
atggccaagg tcggcgaact caaagac                                          27

<210>  18

<211>  27

<212>  DNA

<213>  Artificial Sequence

<220>

<223>  Primer

<400>  18
atggtcggcg aactcaaaga cgatgac                                          27

<210>  19

<211>  47

<212>  DNA

<213>  Artificial Sequence

<220>

<223>  Primer

<400>  19
tcaatgatga tgatgatgat gttcaagcac agcggtgcgc gtgggtg                    47

## Description

### FIELD OF THE INVENTION

The present invention generally relates to forms of a MEK1 peptide and forms of MEK2 peptide, crystals of a MEK1 or MEK2 peptide : ligand : cofactor complex, methods for producing those crystals, three-dimensional structural information derived from crystallographic data and to methods using that structural information.

### BACKGROUND OF THE INVENTION

Mitogen-activated protein (MAP) kinases are thought to act as an integration point for multiple biochemical signals because they are activated by a wide variety of extracellular signals. The pattern of MAP kinase (MAPK) cascade is not restricted to growth factor signaling and it is now known that signaling pathways initiated by phorbol esters, ionophors, heat shock, and ligands for seven transmembrane receptors use distinct MAP kinase cascades with little or no cross-reactivity between them.

Multiple MAP kinases have been described in yeast including SMK1, HOG1, MPK1, FUS3, and KSS1. In mammals, the MAP kinases have been identified as extracellular signal-regulated kinase (ERK), c-Jun amino-terminal kinase (JNK), and p38 kinase (Davis, *Trends Biochem. Sci.* 19: 470 (1994)). These highly conserved MAP kinase isoforms are activated by dual phosphorylation on threonine and tyrosine residues.

A critical protein kinase lies upstream of the MAP kinase-mediated signaling pathway and stimulates the enzymatic activity of MAP kinases. The structure of this protein kinase, denoted MEK1, for MAP kinase /ERK kinase, was elucidated from a complementary DNA sequence and shown to be a protein of 393 amino acids (43.5-kD) that is related most closely in size and sequence to the product encoded by the *Schizosaccharomyces pombe* byr1 gene (Crews *et al*., *Science* 258: 478-480 (1992)). The MEK gene was highly expressed in murine brain, and the product expressed in bacteria phosphorylated the ERK gene product *in vitro.* As an essential component of the MAP kinase signal transduction pathway, MEK1 is involved in many cellular processes such as proliferation, differentiation, transcription regulation and development.

A cDNA encoding the human homolog of MEK1 was cloned from a human T-cell cDNA library, GenBank Accession Number L11284 (Seger *et al. J. Biol. Chem*.267: 25628-25631 (1992)). When overexpressed in COS cells, the predicted 43,439-Da protein led to increased phorbol ester-stimulated MAP kinase kinase activity. The human MEK1 shares 99% amino acid sequence identity with the murine MEK1 and 80% with human MEK2. Human MEK1 and MEK2 encode 393 and 400 amino acid residues, respectively. Both MEK1 and MEK2 were expressed in *E. coli* and shown to be able to activate recombinant human ERK1 *in vitro.* The purified MEK2 peptide stimulated threonine and tyrosine phosphorylation on ERK1 and concomitantly activated ERK1 kinase activity more than 100-fold. The recombinant MEK2 showed lower activity as an ERK activator as compared with MEK2 purified from tissue. However, the recombinant MEK2 can be activated by serum-stimulated cell extract *in vitro.* MEKs, in a manner similar to ERKs, are likely to consist of a family of related proteins playing critical roles in signal transduction. (Zheng *et al., J. Biol. Chem.* May 25; 268(15): 11435-9 (1993))

Constitutive activation of MEK1 results in cellular transformation. This protein kinase therefore represents a likely target for pharmacological intervention in proliferative and inflammatory diseases (Lee *et al*., *Nature* 372, 739-746 (1994); Dudley *et al., Proc. Natl. Acad. Sci. U.S.A.* 92, 7686-7689 (1995)). In order to identify small-molecule inhibitors of this pathway, Sebolt-Leopold *et al.* developed an *in vitro* cascade assay using recombinantly expressed glutathione-S-transferase fusion proteins of MEK1 and ERK1 (Sebolt-Leopold *et al., Nature Med*. 5: 810-816(1999)). Sebolt-Leopold *et al.* also reported the discovery of a highly potent and selective inhibitor of MEK1, 2-(2-chloro-4-iodo-phenylamino)-N-cyclopropylmethoxy-3,4-difluoro-benzamide. This article reported that after treatment with this inhibitor, tumor growth was inhibited as much as 80% in mice with colon carcinomas of both mouse and human origin. Efficacy was achieved with a wide range of doses with no signs of toxicity, and correlated with a reduction in levels of MAPK in excised tumors. It was concluded that these data indicate that MEK inhibitors represent a promising, non-cytotoxic approach to the clinical management of colon cancer.

Influenza A viruses are significant causes of morbidity and mortality worldwide. Annually updated vaccines may prevent disease, and anti-virals are effective treatment early in disease when symptoms are often nonspecific. Viral replication is supported by intracellular signaling events. Using a nontoxic inhibitor of MEK1 and MEK2, and thus an inhibitor of the RAF1/MEK/ERK pathway, Pleschka *et al.* examined the cellular response to infection with influenza A. The inhibitor suppressed both the early and late ERK activation phases after virus infection. Inhibition of the signaling pathway occurred without impairing the synthesis of viral RNA or protein, or the import of viral ribonucleoprotein complexes (RNP) into the nucleus (Pleschka *et al., Nature Cell Biol.* 3: 301-305 (2001)). Instead, it inhibited RAF/MEK/ERK signaling and the export of viral RNP without affecting the cellular mRNA export pathway. It was proposed that ERK regulates a cellular factor involved in the viral nuclear export protein function. An experiment using a nontoxic inhibitor of MEK1 and MEK2 to examine the cellular response to infection with influenza A suggested that local application of MEK inhibitors may have only minor toxic effects on the host while inhibiting viral replication without giving rise to drug-resistant virus variants (Pleschka *et al., Nature Cell Biol.* 3: 301-305 (2001)).

Ryan *et al.* showed that inhibition of MEK1 blocks p53-induced NF-kappa-B activation and apoptosis but not cell-cycle arrest. They demonstrated that p53 activates NF-kappa-B through the RAF/MEK1/p90(rsk) pathway rather than the TNFR1/TRAF2/IKK pathway used by TNFA *(Nature* 404: 892-897 (2000)).

One such means of mediating signal transduction through the MEK1 pathway is to identify enhancers or inhibitors to the MEK1 peptide. Such identification has heretofore relied on serendipity and/or systematic screening of large numbers of natural and synthetic compounds. A superior method of drug development relies on structure assisted drug design. In this case, the three-dimensional structure of a peptide-inhibitor complex is determined and potential enhancers and/or potential inhibitors are screened and/or designed with the aid of computer modeling [Bugg *et al.*, Scientific American, Dec.: 92-98 (1993); West *et al.*, TIPS, 16: 67-74 (1995); Dunbrack *et al.*, Folding & Design, 2: 27-42 (1997)]. However, heretofore the three-dimensional structure of the MEK1 peptide or MEK2 peptide has remained unknown, essentially because no MEK1 peptide or MEK2 peptide crystals have been produced of sufficient quality to allow the required X-ray crystallographic data to be obtained. Therefore, there is presently a need for obtaining a form of the MEK1 peptide or MEK2 peptide that can be crystallized with a ligand (such as an inhibitor) to form a crystal with sufficient quality to allow such crystallographic data to be obtained. Further, there is a need for such crystals. There is also a need for the determination of the three-dimensional structure of such crystals. Finally, there is a need for procedures for related structural based drug design and/or screening based on the crystallographic data.

USP 5,663,314 to Seger *et al*. discloses two types of MEK1 peptides, namely, MKK1a and MKK1b and nucleotide encoding them. MKK1a has 399 amino acid residues and MKK1b has 368 amino acid residues that is identical to MKK1a except lacking amino acid residues from 147 to 172. The modification of the MEK1 peptide by truncation of the NH₂-terminal region or by removal or replacement of the insertion loop-forming region, or by a combination of these modifications to improve the peptide's biophysical characteristics, especially with respect to crystallizability, has not previously been reported.

The citation of any reference herein should not be construed as an admission that such reference is available as "Prior Art" to the instant application.

### SUMMARY OF THE INVENTION

The present invention provides modified MEK1 peptide and modified MEK2 peptide amino acid sequences, and methods for producing such modified MEK1 and MEK2 peptide sequences. In one embodiment, the MEK1 and MEK2 peptides may be modified omitting a significant portion of the NH₂-terminal region of the MEK1 and MEK2 peptides (hereinafter referred to as "NH₂-terminally truncated MEK1 peptide" and "NH₂-terminally truncated MEK2 peptide", respectively, or collectively as "NH₂-terminally truncated MEK1 and MEK2 peptides") and/or by deletion of the MEK1 insertion loop domain and/or by replacement of the MEK1 insertion loop domain with a linker peptide (hereinafter, MEK1 or MEK2 having either one or all of these modifications is referred to as "modified MEK1," "modified MEK2," or collectively as "modified MEK1 and MEK2").

The NH₂-terminally truncated MEK1 peptides lack at least 30 amino acid residues from the NH₂-terminal region of the full-length peptide. Preferably, the NH₂-terminally truncated MEK1 peptides lack at least 30 to at most 70 amino acid residues, more preferably 41 to 61 amino acid residues from the NH₂-terminal region of the full-length peptide, or a conservatively substituted variant thereof.

The NH₂-terminally truncated MEK2 peptides lack at least 34 amino acid residues from the NH₂-terminal region of the full-length peptide of SEQ ID NO: 4. Preferably, the NH₂-terminally truncated MEK2 peptides lack at least 34 to at most 74 amino acid residues, more preferably 45 to 65 amino acid residues from the NH₂-terminal region of the full-length peptide, or a conservatively substituted variant thereof.

In another embodiment, the modified MEK1 peptide may have a deletion of insertion loop-forming amino acid residues, particularly from amino acid 280 to amino acid 323 of SEQ ID NO: 2; or at least 40 amino acids from between amino acid residue 264 and amino acid 310 of SEQ ID NO: 2. This may include a deletion of amino acids (1) from amino acid 264 to amino acid 310 of SEQ ID NO: 2; (2) from amino acid 270 to amino acid 310 of SEQ ID NO: 2; (3) from amino acid 264 to amino acid 305 of SEQ ID NO: 2; (4) amino acid 267 to amino acid 307 of SEQ ID NO: 2; or (5) from amino acid 265 to amino acid 304 of SEQ ID NO: 2.

Alternatively, the MEK1 insertion loop domain may be replaced by with a linker peptide, wherein the linker peptide may have at most 10 amino acid residues. Preferably, the linker peptide has the amino acid sequence of Lys-Asn-Cys-Lys-Thr-Asp.

The modified MEK1 peptides of the invention may be modified by any one or any combination of the NH₂-terminal truncation, the deletion of insertion loop-forming amino acid residues, and the replacement of the MEK1 insertion loop domain with a linker peptide described above.

The invention further provides peptides that are defined by the three-dimensional structural coordinates of the MEK1 or MEK2 peptides as set forth in Table 1 or Table 2 respectively, or of related peptides which possess the structural coordinates having a root mean square deviation of not more than about 1.25 Å away from the core C alpha atoms of the MEK1 or MEK2 peptides as set forth in Table 1 or Table 2.

Additionally, the invention provides peptides that comprise a MEK1, MEK2 or MEK-like cofactor or ligand binding pocket, as defined below.

The invention further provides isolated, purified polynucleotides, which encode the modified MEK1 and/or MEK2 peptide sequences. The polynucleotides may be natural or recombinant.

The invention further provides expression vectors for producing the modified MEK1 and MEK2 peptides in a host cell. It further provides host cells which may be stably transformed and transfected with a polynucleotide encoding the modified MEK1 peptides or modified MEK2 peptides, or a fragment thereof or an analog thereof, in a manner allowing the expression of the modified MEK1 peptides or MEK2 peptides. The present invention additionally includes cells transfected or transformed with an expression vector of the present invention. The present invention also includes methods for expressing a modified MEK1 peptide or modified MEK2 peptide comprising culturing a cell that expresses the modified MEK1 peptide or modified MEK2 peptide in an appropriate cell culture medium under conditions that provide for expression of the peptide by the cell.

Methods of purifying the modified MEK1 peptides or modified MEK2 peptides of the invention from a fermentation broth containing the modified MEK1 peptide or modified MEK2 peptide, respectively, and contaminant proteins other than the MEK1 peptide or MEK2 peptide, respectively, is also provided. The methods comprise subjecting the fermentation broth to immobilized metal chelate chromatography, which may preferably comprise pyrrole-2-carboxylate and/or zinc.

The invention also provides methods of using the purified modified MEK1 peptide or mofidied MEK2 peptide to produce ternary complexes of peptide: ligand: cofactor, and for producing crystals of the peptide: ligand: cofactor complexes containing the modified MEK1 peptide or modified MEK2 peptide. In one embodiment, a method of growing crystals of a peptide: ligand: cofactor complex comprises providing an aqueous solution of a modified MEK1 or MEK2 peptide, a ligand and a cofactor in a solution of ammonium acetate and a N-2-hydroxyethyl-piperazine-N'-2-ethansulfonic acid (HEPES) buffer; providing a precipitant solution comprising (a) if the peptide is MEK1 peptide, polyethylene glycol (PEG), a source of ionic strength, a buffering agent, and a reducing agent; or (b) if the peptide is MEK2 peptide, a source of ionic strength, a buffering agent, and a reducing agent; mixing a droplet of said peptide solution with a droplet of said precipitant solution; suspending the resulting mixed droplet over a well of said precipitant solution at a vapor pressure of the solution in said well being lower than in the resulting solution in the mixed droplet; and allowing the suspended mixed droplet to stand for a prolonged period until a peptide: ligand: cofactor ternary complex crystal grows to a size suitable for X-ray diffraction. The MEK1 peptide may have a concentration from about 10 to about 20 mg/mL and the HEPES further may have a pH from about pH 7 to about pH 8. The MEK2 peptide may have a concentration from about 10 to about 20 mg/mL and the HEPES further may have a pH from about pH 6.8 to about pH 8.8.

The invention also provides the crystal structure of the modified MEK1 peptide in a ternary complex with ligands and cofactors, from which MEK1 structural information may be obtained. Preferably, the ligand is 5-bromo-N-(2,3-dihydroxy-propxy)-3,4-difluoro-2-(2-fluoro-4-iodo-phenylamino)-benzamide and the cofactor is ATP. The crystals preferably diffract to a resolution better than 5.0 Å, preferably 3.0 Å. The crystal structure of the modified MEK1 peptide: 5-bromo-N-(2,3-dihydroxy-propxy)-3,4-difluoro-2-(2-fluoro-4-iodo-phenylamino)-benzamide: ATP complex is the first reported of the MEK1 peptide or any of the related MAPKK family.

Additionally, the invention provides the crystal structure of the modified MEK2 peptide in a ternary complex with ligands and cofactors, from which the MEK2 structural information may be obtained. Preferably, the ligand is {5-[3,4-Difluoro-2-(2-fluoro-4-iodo-phenylamino)-phenyl]-1,3,4-oxadiazol-2-yl}-(2-morpholin-4-yl-ethyl)-amine and the cofactor is ATP. The crystals preferably diffract to a resolution better than 5.0 Å, preferably 3.5 Å. The crystal structure of the modified MEK2: {5-[3,4-Difluoro-2-(2-fluoro-4-iodo-phenylamino)-phenyl]-1,3,4-oxadiazol-2-yl}-(2-morpholin-4-yl-ethyl)-amine: ATP complex is the first reported of the MEK2 peptide family.

The invention further includes the three-dimensional structural coordinates of the MEK1 peptide and MEK2 peptide: ligand: cofactor complexes, as set forth in Tables 1 and 2, or of a related set of structural coordinates having a root mean square deviation of preferably not more than about 1.25 Å away from the core C alpha atoms of the MEK1 or MEK2 the three-dimensional structural coordinates as set forth in Table 1 or Table 2. The structural coordinates reflect the three-dimensional structure of the MEK1 complexes and MEK2 complexes and illustrates to atomic resolution the chemical environment around the MEK1 and MEK2 ligand- and cofactor-binding sites.

Particularly, it has been discovered that MEK1 comprises a peptide ligand-binding pocket that is defined by the structural coordinates of the following amino acid residues within about 4 Å of a MEK1 inhibitor located in the ligand-binding site: G77, N78, G79, G80, K97, I99, L115, L118, V127, F129, I141, M143, C207, D208, F209, G210, V211, S212, L215, I216 and M219 of SEQ ID NO: 2 or a conservatively substituted variant thereof; and is defined by the structural coordinates of the following amino acid residues within about 5 Å of a MEK1 inhibitor located in the ligand-binding site: G77, N78, G79, G80, K97, I99, L115, L118, I126, V127, G128, F129, I141, M143, D190, N195, L206, C207, D208, F209, G210, V211, S212, L215, I216, M219 and F223 of SEQ ID NO: 2, or a conservatively substituted variant thereof.

It has also been discovered that MEK1 peptide comprises a cofactor-binding pocket that is defined by the structural coordinates of the following residues within about 4 Å of a cofactor (e.g. an ATP molecule) located in the cofactor-binding site: L74, G75, A76, G77, N78, G80, V81, V82, A95, K97, V127, M143, E144, H145, M146, G149, S150, D152, Q153, K192, S194, N195, L197, D208 and V224 of SEQ ID NO: 2, or a conservatively substituted variant thereof; and is defined by the structural coordinates of the following residues within about 5 Å of a cofactor located in the cofactor-binding site: L74, G75, A76, G77, N78, G79, 80, V81, V82, A95, K97, V127, M143, E144, H145, M146, D147, G149, S150, D152, Q153, D190, K192, S194, N195, L197, C207, D208, V224 and G225 of SEQ ID NO:2, or a conservatively substituted variant thereof.

Likewise, it has been discovered that MEK2 peptide comprises a ligand-binding pocket that is defined by the structural coordinates of the following amino acid residues within about 4 Å of a MEK2 inhibitor located in the ligand-binding site: G81, N82, G83, G84, K101, I103, L119, L122, V131, F133, I145, M147, C211, D212, F213, G214, V215, S216, L219, I220, M223 of SEQ ID NO: 4, or a conservatively substituted variant thereof; and is defined by structural coordinates of the following amino acid residues within about 5 Å of a MEK2 inhibitor located in the ligand-binding site: G81, N82, G83, G84, K101, I103, L119, L122, I130, V131, G132, F133, I145, M147, D194, N199, L210, C211, D212, F213, G214, V215, S216, L219, I220, M223, F227 of SEQ ID NO: 4, or a conservatively substituted variant thereof.

Additionally, it has been discovered that a MEK2 cofactor-binding pocket is defined by the structural coordinates of the following residues within about 4 Å of a cofactor located in the cofactor-binding site: L78, G79, A80, G81, N82, G84, V85, V86, A99, K101, V131, M147, E148, H149, M150, G153, S154, D156, Q157, K196, S198, N199, L201, D212, V228 of SEQ ID NO: 4, or a conservatively substituted variant thereof; and is defined by the structural coordinates of the following residues within about 5 Å of a cofactor located in the cofactor-binding site: L78, G79, A80, G81, N82, G83, G84, V85, V86, A99, K101, V131, M147, E148, H149, M150, D151, G153, S154, D156, Q157, D194, K196, S198, N199, L201, C211, D212, V228, G229 of SEQ ID NO: 4, or a conservatively substituted variant thereof.

A ligand- or cofactor-binding pocket of a MEK peptide or peptide that is structurally related to MEK1 or MEK2 peptide is also provided, wherein the ligandor cofactor-binding pocket is defined by the atoms found in the structural coordinates of the MEK1 or MEK2 peptide as set forth in Table 1 or Table 2, or in a related set of structural coordinates having a root mean square deviation of not more than preferably about 1.25 Å away from the binding pocket C alpha atoms of a MEK1 or MEK2 binding pockets.

The invention also provides a machine-readable medium having stored thereon data comprising the atomic coordinates as set forth in Table 1 or Table 2, or a related set of structural coordinates having a root mean square deviation of not more than preferably about 1.25 Å away from the core C alpha atoms of the MEK1 or MEK2 structural coordinates as set forth in Table 1 or Table 2

The invention also provides for applying the the atomic coordinates set forth in Table 1 or Table 2, or of a related set of atomic coordinates having a root mean square deviation of not more than preferably about 1.25 Å away from the core C alpha atoms of the MEK1 or MEK2 structural coordinates as set forth in Table 1 or Table 2, to a computer algorithm to generate a three-dimensional representation (also referred to as an "image") of a peptide or peptide binding pocket of the invention. This three-dimensional represenation can be used, for example, in methods for modifying, designing, screening and identifying, and evaluating chemical entities that have the potential to associate with MEK1, MEK2, or a structurally related peptide, and thus have the potential to be an inhibitor or enhancer of MEK1, MEK2, or a structurally related peptide.

Additionally, the invention provides methods for modifying or designing a chemical entity having the potential to associate with a peptide of the invention. The methods include generating a three-dimensional computer representation of the peptide or a binding pocket of the peptide and generating a chemical entity that spatially conforms to the three-dimensional representation of the peptide or the peptide binding pocket. The chemical entity may be generated by a method comprising (i) assembling molecular fragments into the chemical entity; (ii) de novo design of the chemical entity or a fragment thereof; (iii) selecting the chemical entity from a small molecule database; or (iv) modifying a known inhibitor, or portion thereof, which possess the ability to associate with either MEK1, MEK2 or the structurally related peptide.

The invention also provides methods for screening and identifying a potential inhibitor or enhancer of the activity of a peptide of the invention. The methods include generating a three-dimensional computer representation of the peptide or a binding pocket of the peptide; applying an iterative process whereby a chemical entity is applied to the three-dimensional representation to determine whether the chemical entity associates with the peptide or peptide binding pocket; and evaluating the effect(s) of the chemical entity on peptide activity to determine whether the chemical entity functions as an activity inhibitor or enhancer.

Methods for evaluating the potential of a chemical entity to associate with a peptide according to the invention are also provided. The methods include generating a three-dimensional representation of the peptide or a binding pocket of the peptide; applying a three-dimensional representation of chemical entity to the three-dimensional representation; and quantifying the association between the chemical entity and the binding pocket.

Additionally, the invention provides methods of utilizing molecular replacement to obtain structural information about a molecule or a molecular complex of unknown structure comprising crystallizing said molecule or molecular complex; generating an X-ray diffraction pattern from said crystallized molecule or molecular complex; and applying at least a portion of the structural coordinates set forth in Table 1 or Table 2 or having a set of structural coordinates with a root mean square deviation of preferably not more than about 1.25 Å from the core C alpha atoms of the MEK1 or MEK2 structural coordinates as set forth in Table 1 or Table 2, to the X-ray diffraction pattern to generate a three-dimensional electron density map of at least a portion of the molecule or molecular complex whose structure is unknown.

### BRIEF DESCRIPTION OF THE DRAWINGS

The application file contains at least one drawing executed in color. Copies of the patent application publication with color drawings will be provided by the Office upon request and payment of the necessary fee.

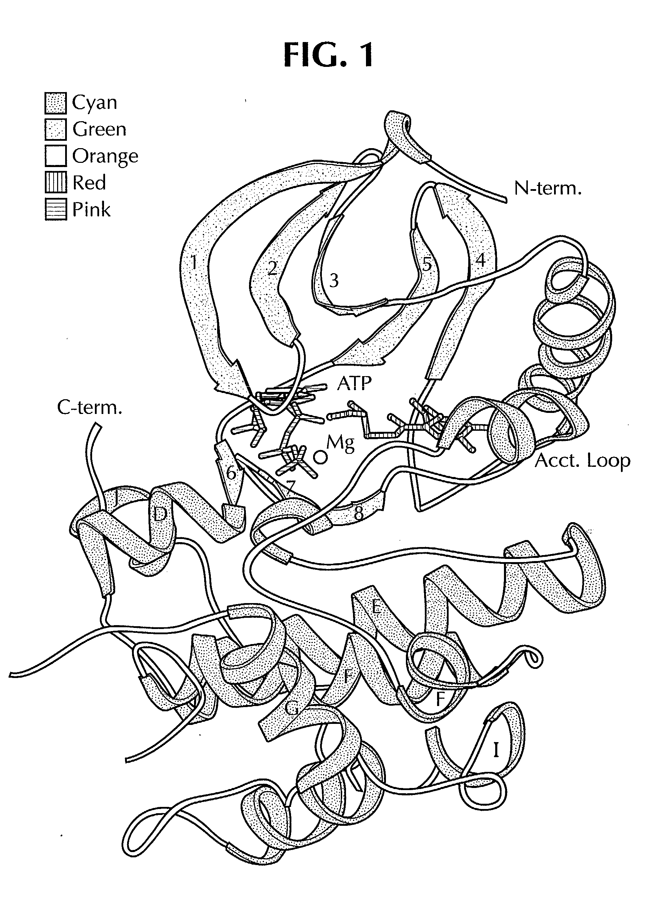

**Figure 1.** Figure 1 is a ribbon representation of the three-dimensional structure of the NH₂-terminally truncated MEK1 peptide structure, in a ternary complex with 5-bromo-N-(2,3-dihydroxy-propxy)-3,4-difluoro-2-(2-fluoro-4-iodo-phenylamino)-benzamide and MgATP. The alpha helical regions of the peptide are colored blue, the beta sheet regions are colored green, the ATP co-factor is purple, the magnesium atom is colored cyan and the inhibitor is colored red. The Mg-ATP molecule is bound at the active site cleft between the NH₂- and COOH-terminal lobes as found in other kinase structures, while the inhibitor binds in a pocket at the back of the cleft formed in part by the activation loop.

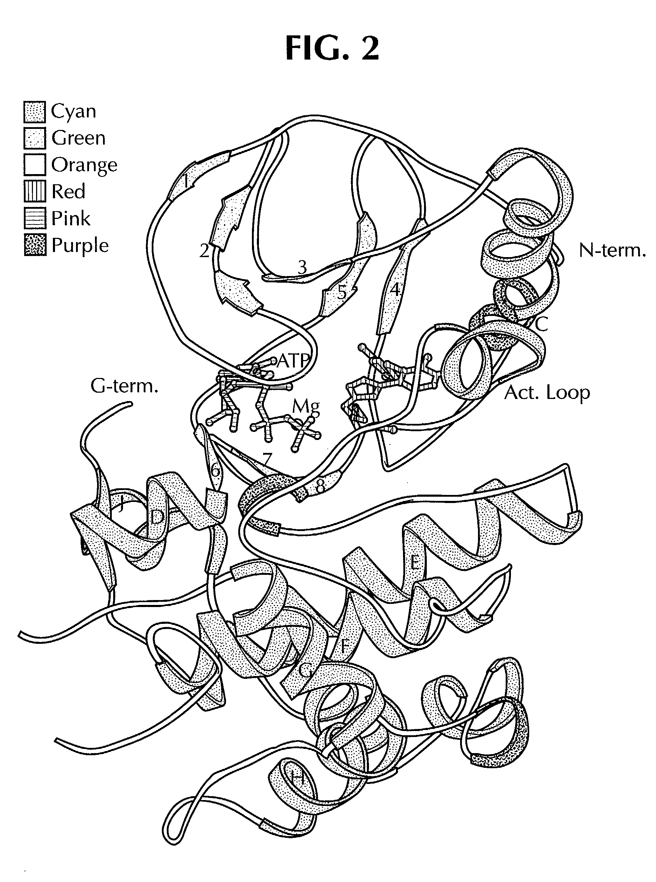

**Figure 2.** Figure 2 is a ribbon representation of the three-dimensional structure of the NH₂-terminally truncated MEK2 peptide structure, in a ternary complex with {5-[3,4-Difluoro-2-(2-fluoro-4-iodo-phenylamino)-phenyl]-1,3,4-oxadiazol-2-yl}-(2-morpholin-4-yl-ethyl)-amine and MgATP. The alpha helical regions of the peptide are colored blue, the beta sheet regions are colored green, the ATP co-factor is purple, the magnesium atom is colored cyan and the inhibitor is colored red. The MgATP molecule is bound at the active site cleft between the NH₂-and COOH-terminal lobes as found in other kinase structures, while the inhibitor binds in a pocket at the back of the cleft formed in part by the activation loop.

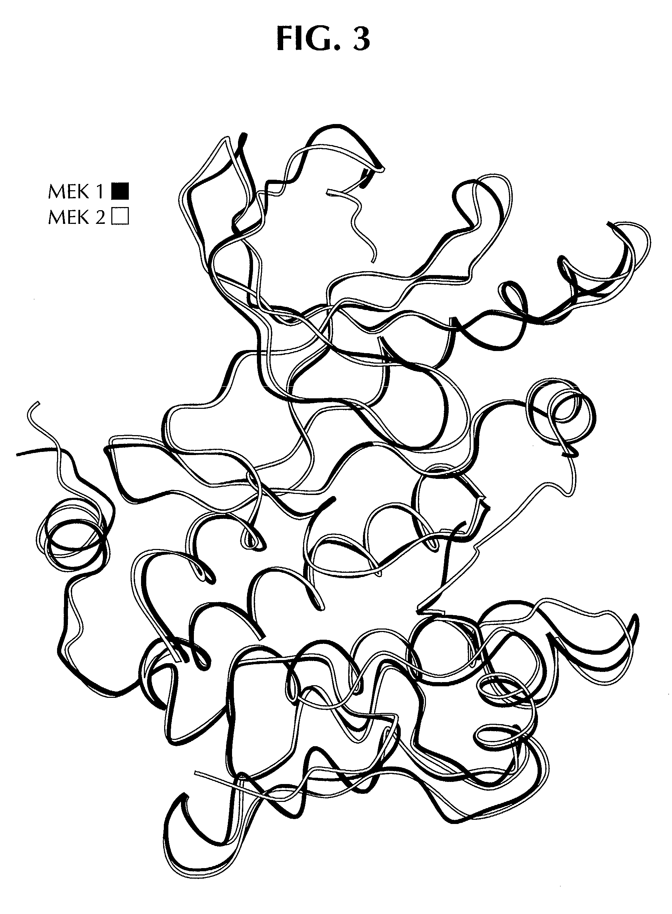

**Figure 3.** Figure 3 is superposition of the three-dimensional structures of MEK1 (blue) and MEK2 (red) peptides using the C alpha atoms of commonly observed residues.

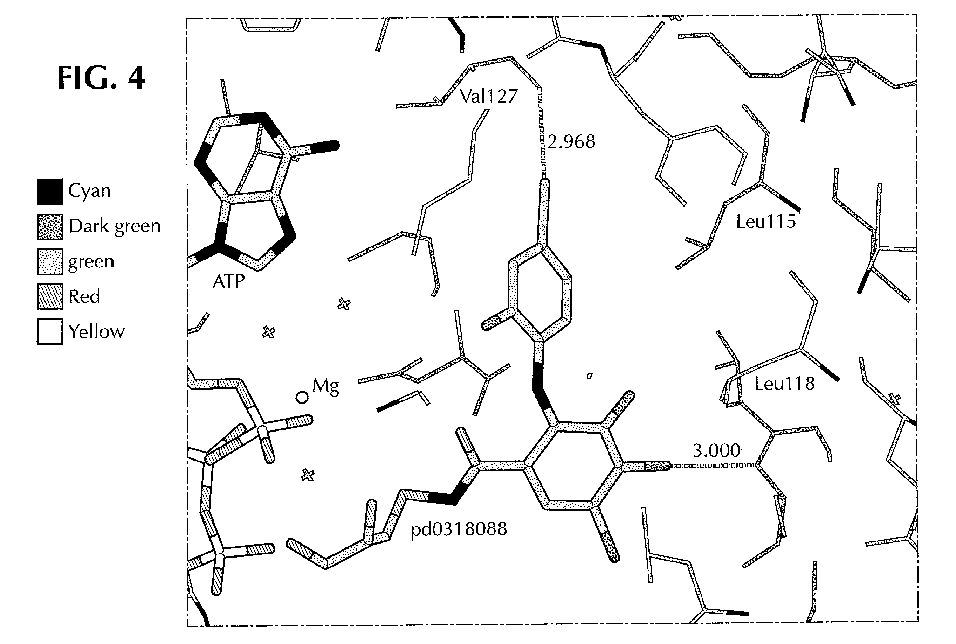

**Figure 4.** Figure 4 illustrates the interactions involved in the orientation of the ligand (referred to as "pd0318088" in the figure) within the MEK1 active site. The back right side of the figure shows the H-donor and acceptor bond formed between the backbone nitrogen of Ser212 and the 4-fluoro group of the inhibitor. Also shown at the top of the figure is the iodine of the inhibitor forming an electrostatic interaction with the backbone carbonyl of Val127. Finally, Leu115, Leu118 and Phe209 participate in forming the prime hydrophobic pocket.

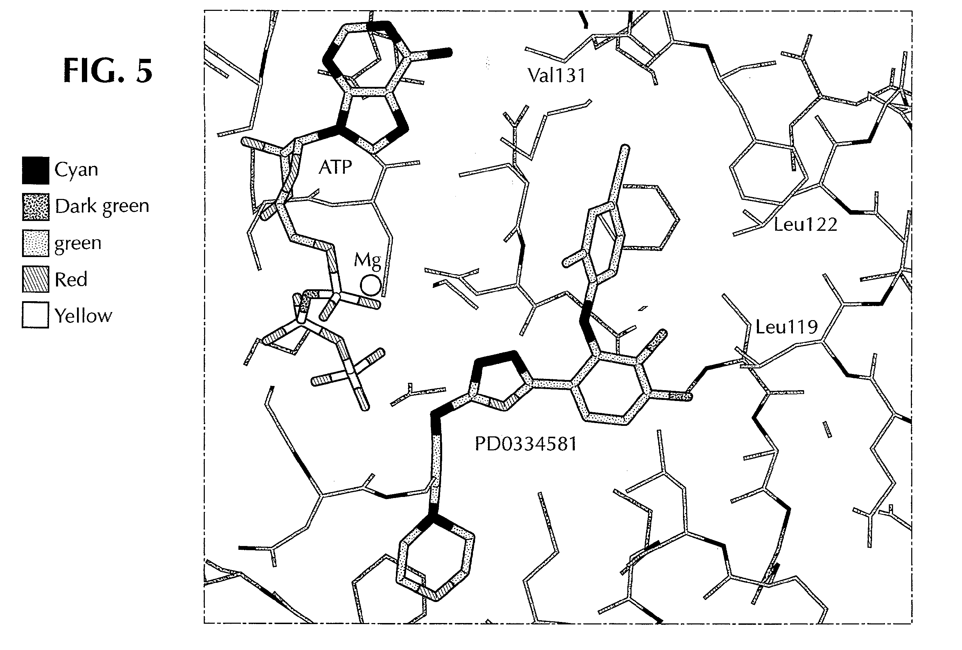

**Figure 5.** Figure 5 illustrates the interactions involved in the orientation of the ligand (referred to as "PD0334581" in the figure) within the MEK2 active site. The back right side of the figure shows the H-donor and acceptor bond formed between the backbone nitrogen of Ser216 and the 4-fluoro group of the inhibitor. Also shown at the top of the figure is the iodine of the inhibitor forming an electrostatic interaction with the backbone carbonyl of Val131. Finally, Leu119, Leu122 and Phe213 participate in forming the prime hydrophobic pocket.

SEQUENCE LISTING
SEQ ID NO: 1 Full-length of MEK1 nucleotide
SEQ ID NO: 2 Full-length of MEK1 peptide
SEQ ID NO: 3 Full-length of MEK2 nucleotide
SEQ ID NO: 4 Full-length of MEK2 peptide
SEQ ID NO: 5 Probe for MEK1
SEQ ID NO: 6 Probe for MEK1
SEQ ID NO: 7 PCR primer for MEK1-C1/MEK1-C1(d280-323)
SEQ ID NO: 8 PCR primer for MEK1-C1/MEK1-C1(d280-323);
MEK1-C2/MEK1-C2(d280-323); and PCR primer for
MEK1-C3/MEK1-C3(d280-323)
SEQ ID NO: 9 PCR primer for MEK1-C2/MEK1-C2(d280-323)
SEQ ID NO: 10 PCR primer for MEK1-C3/MEK1-C3(d280-323)
SEQ ID NO: 11 PCR probe for MEK2
SEQ ID NO: 12 PCR probe for MEK2
SEQ ID NO: 13 PCR primer for MEK2-C1
SEQ ID NO: 14 PCR primer for MEK2-C2

SEQ ID NO: 15 PCR primer for MEK2-C3
SEQ ID NO: 16 PCR primer for MEK2-C4
SEQ ID NO: 17 PCR primer for MEK2-C5
SEQ ID NO: 18 PCR primer for MEK2-C6
SEQ ID NO: 19 PCR reverse primer for MEK2-C1-C6

### DETAILED DESCRIPTION OF PREFERRED EMBODIMENTS

The present invention provides modified MEK1 and modified MEK2 peptides, and crystals of a peptide: ligand: cofactor complex that preferably comprise a ternary (i.e. tertiary) complex of a modified MEK1 peptide or modified MEK2 peptide, or a peptide that is structurally related to MEK1 or MEK2 peptide, a ligand and a cofactor.

According to one embodiment of the invention, the crystals are formed from recombinant human MEK1 peptide or recombinant human MEK2 peptide derived from a circular nucleic acid sequence that preferably has been expressed in a prokaryotic host and purified. The crystals diffract X-rays to a resolution better than 5Å. The information derived from the crystals provides three-dimensional crystallographic structural information for the MEK1 complex and the MEK2 complex, including ligand-binding and cofactor-binding sites of the MEK1 peptide, MEK2 peptide or a peptide that is structurally related to MEK1 or MEK2 peptide. The present invention includes the use of this structural information in, for example, the modification, design, screening and identification, and evaluation of chemical entities that have the potential to associate with MEK1 or MEK2 peptide, or a peptide that is structurally related thereto, and thus may inhibit or enhance MEK1 or MEK2 activity.

### Defintions

As used herein, the terms "comprising" and "including" are used in the conventional, non-limiting sense.

As used herein, "ligand" means a small molecule that binds to or associates specifically with an enzyme and can be used to mean an inhibitor or an activator.

As used herein, "cofactor" means an inorganic molecule, an organic molecule or a coenzyme that is required for enzymatic activity. For example, ATP (adenosine triphosphate) is a cofactor used by a kinase enzyme to transfer a phosphate group, i.e. phosphorylate its substrate.

As used herein, the phrase "root mean square (RMS) deviation" denotes the structural relationship between two or more species of proteins or peptides. It means that the difference in the root mean square of the distance of the three-dimensional structure of one peptide from C-alpha (C_{α}) atoms or backbone trace of the MEK1 peptide or MEK2 peptide in units of Angstroms (Å) unless indicated otherwise. It may be determined by superimposing one of the three-dimensional structures of the species on another, which may be solved by, for example, X-ray crystallography or by NMR and measuring the difference in the root mean square of the distance from C_{α} atoms or backbone trace of the MEK1 or MEK2 peptide to the other peptide in units of Angstroms (Å). The superimposing of three-dimensional structures on one another may be performed using a molecular modeling program, for example CNX™ (Accelrys), XtalView™ (Duncan McRee, Scripps Research Institute) or O™ (Morten Kjeldgaard, Aarhus Univ., Denmark). The closer the relationship between the three-dimensional structures, the smaller will be the value of the RMS deviation. For example, the three-dimensional relationship between the structural coordinates of the C-alpha atoms of two ligand protein co-complex structures is typically between 0.0 - 0.5 Å RMS deviation. An example of the caulation of a RMS deviation, specifically between the MEK1 and MEK2 crystalline structures of the invention, is provided herein in Example 16.

Therefore, one embodiment of this invention is the three-dimensional structures of the modified MEK1 and MEK2 peptides in ternary complexes with a ligand and a cofactor as found in Table 1 and Table 2. An additional embodiment is a "structurally related" peptide, crystals of the structurally related peptide and the three-dimensional structures thereof.

As used herein, a "structurally related" protein or peptide refers to a protein or peptide that is defined by the structural coordinates of the MEK1 or MEK2 peptide as set forth in Table 1 or Table 2 or a related set of structural coordinates having a root mean square deviation of from not more than about 1.5 Å to not more than about 0.50 Å from the core C alpha atoms of the MEK1 or MEK2 peptide structural coordinates as set forth in Table 1 or Table 2. Preferably the root mean square deviation is not more than about 0.50Å, more preferably not more than about 0.75 Å, even more preferably not more than about 1.00 Å, and most preferably not more than about 1.25 Å. An example of such a "structurally related peptide" may be, but is not limited to, MEK5.

Similarly, as used herein, "related set of structural coordinates" refers to a set of structural coordinates having a root mean square deviation in the range of from not more than about 1.5 Å to not more than about 0.50 Å away from the core C alpha atoms of the MEK1 or MEK2 structural coordinates a set forth in Table 1 or Table 2. Preferably the root mean square deviation is not more than about 0.50Å, more preferably not more than about 0.75 Å, even more preferably not more than about 1.00 Å, and most preferably not more than about 1.25 Å.

As used herein, "chemical entity" refers to a chemical compound, a complex of at least two chemical compounds, or a fragment of such a compound or complex. Such entities can be, for example, potential drug candidates and can be evaluated for their ability to inhibit or enhance the activity of MEK1, MEK2, or a structurally related peptide.

As used herein, the term "inhibitor" or "inhibit" (or variations thereof) refers to a ligand such as a compound or substance that lowers, reduces, decreases, prevents, diminishes, stops or negatively interferes with MEK1's or MEK2's activity, or such actions. Often the terms "inhibitor" and "antagonists" can be used interchangeably. Inhibition is typically expressed as a percentage of the enzymes activity in the presence of the inhibitor over the enzymes activity without the inhibitor. Or it may be expressed in terms of IC50, the inhibitor concentration at which 50% of the original enzyme activity is observed.

As used herein, the term "enhancer" or "enhance" (or variations thereof) refers to a ligand such as a compound or substance that improves, increases, stimulates, raises or positively interferes with MEK1 or MEK2 activity, or such actions. Often the terms "enhancer" or "agonists" can be used interchangeably. An enhancer would increase the enzyme's activity.

As used herein, the terms "model" and "modeling" mean the procedure of evaluating (also referred to as "assessing") the affinity of the interaction between a MEK1, MEK2, MEK-like binding pocket and a chemical entity (also referred to as a "candidate compound") based on steric constraints and surface/solvent electrostatic effects.

### MEK1 and MEK2

As used herein, the abbreviation "MEK1" or "Mek1" refers to the polynucleotide encoding the MAP kinase /ERK1 kinase, or the peptide *per se*. The MEK1 peptide is sometimes referred to as MAPKK, MPK1, MEK1, MAP kinase kinase 1 or MAP kinase /ERK1 kinase in the literature and throughout this application. The nucleic acid sequence of the polynucleotide encoding the full-length protein of MEK1 was published by Zheng and Guan (*J. Biol. Chem.* 268, 11435-11439 (1993)) and submitted to GenBank under the accession number L11284. The nucleic acid sequence described therein is provided herein, shown in SEQ ID NO: 1. The corresponding peptide sequence of the full-length protein is provided herein, shown in SEQ ID NO: 2. This peptide sequence was submitted to GenBank by Zheng *et al.* and Seger *et al.* and assigned Accession number NP_002746. It is noted that the numbering of SEQ ID NO:2 provided herein begins at the starting codon ATG (Met).

As used herein, the abbreviation "MEK2" or Mek2" refers to the polynucleotide encoding the MAP kinase kinase 2 or MAP Kinase/ ERK2 kinase, or the peptide *per se.* The MEK2 peptide is sometimes also referred to as MAPKK2 or MPK2. MEK2 is a paralog of MEK1 in the literature and throughout this application. MEK2 peptide, and the polynucleotide encoding the full-length MEK2 peptide were reported by Zheng *et al.* (*J. Biol. Chem.* 268, 11435-11439 (1993)) and their sequences are available under GenBank Accession numbers P36507 and NM_030662, respectively. The nucleic acid sequence encoding MEK2 and amino acid sequence of MEK2 peptide are provided herein as SEQ ID NO: 3 and SEQ ID NO: 4, respectively.

### MEK1 and MEK2 Peptide Binding Pockets

As used herein, "binding pocket," also referred to as, for example, "binding site," "binding domain," "substrate-binding site," "catalytic domain," or "ligand-binding domain," "ligand-binding site," "co-factor-binding site," or "co-factor-binding domain," refers to a region or regions of a molecule or molecular complex, that, as a result of its surface features, including, but not limited to, volume (both internally in cavities or in total), solvent accessibility, and surface charge and hydrophobicity, can associate with another chemical entity or compound. Such regions are of utility in fields such as drug discovery.

As used herein, a "MEK-like" peptide binding pocket refers to a peptide binding pocket defined by the atoms found in the structural coordinates of the MEK1 or MEK2 peptide as set forth in Table 1 or Table 2, or defined by structural coordinates having a root mean square deviation ranging from not more than about 1.5 Å to not more than about about 0.50 Å, preferably of not more than about 1.25 Å, away from the binding pocket C alpha atoms of any one of the MEK1 or MEK2 binding pockets (e.g. the MEK1 or MEK2 cofactor or ligand binding pockets defined above in the Summary of the Invetion section), or a conservatively substituted variant thereof.

As used herein, the term "activity" refers to all activities, i.e., the function of MEK1 or MEK2 in the phosphorylation of its substrate ERK1, as well as to the enzymes' potency. Often the terms "activity" and '"function" can be used interchangeably.

As used herein, the term "associate" refers to the process in which at least two molecules reversibly interact with each other, for example, by binding with each other. This may also refer to the process in which the conformation of the protein changes in response to the presence of a ligand to better accommodate the steric and electrostatic effects of the ligand. Associations between MEK1, MEK2, or a structurally related peptide and a ligand may occur with all or a part of a MEK1, MEK2, or MEK-like binding pocket. The association(s) may be non-covalent, e.g., wherein the juxtaposition is energetically favored by hydrogen bonding, van der Waals interactions or electrostatic interactions, or the association(s) may be covalent.

### MEK1 and MEK2 Peptides

The present invention provides isolated peptides and protein molecules that consist of, consist essentially of or are comprised of the amino acid sequences of the peptides encoded by the nucleic acid sequences disclosed in the SEQ ID NO: 1 and SEQ ID NO: 3, as well as obvious variants of these peptides that the within the art to make and use. Some of these variants are described in detail below.

### Modified MEK1 Peptide

In one embodiment, the invention provides an isolated, substantially pure polypeptide comprising novel modified MEK1 peptides, constructed, for example, so as to omit a significant portion of the flexible NH₂-terminal tail, or a conservatively substituted variant thereof ("NH₂-terminally truncated MEK1 peptides"). The NH₂-terminally truncated MEK1 peptide of the present invention preferably retains the globular core of the corresponding full-length of MEK1 peptide, such that MEK1 peptide can bind to ATP, its natural cofactor. Preferably, the NH₂-terminally truncated MEK1 peptides lack all or a significant portion (minimally 30 amino acids, preferably 41 amino acids, more preferably up to 50 amino acids, and even more preferably up to 61 amino acids) of the flexible, partially disordered NH₂-terminus which includes a portion of the ERK1 binding domain. In addition, the NH₂-terminally truncated MEK1 peptides may have a methionine as the initial amino acid prior to the indicated sequence.

These NH₂-terminally truncated MEK1 peptide may further have a deletion of the MEK1 insertion loop-forming domain, particularly from amino acid 280 to 323 of SEQ ID NO: 2 or at least 40 amino acids from between 264 and 310 of SEQ ID NO: 2. This may include a deletion of amino acids (1) from amino acid 264 to amino acid 310 of SEQ ID NO: 2; (2) from amino acid 270 to amino acid 310 of SEQ ID NO: 2; (3) from amino acid 264 to amino acid 305 of SEQ ID NO: 2; (4) amino acid 267 to amino acid 307 of SEQ ID NO: 2; or (5) from amino acid 265 to amino acid 304 of SEQ ID NO: 2.

The MEK1 insertion loop domain may also be replaced with a linker peptide. Preferably, the linker peptide has at most 10 amino acid residues. More preferably, the linker peptide has the amino acid sequence Lys-Asn-Cys-Lys-Thr-Asp.

Alternatively, the MEK1 peptide may be modified by a deletion of the MEK1 insertion loop-forming domain and/or by replacement of the MEK1 insertion loop domain as described above, without NH₂-terminal truncation.

These modified MEK1 peptides may also have a histidine oligomer tag, for example a histidine hexamer tag (His-Tag) at its COOH-terminus.

For example, the modified MEK1 peptide can be chosen from any of the following: (1) a NH₂-terminally truncated MEK1 peptide comprising amino acids 62-393 of the sequence shown in SEQ ID NO: 2, optionally containing a COOH-terminal His-Tag; (2) a NH₂-terminally truncated MEK1 peptide comprising amino acids 62-393 of SEQ ID NO: 2 with an additional deletion of insertion loop-forming amino acid residues as described above, and optionally containing a COOH-terminal His-Tag; (3) a NH₂-terminally truncated MEK1 peptide comprising amino acids 51-393 of the sequence shown in SEQ ID NO: 2, optionally containing a COOH-terminal His-Tag; (4) a NH₂-terminally truncated MEK1 peptide comprising amino acids 51-393 of SEQ ID NO: 2 with an additional deletion of insertion loop-forming amino acid residues as described above, and optionally containing a COOH-terminal His-Tag; (5) a NH₂-terminally truncated MEK1 peptide comprising amino acids 42-393 of the sequence shown in SEQ ID NO: 2, and which may optionally contain a COOH-terminal His-Tag; (6) a NH₂-terminally truncated MEK1 peptide comprising amino acids 42-393 of SEQ ID NO: 2 with an additional deletion of insertion loop-forming amino acid residues as described above, and optionally containing a COOH-terminal His-Tag; (7) a MEK1 peptide modified to have a deletion of insertion loop-forming amino acid residues as described above; and (8) a MEK peptide modified to replace the insertion loop-forming amino acid residues with a linker peptide, preferably Lys-Asn-Cys-Lys-Thr-Asp, with or without NH₂-terminal truncation described above.

Preferably, the modified MEK1 peptides retain the conserved amino acids described below and comprises approximately 331 to 351 amino acids residues, or approximately 284 to 311 amino acid residues if the NH₂-terminally truncated MEK1 peptides have a deletion within the peptide. The modified MEK1 peptide can be either phosphorylated or unphosphorylated. Similarly the modified MEK1 peptide can comprise one or more selenomethionines substituted for a naturally occurring methionine of the corresponding full-length MEK1 peptide. Of course, general modifications such as additional heavy atom derivatives common in X-ray crystallographic studies may also be performed on the modified MEK1 peptide of the present invention and such modifications are also included as part of the present inventions.

In a preferred embodiment, the modified MEK1 peptide is derived from human MEK1 and lacks the residues from amino acid 1 to amino acid 41 of the N-terminal amino acids of the corresponding full-length MEK1. In a more preferred embodiment, the modified MEK1 peptide lacks the residues from amino acid 1 to amino acid 50 of the N-terminal amino acids of the corresponding full-length MEK1. In the most preferred embodiment, the modified MEK1 peptide lacks the residues from amino acid 1 to amino acid 61 of the N-terminal amino acids of the corresponding full-length MEK1 shown in SEQ ID NO: 2.

The modified MEK1 peptides of the present invention can be derived from any eukaryotic source, but is preferably a vertebrate MEK1, more preferably from a mammalian MEK1, and most preferably human MEK1.

### Modified MEK2 Peptide

In another embodiment, the invention provides an isolated, substantially pure polypeptide comprising novel modified MEK2 peptides, constructed, for example, so as to omit a significant portion of the flexible NH₂-terminal tail, or a conservatively substituted variant thereof ("NH₂-terminally truncated MEK2 peptides"). The NH₂-terminally truncated MEK2 peptide of the present invention preferably retains the globular core of the corresponding full-length of MEK2 peptide, such that MEK2 peptide can bind to ATP, its natural cofactor. Preferably, the NH₂-terminally truncated MEK2 peptides lack all or a significant portion (minimally 34 amino acids to at most 74 amino acids, preferably minimally 45 to at most 65 amino acids) of the flexible, partially disordered NH₂-terminus which includes a portion of the ERK2 binding domain. In addition, the NH₂-terminally truncated MEK2 peptides may have a methionine as the initial amino acid prior to the indicated sequence. These modified MEK2 peptides may also have a histidine oligomer tag, for example a histidine hexamer tag (His-Tag) at its COOH-terminus.

For example, the modified MEK2 peptide can be chosen from any of the following: (1) a NH₂-terminally truncated MEK2 peptide comprising amino acids 46-400 of the sequence shown in SEQ ID NO: 4, optionally containing a COOH-terminal His-Tag; (2) a NH₂-terminally truncated MEK2 peptide comprising amino acids 55-400 of the sequence shown in SEQ ID NO: 4, optionally containing a COON-terminal His-Tag; (3) a NH₂-terminally truncated MEK2 peptide comprising amino acids 66-400 of the sequence shown in SEQ ID NO: 4, and which may optionally contain a COOH-terminal His-Tag; (4) a NH₂-terminally truncated MEK2 peptide comprising amino acids 59-400 of the sequence shown in SEQ ID NO: 4, and which may optionally contain a COOH-terminal His-Tag; (5) a NH₂-terminally truncated MEK2 peptide comprising amino acids 62-400 of the sequence shown in SEQ ID NO: 4, and which may optionally contain a COOH-terminal His-Tag; (6) a NH₂-terminally truncated MEK2 peptide comprising amino acids 64-400 of the sequence shown in SEQ ID NO: 4, and which may optionally contain a COOH-terminal His-Tag.

Preferably, the modified MEK2 peptides retain the conserved amino acids described below and comprises approximately 326 to 366 amino acids residues. The modified MEK2 peptide can be either phosphorylated or unphosphorylated. Similarly the modified MEK2 peptide can comprise one or more selenomethionines substituted for a naturally occurring methionine of the corresponding full-length MEK2 peptide. Of course, general modifications such as additional heavy atom derivatives common in X-ray crystallographic studies may also be performed on the modified MEK2 peptide of the present invention and such modifications are also included as part of the present inventions.

In a preferred embodiment, the modified MEK2 peptide is derived from human MEK2 and lacks the residues from amino acid 1 to amino acid 45 of the N-terminal amino acids of the corresponding full-length MEK2 shown in SEQ ID NO: 4. In a more preferred embodiment, the modified MEK2 peptide lacks the residues from amino acid 1 to amino acid 54 of the N-terminal amino acids of the corresponding full-length MEK2 shown in SEQ ID NO: 4. In the most preferred embodiment, the modified MEK2 peptide lacks the residues from amino acid 1 to amino acid 65 of the N-terminal amino acids of the corresponding full-length MEK2 shown in SEQ ID NO: 4.

The modified MEK2 peptides of the present invention can be derived from any eukaryotic source, but is preferably a vertebrate MEK2, more preferably from a mammalian MEK2, and most preferably human MEK2.

As used herein, a protein or peptide is said to be "isolated" or "purified" when it is substantially free of cellular material or free of chemical precursors or other chemicals. The proteins or peptides of the present invention can be purified to homogeneity or other degrees of purity. The level of purification will be based on the intended use. The critical feature is that the preparation allows for the desired function of the protein or peptide, even if in the presence of considerable amounts of other components.

In some uses, "substantially free of cellular material" includes preparations of the protein or peptide having less than about 30% (by dry weight) other proteins (i.e., contaminating protein), preferably less than about 20% other proteins, more preferably less than about 10% other proteins, or even more preferably less than about 5% other proteins. When the protein or peptide is recombinantly produced, it can also be substantially free of culture medium, i.e., culture medium represents less than about 20% of the volume of the protein preparation.

The language "substantially free of chemical precursors or other chemicals" includes preparations of the protein in which it is separated from chemical precursors or other chemicals that are involved in its synthesis. In one embodiment, the language "substantially free of chemical precursors or other chemicals" includes preparations of the protein having less than about 30% (by dry weight) chemical precursors or other chemicals, preferably less than about 20% chemical precursors or other chemicals, more preferably less than about 10% chemical precursors or other chemicals, or most preferably less than about 5% chemical precursors or other chemicals.

The isolated protein described herein can be purified from cells that naturally express MEK1 or MEK2 peptide or purified from cells that have been altered to express MEK1 or MEK2 (recombinant expression). For example, a nucleic acid molecule encoding the protein is cloned into an expression vector, the expression vector is introduced into a host cell and the protein is then expressed in the host cell. The protein can then be isolated from the cells by an appropriate purification scheme using standard protein purification techniques. Many of these techniques are described in detail below.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. The terms "peptide", "polypeptide" and "protein" are used interchangeably herein. Polypeptides may contain amino acids other than the 20 naturally occurring amino acids. Further, many amino acids, including the terminal amino acids, may be modified by natural processes, such as processing and other post-translational modifications, or by chemical modification techniques well known in the art. Common modifications that occur naturally in polypeptides are described in basic texts, detailed monographs, and the research literature, and they are well known to those of skill in the art.

Accordingly, the polypeptides also encompass derivatives or analogs in which a substituted amino acid residue is not one encoded by the genetic code; in which a substituent group is included, in which the mature polypeptide is fused with another compound, such as a compound to increase the half-life of the polypeptide (for example, polyethylene glycol), or in which the additional amino acids are fused to the mature polypeptide, such as a leader or secretary sequence or a sequence for purification of the mature polypeptide or a pro-protein sequence.

The present invention further provides for fragments of the MEK1 and MEK2 peptides, in addition to proteins and peptides that comprises and consist of such fragments. As used herein, a fragment comprises at least 8 or more contiguous amino acid residues from the protein kinase. Such fragments can be chosen based on the ability to retain one or more of the biological activities of the kinase or could be chosen for the ability to perform a function, e.g. act as an immunogen. Particularly important fragments are biologically active fragments, peptides which are, for example about 8 or more amino acids in length. Such fragments will typically comprise a domain or motif of the kinase, e.g., active site. Further, possible fragments include, but are not limited to, domain or motif containing fragments, soluble peptide fragments, and fragments containing immunogenic structures. Predicted domains and functional sites are readily identifiable by computer programs well known and readily available to those of skill in the art (e.g., by PROSITE analysis).

Known modifications include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, phenylation, racemization, selenoylation, sulfation, transfer-RNA mediated addition of amino acids to proteins such as arginylation, and ubiquitination.

Such modifications are well known to those of skill in the art and have been described in great detail in the scientific literature. Several particularly common modifications, glycosylation, lipid attachment, sulfation, gamma-carboxylation of glutamic acid residues, hydroxylation and ADP-ribosylation, for instance, are described in most basic texts. For example, see *Proteins - Structure and Molecular Properties,* 2nd Ed., T.E. Creighton, W. H. Freeman and Company, New York (1993). Many detailed reviews are available on this subject, such as by Wold, F., *Posttranslational Covalent Modification of Proteins,* B.C. Johnson, Ed., Academic Press, New York 1-12 (1983); Seifter *et al.* (*Meth. Enzymol. 182*: 626-646 (1990)) and Rattan *et al.* (*Ann. N.Y. Acad. Sci. 663: 48-62* (1992)).

The peptides of the present invention can be attached to heterologous sequences to form chimeric or fusion proteins. Such chimeric and fusion proteins comprise a peptide operatively linked to a heterologous protein having an amino acid sequence not substantially homologous to the kinase peptide. "Operatively linked" indicates that the peptide and the heterologous protein are fused in-frame. The heterologous protein can be fused to the NH₂-terminus or COOH-terminus of the kinase peptide. The two peptides linked in a fusion peptide are typically derived from two independent sources, and therefore a fusion peptide comprises two linked peptides not normally found linked in nature. The two peptides may be from the same or different genome.

In some uses, the fusion protein does not affect the activity of the peptide *per se.* For example, the fusion protein can include, but is not limited to, enzymatic fusion proteins, for example beta-galactosidase fusions, yeast two-hybrid GAL fusions, poly-His fusions, MYC-tagged, HI-tagged and Ig fusions. Such fusion proteins, particularly poly-His fusions, can facilitate the purification of recombinant kinase peptide. In certain host cells (e.g., mammalian host cells), expression and/or secretion of a protein can be increased by using a heterologous signal sequence.

A chimeric or fusion protein can be produced by standard recombinant DNA techniques. For example, DNA fragments coding for the different protein sequences are ligated together in-frame in accordance with conventional techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see Ausubel *et al., Current Protocols in Molecular Biology*, 1992). Moreover, many expression vectors are commercially available that already encode a fusion moiety (e.g., a GST protein). A modified MEK1 peptide-encoding nucleic acid can be cloned into such an expression vector such that the fusion moiety is linked in-frame to the peptide.

### Nucleic Acids and Polynucleotides Encoding Modified MEK1 and MEK2 Peptides

The present invention also provides isolated nucleic acid molecules that encode the functional, active kinases of the present invention. Such nucleic acid molecules will consist of, consist essentially of, or comprise a nucleotide sequence that encodes one of the kinase peptides of the present invention, an allelic variant thereof, or an ortholog or paralog thereof.

### Polynucleotides Encoding Modified MEK1 Peptides

In a particular embodiment, the invention also provides isolated, purified polynucleotides that encode novel modified MEK1 peptides, such as NH₂-terminally truncated MEK1 peptides, MEK1 peptides having a deletion of insertion loop-forming domains, and/or MEK1 having the insertion loop domain replaced with a linker peptide.

The polynucleotide may be natural or recombinant. In one embodiment, the nucleic acid sequence encodes a modified MEK1 peptide having an amino acid sequence of amino acids from between amino acids 42 and 62 to amino acid 393 of SEQ ID NO: 2 or a conservatively substituted variant thereof. In particular, the nucleic acid sequence encodes a modified MEK1 peptide having an amino acid sequence of amino acids 42 to 393 of SEQ ID NO: 2 or an amino acid sequence that differs from amino acid 42 to 393 of SEQ ID NO: 2 by only having conservative substitutions. Alternatively, the nucleic acid sequence encodes a modified MEK1 peptide having an amino acid sequence of amino acids 51 to 393 of SEQ ID NO: 2 or an amino acid sequence that differs from amino acid 51 to 393 of SEQ ID NO: 2 by only having conservative substitutions. Or, the nucleic acid sequence encodes a modified MEK1 peptide having an amino acid sequence of amino acids 62 to 393 of SEQ ID NO: 2 or an amino acid sequence that differs from amino acid 62 to 393 of SEQ ID NO: 2 by only having conservative substitutions. Such polynucleotides may be, for example, one having the sequence set forth in SEQ ID NO: 1 with deletion of the portion encoding the first 41-61 amino residues of the sequence of SEQ ID NO: 2.

In addition, the polynucleotide may encode the modified MEK1 peptide to further have a deletion of insertion loop-forming domain, particularly from amino acid 280 to 323 of SEQ ID NO: 2; or at least 40 amino acids from between amino acid residue 264 and amino acid 310 of SEQ ID NO: 2. This may include a deletion of amino acids (1) from amino acid 264 to amino acid 310 of SEQ ID NO: 2; (2) from amino acid 270 to amino acid 310 of SEQ ID NO: 2; (3) from amino acid 264 to amino acid 305 of SEQ ID NO: 2; (4) amino acid 267 to amino acid 307 of SEQ ID NO: 2; or (5) from amino acid 265 to amino acid 304 of SEQ ID NO: 2.

Moreover, the polynucleotide may encode the MEK1 peptide modified to replace the MEK1 insertion loop-forming amino acid residues with a linker peptide, preferably Lys-Asn-Cys-Lys-Thr-Asp, either alone or in combination with the NH₂-terminal truncations described above.

The polynucleotide of the invention also may encode the MEK1 peptides modified to have a deletion of insertion loop-forming amino acid residues as described above, without NH₂-terminal truncation.

Also, any of these amino acid sequences may contain one or more selenomethionines in place of a methionine. Further, these modified MEK1 peptides and their various variants may have a histidine oligomer tag, for example a histidine hexamer tag (His-tag) at their COOH-terminus.

### Polynucleotides Encoding Modified MEK2 Peptides

In another particular embodiment, the invention also provides isolated, purified polynucleotides that encode novel modified MEK2 peptides, such as NH₂-terminally truncated MEK2 peptides. The polynucleotide may be natural or recombinant. In one embodiment, the nucleic acid sequence encodes a modified MEK2 peptide having an amino acid sequence of amino acids from between amino acids 46 and 66 to amino acid 400 of SEQ ID NO: 4 or a conservatively substituted variant thereof. In particular, the nucleic acid sequence encodes a modified MEK2 peptide having an amino acid sequence of amino acids 46 to 400 of SEQ ID NO: 4 or an amino acid sequence that differs from amino acid 46 to 400 of SEQ ID NO: 4 by only having conservative substitutions. Alternatively, the nucleic acid sequence encodes a modified MEK2 peptide having an amino acid sequence of amino acids 55 to 400 of SEQ ID NO: 4 or an amino acid sequence that differs from amino acid 55 to 400 of SEQ ID NO: 4 by only having conservative substitutions. Or, the nucleic acid sequence encodes a modified MEK2 peptide having an amino acid sequence of amino acids 66 to 400 of SEQ ID NO: 4 or an amino acid sequence that differs from amino acid 66 to 400 of SEQ ID NO: 4 by only having conservative substitutions. Alternatively, the nucleic acid sequence encodes a modified MEK2 peptide having an amino acid sequence of amino acids 59 to 400 of SEQ ID NO: 4 or an amino acid sequence that differs from amino acid 59 to 400 of SEQ ID NO: 4 by only having conservative substitutions. Or, the nucleic acid sequence encodes a modified MEK2 peptide having an amino acid sequence of amino acids 62 to 400 of SEQ ID NO: 4 or an amino acid sequence that differs from amino acid 62 to 400 of SEQ ID NO: 4 by only having conservative substitutions. Additionally, the nucleic acid sequence encodes a modified MEK2 peptide having an amino acid sequence of amino acids 64 to 400 of SEQ ID NO: 4 or an amino acid sequence that differs from amino acid 64 to 400 of SEQ ID NO: 4 by only having conservative substitutions. Such polynucleotides may be, for example, one having the sequence set forth in SEQ ID NO: 3 with deletion of the portion encoding the first 45-65 amino residues of the sequence of SEQ ID NO: 4.

Also, any of these amino acid sequences may contain one or more selenomethionines in place of a methionine. Further, these modified MEK2 peptides and their various variants may have a histidine oligomer tag, for example a histidine hexamer tag (His-tag) at their COOH-terminus.

As used herein, an "isolated" nucleic acid molecule is one that is separated from other nucleic acid present in the natural source of the nucleic acid. Preferably, an "isolated" nucleic acid is free of sequences which naturally flank the nucleic acid (i.e., sequences located at the 5' and 3' ends of the nucleic acid) in the genomic DNA or cDNA of the organism from which the nucleic acid is derived. However, there can be some flanking nucleotide sequences, for example up to about 5KB, particularly contiguous peptide encoding sequences and peptide encoding sequences within the same gene but separated by introns in the genomic sequence. The important point is that the nucleic acid is isolated from remote and unimportant flanking sequences such that it can be subjected to the specific manipulations described herein such as recombinant expression, preparation of probes and primers, and other uses specific to the nucleic acid sequences.

Moreover, an "isolated" nucleic acid molecule, such as a cDNA molecule, can be substantially free of other cellular material, or culture medium when produced by recombinant techniques, or chemical precursors or other chemicals when chemically synthesized. However, the nucleic acid molecule can be fused to other coding or regulatory sequences and still be considered isolated.

For example, recombinant DNA molecules contained in a vector are considered isolated. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the isolated DNA molecules of the present invention. Isolated nucleic acid molecules according to the present invention further include such molecules produced synthetically.

The preferred classes of nucleic acid molecules that are comprised of the nucleotide sequences of the present are the full-length cDNA molecules and genes and genomic clones since some of the nucleic acid molecules provided in SEQ ID NO: 1 are fragments of the complete gene that exists in nature. A brief description of how various types of these nucleic acid molecules can be readily made/isolated is provided herein.

Full-length genes may be cloned from known sequence using any one of a number of methods known in the art. For example, a method which employs XL-PCR (Perkin-Elmer, Foster City, Calif.) to amplify long pieces of DNA may be used. Other methods for obtaining full-length sequences are known in the art.

The isolated nucleic acid molecules can encode the active protein plus additional amino or carboxyl-terminal amino acids, or amino acids interior to the mature peptide (when the mature form has more than one peptide chain, for instance). Such sequences may play a role in processing of a protein from precursor to an active form, facilitate protein trafficking, prolong or shorten protein half-life or facilitate manipulation of a protein for assay or production, among other things. As generally is the case *in situ,* the additional amino acids may be processed away from the mature active protein by cellular enzymes.

As mentioned above, the isolated nucleic acid molecules include, but are not limited to, the sequence encoding the active kinase alone or in combination with coding sequences, such as a leader or secretary sequence (e.g., a pre-pro or pro-protein sequence), the sequence encoding the active kinase, with or without the additional coding sequences, plus additional non-coding sequences, for example introns and non-coding 5' and 3' sequences such as transcribed but non-translated sequences that play a role in transcription, mRNA processing (including splicing and polyadenylation signals), ribosome binding and stability of mRNA. In addition, the nucleic acid molecule may be fused to a marker sequence encoding, for example, a peptide that facilitates purification.

Isolated nucleic acid molecules can be in the form of RNA, such as mRNA, or in the form of DNA, including cDNA and genomic DNA, obtained by cloning or produced by chemical synthetic techniques or by a combination thereof. The nucleic acid, especially DNA, can be double-stranded or single-stranded. Single-stranded nucleic acid can be the coding strand (sense strand) or the non-coding strand (antisense strand).

The invention further provides nucleic acid molecules that encode fragments of the peptides of the present invention and that encode obvious variants of the kinase proteins of the present invention that are described above. Such nucleic acid molecules may be naturally occurring, such as allelic variants (same locus), paralogs (different locus), and orthologs (different organism), or may be constructed by recombinant DNA methods or by chemical synthesis. Such non-naturally occurring variants may be made by mutagenesis techniques, including those applied to nucleic acid molecules, cells, or organisms. Accordingly, as discussed above, the variants can contain nucleotide substitutions, deletions, inversions and insertions. Variation can occur in either or both the coding and non-coding regions. The variations can produce both conservative and non-conservative amino acid substitutions.

A fragment comprises a contiguous nucleotide sequence greater than 12 or more nucleotides. Further, a fragment could be at least 30, 40, 50, 100, 250 or 500 nucleotides in length. The length of the fragment will be based on its intended use. For example, the fragment can encode epitope bearing regions of the peptide, or can be useful as DNA probes and primers. Such fragments can be isolated using the known nucleotide sequence to synthesize an oligonucleotide probe. A labeled probe can then be used to screen a cDNA library, genomic DNA library, or mRNA to isolate nucleic acid corresponding to the coding region. Further, primers can be used in PCR reactions to clone specific regions of gene.

A probe/primer typically comprises substantially a purified oligonucleotide or oligonucleotide pair. The oligonucleotide typically comprises a region of nucleotide sequence that hybridizes under stringent conditions to at least about 12, 20, 25, 40, 50 or more consecutive nucleotides.

Orthologs, homologs, and allelic variants can be identified using methods known in the art. As described above, these variants comprise a nucleotide sequence encoding a peptide that is typically 60%, preferably 65%, more preferably 70%, or even more preferably 75% or more homologous to the nucleotide sequence provided in SEQ ID NO: 1 or SEQ ID NO: 3 or a fragment of this sequence. In one preferred embodiment, the variants comprise a nucleotide sequence encoding a peptide that is at least 80%, preferably 85%, more preferably 90%, even more preferably 95% or more homologous to the nucleotide sequence provided in SEQ ID NO: 1 or SEQ ID NO: 3 or a fragment of this sequence. Such nucleic acid molecules can readily be identified as being able to hybridize under moderate to stringent conditions, to the nucleotide sequence shown in SEQ ID NO: 1 or SEQ ID NO: 3 or a fragment of the sequence.

As used herein, the term "hybridizes under stringent conditions" is intended to describe conditions for hybridization and washing under which nucleotide sequences encoding a peptide at least 50%, preferably at least 55% homologous to each other typically remain hybridized to each other. The conditions can be such that sequences at least 65%, preferably at least 70%, or more preferably at least 75% homologous to each other typically remains hybridized to each other. Standard hybridization conditions from moderate to highly stringent conditions are known to those skilled in the art (See e.g., *Current Protocols in Molecular Biology,* John Wiley & Sons, N.Y. (1989), 6.3.1-6.3.6). Moderate hybridization conditions are defined as equivalent to hybridization in 2X sodium chloride/sodium citrate (SSC) at 30 °C, followed by one or more washes in 1X SSC, 0.1% SDS at 50-60 °C. Highly stringent conditions are defined as equivalent to hybridization in 6X sodium chloride/sodium citrate (SSC) at 45 °C, followed by one or more washes in 0.2X SSC, 0.1% SDS at 50-65 °C.

The nucleic acid molecules of the present invention are useful for probes, primers, chemical intermediates, and in biological assays. The nucleic acid molecules are useful as a hybridization probe for cDNA and genomic DNA to isolate full-length cDNA and genomic clones encoding the peptide described herein and to isolate cDNA and genomic clones that correspond to variants (alleles, orthologs, etc.) producing the same or related peptides described herein.

The probe can correspond to any sequence along the entire length of the nucleic acid molecules provided in the SEQ ID NO: 1 or SEQ ID NO: 3. Accordingly, it could be derived from 5' noncoding regions, the coding region, and 3' noncoding regions. However, as discussed, fragments are not to be construed as those which may encompass fragments disclosed prior to the present invention.

The nucleic acid molecules are also useful as primers for PCR to amplify any given region of a nucleic acid molecule and are useful to synthesize antisense molecules of desired length and sequence.

The nucleic acid molecules are also useful for constructing recombinant vectors. Such vectors include expression vectors that express a portion of, or all of, the peptide sequences. Vectors also include insertion vectors, used to integrate into another nucleic acid molecule sequence, such as into the cellular genome, to alter *in situ* expression of a gene and/or gene product. For example, an endogenous coding sequence can be replaced via homologous recombination with all or part of the coding region containing one or more specifically introduced mutations. The nucleic acid molecules are also useful for expressing antigenic portions of the proteins; useful as probes for determining the chromosomal positions of the nucleic acid molecules by means of *in situ* hybridization methods; useful for designing ribozymes corresponding to all, or a part, of the mRNA produced from the nucleic acid molecules described herein; and useful for constructing host cells expressing a part, or all, of the nucleic acid molecules and peptides. The nucleic acid molecules are also useful for constructing transgenic animals expressing all, or a part, of the nucleic acid molecules and peptides; and useful for making vectors that express part, or all, of the peptides. The nucleic acid molecules are further useful as hybridization probes for determining the presence, level, form and distribution of nucleic acid expression. Accordingly, the probes can be used to detect the presence of, or to determine levels of, a specific nucleic acid molecule in cells, tissues, and in organisms. The nucleic acid whose level is determined can be DNA or RNA. Accordingly, probes corresponding to the peptides described herein can be used to assess expression and/or gene copy number in a given cell, tissue, or organism. These uses are relevant for diagnosis of disorders involving an increase or decrease in kinase protein expression relative to normal results.

*In vitro* techniques for detection of mRNA include Northern hybridizations and *in situ* hybridizations. *In vitro* techniques for detecting DNA includes Southern hybridizations and *in situ* hybridization.

Probes can be used as a part of a diagnostic test kit for identifying cells or tissues that express a kinase protein, such as by measuring a level of a receptor-encoding nucleic acid in a sample of cells from a subject e.g., mRNA or genomic DNA, or determining if a receptor gene has been mutated.

### Vectors And Host Cells For Producing Modified MEK1 and MEK2 Peptides

The present invention also provides for an expression vector for producing the modified MEK1 and MEK2 peptides described above in a host cell. It further provides a host cell which may be stably transformed and transfected with a polynucleotide encoding the modified MEK1 and MEK2 peptides described above, or a fragment thereof or an analog thereof, in a manner allowing the expression of the modified MEK1 and MEK2 peptides. The present invention also provides expression vectors comprising the nucleic acids of the present invention operatively associated with an expression control sequence.

### MEK1 Expresion Vectors

The expression vector may contain a nucleic acid encoding a modified MEK1 peptide having an amino acid sequence from between amino acids 42 and 62 to amino acid 393 of SEQ ID NO: 2 or a conservatively substituted variant thereof. In one embodiment, the expression vector contains a nucleic acid encoding a modified MEK1 peptide having an amino acid sequence of amino acids 42 to 393 of SEQ ID NO: 2 or an amino acid sequence that differs from amino acid 42 to 393 of SEQ ID NO: 2 by only having conservative substitutions. In an alternative embodiment, the expression vector contains a nucleic acid encoding a modified MEK1 peptide having an amino acid sequence between amino acids 51 to 393 of SEQ ID NO: 2 or an amino acid sequence that differs from amino acids 51 to 393 of SEQ ID NO: 2 by only having conservative substitutions. In still another embodiment, the expression vector contains a nucleic acid encoding a NH₂-terminally truncated MEK1 peptide having an amino acid sequence of amino acids 62 to 393 of SEQ ID NO: 2 or an amino acid sequence that differs from amino acids 62 to 393 of SEQ ID NO: 2 by only having conservative substitutions. Such polynucleotides may be, for example, one having the sequence set forth in SEQ ID NO: 1 with deletion of the portion encoding the first 41 - 61 amino residues of the sequence of SEQ ID NO: 2.

In addition, the expression vector may contain a nucleic acid encoding a modified MEK1 having a NH₂-terminal truncation and a deletion of insertion loop-forming amino acid residues, particularly from amino acid 280 to amino acid 323 SEQ ID NO: 2; or at least 40 amino acids from between amino acid residue 264 and amino acid 310 of SEQ ID NO: 2. This may include a deletion of amino acids (1) from amino acid 264 to amino acid 310 of SEQ ID NO: 2; (2) from amino acid 270 to amino acid 310 of SEQ ID NO: 2; (3) from amino acid 264 to amino acid 305 of SEQ ID NO: 2; (4) amino acid 267 to amino acid 307 of SEQ ID NO: 2; or (5) from amino acid 265 to amino acid 304 of SEQ ID NO: 2. Further, such polynucleotide may encode the MEK1 peptide modified to have a deletion of insertion loop-forming amino acid residues as described above without NH₂-terminal truncation.

Also, the expression vector may contain a nucleic acid encoding a modified MEK1 peptide wherein the insertion loop-forming amino acid residues are replaced with a linker peptide, preferably Lys-Asn-Cys-Lys-Thr-Asp, with or without NH₂-terminal truncation as described above.

Also, any of these expression vectors may optionally contain a nucleic acid encoding a modified MEK1 peptide having one or more selenomethionines in place of a methionine. The expression vector may also optionally contain a nucleic acid encoding a modified MEK1 peptide having a histidine oligomer tag, for example a histidine hexamer tag (His-tag) at its COOH-terminus.

### MEK2 Expresion Vectors

The expression vectors of the invention may also contain a nucleic acid encoding a modified MEK2 peptide having an amino acid sequence from between amino acids 46 and 66 to amino acid 400 of SEQ ID NO: 4 or a conservatively substituted variant thereof. In particular, the expression vector may contain a nucleic acid sequence encoding a modified MEK2 peptide having an amino acid sequence of amino acids 46 to 400 of SEQ ID NO: 4 or an amino acid sequence that differs from amino acid 46 to 400 of SEQ ID NO: 4 by only having conservative substitutions. Alternatively, the expression vector may contain a nucleic acid sequence encoding a modified MEK2 peptide having an amino acid sequence of amino acids 55 to 400 of SEQ ID NO: 4 or an amino acid sequence that differs from amino acid 55 to 400 of SEQ ID NO: 4 by only having conservative substitutions. Or, the expression vector may contain a nucleic acid sequence encoding a modified MEK2 peptide having an amino acid sequence of amino acids 66 to 400 of SEQ ID NO: 4 or an amino acid sequence that differs from amino acid 66 to 400 of SEQ ID NO: 4 by only having conservative substitutions. Alternatively, the expression vector may contain a nucleic acid sequence encoding a modified MEK2 peptide having an amino acid sequence of amino acids 59 to 400 of SEQ ID NO: 4 or an amino acid sequence that differs from amino acid 59 to 400 of SEQ ID NO: 4 by only having conservative substitutions. Or, the expression vector may contain a nucleic acid sequence encoding a modified MEK2 peptide having an amino acid sequence of amino acids 62 to 400 of SEQ ID NO: 4 or an amino acid sequence that differs from amino acid 62 to 400 of SEQ ID NO: 4 by only having conservative substitutions. Additionally, the expression vector may contain a nucleic acid sequence encoding a modified MEK2 peptide having an amino acid sequence of amino acids 64 to 400 of SEQ ID NO: 4 or an amino acid sequence that differs from amino acid 64 to 400 of SEQ ID NO: 4 by only having conservative substitutions. Such expression vectors may be, for example, one containing a nucleic acid sequence encoding the sequence set forth in SEQ ID NO: 3 with deletion of the portion encoding the first 45-65 amino residues of the sequence of SEQ ID NO: 4.

Also, any of these expression vectors may optionally contain a nucleic acid encoding a modified MEK2 peptide having one or more selenomethionines in place of a methionine. The expression vector may also optionally contain a nucleic acid encoding a modified MEK2 peptide having a histidine oligomer tag, for example a histidine hexamer tag (His-tag) at its COOH-terminus.

The present invention further includes a cell transfected or transformed with an expression vector of the present invention. Any of the cells mentioned below may be employed in this method. In one embodiment, the cell is a prokaryotic cell. Preferably, the prokaryotic cell is an *E. coli* cell. In another embodiment the cell is an eukaryotic cell, such as an insect cell or a vertebrate cell, which may be, for example, a mammalian cell.

The present invention also includes methods for expressing the modified MEK1 and MEK2 peptides comprising culturing a cell that expresses the modified MEK1 or MEK2 peptide in an appropriate cell culture medium under conditions that provide for expression of the protein by the cell. Any of the cells mentioned above may be employed in this method. In a particular embodiment, the cell is a yeast cell which has been manipulated to express a modified MEK1 or MEK2 peptide of the present invention. In a preferred embodiment, the prokaryotic cell is an *E. coli* cell which has been manipulated to express a modified MEK1 or MEK2 peptide of the present invention.

The invention also provides vectors containing the nucleic acid molecules described herein. The term "vector" refers to a vehicle, preferably a nucleic acid molecule that can transport the nucleic acid molecules. When the vector is a nucleic acid molecule, the nucleic acid molecules are covalently linked to the vector nucleic acid. The vector preferably includes a plasmid, single or double stranded phage, a single or double stranded RNA or DNA viral vector, or artificial chromosome, such as aBAC, PAC, YAC, or MAC, which are commercially available from, for example, Qiagen (Valencia, CA). Various expression vectors known in the art can be used to express polynucleotide encoding the MEK1 or MEK2 peptide.

A vector can be maintained in the host cell as an extrachromosomal element where it replicates and produces additional copies of the nucleic acid molecules. Alternatively, the vector may integrate into the host cell genome and produce additional copies of the nucleic acid molecules when the host cell replicates.

The invention provides vectors for the maintenance (cloning vectors) or vectors for expression (expression vectors) of the nucleic acid molecules. The vectors can function in prokaryotic or eukaryotic cells or in both (shuttle vectors).

Expression vectors contain cis-acting regulatory regions that are operably linked in the vector to the nucleic acid molecules such that transcription of the nucleic acid molecules is allowed in a host cell. The nucleic acid molecules can be introduced into the host cell with a separate nucleic acid molecule capable of affecting transcription. Thus, the second nucleic acid molecule may provide a trans-acting factor interacting with the cis-regulatory control region to allow transcription of the nucleic acid molecules from the vector. Alternatively, a trans-acting factor may be supplied by the host cell. Finally, a trans-acting factor can be produced from the vector itself. It is understood, however, that in some embodiments, transcription and/or translation of the nucleic acid molecules can occur in a cell-free system.

The regulatory sequence to which the nucleic acid molecules described herein can be operably linked include promoters for directing mRNA transcription. These include, but are not limited to, the left promoter from bacteriophage λ, the lac, TRP, and TAC promoters from *E. coli,* the early and late promoters from SV40, the CMV immediate early promoter, the adenovirus early and late promoters, and retrovirus long-terminal repeats.

In addition to control regions that promote transcription, expression vectors may also include regions that modulate transcription, such as repressor binding sites and enhancers. Examples include the SV40 enhancer, the cytomegalovirus immediate early enhancer, polyoma enhancer, adenovirus enhancers, and retrovirus LTR enhancers.

In addition to containing sites for transcription initiation and control, expression vectors can also contain sequences necessary for transcription termination and, in the transcribed region a ribosome-binding site for translation. Other regulatory control elements for expression include initiation and termination codons as well as polyadenylation signals. The person of ordinary skill in the art would be aware of the numerous regulatory sequences that are useful in expression vectors. Such regulatory sequences are described, for example, in Sambrook *et al., (Molecular Cloning: A Laboratory Manual. 2nd. ed.,* Cold Spring Harbor Laboratory Press, Cold Spring Harbor, NY, (1989)).

A variety of expression vectors can be used to express a nucleic acid molecule. Such vectors include chromosomal, episomal, and virus-derived vectors, for example vectors derived from bacterial plasmids, from bacteriophage, from yeast episomes, from yeast chromosomal elements, including yeast artificial chromosomes, from viruses such as baculoviruses, papovaviruses such as SV40, Vaccinia viruses, adenoviruses, poxviruses, pseudorabies viruses, and retroviruses. Vectors may also be derived from combinations of these sources such as those derived from plasmid and bacteriophage genetic elements, e.g., cosmids and phagemids. Appropriate cloning and expression vectors for prokaryotic and eukaryotic hosts are described in Sambrook *et al., Molecular Cloning: A Laboratory Manual. 2nd. ed.,* Cold Spring Harbor Laboratory Press, Cold Spring Harbor, NY, (1989).

The regulatory sequence may provide constitutive expression in one or more host cells (i.e. tissue specific) or may provide for inducible expression in one or more cell types such as by temperature, nutrient additive, or exogenous factor such as a hormone or other ligand. A variety of vectors providing for constitutive and inducible expression in prokaryotic and eukaryotic hosts are known to those of ordinary skill in the art.

The nucleic acid molecules can be inserted into the vector nucleic acid by well-known methodology. Generally, the DNA sequence that will ultimately be expressed is joined to an expression vector by cleaving the DNA sequence and the expression vector with one or more restriction enzymes and then ligating the fragments together. Procedures for restriction enzyme digestion and ligation are known to those of ordinary skill in the art.

The vector containing the appropriate nucleic acid molecule can be introduced into an appropriate host cell for propagation or expression using well-known techniques. Bacterial cells include, but are not limited to, *E. coli, Streptomyces*, and *Salmonella typhimurium.* Eukaryotic cells include, but are not limited to, yeast, insect cells such as *Drosophila*, animal cells such as COS and CHO cells, and plant cells.

As described herein, it may be desirable to express a peptide of the present invention as a fusion protein. Accordingly, the invention provides fusion vectors that allow for the production of such peptides. Fusion vectors can increase the expression of a recombinant protein, increase the solubility of the recombinant protein, and aid in the purification of the protein by acting for example as a ligand for affinity purification. A proteolytic cleavage site may be introduced at the junction of the fusion moiety so that the desired peptide can ultimately be separated from the fusion moiety. Proteolytic enzymes include, but are not limited to, factor Xa, thrombin, and enterokinase. Typical fusion expression vectors include pRS (Sikorski, et al., *Genetics* 122(1): 19-27 (1989)), pGEX (Smith *et al., Gene 67:* 31-40(1988)), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein. Examples of suitable inducible non-fusion *E. coli* expression vectors include pTrc (Amann *et al., Gene 69*: 301-315 (1988)) and pET 11d (Studier *et al., Gene Expression Technology: Methods in Enzymology* 185: 60-89 (1990)).

Recombinant protein expression can be maximized in a host bacteria by providing a genetic background wherein the host cell has an impaired capacity to proteolytically cleave the recombinant protein. (Gottesman, S., *Gene Expression Technology: Methods in Enzymology* 185, Academic Press, San Diego, California 119-128 (1990)). Alternatively, the sequence of the nucleic acid molecule of interest can be altered to provide preferential codon usage for a specific host cell, for example *E. coli.* (Wada *et al., Nucleic Acids Res. 20*: 2111-2118 (1992)).

The nucleic acid molecules can also be expressed by expression vectors that are operative in yeast. Examples of vectors for expression in yeast e.g., *S. cerevisiae* include pYepSec1 (Baldari, *et al., EMBO J. 6*: 229-234 (1987)), pMFa (Kurjan *et al., Cell 30:* 933-943 (1982)), pJRY88 (Schultz *et al., Gene 54*: 113-123 (1987)), and pYES2 (Invitrogen Corporation, San Diego, CA).

The nucleic acid molecules can also be expressed in insect cells using, for example, baculovirus expression vectors. Baculovirus vectors available for expression of proteins in cultured insect cells (e.g., Sf9 cells) include the pAc series (Smith *et al., Mol. Cell Biol. 3:* 2156-2165 (1983)) and the pVL series (Lucklow *et al., Virology 170:* 31-39 (1989)). In certain embodiments of the invention, the nucleic acid molecules described herein are expressed in mammalian cells using mammalian expression vectors. Examples of mammalian expression vectors include pCDM8 (Seed, B. *Nature 329:* 840 (1987)) and pMT2PC (Kaufman *et al*., *EMBO J. 6*: 187-195 (1987)).

The expression vectors listed herein are provided by way of example only of the well-known vectors available to those of ordinary skill in the art that would be useful to express the nucleic acid molecules. Preferred vectors include the pET24b (Novagen, Madison, WI), pAcSG2 (Pharmingen, San Diego, CA), and pFastBac (Life Technologies, Gaithersburg, MD). The person of ordinary skill in the art would be aware of other vectors suitable for maintenance propagation or expression of the nucleic acid molecules described herein. These are found for example in Sambrook, J., Fritsh, E. F., and Maniatis, T. *Molecular Cloning: A Laboratory Manual. 2nd, ed., Cold Spring Harbor Laboratory*, Cold Spring Harbor Laboratory Press, Cold Spring Harbor, NY, 1989.

The invention also encompasses vectors in which the nucleic acid sequences described herein are cloned into the vector in reverse orientation, but operably linked to a regulatory sequence that permits transcription of antisense RNA. Thus, an antisense transcript can be produced to all, or to a portion, of the nucleic acid molecule sequences described herein, including both coding and non-coding regions. Expression of this antisense RNA is subject to each of the parameters described above in relation to expression of the sense RNA (regulatory sequences, constitutive or inducible expression, tissue-specific expression).

The invention also relates to recombinant host cells containing the vectors described herein. Host cells therefore include, for example, prokaryotic cells, lower eukaryotic cells such as yeast, other eukaryotic cells such as insect cells, and higher eukaryotic cells such as mammalian cells. Preferred host cells of the instant invention include *E. coli* and Sf9.

The recombinant host cells are prepared by introducing the vector constructs described herein into the cells by techniques readily available to the person of ordinary skill in the art. These include, but are not limited to, calcium phosphate transfection, DEAE-dextran-mediated transfection, cationic lipid-mediated transfection, electroporation, transduction, infection, lipofection, and other techniques such as those found in Sambrook, *et al.* (*Molecular Cloning: A Laboratory Manual. 2nd, ed., Cold Spring Harbor Laboratory*, Cold Spring Harbor Laboratory Press, Cold Spring Harbor, NY, 1989).

Host cells can contain more than one vector. Thus, different nucleotide sequences can be introduced on different vectors of the same cell. Similarly, the nucleic acid molecules can be introduced either alone or with other nucleic acid molecules that are not related to the nucleic acid molecules such as those providing trans-acting factors for expression vectors. When more than one vector is introduced into a cell, the vectors can be introduced independently, co-introduced or joined to the nucleic acid molecule vector.

In the case of bacteriophage and viral vectors, these can be introduced into cells as packaged or encapsulated virus by standard procedures for infection and transduction. Viral vectors can be replication-competent or replication-defective. In the case in which viral replication is defective, replication will occur in host cells providing functions that complement the defects.

Vectors generally include selectable markers that enable the selection of the subpopulation of cells that contain the recombinant vector constructs. The marker can be contained in the same vector that contains the nucleic acid molecules described herein or may be on a separate vector. Markers include tetracycline or ampicillin-resistance genes for prokaryotic host cells and dihydrofolate reductase or neomycin resistance for eukaryotic host cells. However, any marker that provides selection for a phenotypic trait will be effective.

While the active protein kinases can be produced in bacteria, yeast, mammalian cells, and other cells under the control of the appropriate regulatory sequences, cell- free transcription and translation systems can also be used to produce these proteins using RNA derived from the DNA constructs described herein.

Where secretion of the peptide is desired, appropriate secretion signals are incorporated into the vector. The signal sequence can be endogenous to the proteins or heterologous to these proteins.

It is also understood that depending upon the host cell in recombinant production of the peptides described herein, the peptides can have various glycosylation patterns, depending upon the cell, or maybe non-glycosylated as when produced in bacteria. In addition, the peptides may include an initial modified methionine in some cases as a result of a host-mediated process.

The recombinant host cells expressing the peptides described herein have a variety of uses. First, the cells are useful for producing the MEK1 and MEK2 peptides or a peptide that can be further purified to produce desired amounts of the peptide or fragments. Thus, host cells containing expression vectors are useful for peptide production.

Host cells are also useful for conducting cell-based assays involving the protein or protein fragments. Thus, a recombinant host cell expressing a native protein is useful for assaying compounds that stimulate or inhibit protein function.

Host cells are also useful for identifying protein mutants in which these functions are affected. If the mutants naturally occur and give rise to a pathology, host cells containing the mutations are useful to assay compounds that have a desired effect on the mutant protein (for example, stimulating or inhibiting function) which may not be indicated by their effect on the native protein.

Genetically engineered host cells can be further used to produce non-human transgenic animals. A transgenic animal is preferably a mammal, for example a rodent, such as a rat or mouse, in which one or more of the cells of the animal include a transgene. A transgene is exogenous DNA which is integrated into the genome of a cell from which a transgenic animal develops and which remains in the genome of the mature animal in one or more cell types or tissues of the transgenic animal. These animals are useful for studying the function of the MEK1 or MEK2 peptide and identifying and evaluating modulators of the protein activity. Other examples of transgenic animals include non-human primates, sheep, dogs, cows, goats, chickens, and amphibians.

### Peptide Purification of Modified MEK1 and MEK2

The purification conditions and methods listed herein are provided to elucidate the approach used in the purification of the MEK1 and MEK2 peptides and for the formation, for example, of the MEK1: ligand: cofactor and MEK2:ligand:cofactor complexes. Of course those of ordinary skill in the art would be aware of other purification conditions and techniques that may be suitable for the purification of the modified MEK1 and MEK2 proteins described herein. For examples see, Methods in Enzymology, Volume 182; Guide to Protein Purification, edited by M.P. Duetscher; Academic Press (1990).

### Purification of Modified MEK1

The invention provides a multiple step method for purifying the modified MEK1 peptide, described herein, to near homogeneity. The modified MEK1 peptide, preferably the MEK1 peptide with a poly-histidine tag at the COOH-terminus, may be advantageously purified by employing immobilized metal affinity chromatography (IMAC). The immobilized metal may be nickel, zinc, cobalt, or copper. Preferably, the immobilized metal is cobalt, as found in the TALON™ metal affinity resin from ClonTech. The IMAC step may be performed on the soluble fraction of the fermentation broth containing the expressed MEK1 peptide, which is obtained after the host cell lysis. The host cell lysis processes are known to those skilled in the art and may be selected for by them without difficulty. For example, when *E. coli* is used as host cell for expressing the MEK1 peptides, *E. coli* cell lysis may be enzymatically performed, for example by using lysozyme. Alternatively, mechanical processes using a french press, sonicator, or bead bill may be employed. Preferably, a mechanical lysis using a bead mill (DynoMill KDL) is used to perform *E*. *coli* cell lysis.

The modified MEK1 peptide, preferably the MEK1 peptide containing the COOH-terminal His-Tag, may be purified by immobilized metal chelate (IMAC) chromatography. The IMAC step may be accomplished with the following resins, but not limited to: Ni-NTA™ resin from Qiagen, HisTrap™ resin from Pharmacia, POROS™ MC resin from Applied Biosystems or TALON resin from Clontech. Preferably, TALON resin is used. More preferably, the IMAC step is accomplished by the use of resin in a ratio of about 2 mL resin to about 1 g wet weight of whole cells, prior to cell lysis.

An additional aspect of the invention includes the use of a transition state analogue and a metal cation to displace a contaminant protein during the IMAC purification. When *E. coli* is employed as a host cell for expressing the MEK1 peptides, contaminant proteins such as *E. coli* prolyl isomerase are contained in the fermentation broth, and are preferably removed before crystallization. For the invention, a transition state analogue and a metal cation are employed to replace the contaminant protein during MEK1-binding and contaminant removal steps of the IMAC purification. The transition state analogue may be, but is not limited to, pyrrole-2-carboxylate, Δ-1-pyrroline-2-carboxylate, or tetrahydrofuran-2-carboxylate. Preferably, the transition state analogue is pyrrole-2-carboxylate. The transition state analogue, if present, has a concentration of from about 0.1 mM to about 20.0 mM, and even more preferably has a concentration of about 1.0 mM. As a source of the metal cation, a form of the zinc cation may be employed. It may be used in any salt form, for example zinc acetate, zinc chloride or zinc sulfate. Preferably, zinc is used in the form of zinc chloride at a concentration of about 0.05 mM.

The MEK1-binding and contaminant removal steps of the IMAC purification may be performed in the presence of any suitable buffering agent. For example, the buffering agent may be, but is not limited to, Tris [Tris(hydroxymethyl)-aminomethane], HEPES (N-2-hydroxyethyl-piperazine-N'-2-ethanesulfonic acid), potassium phosphate, citrate-phosphate, sodium phosphate, ammonium monobasic phosphate, or MOPS (3-(N-morpholino) propanesulfonic acid). Preferably, the buffering agent is about 50.0 mM ammonium or potassium monobasic phosphate having a pH of about 8.0.

The binding and contaminant removal steps of the IMAC purification may be performed in the presence of any suitable contaminating protein-displacing agents. The contaminating protein-displacing agent may be, but is not limited to, imidazole, or histidine. Preferably, the contaminating protein-displacing agent is about 5.0 mM imidazole.

The binding and contaminant removal steps of the IMAC purification can be performed in the presence of any suitable reducing agents. The reducing agent may be, but is not limited to, 2-mercaptoethanol, or TCEP (Tris[2-Carboxyethylphosphine] hydrochloride). Preferably, the reducing agent is about 2.0 mM TCEP.

The binding and contaminant removal steps of the IMAC purification may be performed in the presence of a detergent. The detergent may be, but is not limited to, CHAPS (3-([3-cholamidopropyl]-dimethylammonio)-1-propanesulfonate), Triton X-100, Nonidet P-40, Tween 20, or Tween 80. Preferably, the detergent is about 10.0 mM CHAPS.

The binding and contaminant removal steps of the IMAC purification may be performed in the presence of an ion source. The ion source may be, but is not limited to, KCl, NaCl, or sodium sulfate. Preferably, an ion source is about 0.3 M NaCl.

The elution of MEK1 from the IMAC resin may be accomplished by several modes, which are known in the art. For example, the elution of MEK1 from the IMAC column may be accomplished by using, for example, EDTA, histidine, or imidazole, or by reducing the pH. Preferably, the elution of MEK1 may be accomplished with about 100 mM EDTA.

The elution step of the IMAC purification may be performed in the presence of any suitable buffering agent(s). The buffering agent may be, but is not limited to, Tris, ammonium monobasic phosphate, HEPES, or MOPS. Preferably, the buffering agent may be about 20.0 mM HEPES or (pH of about 8.0). The elution of MEK1 also may be performed in the presence of any suitable organic agents. The organic agent may be, but is not limited to, glycerol or ethylene glycol. Preferably, the organic agent is about 10% glycerol.

The invention also provides a method for further purifying the protein using cation and anion exchange chromatography, followed by size exclusion chromatography (SEC). The cation exchange chromatography could occur prior to the anion exchange chromatography, or alternatively, the anion exchange chromatography could occur prior to the cation exchange chromatography. Further, the ion exchange steps could be eliminated all together. The preferred method is to perform the cation exchange step prior to the anion exchange step, which is followed by protein concentration and size exclusion chromatography.

The cation exchange step may be performed using several types of chromatography resins. For example, the cation exchange resin may be, but is not limited to, S-Sepharose™, MonoS™, POROS™ HS, or POROS™ S. Preferably, the cation exchange resin is POROS™ HS. The cation exchange step may be performed in the presence of any suitable buffering agent(s). The buffering agent may include, but is not limited to, phosphate, malonic acid, butanedioic acid, acetic acid, MES (2-(N-morpholino)-ethanesulfonic acid), or HEPES. Preferably, the buffering agent is about 20.0 mM MES (pH of about 6.4).

The cation exchange step may be performed in the presence of any suitable reducing agent(s). The reducing agent may be, but is not limited to, 2-mercaptoethanol, TCEP, or dithiothreitol (DTT). Preferably, the reducing agent is about 2.0 mM TCEP.

The cation exchange step may be performed in the presence of any suitable organic agent(s). The organic agent may be, but is not limited to, glycerol or ethylene glycol. Preferably, the organic agent is about 20.0% ethylene glycol.

Elution of the MEK1 from the cation exchange resin may be accomplished by several different modes that are known in the art. For example, the MEK1 may be eluted by increasing the pH. Alternatively, the MEK1 may be eluted by increasing the salt concentration by using, for example, NaC1, KC1, ammonium acetate, or sodium sulfate. Preferably, ammonium acetate may be used to cause the elution by an increase of the salt concentration.

The anion exchange step may be performed using several types of chromatography resin(s). The anion exchange resin may be, but is not limited to, Q-Sepharose™, DEAE-Sepharose™, MonoQ™, POROS™ HQ, or POROS™ PI. Preferably, the exchange resin is POROS™ HQ.

The anion exchange step may be performed in the presence of any suitable buffering agent(s). The buffering agent may be, but is not limited to, HEPES, Tris, bis-Tris, bis-Tris Propane, N-methyldiethanolamine, 1,3-diaminopropane, ethanolamine, piperazine, or ammonium monobasic phosphate. Preferably, the buffering agent is about 20.0 mM Tris (pH of about 8.0).

The anion exchange step may be performed in the presence of any suitable reducing agent(s). The reducing agent may be, but is not limited to, 2-mercaptoethanol, TCEP, or DTT. Preferably, the reducing agent is about 2.0 mM TCEP.

The anion exchange step may be performed in the presence of any suitable salt(s). The salt may be NaCl, KCl, ammonium acetate, or sodium sulfate. Preferably, the salt is about 10.0 mM ammonium acetate.

The SEC step may be performed using various types of chromatography resins. For example, the suitable SEC resin may include, but is not limited to, Sephadex™ G-100, Sephadex™ G-200, Sephacryl™ S-100, Sephacryl™ S-200, Superdex™ 75, or Superdex™ 200. Preferably, the SEC resin is Superdex™ 200.

The SEC step may performed in the presence of any suitable buffering agent(s). The buffering agent may be, but is not limited to, phosphate, HEPES, MES, Tris, bis-Tris, or bis-Tris propane. Preferably, the buffering agent is about 20.0 mM HEPES (pH of about 7.5).

The SEC step may be performed in the presence of any suitable reducing agent(s). The reducing agent may be, but is not limited to, 2-mercaptoethanol, TCEP, or DTT. Preferably, the reducing agent is about 2.0 mM TCEP.

The SEC step may be performed in the presence of any suitable salt(s). The salt may be, but is not limited to, NaCl, KCl, ammonium acetate, or sodium sulfate. Preferably, the salt is about 150.0 mM ammonium acetate.

The SEC step may be performed in the presence of any suitable chelating agent(s). The chelating agent may be, but is not limited to, sodium citrate or EDTA. Preferably, the salt is about 0.5 mM EDTA.

The invention also provides a method for forming a MEK1: ligand complex. The MEK1: ligand complex formation may be performed at any point during the MEK1 purification. Preferably, the MEK1: ligand complex formation step may be performed prior to the SEC step. Alternatively, the MEK1: ligand complex formation step could be performed after the protein has been completely purified. The MEK1: ligand complex formation step may be performed at various concentrations of MEK1. For example, the concentration of MEK1 may be in the range of about 0.02 mg/ml to about 2 mg/ml. Preferably, the concentration of MEK1 is about 0.2 mg/ml to about 0.3 mg/ml. Further, the MEK1: ligand complex formation step may be performed at various molar ratios of MEK1 to ligand. For example, the molar ratio of MEK1 to ligand may be in the range of about 1:1 to about 1:1000. Preferably, the molar ratio of MEK1 to ligand is about 1:10.

### Purification of Modified MEK2

The invention also provides a multiple step method for purifying the modified MEK2 peptide, described herein, to near homogeneity. The modified MEK2 peptide, preferably the MEK2 peptide with a poly-histidine tag at the COOH-terminus, may be advantageously purified by employing immobilized metal affinity chromatography (IMAC). The immobilized metal may be, for example, nickel, zinc, cobalt, or copper. Preferably, the immobilized metal is cobalt, as found in the TALON™ metal affinity resin from ClonTech. The IMAC step may be performed on the soluble fraction of the fermentation broth containing the expressed MEK2 peptide, which is obtained after the host cell lysis. The host cell lysis processes are known to those skilled in the art and may be selected for by them without difficulty. For example, when *E*. *coli* is used as host cell for expressing the MEK2 peptides, *E*. *coli* cell lysis may be enzymatically performed, for example by using lysozyme. Alternatively, mechanical processes using a french press, sonicator, or bead bill may be employed. Preferably, a mechanical lysis using a bead mill (DynoMill KDL) is used to perform *E*. *coli* cell lysis.

The modified MEK2 peptide, preferably the MEK2 peptide containing the COOH-terminal His-Tag, may be purified by immobilized metal chelate (IMAC) chromatography. The IMAC step may be accomplished with, for example, the following resins: Ni-NTA™ resin from Qiagen, HisTrap™ resin from Pharmacia, POROS™ MC resin from Applied Biosystems or TALON resin from Clontech. Preferably, TALON resin is used. More preferably, the IMAC step is accomplished by the use of resin in a ratio of about 1 mL resin to about 5 g wet weight of whole cells, prior to cell lysis.

An additional aspect of the invention includes the use of a transition state analogue and metal to displace a contaminant protein during the IMAC purification. When *E*. *coli* is employed as a host cell for expressing the MEK2 peptides, contaminant proteins such as *E*. *coli* prolyl isomerase are contained in the fermentation broth, and are preferably removed before crystallization. For the invention, a transition state analogue and a divalent metal cation are employed to displace the contaminant protein during the MEK2-binding and contaminant removal steps of the IMAC purification. The transition state analogue may be, but is not limited to, pyrrole-2-carboxylate, Δ-1-pyrroline-2-carboxylate, or tetrahydrofuran-2-carboxylate. Preferably, the transition state analogue is pyrrole-2-carboxylate. The transition state analogue, if present, has a concentration of from about 0.1 mM to about 20.0 mM, and even more preferably has a concentration of about 1.0 mM. As the divalent metal cation, zinc may be employed. It may be used in any salt form, for example zinc acetate, zinc chloride or zinc sulfate. Preferably, zinc is used in the form of zinc chloride at a concentration of about 0.05 mM.

The MEK2-binding and contaminant removal steps of the IMAC purification may be performed in the presence of any suitable buffering agent. For example, the buffering agent may be, but is not limited to, Tris [Tris(hydroxymethyl)-aminomethane], HEPES (N-2-hydroxyethyl-piperazine-N'-2-ethanesulfonic acid), potassium phosphate, citrate-phosphate, sodium phosphate, or MOPS (3-(N-morpholino) propanesulfonic acid). Preferably, buffering agent is about 50.0 mM potassium phosphate having pH of about 8.0.

The binding and contaminant removal steps of the IMAC purification may be performed in the presence of any suitable contaminating protein-displacing agents. The contaminating protein-displacing agent may be, but is not limited to, imidazole, or histidine. Preferably, the contaminating protein-displacing agent is about 5.0 mM imidazole.

The binding and contaminant removal steps of the IMAC purification can be performed in the presence of any suitable reducing agents. The reducing agent may be, but is not limited to, 2-mercaptoethanol, or TCEP (Tris[2-Carboxyethylphosphine] hydrochloride). Preferably, the reducing agent is about 2.0 mM TCEP.

The binding and contaminant removal steps of the IMAC purification may be performed in the presence of a detergent. The detergent may be, but is not limited to, CHAPS (3-([3-cholamidopropyl]-dimethylammonio)-1-propanesulfonate), Triton X-100, Nonidet P-40, Tween 20, or Tween 80. Preferably, the detergent is about 10.0 mM CHAPS.

The binding and contaminant removal steps of the IMAC purification may be performed in the presence of an ion source. The ion source may be, but is not limited to, KCl, NaCl, or sodium sulfate. Preferably, an ion source is about 0.3 M NaCl.

The elution of MEK2 from the IMAC resin may be accomplished by several modes, which are known in the art. For example, the elution of MEK2 from the IMAC column may be accomplished by using, for example, EDTA, histidine, or imidazole, or by reducing the pH. Preferably, the elution of MEK2 may be accomplished with about 0.1 M EDTA.

The elution step of the IMAC purification may be performed in the presence of any suitable buffering agent(s). The buffering agent may be, but is not limited to, Tris, phosphate, HEPES, or MOPS. Preferably, the buffering agent may be about 20.0 mM HEPES (pH of about 8.0). The elution of MEK2 also may be performed in the presence of any suitable organic agents. The organic agent may be, but is not limited to, glycerol or ethylene glycol. Preferably, the organic agent is about 10% glycerol.

The invention also provides a method for further purifying the protein using cation exchange chromatography, followed by concentration of the protein and size exclusion chromatography (SEC).

The cation exchange step may be performed using several types of chromatography resins. For example, the cation exchange resin may be, but is not limited to, S-Sepharose™, MonoS™, POROS™ HS, or POROS™ S. Preferably, the cation exchange resin is POROS™ HS. The cation exchange step may be performed in the presence of any suitable buffering agent(s). The buffering agent may include, but is not limited to, phosphate, malonic acid, butanedioic acid, acetic acid, MES (2-(N-morpholino)-ethanesulfonic acid), or HEPES. Preferably, the buffering agent is about 20.0 mM MES (pH of about 6.4).

The cation exchange step may be performed in the presence of any suitable reducing agent(s). The reducing agent may be, but is not limited to, 2-mercaptoethanol, TCEP, or dithiothreitol (DTT). Preferably, the reducing agent is about 2.0 mM TCEP.

The cation exchange step may be performed in the presence of any suitable organic agent(s). The organic agent may be, but is not limited to, glycerol or ethylene glycol. Preferably, the organic agent is about 20.0% ethylene glycol.

Elution of the MEK2 from the cation exchange resin may be accomplished by several different modes that are known in the art. For example, the MEK2 may be eluted by increasing the pH. Alternatively, the MEK2 may be eluted by increasing the salt concentration by using, for example, NaCl, KCl, ammonium acetate, or sodium sulfate. Preferably, ammonium acetate may be used to cause the elution by an increase the salt concentration.

The SEC step may be performed using various types of chromatography resins. For example, the suitable SEC resin may include, but is not limited to, Sephadex™ G-100, Sephadex™ G-200, Sephacryl™ S-100, Sephacryl™ S-200, Superdex™ 75, or Superdex™ 200. Preferably, the SEC resin is Superdex™ 200.

The SEC step may performed in the presence of any suitable buffering agent(s). The buffering agent may be, but is not limited to, phosphate, HEPES, MES, Tris, bis-Tris, or bis-Tris propane. Preferably, the buffering agent is about 20.0 mM HEPES (pH of about 7.5).

The SEC step may be performed in the presence of any suitable reducing agent(s). The reducing agent may be, but is not limited to, 2-mercaptoethanol, TCEP, or DTT. Preferably, the reducing agent is about 2.0 mM TCEP.

The SEC step may be performed in the presence of any suitable salt(s). The salt may be, but is not limited to, NaCl, KCl, ammonium acetate, or sodium sulfate. Preferably, the salt is about 150.0 mM ammonium acetate.

The SEC step may be performed in the presence of any suitable chelating agent(s). The chelating agent may be, but is not limited to, sodium citrate or EDTA. Preferably, the salt is about 0.1 mM EDTA.

The invention also provides a method for forming a MEK2: ligand complex. The MEK2: ligand complex formation may be performed at any point during the MEK2 purification. Preferably, the MEK2: ligand complex formation step may be performed following the SEC step. Alternatively, the MEK2: ligand complex formation step could be performed prior to the SEC step. The MEK2: ligand complex formation step may be performed using various concentrations of MEK2. For example, the concentration of MEK2 may be in the range of about 0.02 mg/ml to about 2 mg/ml. Preferably, the concentration of MEK2 is about 0.2 mg/ml to about 0.3 mg/ml. Further, the MEK2: ligand complex formation step may be performed at various molar ratios of MEK2 to ligand. For example, the molar ratio of MEK2 to ligand may be in the range of about 1:1 to about 1:1000. Preferably, the molar ratio of MEK2 to ligand is about 1:10.

### Crystallization of Modified MEK1 and MEK2 Peptide Complexes

The present invention further includes methods of using modified MEK1 and MEK2 peptides, such as NH₂-terminally truncated MEK1 and MEK2 peptides, to grow crystals of a MEK1 or MEK2 peptide: ligand: cofactor complex. The crystallization conditions and methods listed herein are provided to elucidate one approach used in the crystallization of the MEK1 or MEK2 peptide: ligand: cofactor complexes. Of course those of ordinary skill in the art would be aware of other crystallization conditions and techniques that may be suitable for the crystallization of the modified MEK1 proteins described herein. For examples see, McPherson, A., Crystallization of Biological Macromolecules, Cold Spring Harbor Laboratory Press (1999).

### Crystallization of Modified MEK1 Peptide Complexes

Generally, the crystallization of modified MEK1 peptide complexes comprises contacting a NH₂-terminally truncated MEK1 peptide with a ligand and a cofactor, wherein a stable ternary complex of a MEK1 peptide: ligand: cofactor is formed, and then growing a crystal of the MEK1 peptide: ligand: cofactor complex by adding the solution of the ternary complex to a precipitating solution. For example, in order to produce crystals of a MEK1 peptide: ligand: cofactor complex, a solution ("MEK1 solution") containing the MEK1 peptide, a ligand and a cofactor and a precipitant solution are provided. The concentration of MEK1 in the MEK1 solution is from about 2 mg/mL to about 40 mg/mL, preferably from about 10 mg/mL to about 20 mg/mL, and more preferably is about 15 mg/mL. The concentration of ligand is from about 2- and about 20-fold in excess that of the MEK1 concentration, preferably, is from about 5- and about 15-fold in excess that of the MEK1 concentration, and more preferably, is about 10-fold excess. The concentration of cofactor in the MEK1 solution is from about 1 mM to about 100 mM, preferably from about 2.5 mM to about 25 mM and, more preferably, is about 5 mM.

The cofactor may include, but is not limited to, an ATP-cation, non-hydrolyzable ATP analogue such as AMP-PNP (adenylyl-imidodiphosphate), or ATP-Gamma-S (Adenosine 5'-O-3-thiotriphosphate). The ATP-cation may include, but is not limited to, a lithium, sodium, magnesium, or potassium salt of ATP. Preferably, the ATP-cation is a sodium or magnesium salt of ATP. More preferably, the ATP-cation is a magnesium salt of ATP ("Mg-ATP").

The MEK1 solution may comprise, but is not limited to, MEK1, ligand, cofactor, a buffering agent, a reducing agent and a source of ionic strength. The concentrations of the protein, ligand and cofactor are described above. The buffering agent may be, but is not limited to, phosphate, MES, HEPES (N-2-hydroxyethyl-piperazine-N'-2-ethanesulfonic acid), Tris, bis-Tris, or bis-Tris propane. Preferably, the buffering agent is HEPES of a concentration between about 10 mM and about 100 mM and a pH value between about 6.8 and about 8.8. More preferably, the buffering agent is about 20 mM HEPES (pH 7.8). The reducing agent may be, but is not limited to, 2-mercaptoethanol, TCEP or DTT. Preferably, the reducing agent is TCEP at a concentration between about 0.1 mM and about 10 mM. More preferably, the reducing agent is about 2.0 mM TCEP. The MEK1 solution may contain a salt as source of ionic strength. The salt may be, but is not limited to, NaCl KCl, ammonium acetate or sodium sulfate. Preferably, the salt is ammonium acetate at a concentration between about 5 mM and about 500 mM. More preferably, the salt is about 150 mM ammonium acetate.

The MEK1 solution may optionally contain any suitable chelating agent(s). The chelating agent may be sodium citrate. When the MEK1 solution contains a chelating agent, about 0.5 mM EDTA is preferably used as the chelating agent.

In addition, any precipitating solution ("MEK1 precipitant solution") may also be used in the crystallization of MEK1 peptide : ligand : cofactor complex. When mixed with the MEK1 solution described above, the precipitating solution preferably causes the ternary complex to form well-diffracting crystals. The MEK1 precipitant solution may comprise a variety of components designed to stabilize the formation of the MEK1 peptide: ligand: cofactor complex as a crystalline solid. For example, the precipitant solution may include, but is not limited to, a source of ionic strength, a source of polyethylene glycol (PEG), a buffering agent, and a reducing agent.

The buffering agent of the MEK1 precipitant solution may be, but is not limited to, phosphate, acetate, succinate, malonate, malate, imidazole, MES, Tris, or bis-Tris propane, or any combination thereof. Preferably, the buffering agent is a mixed buffer system of imidazole/ malate/ phosphate buffer. The concentrations of the buffers may be in the ranges of about 1 mM - about 100 mM for imidazole, about 10 mM - about 1000 mM for malate, and about 40 mM - about 1400 mM for phosphate. The mixed buffer system may have a pH value between about pH 3.0 and about pH 7.0. For the phosphate buffer, ammonium phosphate monobasic may be preferably used. Most preferably, the mixed buffer system comprises about 10 mM imidazole, about 100 mM malate, and about 400 mM ammonium phosphate and has a pH value between about pH 4.5 and about pH 5.5.

The source of ionic strength of the MEK1 precipitant solution may be, but is not limited to, NaCl, KCl, ammonium sulfate, lithium sulfate, ammonium phosphate, or sodium potassium phosphate. The mixed buffer system of the MEK1 precipitant solution may serve as an ionic strength source. Preferably, monobasic ammonium phosphate is added, without adjustment of the pH, to a concentration of 100 -1000 mM, or more preferably to a concentration of 200 - 600 mM to a solution of the 10 mM imidazole/100 mM malate acid buffer solution that has had the pH value adjusted to pH 7.0 with a 50% (v/v) solution of KOH. The resulting solution preferably has the appropriate ionic strength and a final pH value in the range of about pH 4.5 to about pH 5.5.

The MEK1 precipitant solution also may contain any suitable reducing agent(s). The reducing agent may include, but is not limited to, 2-mercaptoethanol, TCEP or DTT at a concentration of about 0.1 mM to about 100 mM. Preferably, DTT may be used. Most preferably, about 20 mM DTT may be used.

Many possible method could be used to grow the crystals of the MEK1 peptide: ligand: cofactor complex, including, but is not limited to, hanging-drop vapor diffusion, sitting-drop vapor diffusion, microbatch, batch, or counter diffusion in gels or oils. Preferably, the crystallization may be performed by hanging-drop vapor diffusion. When using the method of hanging drop vapor diffusion to grow the MEK1: ligand: cofactor crystals, the MEK1 solution is mixed with a droplet of the MEK1 precipitant solution to obtain a mixed droplet solution. The mixed droplet solution is then suspended over a well of precipitant solution in a sealed container. For example, about 1 µL of the MEK1 solution is mixed with the MEK1 precipitant solution in a ratio from about 1:4 to about 4:1, and preferably from about 1:2 to about 2:1. More preferably, the ratio of the MEK1 solution to the MEK1 precipitant solution is about 1:1. In one embodiment, the mixed droplet may be suspended over a well solution containing between 0.6 mL and 1.2 mL, and more preferably about 1.0 mL of precipitant solution. The crystallization temperature may be between about 4°C and about 20°C, preferably is about 13°C, and more preferably the crystals are grown at 13°C for 3-5 days and then moved to room temperature.

The mixed droplet solution is allowed to stand suspended over the well solution containing the MEK1 precipitant solution at the temperature described above for a period of about 5 days to about 5 weeks until the MEK1 peptide: ligand: cofactor crystals reach a size appropriate for crystallographic data collection, preferably between about 0.05 x about 0.05 x about 0.1 mm to about 0.3 x about 0.3 x about 0.5 mm.

Standard micro and/or macro seeding may also be used to obtain a crystal of X-ray diffraction quality, i.e. a crystal that will diffract to a resolution greater than 5.0 Å. In the preferred form, no seeding is used to grow diffraction quality crystals.

After the desired growth is achieved, the crystals of MEK1 peptide: ligand: cofactor complex, may be harvested and bathed in a cryoprotective solution. The cryoprotective solution may comprise a variety of components designed to stabilize the formation of a vitreous solid containing the MEK1 peptide: ligand: cofactor complex as a crystalline solid at a temperature of about 110 Kelvin. The cryoprotective solution may comprise, but is not limited to, a suitable source of low molecular weight solution of ethylene glycol or polyethylene glycol (PEG), a diluting agent and a cryo oil solution made up of about 70% Paratone-N oil and 30% mineral oil. The low molecular weight ethylene glycol or polyethylene glycol solution may have a molecular weight of about 100 to about 1000, more preferably about 200 to 600, and most preferably a 100% solution of ethylene glycol is used. The ethylene glycol or PEG may be used as a 100% (w/v) solution and then diluted with the crystallization well solution to a final concentration of between 5 and 25%. Most preferably the PEG solution will be made to a final concentration of 10% with the well solution. During the cryo preparation process, the crystal may first be removed from the mother liquor and then bathed in the 10% PEG solution for 1 -20 minutes. Most preferably the crystal will placed in the PEG/ well solution for about 2 minutes. The crystal may then be removed to a Paratone-N and mineral oil mixture and manipulated to in the oil to remove the aqueous solution prior to quickly placing the cryo-protected crystal into a liquid nitrogen bath.

Although any ligand for the NH₂-terminally truncated MEK1 peptide may be used, preferably the ligand comprises a MEK1 or MEK2 inhibitor. Preferably, the ligand is 5-bromo-N-(2,3-dihydroxy-propoxy)-3,4-difluoro-2-(2-fluoro-4-iodo-phenylamino)-benzamide having the following chemical structure:

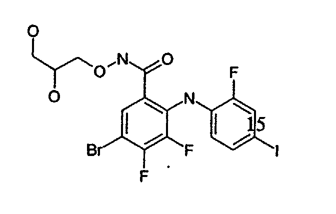

A crystal of the present invention may take a variety of crystal forms, all of which are included in the present invention. In one embodiment the crystals may be triclinic, monoclinic, orthorhombic, tetragonal, cubic, trigonal or hexagonal. In a preferred embodiment the crystal has hexagonal symmetry. In a more preferred embodiment the crystal has hexagonal symmetry and has the space group P6₂. In the most preferred form the crystal has the space group P6₂ and has a unit cell consisting of approximately: a = b = 81.4 ± 0.3 Å; c = 129.0 ± 0.3 Å; α = β = 90.0°; γ = 120.0°.

### Crystallization of Modified MEK2 Peptide Complexes

Generally, the crystallization of modified MEK2 peptide complexes comprises contacting a NH₂-terminally truncated MEK2 peptide with a ligand and a cofactor, wherein a stable ternary complex of a MEK2 peptide: ligand: cofactor is formed, and then growing a crystal of the MEK2 peptide: ligand: cofactor complex by adding the solution of the ternary complex to a precipitating solution. For example, in order to produce crystals of a MEK2 peptide: ligand: cofactor complex, a solution ("MEK2 solution") containing the MEK2 peptide, a ligand and a cofactor and a precipitant solution are provided. The concentration of MEK2 in the MEK2 solution is from about 2 mg/mL to about 40 mg/mL, preferably from about 10 mg/mL to about 20 mg/mL, and more preferably is about 15 mg/mL. The concentration of ligand is from about 2- and about 20-fold in excess that of the MEK2 concentration, preferably, is from about 5- and about 15-fold in excess that of the MEK2 concentration, and more preferably, is about 10-fold excess. The concentration of cofactor in the MEK2 solution is from about 1 mM to about 100 mM, preferably from about 2.5 mM to about 25 mM and, more preferably, is about 5 mM.

The cofactor may include, but is not limited to, an ATP-cation, non-hydrolyzable ATP analogue such as AMP-PNP (adenylyl-imidodiphosphate), or ATP-Gamma-S (Adenosine 5'-O-3-thiotriphosphate). The ATP-cation may include, but is not limited to, a lithium, sodium, magnesium, or potassium salt of ATP. Preferably, the ATP-cation is a sodium or magnesium salt of ATP. More preferably, the ATP-cation is a magnesium salt of ATP ("Mg-ATP").

The MEK2 solution may comprise, but is not limited to, MEK2, ligand, cofactor, a buffering agent, a reducing agent and a source of ionic strength. The concentrations of the protein, ligand and cofactor are described above. The buffering agent may be, but is not limited to, phosphate, MES, HEPES (N-2-hydroxyethyl-piperazine-N'-2-ethanesulfonic acid), Tris, bis-Tris, or bis-Tris propane. Preferably, the buffering agent is HEPES of a concentration between about 10 mM and about 100 mM and a pH value between about pH 6.8 and about pH 8.8. More preferably, the buffering agent is about 20 mM HEPES (pH 7.5). The reducing agent may be, but is not limited to, 2-mercaptoethanol, TCEP or DTT. Preferably, the reducing agent is TCEP at a concentration between about 0.1 mM and about 10 mM. More preferably, the reducing agent is about 2.0 mM TCEP. The MEK2 solution may contain a salt as source of ionic strength. The salt may be, but is not limited to, NaCl, KCl, ammonium acetate or sodium sulfate. Preferably, the salt is ammonium acetate at a concentration between about 5 mM and about 500 mM. More preferably, the salt is about 150 mM ammonium acetate.

The MEK2 solution may optionally contain any suitable chelating agent(s). The chelating agent may be sodium citrate. When the MEK2 solution contains a chelating agent, about 0.1 mM EDTA is preferably used as the chelating agent.

In addition, any precipitating solution ("MEK2 precipitant solution") may also be used in the crystallization of MEK2 peptide : ligand : cofactor complex. When mixed with the MEK2 solution described above, the precipitating solution preferably causes the ternary complex to form well-diffracting crystals. The MEK2 precipitant solution may comprise a variety of components designed to stabilize the formation of the MEK2 peptide: ligand: cofactor complex as a crystalline solid. For example, the precipitant solution may include, but is not limited to, a source of ionic strength, a buffering agent, and a reducing agent.

The buffering agent of the MEK2 precipitant solution may be, but is not limited to, sodium / potassium phosphate, ammonium phosphate, acetate, imidazole, MES, HEPES, Tris, or bis-Tris propane, or any combination thereof. Preferably, the buffering agent is also the source of ionic strength using a mixed solution of sodium monobasic phosphate and potassium dibasic phosphate buffered to about pH 6 to about pH 8. The concentration of the sodium potassium phosphate buffer may preferably be in the range of about 1.4 to about 1.9 M sodium monobasic phosphate and potassium dibasic phosphate. Most preferably, the mixed phosphate buffer system may be in the range of about 1.4 to about 1.9 M sodium monobasic phosphate and potassium dibasic phosphateand buffered to about pH 6.7 to about pH 7.1.

The MEK2 precipitant solution also may contain any suitable reducing agent(s). The reducing agent may include, but is not limited to, 2-mercaptoethanol, TCEP or DTT at a concentration of about 0.1 mM to about 100 mM. Preferably, DTT may be used. Most preferably, about 20 mM DTT may be used.

Many possible method could be used to grow the crystals of the MEK2 peptide: ligand: cofactor complex, including, but is not limited to, hanging-drop vapor diffusion, sitting-drop vapor diffusion, microbatch, batch, or counter diffusion in gels or oils. Preferably, the crystallization may be performed by hanging-drop vapor diffusion. When using the method of hanging drop vapor diffusion to grow the MEK2: ligand: cofactor crystals, the MEK2 solution is mixed with a droplet of the MEK2 precipitant solution to obtain a mixed droplet solution. The mixed droplet solution is then suspended over a well of precipitant solution in a sealed container. For example, about 1 µL of the MEK2 solution is mixed with the MEK2 precipitant solution in a ratio from about 1:4 to about 4:1, and preferably from about 1:2 to about 2:1. More preferably, the ratio of the MEK2 solution to the MEK2 precipitant solution is about 1:1. In one embodiment, the mixed droplet may be suspended over a well solution containing between 0.6 mL and 1.2 mL, and more preferably about 1.0 mL of precipitant solution. The crystallization temperature may be between about 4°C and about 20°C, and preferably is about 13°C. The mixed droplet solution is allowed to stand suspended over the well solution containing the MEK2 precipitant solution at the temperature described above for a period of about 5 days to about 5 weeks until the MEK2 peptide: ligand: cofactor crystals reach a size appropriate for crystallographic data collection, preferably between about 0.05 x about 0.05 x about 0.1 mm to about 0.3 x about 0.3 x about 0.5 mm.

Standard micro and/or macro seeding may also be used to obtain a crystal of X-ray diffraction quality, i.e. a crystal that will diffract to a resolution greater than 5.0 Å. In the preferred form, no seeding is used to grow diffraction quality crystals.

After the desired growth is achieved, the crystals of MEK2 peptide: ligand: cofactor complex, may be harvested and bathed in a cryoprotective solution. The cryoprotective solution may comprise a variety of components designed to stabilize the formation of a vitreous solid containing the MEK2 peptide: ligand: cofactor complex as a crystalline solid at a temperature of about 110 Kelvin. The cryoprotective solution may comprise, but is not limited to, a low molecular weight polyethylene glycol (PEG), glycerol or ethylene glycol, and / or a cryo oil solution made up of about 70% Paratone-N oil and 30% mineral oil. The cryo solution may be used by combining the ethylene glycol with the Paratone-N and mineral oil mixture as was done with MEK1 or by using either the Paratone-N / mineral oil mixture or the ethylene glycol independently. In a preferred form, the ethylene glycol was used at a concentration of about 5 -30% made by mixing a 100% solution of ethylene glycol with the well solution from the crystallization condition. In the most preferred form, the crystals may be removed from the mother-liquor crystal growth solution directly to the Paratone-N and mineral oil mixture using a mounted loop and manipulated in the oil to remove the aqueous solution prior to quickly placing the cryo-protected crystal into a liquid nitrogen bath.

Although any ligand for the NH₂-terminally truncated MEK2 peptide may be used, preferably the ligand comprises a MEK2 or MEK2 inhibitor. Preferably, the ligand is {5-[3,4-Difluoro-2-(2-fluoro-4-iodo-phenylamino)-phenyl]-1,3,4-oxadiazol-2-yl}-(2-morpholin-4-yl-ethyl)-amine having the following chemical structure:

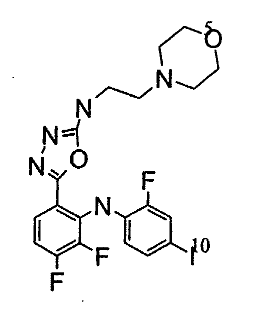

A crystal of the present invention may take a variety of crystal forms, all of which are included in the present invention. In one embodiment the crystals may be triclinic, monoclinic, orthorhombic, tetragonal, cubic, trigonal or hexagonal. In a preferred embodiment the crystal has hexagonal symmetry. In a more preferred embodiment the crystal has hexagonal symmetry and has the space group P6₁22. In the most preferred form the crystal has the space group P6₁22 and has a unit cell consisting of approximately: a = b = 161.89 ± 0.3 Å; c = 122.99 ± 0.3 Å; α = β = 90.0°; γ = 120.0°.

### X-ray data collection and structural determination

The present invention further includes methods for collecting X-ray diffraction data on the MEK1 and MEK2 peptide: ligand: cofactor crystals. The data collection conditions and methods listed herein are provided to elucidate the approach used for structural determination of the MEK1 and MEK2 peptide: ligand: cofactor crystals. Of course those of ordinary skill in the art would be aware of other conditions and techniques that may be suitable for the X-ray data collection and structural determination of the modified MEK1 or MEK2 protein crystals described herein. For examples see, Glusker, J., Crystal Structure Analysis for Chemists and Biologists, Wiley-VCH Press (1994) or the International Tables for X-Ray Crystallography, Volume F, edited by M.G. Rosssman and E. Arnold, Kluwer Academic Publishers (2001).

Generally, collecting the X-ray diffraction data for the MEK1 or MEK2 peptide: ligand: cofactor complex crystals comprises mounting the crystals in a cryo loop, bathing the crystals in a cryo protectant solution, rapidly cooling the crystals to about 100 K and collecting diffraction data in the oscillation mode. The source of X-rays may be, but is not limited to, a rotating anode home source such as a Rikagu RU-H3R generator, or a high energy synchrotron source such as that found on beamline 17 (17-ID or 17-BM, IMCA-CAT) at the Argonne National Laboratory Advanced Photon Source. The preferred method of data collection is to collect an initial data set using the home source to evaluate the crystal quality and then collecting a complete data set at IMCA-CAT. The method of detecting and quantitating the diffraction data may be performed by using, for example, an image plate such as a R-Axis IV⁺⁺ from MSC/Rigaku, or a charge-coupled device like a MAR-CCD, or ADSC Quantum 210 X-ray detector. Preferably, the resulting MEK1 crystal diffracts X-rays for the determination of the atomic coordinates of the MEK1 peptide: ligand: cofactor complex, to a resolution better than 5.0 Å, more preferably to a resolution better than 3.0 Å, and even more preferably to a resolution of better than 2.5 Å. Preferably, the resulting MEK2 crystal diffracts X-rays for the determination of the atomic coordinates of the MEK2 peptide: ligand: cofactor complex, to a resolution better than 5.0 Å, more preferably to a resolution better than 3.5 Å.

Once the data is collected, it is generally corrected for Lorenz and polarization effects and converted to indexed structure factor amplitudes using data processing software, for example DENZO or HKL2000 (Otwinowski, Z. and Minor, W., Processing of X-ray diffraction data collected in oscillation mode, *Methods Enzymol.* 276: 307-326 (1997)), d*Trek (Rigaku MSC) or Mosfilm (Leslie, A.G.W., Joint CCP4 + ESF-EAMCB Newsletter on Protein Crystallography, No. 26 (1992)). The preferred processing software may be HKL2000. The scaled and reduced diffraction data from the crystal may be used to determinae the three-dimensional crystal structure using one or more of the following methods or by other similar methods not included in this list: Fourier difference methods, molecular replacement (MR), multiwavelength anomalous dispersion (MAD), single-wavelength anomalous dispersion (SAD), single isomorphous replacement with anomalous scattering (SIRAS) or multiple isomorphous replacement (MIR).

### MEK1 and MEK2 Crystallographic Structural Analysis

The present invention further includes methods for solving the three-dimensional structural coordinates of the MEK1 and MEK2 peptide: ligand: cofactor crystalline complexes using the X-ray diffraction data. The methods used for structural determination are provided to elucidate the approach used for the structural determination of the crystalline MEK1 and MEK2 peptide: ligand: cofactor crystalline complexes. Those of ordinary skill in the art would be aware of other conditions and techniques that may be suitable for the X-ray structural determination of the modified MEK1 and MEK2 protein complexes described herein. For examples see, Glusker, J., Crystal Structure Analysis for Chemists and Biologists, Wiley-VCH Press (1994) or the International Tables for X-Ray Crystallography, Volume F, edited by M.G. Rosssman and E. Arnold, Kluwer Academic Publishers (2001)..

In the structural determination of the first MAPK kinase, it was necessary to use methods other than molecular replacement to solve the phase problem. In this case, an iodine atom contained within the bound ligand/inhibitor was used for SAD phase estimation. Other methods like MIR, MAD or SAD could have alternatively been used. In a preferred embodiment, the data may be scaled isomorphously using the SCALEPACK module in HKL2000 (Otwinowski et al, *Methods Enzymol*. 276:307-326 (1997)). Alternatively, the data could have been scaled using d*Trek (Rigaku MSC), or SCALA (COLLABORATIVE COMPUTATIONAL PROJECT, No. 4, "The CCP4 Suite: Programs for Protein Crystallography" *Acta Cryst.* D50, 760-763 (1994)). All subsequent calculations may be done using the CNX package (Accelrys, San Diego, CA.). Alternatively, the structural coordinates could have been identified using the CCP4 program suite, or a combination of other software packages known to those skilled in the art.

After the structures of MEK1 and MEK2 were solved, the three-dimensional structures were prepared for use in structure-based drug design. The preparation and analysis of the MEK1 and MEK2 templates may be performed by using, for example, SYBYL®, GRIN/GRID®, MolCad®, GOLD®, FlexX®. Additionally, suitable computer modeling software can optionally be used to perform structural determination. Such software includes, but is not limited to, QUANTA® (Accelrys, San Diego, CA), CHARMm® (Accelrys), INSIGHT® (Accelrys), SYBYL® (Tripos, Inc., St. Louis), MacroModel® (Schrödinger, Inc.) and ICM (MolSoft, LLC), with SYBYL® being the most preferable program. The computer program may be used alone or combined with a docking computer program such as GRAMM (Ilya A. Vakser, Rockefeller Univ.), FlexX® (Tripos Inc.), Flexidock® (Tripos Inc.), GOLD (commercially available via Cambridge Crystallographic Data Centre, Cambridge, UK), DOCK (Irwin Kuntz, Department of Pharmaceutical Chemistry at the University of California, San Francisco), or AutoDock® (Molecular Graphics Laboratory). These docking computer programs scan known databases of small molecules to find core compounds that roughly fit the binding sites. GOLD® may most preferably be used

If necessary, crystallographic data in PDB (Protein DataBank) files can be "cleaned up" by modifying the atom types of the inhibitor and cofactor and any water molecules that are present so that the water molecules find their lowest energy rotamer. Suitable software for performing this "clean up" include, but are not limited to, SYBYL®, WATCHECK (part of CCP4 suite, COLLABORATIVE COMPUTATIONAL PROJECT, No. 4, "The CCP4 Suite: Programs for Protein Crystallography." *Acta Cryst*. D50, 760-763 (1994)), and REDUCE (Word, *et al*., "Asparagine and glutamine: using hydrogen atom contacts in the choice of side chain amide orientation" *J*. *Mol. Bio*. 285: 1733-45 (1999)), with REDUCE, or any software performing the equivalent function as REDUCE, being the most preferred software. Any suitable docking computer program may be used to further validate the refined protein structure by adding all of the hydrogens in the most favorable protonation state as well as rotating all water molecules into orientations that give the optimal interactions with the protein.

Further, the binding sites may be characterized using, for example, GRIN/GRID® (Molecular Discovery Limited), MOLCAD® (Tripos, Inc.) contouring, CAVEAT (P.A. Bartlett, *et al*., CAVEAT: A program to facilitate the structure-derived design of biologically active molecules, in molecular recognition in chemical and biological problems, special Publication, *Royal Chem. Soc.*, 78, 182-196 (1989), available from the University of California, Berkely, CA), GRASP (A. Nicholls, Columbia Unviersity), SiteID® (Tripos, Inc.), INSIGHT®, or SYBYL®. These softwares may be used individually or in combination. For example, the combination of GRID/GRID®, MolCad® (Tripos, Inc.) and SYBYL® may be preferably used.

According to one embodiment of the invention, it has been discovered that the MEK1 peptide comprises a ligand-binding pocket that is defined by the structural coordinates of the following amino acid residues within 4 Å of a MEK1 inhibitor located in the ligand-binding site: G77, N78, G79, G80, K97, I99, L115, L118, V127, F129, I141, M143, C207, D208, F209, G210, V211, S212, L215, I216, M219 of SEQ ID NO: 2; or by structural coordinates of the following amino acid residues within 5 Å of a MEK1 inhibitor located in the ligand-binding site: G77, N78, G79, G80, K97, I99, L115, L118, I126, V127, G128, F129, I141, M143, D190, N195, L206, C207, D208, F209, G210, V211, S212, L215, I216, M219, F223 of SEQ ID NO: 2.

Further, it has been discovered that the MEK1 peptide comprises a cofactor-binding pocket that is defined by the structural coordinates of the following residues within 4 Å of the ATP molecule in the cofactor-binding site: L74, G75, A76, G77, N78, G80, V81, V82, A95, K97, V127, M143, E144, H145, M146, G149, S150, D152, Q153, K192, S194, N195, L197, D208, V224 of SEQ ID NO: 2; or structural coordinates of the following residues within 5 Å of the ATP molecule in the cofactor-binding site: L74, G75, A76, G77, N78, G79, 80, V81, V82, A95, K97, V127, M143, E144, H145, M146, D147, G149, S150, D152, Q153, D190, K192, S194, N195, L197, C207, D208, V224, G225 of SEQ ID NO: 2.

It has also been discovered that the MEK2 peptide comprises a ligand-binding pocket that is defined by the structural coordinates of the following amino acid residues within 4 Å of a MEK2 inhibitor located in the ligand-binding site: G81, N82, G83, G84, K101, I103, L119, L122, V131, F133, I145, M147, C211, D212, F213, G214, V215, S216, L219, I220, M223 of SEQ ID NO: 4, or a conservatively substituted variant thereof; and is defined by structural coordinates of the following amino acid residues within 5 Å of a MEK2 inhibitor located in the ligand-binding site: G81, N82, G83, G84, K101, I103, L119, L122, I130, V131, G132, F133, I145, M147, D194, N199, L210, C211, D212, F213, G214, V215, S216, L219, I220, M223, F227 of SEQ ID NO: 4, or a conservatively substituted variant thereof.

Additionally, it has been discovered that the MEK2 peptide comprises a co-factor-binding pocket that is defined by the structural coordinates of the following residues within 4 Å of the ATP molecule in the cofactor-binding site: L78, G79, A80, G81, N82, G84, V85, V86, A99, K101, V131, M147, E148, H149, M150, G153, S154, D156, Q157, K196, S198, N199, L201, D212, V228 of SEQ ID NO: 4, or a conservatively substituted variant thereof; and is defined by the structural coordinates of the following residues within 5 Å of the ATP molecule in the cofactor-binding site: L78, G79, A80, G81, N82, G83, G84, V85, V86, A99, K101, V131, M147, E148, H149, M150, D151, G153, S154, D156, Q157, D194, K196, S198, N199, L201, C211, D212, V228, G229 of SEQ ID NO: 4, or a conservatively substituted variant thereof.

Those of skill in the art will recognize that a set of structural coordinates for a peptide or a peptide: ligand: cofactor complex or a subset thereof, is a relative set of points in space that defines a complex three dimensional surface. As such it is possible to represent the same surface using an entirely different set of coordinates. Also, due to small errors in the measurement of all crystallographic data, slight variations in the individual coordinates will have little or no effect on the overall surface. Thus, a binding pocket could be generated from the structural coordinates provided in Table 1 or Table 2 from some variation of the structural coordinate that still retains similar surface features, including, but not limited to, volume (both internally in cavities or in total), solvent accessibility, and surface charge and hydrophobicity. In addition, the structural coordinates could be modified by crystallographic permutations, including, but not limited to, fractionalization, integer addition or substraction, inversion or any combination thereof.

In addition, it would be apparent to one skilled in the art that the binding pockets described in detail above could be modified in order to obtain somewhat different three-dimesional coordiates.

It should also be recognized that minor modification of any or all of the components of the peptide: ligand: cofactor complexes that results in the generation of structural coordinates that still retains the basic features of the three-dimensional structure should be considered part of the invention.

### Computers, Computer Software, Computer Modeling

Once the atomic coordinates are known, a computer may be used for producing a three-dimensional representation of a MEK1 peptide, a MEK2 peptide, or a structurally related peptide. Likewise, the atomic coordinates, or related set of structural coordinates, may be used to generate a three dimensional represenation of a MEK1 peptide binding pocket, MEK-2 peptide binding pocket or MEK-like peptide binding pocket. Thus, another aspect of the invention involves using the structural coordinates generated from the MEK1 and MEK2 complexes as set forth in Table 1 or Table 2, or a related set of structural coordinates, to generate three-dimensional representations of MEK1 peptide, MEK2 peptide, or a structurally related peptide, or a MEK1, MEK2, or MEK-like peptide binding pocket. This is achieved through the use of commercially available software that is capable of generating three-dimensional graphical representations of molecules or portions thereof from a set of atomic coordinates.

Suitable computers are known in the art and typically include a central processing unit (CPU), and a working memory, which can be random-access memory, core memory, mass-storage memory, or a combination thereof. The CPU may encode one or more programs. Computers also typically include display, input and output devices, such as one or more cathode-ray tube display terminals, keyboards, modems, input lines and output lines. Further, computers may be networked to computer servers (the machine on which large calculations can be run in batch) and file servers (the main machine for all the centralized databases).

Machine-readable media containing data, such as the atomic coordinates set forth in Table 1 and Table 2, or a related set of atomic coordinates, may be inputted using various hardware, including modems, CD-ROM drives, disk drives, or keyboards.

Machine-readable data medium can be, for example, a floppy diskette, hard disk, or an optically-readable data storage medium, which can be either read only memory, or rewritable, such as a magneto-optical disk.

Output hardware, such as a CRT display terminal, may be used for displaying a graphical representation of the three-dimensional structural coordinates of the MEK1 or MEK2 peptides as set forth in Tablel or Table 2 respectively, of a structurally related peptide, or of a MEK1, MEK2, or MEK-like binding pocket, as described herein. Output hardware may also include a printer and disk drives.

The CPU coordinates the use of the various input and output devices, coordinates data access from storage and access to and from working memory, and determines the sequence of data processing steps. A number of programs may be used to process the machine-readable data. Such programs are discussed herein in reference to the computational methods of drug discovery.

In a preferred embodiment of the invention, atomic coordinates capable of being processed into a three-dimensional representation of a molecule or molecular complex that comprises a MEK1, MEK2, or MEK-like peptide binding pocket are stored in a machine-readable storage medium. As described below, the three-dimensional structure of a molecule or molecular complex comprising a MEK1, MEK2, or MEK-like peptide binding pocket is useful for a variety of purposes, such as in drug discovery and drug design. For example, the three-dimensional structure derived from the atomic coordinate data may be computationally evaluated for its ability to associate with chemical entities.

### MEK1 or MEK2 Activity Inhibitors and/or Enhancers

The association of natural ligands with their corresponding binding pockets on receptors or enzymes is the basis of many biological mechanisms of action. Similarly, many drugs exert their biological effects *via* an interaction with the binding pockets of a receptor or enzyme. An understanding of such associations can lead to the design of drugs having more favorable and specific interactions with their target receptors or enzymes, and thus, improved biological effects. Therefore, information related to ligand association with the MEK1, MEK2, or MEK-like peptide binding sites is valuable in designing and/or identifying potential inhibitors or enhancers of MEK1, MEK2, or peptides structurally related thereto. Further, the more specific the design of a potential drug, the more likely that the drug will not interact with similar proteins, thus, minimizing potential side effects due to unwanted cross interactions.

Computer programs can be employed to estimate the attraction, repulsion, and steric hindrance of a ligand to a MEK1, MEK2, or MEK-like binding pocket. For example, one can screen computationally small molecule databases for chemical entities or compounds that can bind in whole, or in part, to a MEK1, MEK2, or MEK-like binding pocket. In this screening, the quality of fit of such entities or compounds to the binding site may be judged either by shape complementarity or by estimated interaction energy (Meng, et al., *J. Comp. Chem., 13*:505-524 (1992)). Generally, the tighter the fit, e.g., the lower the steric hindrance and/or the greater the attractive force, the more potent the drug is projected to be since these properties are consistent with a tighter-binding constant.

The present invention provides methods for screening candidate compounds as potential therapeutic agents for the treatment of various diseases associated with MEK1 and MEK2, including, but not limited to, disease states that involve diverse cellular processes including, but not limited to, apoptosis, differentiation, angiogenisis and inflammation. Such disease states include, for example, cancer, psoriasis, arthritis, septic shock, viral infections and cardiovascular disease.

More specifically, the present invention provides methods of using the three-dimensional representations of the MEK1, MEK2, and structurally related peptides, or binding pockets thereof, generated from the X-ray crystallographic data, or a related set of structural coordinates, to model the binding of candidate compounds. The methods include methods for screening and identifying potential inhibitors or enhancers of MEK1, MEK2, or a structurally peptide; and for the design of or modification of chemical entities having the potential to associate with MEK1, MEK2, a structurally related peptide, or binding pocket thereof.

The compound design or modification process begins after the structure of the target, e.g., a MEK1 or MEK2 peptide, is resolved to of greater than 5.0 Å, preferably greater than 3.5 Å. As described above, the data generated from the resolved crystal structure is applied to a computer algorithm to generate a three-dimensional representation and, ultimately, model, of the MEK1, MEK2 or structurally related peptide and MEK1, MEK2, MEK-like peptide binding pockets. Resolving the MEK1 and MEK2 three-dimensional structures using the X-ray crystallographic coordinates, as described above, enables one to determine whether a compound could occupy the ligand or cofactor binding site, as demonstrated in Figures 4 and 5, or a MEK-like binding pocket.

After a three-dimensional representation of the MEK1 or MEK2 petpide molecule, a structurally related peptide molecule, or a MEK1, MEK2 or MEK-like binding pocket is generated, a ligand having the potential to associate with the peptide or binding pocket is generated by, for example, (i) assembling molecular fragments into the chemical entity; (ii) de novo design of the chemical entity; (iii) selecting a chemical entity from a small molecule database; or (iv) modifying a known inhibitor, or portion thereof, of MEK1 or MEK2 activity.

If a chemical entity is designed, the following factors may be considered. First, the entity must be capable of physically and structurally associating with some or the entire MEK1, MEK2, or MEK-like binding pocket. Second, the entity must be able to assume a conformation that allows it to associate with a MEK1, MEK2, or MEK-like binding pocket directly. Although certain portions of the entity will not directly participate in these associations, those portions of the entity may still influence the overall conformation of the molecule. This, in turn, may have a significant impact on potency. Such conformational requirements include the overall three-dimensional structure and orientation of the chemical entity in relation to all or a portion of the binding pocket, and the spacing between functional groups of an entity comprising several chemical entities that directly interact with the MEK1, MEK2, or MEK-like binding pocket.

The design of new compounds or the modification of known compounds may involve synthesizing or modifying compounds, or fragments thereof, *via* computer programs which build and link fragments or atoms into a target binding site(s) based upon steric and electrostatic complementarity, without reference to substrate analog structures. The computer program analyzes molecular structure and interactions. The computer analysis can be performed, for example, with one or more of the following computer programs: QUANTA®, CHARMM®, INSIGHT®, SYBYL®, MACROMODEL® or ICM [Dunbrack *et al.*, 1997, supra]. Selected compounds, or fragments thereof, may be positioned in a variety of orientations, or docked, within the MEK1, MEK2, or MEK-like binding pocket(s) as defined by the atomic coordinates. If compounds have been selected, then they may be assembled into a single complex. If fragments have been selected, then they may be assembled into a single compound. Assembly may be preceded by visual inspection of the relationship of the compounds or fragments to each other on the three-dimensional MEK1, MEK2, or MEK-like peptide binding pocket representation displayed on a computer screen in relation to the atomic coordinates. This visual image step may be followed by manual model building using appropriate software programs. Alternatively, compounds may be designed as a whole using either empty binding pocket(s) or binding pocket(s) containing the natural ligand(s).

Computer programs that may be used in the design or modification of the potential ligand include, but are not limited to, alone or in combination, QUANTA (Accelrys Inc.) and/or SYBYL® (Tripos, Inc.) and/or a docking computer program such as GOLD (commercially available *via* Cambridge Crystallographic Data Centre, Cambridge, UK; Jones, G., *J. Mol. Biol. 245:* 43-53 (1995)), FlexX (Tripos, Inc.), GRAMM (Ilya A. Vakser, Rockefeller Univ.), Flexidock (Tripos, Inc.), Dock (Ewing, T.J.A. *et al., J. Comput.-Aided Mol. Des. 15:* 411-428 (2001)), or AutoDock (Molecular Graphics Laboratory (Scripps Research Inst.); Goodsell, D.S., *J. Mol. Recognit. 9:* 1-5 (1996)). In addition, other related computer programs may be used.

The potential inhibitory or binding effect of the chemical entity on a MEK1, MEK2, or MEK-like peptide binding pocket may be analyzed prior to its actual synthesis and testing through the use of computer modeling techniques. The "modeling" includes applying an iterative or rational process to individual or multiple potential ligands, or fragments thereof, to evaluate their association with the MEK1, MEK2, or MEK-like binding pocket and to evaluate their inhibition and/or enhancement of MEK1 or MEK2 activity. This procedure may include, for example, computer fitting a potential ligand into a MEK1, MEK2, or MEK-like peptide binding site(s) to ascertain how well the shape and chemical structure of the potential ligand complements or interferes with the peptide. Computer programs, such as, for example, the program GOLD®, may also be used to estimate the attraction, repulsion and steric hindrance of the ligand to the MEK1, MEK2, or MEK-like binding sites. Generally, the tighter the fit, e.g., the lower the steric hindrance and/or the greater the attractive force, the more antagonistic or agonistic the potential ligand will be since these properties are consistent with a tighter-binding constant. If the theoretical structure, i.e., computational structure, indicates insufficient interaction and association, further testing may not be necessary. However, if computer modeling indicates a strong interaction, then the ligand may be synthesized and tested for its ability to bind to a MEK1, MEK2, or MEK-like binding site(s). Thus, a potential inhibitor or enhancer may be identified and selected, based on its computational ability to positively associate with the amino acid residues found within any one or all of the binding sites.

Suitable computer programs to be used for computer modeling include, but are not limited to, QUANTA®, CharmM®, INSIGHT®, SYBYL®, MacroModel® and ICM (Dunbrack *et al*., 1997, supra). SYBYL® may be preferably used. The computer program may be used alone or combined with a docking computer program such as GRAM®, FlexX®, Flexidock®, GOLD® or AUTO DOCK [Dunbrack *et al*., 1997, supra]. For this purpose, GOLD® may be preferably used.

The screening method and subsequent identification of potential ligands, may be accomplished *in vivo, in vitro* or *ex vivo.* Initial ligand computation analysis is optional. Instead, or additionally, high-throughput screening may be employed which may be capable of full automation at robotic workstations such that large collections of compound libraries may be screened.

In one embodiment of the screening and identification method, the initial computer modeling is performed with one or more of the following docking computer modeling programs: Dock (Ewing, T.J.A. *et al*., *J. Comput.-Aided Mol. Des. 15:* 411-428 (2001)), AutoDock (Molecular Graphics Laboratory; Goodsell, D.S., *J. Mol. Recognit. 9:* 1-5 (1996)), GOLD (commercially available *via* Cambridge Crystallographic Data Centre, Cambridge, UK; Jones, G., *J. Mol. Biol. 245:* 43-53 (1995)) or FlexX (Tripos, Inc.). Potential ligands initially identified by the docking program(s) are elaborated using standard modeling methods as found in, for example, SYBYL® (Tripos, Inc.), QUANTA (Accelrys Inc.), INSIGHT®-II (Accelrys Inc.), GRIN/GRID (Molecular Discovery Ltd.), UNITY® (Tripos, Inc.), LigBuilder (Want, R., *J. Mol. Model 6:* 498-516 (2000)), or SPROUT (developed and distributed by ICAMS (Institute for Computer Applications in Molecular Sciences) at the University of Leeds, United Kingdom (Gillet, V. *et al., J. Comput. Aided Mol. Design 7:* 127-153 (1993))).

After a potential activity inhibitor and/or enhancer is identified, it can either be selected from commercial libraries of compounds or alternatively the potential inhibitor and/or enhancer may be synthesized and assayed to determine its effect(s) on the activity of MEK1, MEK2, or a structurally related peptide. Optionally, the assay may be radioactive. However, in a preferred embodiment, the assay is a nonradioactive ELISA.

In one embodiment of screening and identifying potential ligands *via* computer modeling, the method comprises: (a) generating a three-dimensional representation of MEK1 peptide, MEK2 peptide, a structurally related peptide or a MEK-1, MEK-2 or MEK-like peptide binding pocket; (b) designing and/or building (e.g. computationally) *de novo* potential ligands; and (b) identifying the ligands that associate with the MEK1, MEK2, or MEK-like binding site(s). Such ligands may be identified by, for example, contacting the ligand with a cell that expresses MEK1 or MEK2. A MEK1 or MEK2 inhibitor may be identified, for example, as a compound that inhibits the MEK1 or MEK2 catalyzed phosphorylation of ERK1 in the cell. The cell may be a eukaryotic cell, including, but not limited to, a yeast cell or vertebrate. Preferably, the cell is a mammalian cell. More preferably, the cell is a human cell. The protein assay can be an *in vitro, in situ* or *in vivo,* but is preferably an *in vitro* assay. In one such embodiment, the MEK1 or MEK2 catalyzed phosphorylation of ERK1 may be determined by Western blot analysis of ERK di-phosphorylation with ERK phospho-specific antibodies as a direct read-out of MEK activity in the cell. The inhibitory activity of MEK1 or MEK2 ligands may also be screened by *in vitro, ex vivo* or *in vivo* assays. In another embodiment, the assay is performed using a glutathione-S-transferase fusion protein of kinase-inactive ERK1 (GSTERK1K71R) as substrate.

In an alternative embodiment of screening and identifying potential ligands *via* computer modeling, the method comprises: (a) generating a three-dimensional representation of MEK1, MEK2, a structurally related peptide or a MEK-1, MEK-2 or MEK-like peptide binding pocket; (b) building (e.g. computationally) and, optionally, modifying, known potential ligands; and (c) identifying the ligands that associate with the MEK-1, MEK-2 or MEK-like peptide binding pocket binding site(s).

In an alternative embodiment, the compound screening and identification method comprises evaluating the ability of *de novo* compounds to function as MEK1 or MEK2 activity inhibitors and/or enhancers by, for example: (a) generating a MEK1, MEK2, or MEK-like virtual binding cavity, the binding cavity defined by the binding sites; (b) designing (e.g. computationally) a compound structure that spatially conforms to the binding cavity; (c) synthesizing the compound and, optionally, analogs thereof, and (d) testing to determine whether the compound binds to at least one of the binding sites.

In an alternative embodiment, the compound screening and identification method comprises evaluating the ability of known compounds to function as MEK1 or MEK2 activity inhibitors and/or enhancers by, for example: (a) generating a MEK1, MEK2, or MEK-like virtual binding cavity defined by the binding sites; (b) generating (e.g. computationally) and, optionally, modifying, a known compound structure; (c) determining whether that compound spatially conforms to the binding cavity; (d) synthesizing the compound and, optionally, analogs thereof; and (e) testing to determine whether the compound binds to at least one of the binding sites by.

In another embodiment, wherein a potential ligand has been selected, the identification method comprises: (a) generating a three-dimensional representation of MEK1, MEK2, or a structurally related peptide with the potential ligand bound thereto; (b) modifying the potential ligand based on the three-dimensional representation; and (c) generating a second three-dimensional representation with the modified potential ligand bound thereto. Then, one can test the potential ligand in a biochemical assay known in the art, if desired.

In addition, when a potential ligand is identified, a supplemental crystal may be grown comprising the ligand in complex with MEK1, MEK2, or a structurally related peptide, and optionally a cofactor. Molecular replacement analysis, for example, may be used to determine the three-dimensional structure of the supplemental crystal. Molecular replacement analysis may also be used in the initial crystal structure determination.

It should be understood that in all of the structure-based drug design strategies provided herein, a number of iterative cycles of any or all of the steps may be performed to optimize the selection.

Thus, according to another embodiment, the invention provides compounds that associate with a MEK1, MEK2 and MEK-like peptide binding pocket(s) produced or identified by any one or a combination of the methods set forth above.

### MEK1 and MEK2 Variants

As mentioned above, the present invention also provides and enables obvious variants of the amino acid sequence of the proteins of the present invention, such as naturally occurring mature forms of the proteins, allelic/sequence variants of the proteins, non-naturally occurring recombinantly derived variants of the proteins, and orthologs and paralogs of the proteins. Such variants can be generated using techniques that are known by those skilled in the fields of recombinant nucleic acid technology and protein biochemistry. It is understood, however, that variants exclude any proteins or peptides disclosed prior to the invention.

Such variants can readily be identified/made using molecular techniques and the sequence information disclosed herein. Further, such variants can readily be distinguished from other proteins based on sequence and/or structural homology to the proteins of the present invention. The degree of homology/identity present will be based primarily on whether the peptide is a functional variant or non-functional variant, the amount of divergence present in the paralog family, and the evolutionary distance between the orthologs. An alternative method to using the primary sequence for describing the structural relationship between two proteins or peptides is to use the three-dimensional structures of the two related proteins. In this method, the two structures are solved by X-ray crystallography or by NMR, and then the similarity is determined by comparing the root mean square (RMS) deviation of the backbone C-alpha trace of the two species.

To determine the percent identity of two amino acid sequences or two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In one aspect of the invention, the length of a reference sequence aligned for comparison purposes is at least 30%, preferably 40%, more preferably 50%, even more preferably 60%. In one preferred embodiment, it is preferably at least 70%, more preferably 80%, or most preferably 90% or more of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid 'identity' is equivalent to amino acid or nucleic acid 'homology'). The percent identity between the two sequences is a function of the number of identical positions shared by the sequences, taking into account the number of gaps, and the length of each gap, which need to be introduced for optimal alignment of the two sequences.

The comparison of sequences and determination of percent identity and similarity between two sequences can be accomplished using a mathematical algorithm. (*Computational Molecular Biology,* Lesk, A.M., ed., Oxford University Press, New York, 1988; *Biocomputing: Informatics and Genome Projects,* Smith, D.W., ed., Academic Press, New York, 1993; *Computer Analysis of Sequence Data, Part 1,* Griffin, A.M., and Griffin, H.G., eds., Humana Press, New Jersey, 1994; *Sequence Analysis in Molecular Biology,* von Heinje, G., Academic Press, 1987; and *Sequence Analysis Primer,* Gribskov, M. and Devereux, J., eds., M Stockton Press, New York, 1991). In a preferred embodiment, the percent identity between two amino acid sequences is determined using the Needleman and Wunsch (*J. Mol. Biol.* (48): 444-453 (1970)) algorithm which has been incorporated into commercially available computer programs, such as GAP in the GCG software package, using either a Blossom 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences can be determined using the commercially available computer programs including the GAP program in the GCG software package (Devereux, J., *et al., Nucleic Acids Res. 12(1)*: 387 (1984)), the NWS gap DNA CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Meyers and W. Miller (CABIOS, 4: 11-17 (1989)) which has been incorporated into commercially available computer programs, such as ALIGN (version 2.0), using a PAM120 weight residue table, a gap length penalty of 12 and a gap penalty of 4.

The nucleic acid and protein sequences of the present invention can further be used as a "query sequence" to perform a search against sequence databases to, for example, identify other family members or related sequences. Such searches can be performed using commercially available search engines, such as the BLASTN and BLASTX programs (version 2.0) of Altschul, *et al*. (*J. Mol. Biol*. 215: 403-10 (1990)). BLAST nucleotide searches can be performed with the BLASTN program, score = 100, wordlength = 12 to obtain nucleotide sequences homologous to the nucleic acid molecules of the invention. BLAST protein searches can be performed with the BLASTX program, score *=* 50, wordlength = 3 to obtain amino acid sequences homologous to the proteins of the invention. To obtain gapped alignments for comparison purposes, Gapped BLAST can be utilized as described in Altschul *et al.* (*Nucleic Acids Res.* 25(17): 3389-3402 (1997)). When utilizing BLAST programs, the default parameters of the respective programs (e.g., BLASTX and BLASTN) can be used.

Full-length clones comprising one of the proteins of the present invention can readily be identified as having complete sequence identity to one of the kinases of the present invention as well as being encoded by the same genetic locus as the MEK1 or MEK2 peptide provided herein.

Allelic variants of a peptide can readily be identified as having a high degree (significant) of sequence homology/identity to at least a portion of the protein as well as being encoded by the same genetic locus as the MEK1 or MEK2 peptide provided herein. As used herein, two proteins (or a region of the proteins) have significant homology when the amino acid sequences are typically at least 70%, preferably 75%, more preferably 80%, or even more preferably 85% or more homologous. In one preferred embodiment, it is at least 90%, or preferably 95% or more homologous. A significantly homologous amino acid sequence, according to the present invention, will be encoded by a nucleic acid sequence that will hybridize to a protein encoding nucleic acid molecule under stringent conditions as more fully described below.

Paralogs of a protein can readily be identified as having some degree of significant sequence homology/identity to at least a portion of the MEK1 or MEK2 peptide, as being encoded by a gene from same species, and as having similar activity or function. Two proteins will typically be considered paralogs when the amino acid sequences are typically at least 70%, preferably 75%, more preferably 80%, even more preferably 85% or more homologous through a given region or domain. In one preferred embodiment, two proteins will typically be considered paralogs when the amino acid sequences are typically 90% or more, preferably 95% or more homologous through a given region or domain. Such paralogs will be encoded by a nucleic acid sequence that will hybridize to a kinase peptide encoding nucleic acid molecule under stringent conditions as more fully described below. An example of a paralog is the relationship between MEK2 and MEK1. These proteins are 80% homologous overall, 85% homologous in the kinase domain and 100% homologous in the ATP- and ligand-binding domain. In addition, as MEK2 is inhibited by the MEK1 inhibitors, and as the active sites have been found to be identical, the three-dimensional structure or X-ray crystallographic coordinates of the MEK1 peptide may be used to identify MEK2 inhibitors. Conversely, the three-dimensional structure or X-ray crystallographic coordinates of the MEK2 peptide may be used to identify MEK1 inhibitors.

Orthologs of a protein can readily be identified as having some degree of significant sequence homology/identity to at least a portion of the protein as well as being encoded by a gene from another organism. Preferred orthologs will be isolated from mammals, preferably human, for the development of human therapeutic targets and agents, or other invertebrates, particularly insects of economical/agriculture importance, e.g. members of the Lepidopteran and Coleopteran orders, for the development of insecticides and insecticidal targets. Such orthologs will be encoded by a nucleic acid sequence that will hybridize to a MEK1 or MEK2 encoding nucleic acid molecule under moderate to stringent conditions, as more described below, depending on the degree of relatedness of the two organisms yielding the proteins.

Non-naturally occurring variants of the MEK1 or MEK2 peptide of the present invention can readily be generated using recombinant techniques. Such variants include, but are not limited to deletions, additions and substitutions in the amino acid sequence of the protein. For example, one class of substitutions is conserved amino acid substitution. Such substitutions are those that substitute a given amino acid in a protein by another amino acid of like characteristics. Typically seen as conservative substitutions are the replacements, one for another, among the aliphatic amino acids Ala, Val, Leu, and Ile; interchange of the hydroxyl residues Ser and Thr; exchange of the acidic residues Asp and Glu; substitution between the amide residues Asn and Gln; exchange of the basic residues Lys and Arg; and replacements among the aromatic residues Phe, Tyr. Guidance concerning which amino acid changes are likely to be phenotypically silent are found in Bowie *et al., Science 247*:1306-1310 (1990).

Variants can be fully functional or can lack function in one or more activities. Fully functional variants typically contain only conservative variation or variation in non-critical residues or in non-critical regions. Functional variants can also contain substitution of similar amino acids, which result in no change or an insignificant change in function. Alternatively, such substitutions may positively or negatively affect function to some degree.

Amino acids that are essential for function can be identified by methods known in the art, such as site-directed mutagenesis or alanine-scanning mutagenesis (Cunningham *et al., Science 244*:1081-1085 (1989)). The latter procedure introduces single alanine mutations at every residue in the molecule. The resulting mutant molecules are then tested for biological activity such as receptor binding or *in vitro* proliferative activity. Sites that are critical for binding can also be determined by structural analysis such as X-ray crystallography, nuclear magnetic resonance or photoaffinity labeling (Smith *et al., J. Mol. Biol. 224*:899-904 (1992); de Vos *et al. Science 255*:306-312 (1992)).

The following examples illustrate preferred embodiments and aspects of the invention, and are intended to be non-limiting.

### EXAMPLES

### Example 1: Cloning of modified MEK1s

Human MEK1 cDNA (GenBank Accession Number L11284; SEQ ID NO: 1) was modified via PCR and subcloned into pET24b (Novagen) at 5'*Nde1*/3'*HindIII* sites. Wild type MEK1 was modified via PCR for cloning into pET21b and pET24b expression vectors by the addition of 5'Ndel and 3'HindIII restriction sites with the following oligonucleotides:

5'CATATGCCGAAAAAGAAGCCGACCCCGATCCAG3' (SEQ ID NO: 5)

5'AAGCTTGACGCCAGCAGCATGGGTT3' (SEQ ID NO: 6)

Conditions for PCR were as follows: 20ng MEK DNA, 20pmoles of both 5' and 3' primers, 200µM dNTP mix, 1X PCR 10X Vent buffer, 1 unit Vent DNA polymerase in a reaction volume of 100µl. Reactions were run at 95°C for 5 min (1 cycle), followed by 25 cycles of 95°C 1 min, 56°C 1 min, 72°C for 1 min 30 sec. Wild-type MEK1 ("MEK1(wt)") sequence was altered on the region spanning from bp 73 to bp 99 for tRNA bias in *E. coli,* with the following base changes, highlighted in bold and underlined: ^{5'}ATGCC**G**AA**A** AAGAAGCCGAC**C**CC**G**ATC^{3'} (amino acid residues 73-99 of SEQ ID NO: 2). A NH₂-terminally truncation of MEK1 was also generated by PCR, designated C1 ("MEK1-C1"), encoding amino acids 51-393 of SEQ ID NO: 2. The MEK1-C1 clone was subcloned into pET21b (Novagen, Madison, WI) at the 5'*Nde1*/3'*Xho1* sites. Additionally, both MEK1 (wt) and MEK1-C 1 clones were modified by deletion of a proline-rich insertion loop, comprised of residues 280-323 to give MEK1 (wt)(d280-323) and MEK1-C1 (d280-323), respectively. This deletion was created by introducing an internal *HindIII* site which encoded residues 324 and 325 with the following sequence: AAG(K280)CTT(L323). Fragment A, containing the coding sequence NH₂-terminally to the deletion (amino acids 1-279 for MEK1 (wt), and residues 51-279 for the MEK1-C1), was modified by PCR to contain a *HindIII* site on the 3' end downstream of amino acid 279 (V). This fragment was then ligated to fragment B, a 0.2kb fragment containing sequence for residues 324-393, including the codon changes for amino acids 324 and 325 to become a *HindIII* site. Both MEK1 (wt)(d280-323) and MEK1-C1 (d280-323) clones were subcloned into pET24b (Novagen, Madison, WI) at 5'*Nde1*/3'*Xho1* sites. All modified MEK cDNA sequences were verified using MEK1-specific sequencing primers in an automated sequencing apparatus (ABI technologies). Once a nucleotide sequence was obtained, it was compared to the deposited sequence for MEK1 to confirm sequence integrity.

By following a similar procedure, the following NH₂-terminally truncated MEK1 peptides were prepared:
MEK1-C2: NH₂-terminally truncated MEK1 having peptide of amino acid residues 62 to 393 of the sequence of SEQ ID NO: 2.
MEK1-C2 (d280-323): MEK1-C2 having a deletion from amino acid residues from 280 to 323 of the sequence of SEQ ID NO: 2.
MEK1-C3: NH₂-terminally truncated MEK1 having peptide of amino acid residues 42 to 393 of the sequence of SEQ ID NO: 2.
MEK1-C3 (d280-323): MEK1-C3 having a deletion from amino acid residues from 280 to 323 of the sequence of SEQ ID NO: 2.
MEK1 (d280-323): MEK1 having a deletion from amino acid residues from 280 to 323 of the SEQ ID NO: 2.
MEK1 (d264-310): MEK1 having a deletion from amino acid residues from 264 to 310 of the SEQ ID NO: 2.
MEK1 (d270-310): MEK1 having a deletion from amino acid residues from 270 to 310 of the SEQ ID NO: 2.
MEK1 (d264-305): MEK1 having a deletion from amino acid residues from 264 to 305 of the SEQ ID NO: 2.
MEK1 (d267-307): MEK1 having a deletion from amino acid residues from 267 to 307 of the SEQ ID NO: 2.
MEK1 (d265-304): ME The software may be used to add hydrogens to the PDB molecular structure file in standardized geometry with optimization of orientations of OH, SH, NH₃⁺, Met methyls, Asn and Gln sidechain amides, and His rings.K1 having a deletion from amino acid residues from 265 to 304 of the SEQ ID NO: 2.

The following primers were used for PCR of the modified MEK1s.
MEK1-C1 and MEK1-C1 (d280-323): 
For fragment A: 5' CTTGCATATG GAGGCCTTTC TTACCCAGA 3' (SEQ
ID NO: 7)
 
For fragment B: 5' CTTGAAGCTT CACCTGGCAC CCAAACATC 3' (SEQ
ID NO: 8)
MEK1-C2 and MEK1-C2 (d280-323): 
For fragment A: 5' CTTGCATATG GAACTGAAGG ATGACGACTT 3'
(SEQ ID NO: 9)
 
For fragment B: 5' CTTGAAGCTT CACCTGGCAC CCAAACATC 3'
(SEQ ID NO: 8)
MEK1-C3 and MEK1-C3 (d280-323):

For fragment A: 5' CTTGCATATG CTTGATGAGC AGCAGCGAA 3'
(SEQ ID NO: 10)

For fragment B: 5' CTTGAAGCTT CACCTGGCAC CCAAACATC 3'
(SEQ ID NO: 8)

### Example 2: Cloning of modified MEK2s

Human MEK2 cDNA (GenBank Accession Number NM_030662; SEQ ID NO: 3) was modified via PCR and subcloned into pET24b (Novagen) at 5'Nde1/3'HindIII sites. Wild type MEK2 was modified via PCR for cloning into pET24b expression vectors by the addition of 5'Nde1 and 3'HindIII restriction sites with the following oligonucleotides:

5’CATATG AAGCCGGTGCTGCCGGCGCTCACCATC3’ (SEQ ID NO:
11) 5’AAGCTTGGCCACTGTCACACGGCGGTG3’ (SEQ ID NO: 12)

Conditions for PCR were as follows: 20ng MEK DNA, 20pmoles of both 5' and 3' primers, 200µM dNTP mix, 1X PCR 10X Vent buffer, 1 unit Vent DNA polymerase in a reaction volume of 100µl. Reactions were run at 95°C for 5 min (1 cycle), followed by 25 cycles of 95°C 1 min, 56°C 1 min, 72°C for 1 min 30 sec.

Six N-termina truncations of wild type human MEK2 cDNA were generated via PCR amplification and cloned into pET201 TA-TOPO vector (Invitrogen). Each is fused with a C-terminal His6 tag.

Sequencing of the template DNA pET24b MEK2 wild type revealed a silent mutation on amino acid residual V64: GTC to GTT.

Conditions for PCR were as follows: pET24b MEK2 wild type DNA 1ng/ul, 5' and 3' primers 1 µM, 1x Pfx Amplification Buffer, 0.3 mM dNTP mixture, 5 unit Platinum Pfx DNA polymerase in a reaction volume of 100µl. Reactions were run at 94°C for 3 min (1 cycle), followed by 35 cycles of 94°C 1 min, 55°C 30 seconds, 68°C 1 min 30 sec, followed by 1 cycle of 68°C 10 min. The 3' A overhangs of the PCR products were added by 1 cycle of 68°C 10 min in the above PCR mixture plus 0.1 mM dATP and 2.5 unit of Taq DNA Polymerase.

Six varied NH₂-terminal truncations were used in the creation of the modified MEK2 peptides. Each of the six constructs possessed a Hexahistidine tag fused to the COOH-terminal:
MEK2-C1: amino acid residues 46 to 400 of the sequence of SEQ ID NO:3;
MEK2-C2: amino acid residues 55 to 400 of the sequence of SEQ ID NO:3;
MEK2-C3: amino acid residues 66 to 400 of the sequence of SEQ ID NO:3;
MEK2-C4: amino acid residues 59 to 400 of the sequence of SEQ ID NO:3;
MEK2-C5: amino acid residues 62 to 400 of the sequence of SEQ ID NO:3;
MEK2-C6: amino acid residues 64 to 400 of the sequence of SEQ ID NO:3;

The following primers were used for PCR generation of the MEK2 N-terminal truncations:

MEK2-C1: forward primer: 5' ATGCTTGACGAGCAGCAGAAGAAG 3'
(SEQ ID NO: 13)

MEK2-C2: forward primer: 5' ATGGAAGCCTTTCTCACCCAGAAAGCC
3' (SEQ ID NO: 14)

MEK2-C3: forward primer: 5' ATGGCCTTTCTCACCCAGAAAGCC 3'
(SEQ ID NO: 15)

MEK2-C4: forward primer: 5' ATGACCCAGAAAGCCAAGGTTGG 3'
(SEQ ID NO: 16)

MEK2-C5: forward primer: 5'
ATGGCCAAGGTCGGCGAACTCAAAGAC 3' (SEQ ID NO: 17)

MEK2-C6: forward primer: 5'
ATGGTCGGCGAACTCAAAGACGATGAC 3' (SEQ ID NO: 18)

The common reverse primer for all the above six MEK2 trunctions:

5'
TCAATGATGATGATGATGATGTTCAAGCACAGCGGTGCGCGTGGGTG 3'
(SEQ ID NO: 19)

Sequences for the above six MEK2 truncations were verified using pET201 vector sequencing primers (T7 promoter and T7 Terminator primers) in an automated sequencing apparatus (ABI technologies). Once a nucleotide sequence was obtained, it was compared to the deposited sequence for MEK2 to confirm sequence integrity.

### Example 3: Large scale expression of the modified MEK1s in E. coli

The recombinant MEK1 constructs obtained from Example 1 were grown at 37°C in *E. coli* BL21 (DE3) in a 10-liter fermentor containing rich media. A starter culture of Kanamycin resistant MEK1 was grown by inoculating a 1 L shake flask of LB media with 100 µL of glycerol stock. The culture was grown overnight at 30°C in a shaking incubator set to 250 rpm. The next morning the entire liter of culture was added to a 10-liter fermentor containing rich media prepared using the recipe (260.0 g yeast extract (Difco 0127), 260.0 g BBL Acidicase peptone, 260.0 g casitone (Difco 0259) dissolved in distilled water to 4.0 L. Add 260.0 g dry gelatone (Difco 0657) then 26.0 g KH₂PO₄ anhydrous, 26.0 g K₂HPO₄ anhydrous, 26.0 g Na₂HPO₄.7H₂O, dissolved with distilled water to 2.0 L and added 0.5% (v/v) glycerol) or Super Broth (KD Medical, Columbia, MD). At an optical density at 600nm of 10, the temperature was decreased to 22-25°C for one hour and recombinant protein expression was induced by addition of IPTG (isopropylthio-beta-D-galactoside) to a concentration of 1.0 mM. For the Rich media preparation without glycerol supplementation the fermentor was harvested eighteen hours after induction. For the Super Broth preparation or the Rich media preparation with glycerol supplementation the fermentor was harvested thirty-six hours after induction. A typical yield was about 700g whole cells / 10 L fermentation.

The cell pellet was resuspended 1:5 (w/v) in 50mM K₂HPO₄, 2mM TCEP (Tri(2-carboxyethyl)phosphine), 300mM NaCl, 5 mM MgCl₂, 10mM CHAPS (3-([3-cholamidopropyl]dimethylammonio)-1-propanesulfonate), at pH 8.0. Cell disruption was carried out using a Dyno-Mill KDL. Fifty µL of Benzonase (EM Industries, Hawthorne, NY) was included during lysis. The lysis was clarified by centrifugation for 45 minutes at 13,600 times g at 4°C.

### Example 4: Large scale expression of the modified MEK2s in E. coli

The recombinant MEK2 constructs obtained from Example 2 were grown at 37°C in *E*. *coli* BL21 (DE3) in a 10-liter fermentor containing rich media. A starter culture of Kanamycin resistant MEK1 was grown by inoculating a 1 L shake flask of LB media with 100 µL of glycerol stock. The culture was grown overnight at 30°C in a shaking incubator set to 250 rpm. The next morning the entire liter of culture was added to a 10-liter fermentor containing rich media prepared using the recipe (260.0 g yeast extract (Difco 0127), 260.0 g BBL Acidicase peptone, 260.0 g casitone (Difco 0259) dissolved in distilled water to 4.0 L. Add 260.0 g dry gelatone (Difco 0657) then 26.0 g KH₂PO₄ anhydrous, 26.0 g K₂HPO₄ anhydrous, 26.0 g Na₂HPO₄.7H₂O, dissolved with distilled water to 2.0 L and added 0.5% (v/v) glycerol) or Super Broth (KD Medical, Columbia, MD). At an optical density at 600nm of 10, the temperature was decreased to 22-25°C for one hour and recombinant protein expression was induced by addition of IPTG (isopropylthio-beta-D-galactoside) to a concentration of 1.0 mM. For the Rich media preparation without glycerol supplementation the fermentor was harvested eighteen hours after induction. For the Super Broth preparation or the Rich media preparation with glycerol supplementation the fermentor was harvested thirty-six hours after induction. A typical yield was about 700g whole cells / 10 L fermentation.

The cell pellet was resuspended 1:3 (w/v) in 50mM K₂HPO₄, 2mM TCEP (Tri(2-carboxyethyl)phosphine), 5 mM Imidazole, 300mM NaCl, 5 mM MgCl₂, 2mM pyrrole-2-carboxylate and 100µM ZnCl₂, 10mM CHAPS (3-([3-cholamidopropyl]dimethylammonio)-1-propanesulfonate), at pH 8.0. Cell disruption was carried out using a Dyno-Mill KDL. Fifty µL of Benzonase (EM Industries, Hawthorne, NY) was included during lysis. The lysis was clarified by centrifugation for 45 minutes at 13,600 times g at 4°C.

### Example 5: Purification of modified MEK1s

The cell lysate was mixed 1:1 with 2X binding buffer (50mM K₂HPO₄, 10mM imidazole, 4mM TCEP, 300mM NaCl, 10mM CHAPS, 2mM pyrrole-2-carboxylate and 100µM ZnCl₂, pH=8.0) and combined with washed TALON™ metal affinity resin (ClonTech, Cat # 8908-2) at a ratio of 2mL of resin per 1g of cells. The mixture was stirred at 4°C for 1 hour, then batch-loaded into a 5-L Amicon (Vantage-S) column. Any additional unbound proteins/impurities were eluted with the washing buffer (50mM K₂HPO₄, 5mM imidazole, 2mM TCEP, pH 8.0 and 300mM NaCl, 1mM pyrrole-2-carboxylate, 50µM ZnCl₂) until a baseline reading was reached. The protein was eluted with 4-5 column volumes of elution buffer (20mM HEPES, 100mM EDTA disodium salt, 2mM TCEP, pH 8.0 and 10%v/v glycerol) and concentrated 10-fold using a Millipore (S1Y10) spiral cartridge. The protein concentrate was then diluted 10-fold in HS buffer A (20mM MES (2-(N-morpholino)-ethanesulfonic acid), 2mM TCEP, pH 6.4 and 20% ethylene glycol) and loaded onto a pre-equilibrated HS column (POROS HS/20 25X100, Applied Biosystems). The protein was eluted with a linear gradient from 0-100% HS Buffer B (Buffer A and 1M ammonium acetate). The protein containing fractions were pooled together and concentrated using a Millipore ultrafiltration stir-cell (YM10 membrane). The protein concentrate was then diluted 50X in HQ Buffer A (20mM TRIS Tris(hydroxymethyl) aminomethane), 2mM TCEP, pH 8.0 and 10mM ammonium acetate) and loaded on to a pre-equilibrated HQ column (POROS HQ/20 16X100, Applied Biosystems). The protein was eluted in the unbound fraction and was immediately concentrated using a Millipore ultrafiltration stir-cell (YM10 membrane). The protein:inhibitor complexes were purified by adding a 10-fold molar excess of the inhibitor was and stirring overnight at 4°C. The sample was then filtered using a 0.22µm polyethersulfone filter, further concentrated using a YM10 membrane fitted stir-cell, centrifuged at 8000 rpm for 30 minutes at 4°C, and loaded onto a pre-equilibrated size exclusion column (HiLoad 26/60 Superdex 200 prep grade, Pharmacia, # 17-1071-01). The protein was eluted in approximately 5 hours using the size exclusion chromatography buffer (20mM HEPES, 0.5mM EDTA disodium salt, 2mM TCEP, pH 7.5 and 150 ammonium acetate and 50nM inhibitor). The inhibitor used to form the ternary complex was 5-bromo-N-(2,3-dihydroxy-propoxy)-3,4-difluoro-2-(2-fluoro-4-iodo-phenylamino)-benzamide. The protein-containing fractions were pooled together, concentrated using a Millipore ultrafiltration stir-cell (YM10 membrane), centrifuged at 14,000 rpm for 10 minutes at 4°C, aliquoted into microcentrifuge tubes, and snap-frozen in liquid nitrogen. The protein aliquots were stored at -80°C until needed for crystallization. The same procedure was followed for the purification of the apo protein except that the concentrated HQ flow-through was loaded directly onto the pre-equilibrated size exclusion column.

### Example 6: Purification of modified MEK2s

The cell lysate was combined with washed TALON™ metal affinity resin (ClonTech, Cat # 8908-2) at a ratio of 1mL of resin per 5g of cells. The mixture was stirred at 4°C for 1 hour, then batch-loaded into a Biorad Econo column. Any additional unbound proteins/impurities were eluted with the washing buffer (50mM K₂HPO₄, 5mM imidazole, 2mM TCEP, pH 8.0 and 300mM NaCl, 1mM pyrrole-2-carboxylate, 50µM ZnCl₂) until a baseline reading was reached. The protein was eluted with 4-5 column volumes of elution buffer (20mM HEPES, 100mM EDTA disodium salt, 2mM TCEP, pH 7.5 and 10%v/v glycerol). The eluted protein was then diluted 10-fold in HS buffer A (20mM MES (2-(N-morpholino)-ethanesulfonic acid), 2mM TCEP, pH 6.4 and 20% ethylene glycol) and loaded onto a pre-equilibrated HS column (POROS HS/20 25X100, Applied Biosystems). The protein was eluted with a linear gradient from 0-100% HS Buffer B (Buffer A and 1M ammonium acetate. The protein containing fractions were pooled together and concentrated using a Millipore ultrafiltration stir-cell (YM10 membrane). The protein concentrate was then loaded onto a pre-equilibrated size exclusion column (HiLoad 26/60 Superdex 200 prep grade, Pharmacia, # 17-1071-01). The protein was eluted in approximately 5 hours using SEC buffer (20mM HEPES, 0.1mM EDTA disodium salt, 2mM TCEP, pH 7.5 and 150 ammonium acetate). The protein containing fractions were pooled and protein:ligand complex was formed by adding 10-fold molar excess of the ligand, {5-[3,4-Difluoro-2-(2-fluoro-4-iodo-phenylamino)-phenyl]-1,3,4-oxadiazol-2-yl}-(2-morpholin-4-yl-ethyl)-amine (dissolved in DMSO), dropwise into the protein solution under constant stirring. After stirring overnight at 4°C, the complex was concentrated using a YM10 membrane fitted stir-cell, centrifuged at 8000 rpm for 30 minutes at 4°C, aliquoted into microcentrifuge tubes, and snap-frozen in liquid nitrogen. The protein aliquots were stored at -80°C until needed for crystallization.

### Example 7: In vivo and in vitro MEK1 and MEK2 activity assays

The kinase activity of the modified MEK1 peptides was assayed using a glutathione-S-transferase fusion protein of kinase-inactive ERK1 (GSTERK1K71R) as substrate. The modified MEK peptides (50ng) were assayed in 20mM HEPES (N-2-hydroxyethyl-piperazine-N'-2-ethanesulfonic acid) pH 7.5, 10mM MgCl₂, 1mM EGTA (ethylene glycol bis(2-aminoethyl ether)-N,N,N'N'-tetraacetic acid), 10µM ATP, with 5µCi [γ-³²P] ATP. Appropriate samples had 0.5 units of Raf-1 (UBI) added, and the reaction started with the addition of 1 µg GSTERK1 (K71R). The reactions were incubated at room temperature for 20 minutes, then quenched with 5µl 6x Laemmli sample buffer. The samples were then resolved by SDS-PAGE, and the phosphoproteins visualized by autoradiography. Radiolabel incorporated into ERK was quantitated by excision of the protein band from the gel and counting in a standard scintillation counter. Initial experiments with the modified MEK1s generated the following specific activities in units of pmol PO4 min-1 mg-1 after Raf activation: WT: 8380.6, W 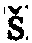 TPR: 40.1, C1FL: 773.3, C1 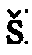 PR: 35.9, C2FL: 537.5, C2 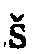 PR: 24.1, C3FL: 441.7 and C3 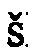 PR: 26.9.

In the evaluation of ligand that has been designed using the three-dimensional structural information of the MEK1: ligand: cofactor complexes, 5-bromo-N-(2,3-dihydroxy-proxy)-3,4-difluoro-2-(2-fluoro-4-iodo-phenylamino)-benzamide was added to 96 well format plates with filter bottomed wells. Kinase-inactive ERK1 (K71R mutant) in HEPES buffer is then added to each well. After subsequent addition of MEK1 (2D mutant) diluted in a Tris buffer before being added to the plate, and the reaction is initiated by the addition of radioactive ATP, diluted in 0.05% Tween 20. After 1 hour incubation at room temperature, ice-cold 20% TCA is added to each well to stop the reaction and to precipitate the protein in solution. Filtration is done the following day, followed by scintillation counting of the incorporated radioactivity using a Perkin Elmer Wallac microBeta 1450 counter. Inhibition is expressed as a percentage of the vehicle control.

### (1) Evaluation of ex vivo tissue samples for pMAPK levels

A lysis buffer of 50mM glycerol phosphate, 10mM HEPES, pH7.4, 1% Triton x-100, 70mM NaCl, 1mM Na₃VO₄, 100µM PMSF, 10µM leupeptin, and 10µM pepstatin. (Only add the inhibitors when ready to use) was prepared. Tumors are removed at -70° and placed onto dry ice immediately. 40-50 mg of each tumor is weighed and sliced using a scalpel. The mixture was homogenized with polytron and centrifuged at 2500 rpm at 4°□ for 15 minutes. A supernatant was transferred to a 15 mL Falcon tube. The protein assay was then run and protein was normalized to about 15µg. 5µL - 10µL of 6X laemmli was added to each tube depending on the amount of lysate. The mixture was boiled for 3 minutes, spin and run on 10% tris-gly gels at 140V (Bio-Rad). Transfer was accomplished at 125V for 1.5 - 2 hours. The membrane was rinsed in water, and was then placed into blocking solution of TBST (Tris-buffered saline-Tween 20), 1%BSA and 1% ovalbumin. The membrane was rinsed three times for 5 minutes in TBST and was then placed into the primary antibody (Promega) for 3-4 hours. The membrane was then washed for 5 minutes in TBST and was placed into the secondary antibody (Bio-Rad Goat anti-rabbit HRP) for 1.5 hours. The blots were visualized by ECL (Pierce). Protein bands, identified as phosphorylated MAPK were measured using densitometry using a Biorad Flur-S MultiImager Max.

### (2) In vivo Colon26 Cellular MAP Kinase Western Assay:

Day 1 Colon26 (murine) cells were seeded in twelve-well plates at roughly 2x10⁵ cells/well (1.5 ml/well). The cells were grown in media consisting of DMEM/F12 #11330-032 (Gibco), 10% FBS and 1% antibiotic/antimycotic and incubated at 37°C, 5% CO₂ overnight.
Day 2 Compounds were prepared in DMSO at concentrations of (1, 0.3, 0.1, 0.03, 0.01, 0.003, and 0.001 µM) in a 96-well plate. The DMSO concentration in the cell preparations should never be greater than 0.2 %; therefore, a total of 3 µl of each DMSO solution was added to a 1.5 ml well containing cells. The cells were then incubated at 37° in a 5% CO₂ incubator for one hour.

The plates and eppendorf tubes were kept on ice. The cells were washed in 1 ml PBS (containing 1mM Na₃VO₄) followed by removal of PBS. The state of threonine and tyrosine phosphorylation of cellular MAPK was determined after treatment by lysing cells in 100 uLµ of a solution containing 70 mM NaCl, 50 mM glycerol phosphate, 10 mM HEPES, pH 7.4, 1% Triton X-100, 1 mM Na₃VO₄, 100 µM PMSF, 10 µM leupeptin and 10 µM pepstatin. The cells were scraped from the wells and transferred to an eppendorf tube. The eppendorf tubes were spun at 13,000 rpm at 4 degrees for 5 min. The supernatant was then transferred to cold eppendorf tubes and the pellet was discarded. The Pierce BCA protein assay was then run and proteins were standardized to 20 µg. Either a 10% tris-glycine 10 well or 15 well gel was loaded and the gel was run at 130V for 1.5 hours. Transfer was accomplished at 125V for 2 hours or at 25V overnight. The membrane was rinsed in water, and was then placed into blocking solution for 1-18 hours. The membrane was rinsed three times for 5 minutes in TBST and was then placed into the primary antibody (pMAPK) for 3-18 hours. The membrane was then washed for 5 minutes in TBST and was placed into the secondary antibody (Bio-Rad Goat anti-rabbit HRP) for one hour. The membrane was finally washed three times for 15 minutes in TBST. The blots were visualized by ECL (Pierce). Protein bands, identified as phosphorylated MAPK were measured using densitometry using a Biorad Flur-S MultiImager Max. To evaluate total MAPK levels, blots were subsequently 'stripped' and re-probed with a 1:1 mixture of polyclonal antibodies recognizing unphosphorylated ERK1 and ERK2 (Santa Cruz Biotechnology, Santa Cruz, California).

### (3) Materials List:

TBST buffer 0.9% NaCl, 10mM Tris, 0.1% Tween-20, pH 7.5
pMAPK Promega pMAPK: (1:5,000 in 0.5% BSA) #V8031-Polyclonal
Secondary BioRad Goat-anti-Rabbit HRP (1:10,000 in 0.5% BSA)
Catalogue # 170-6515
Blocking Solution: 1% BSA, 1%Ovalbumin in TBST, filter sterilized with 0.01% Na
Azide

### Example 8: Crystallization of the MEK1: ligand: cofactor complexes

The purified protein: ligand complexes (MEK1-C2, MEK1-C1, MEK1-C1(d280-323)) described in Example 4 were quick-thawed and incubated overnight at 4°C with a cofactor solution consisting of 5 mM Mg-ATP dissolved in the size exclusion chromatography buffer as described above. After incubation, the protein: ligand: cofactor complexes were centrifuged for 30 minutes at 14,000 x g at 4°C. Crystals of the MEK1 C2 peptide: ligand: cofactor complexes were grown by hanging drop vapor diffusion at 13°C using 1 µL drops of the ternary complex, with a protein to precipitant ratio of 1:1 over a 1 mL well solution. The precipitant used was 6-16% (w/v) PEG 8K, 150-450 mM NH₄H₂PO₄, 20 mM DTT, and 100 mM imidazole/malate buffer, pH 7.0. The crystals grew to about 0.2 x 0.1 x 0.1 mm in about 8 days.

Alternatively, 60 µL of the purified apo protein solution described in Example 4 was combined with about 0.2 mg of an inhibitor for a period between 2 - 8 hours. The solution of the MEK1 peptide: ligand complex was then incubated overnight at 4°C with a cofactor solution consisting of 5 mM Mg-ATP dissolved in the size exclusion chromatography buffer and crystallized as described above.

The crystals were prepared for low temperature data collection by bathing the crystal in a cryoprotectant solution consisting of 10% ethylene glycol diluted with the well solution. The procedure comprises adding a 1 µL drop of the 100% ethylene glycol solution to the inverted coverslip containing the crystal. The cryo solution was then thoroughly mixed with 9µL of the well solution to create a 10% ethylene glycol cryo solution. The mixed cryo solution was then added in a ratio of 1:1 (v/v) to the drop containing the crystal and allowed to slowly mix by diffusion. The drop containing the crystal, called the mother liquor, is now composed of 5% ethylene glycol. After about 2 minutes, the crystal was removed from the cryo/ mother liquor solution and transferred into a mixture of 70% Paratone-N obtained from Hampton Research, Laguna Niguel, CA and 30% light, white mineral oil mixture using a mounted cryo loop, also obtained from Hampton Research, Laguna Niguel, CA. The crystal was then manipulated in the oil mixture to completely remove the aqueous solution and then quickly cooled by immersion directly into liquid nitrogen. Alternatively, the crystals may be dipped in a stream of cold nitrogen gas at about 100 K.

### Example 9: Crystallization of the MEK2: ligand: cofactor complexes

The purified protein: ligand complexes MEK2-C2, described in Example 6 were quick-thawed and incubated overnight at 4°C with a cofactor solution consisting of 5 mM Mg-ATP dissolved in the size exclusion chromatography buffer as described above. After incubation, the protein: ligand: cofactor complexes were centrifuged for 30 minutes at 14,000 x g at 4°C. Crystals of the MEK2 peptide: ligand: cofactor complexes were grown by hanging drop vapor diffusion at 13°C using 1 µL drops of the ternary complex, with a protein to precipitant ratio of 1:1 over a 1 mL well solution. The precipitant used was 1.4-1.85 M NaH₂PO4/ K₂HPO4, 0-20 mM DTT, pH 6.7-7.1. The crystals grew to about 0.2 x 0.1 x 0.1 mm in about 8 days.

The crystals were prepared for low temperature data collection by bathing the crystal in a cryoprotectant solution consisting of a mixture of 70% Paratone-N obtained from Hampton Research, Laguna Niguel, CA and 30% light, white mineral oil mixture using a mounted cryo loop, also obtained from Hampton Research, Laguna Niguel, CA. The crystal was then manipulated in the oil mixture to completely remove the aqueous solution and then quickly cooled by immersion directly into liquid nitrogen. Alternatively, the crystals may be dipped in a stream of cold nitrogen gas at about 100 K.

### Example 10: Crystallographic data collection of the MEK1 and MEK2: ligand: cofactor complex crystals

### (1) In-house analysis and collection of diffraction data:

The initial data set used for phase determination was collected at 100 Kelvin on an R-AXIS IV⁺⁺ mounted on a Rigaku RU-H3R rotating anode X-ray source operating at 50 kV and 100 mA equipped with an Osmic confocal mirror. The detector was positioned at a 2 theta value of 0 degrees with a crystal to detector distance of 225 mm. A rotation angle of 0.5 degree per frame was used with an exposure time of 10 minutes per frame. The inverse-beam method was used with two 80° sweeps collected 180° apart.

### (2) Synchrotron data collection:

Synchrotron data sets of MEK1 and MEK2 were collected 2.4 Å and 3.2 Å resolution respectively at 100 K on the 17-ID beamline (IMCA) at the Advanced Photon Source Argonne, IL. A wavelength of 1.0 Å was used with crystal to detector distance of 170 mm, with a rotation angle of 0.5 degrees per frame and an exposure time of 10 sec per frame using a MAR CCD for the MEK1 data collection. A wavelength of 1.0 Å was used with crystal to detector distance of 200 mm, with a rotation angle of 0.5 degrees per frame and an exposure time of 10 sec per frame using an ADSC QUANTUM 210 CCD detector was used for the MEK2 data collection.

### Example 11: Structural determination of the initial MEK1: ligand: cofactor complex

Typical crystals of the ternary complex belong to the hexagonal space group, P6₂ with unit cell dimensions of approximately a=b=81.4, c=129.2 Å with one molecule per asymmetric unit.

The initial crystal structure of MEK1 was solved using the SAD method using in-house data from the anomolous signal created by the Iodine atom in the ligand. The data collection statistics are a total of 6699 anomalous pairs out of 25336 measured reflections were found in the resolution range of 36 - 3.4 Å. About 83% of the reflections were measured with a redundancy of 4 and 11% of the reflections were measured with a redundancy of 3, with an overall R_{symm}=0.051.

All data were processed with HKL2000 and scaled with SCALEPACK (Otwinowski *et al*., "Processing of x-ray diffraction data collected in oscillation mode," *Methods Enzymol.* 276:307-326 (1997)). All subsequent calculations were done with the CNX package (Molecular Simulations Incorporated). A single position of an iodine atom was found from 3.5 Å anomalous difference Patterson map and later confirmed with the automated Patterson search program. Ten cycles of heavy atom parameters (positional as well as thermal) and phase calculations were performed. An overall figure-of-merit (FOM) was 0.258 for the reflections between 36 and 3.5 Å. Density modification was used to minimize any ambiguity in phase information. A solvent content of 64% was assumed according to a Matthews analyses (Matthews, B.W. *J. Mol. Biol.* 491-497 (1968)). After the density modification procedure, the new phases had FOM=0.858. In order to find a correct enantiomorph similar calculations were done in P6₅ space group with flipped positional coordinates of iodine atom. Analyses of the electron density calculated in both space groups confirmed P6₂ as a correct one. Phases were extended to 2.38 Å after higher resolution data became available. A 2.38 Å electron density map calculated with these new phases was used for protein model tracing and fitting of the inhibitor and ATP, with the QUNATA-2000 graphical application (Molecular Simulations Incorporated). Atomic models at different stages of completeness were refined with CNX refinement programs (minimize & individual). For the 2.38 Å resolution native data set collected at a wavelength of 1.0 Å at the 17ID line of the APS, a complete sweep of 720 degrees of data was collected. In order to minimize the radiation induced crystal decay only the first 240 out of 720 frames were processed with the HKL2000 and scaled with SCALEPACK. Unit cell dimensions for this crystal were estimated as a=b=81.6, c=129.2 Å. Data included 18590 unique reflections out of 35816 measured in the 36 to 2.38 Å resolution range (about 95 % coverage) with R_{symm=}0.044.

### Example 12: Refinement of the MEK1: ligand: cofactor structure complexes

Positional and simulated annealing refinements to the initial structural coordinates were carried out using the multi-stage maximum likelihood minimization procedure implemented in the program X-PLOR version 98.1 (Accelrys, San Diego, CA.) against data from 25-2.4Å. One round of simulated annealing using the slow-cool procedure (3000 K to 300 K in steps of 25 K) followed by several cycles of standard positional refinement reduced the R_{work} to 0.372 (R_{free} = 0.428). The (2Fo - Fc) and (Fo - Fc) different electron density maps clearly revealed most of the side chains of the model and the binding sites for inhibitor compound (5-bromo-N-(2,3-dihydroxy-propoxy)-3,4-difluoro-2-(2-fluoro-4-iodo-phenylamino)benzamide), ATP and Mg²⁺. The first round of model rebuilding and positional refinement, followed by individually restrained B-factor refinement reduced the R_{work} to 0.302 (R_{free} = 0.347). Application of bulk solvent correction further reduced the R_{work} to 0.277 (R_{free} = 0.319). The successive rounds of model rebuilding, adding solvent molecules and modeling in the inhibitor and MgATP, followed by positional and restrained individual B refinements produced the current model with R_{work} of 0.223 (R_{free} = 0.276). The current model contains 2159 non-hydrogen protein atoms corresponding to 276 residues of the NH₂-terminally truncated MEK1 in a ternary complex with one ATP molecule, one Mg²⁺ ion, one inhibitor molecule and 80 water molecules.

The crystal coordinates of the MEK1 peptide (MEK1-C1): Mg-ATP: 5-bromo-N-(2,3-dihydroxy-propoxy)-3,4-difluoro-2-(2-fluoro-4-iodo-phenylamino)benzamide are shown in Table 1.

### Example 13: Structural Determination and Refinement of MEK2: ligand: cofactor structure complexes

Typical crystals of the ternary MEK2 complex belong to the hexagonal space group, P6₁22 with unit cell dimensions of approximately a=b=161.89, c=122.99 Å with two molecules per asymmetric unit. All data were processed with HKL2000 and scaled with SCALEPACK (Otwinowski *et al*., "Processing of x-ray diffraction data collected in oscillation mode," *Methods Enzymol.* 276:307-326 (1997)). For the ternary co-complex of MEK2-C2, MgATP and {5-[3,4-Difluoro-2-(2-fluoro-4-iodo-phenylamino)-phenyl]-1,3,4-oxadiazol-2-yl}-(2-morpholin-4-yl-ethyl)-amine, a total of 152,386 measured reflections were identified, with 16,131 unique reflections in the resolution range of 36 - 3.2 Å, with an overall R_{symm}=0.088.

The structure was solved by molecular replacement using the MEK1: MgATP: inhibitor complex coordinates from Table 1 as a search model using the program Molrep in CCP4 (Collaborative Computational Project, Number 4. 1994. "The CCP4 Suite: Programs for Protein Crystallography". Acta Cryst. D50, 760-763.) A rigid body refinement was performed, followed by one cycle of positional refinement using Refinac in CCP4. A 3.2 Å electron density map was calculated and used for manual fitting of the protein using the program QUNATA-2000 graphical application (Accelrys, San Diego, CA.). Atomic models at different stages of completeness were refined using the CNX refinement programs (minimize & individual).

Positional and simulated annealing refinements to the initial structural coordinates were carried out using the multi-stage maximum likelihood minimization procedure implemented in the program CNX version 2002 (Accelrys, San Diego, CA.) against data from 25-3.2Å. One round of simulated annealing using the slow-cool procedure (2500 K to 300 K in steps of 25 K) followed by several cycles of standard positional refinement reduced the R_{work} to 0.338 (R_{free} = 0.439). The (2Fo - Fc) and (Fo - Fc) different electron density maps clearly revealed most of the side chains of the model and the binding sites for inhibitor compound {5-[3,4-Difluoro-2-(2-fluoro-4-iodo-phenylamino)-phenyl]-1,3,4-oxadiazol-2-yl}-(2-morpholin-4-yl-ethyl)-amine, ATP and Mg²⁺. The first round of model rebuilding and positional refinement was followed by modeling in the inhibitor and MgATP. Then positional and group restrained B-factor refinement reduced the R_{work} to 0.29 (R_{free} = 0.37). The crystal coordinates of the MEK2 peptide (MEK2-C2): Mg-ATP: {5-[3,4-Difluoro-2-(2-fluoro-4-iodo-phenylamino)-phenyl]-1,3,4-oxadiazol-2-yl}-(2-morpholin-4-yl-ethyl)-amine are provided in Table 2.

### Example 14: Analysis of the crystal structures of the MEK1: ligand: cofactor complexes

The crystal structure of the ternary complex was obtained in the PDB file format and analyzed using software that performed the functions performed by SYBYL® and REDUCE® software. Initially, the structural data were processed by modifying the atom types of the ligand and cofactor and any waters that were present. The software was then used to titrate the protein by adding all of the hydrogens in the most favorable protonation state, as well as by rotating all the water molecules into orientations that gave the optimal interactions with the protein. After this modification, the protein complexes were ready for binding site characterization and docking simulation. The ligand and cofactor binding sites were characterized using GRIN/GRID® and MOLCAD® contouring. In this procedure, a 4 and 5 Å radius was traced around each atom of the ligand and the cofactor and all the amino acid residues that fell within that radius were identified.

After analysis of the binding site characterization and the docking studies using the X-ray crystallographic structural data, it was discovered that the MEK1 peptide comprises a ligand-binding pocket that is defined by the structural coordinates of the following amino acid residues within 4 Å of the MEK1 inhibitors located in the ligand-binding site: G77, N78, G79, G80, K97, I99, L115, L118, V127, F129, I141, M143, C207, D208, F209, G210, V211, S212, L215, I216, M219 of SEQ ID NO: 2. Further, it was discovered that the MEK1 peptide has a ligand-binding pocket that is defined by structural coordinates of the following amino acid residues within 5 Å of the MEK1 inhibitors located in the ligand-binding site: G77, N78, G79, G80, K97, 199, L115, L118, I126, V127, G128, F129, I141, M143, D190, N195, L206, C207, D208, F209, G210, V211, S212, L215, I216, M219, F223 of SEQ ID NO: 2.

It was also discovered that the MEK1 peptide comprises a cofactor-binding pocket that is defined by the structural coordinates of the following residues within 4 Å of the ATP molecule in the cofactor-binding site: L74, G75, A76, G77, N78, G80, V81, V82, A95, K97, V127, M143, E144, H145, M146, G149, S150, D152, Q153, K192, S194, N195, L197, D208, V224 of SEQ ID NO: 2. Further, it was discovered that the MEK1 peptide comprises a cofactor-binding pocket that is defined by structural coordinates of the following residues within 5 Å of the ATP molecule in the cofactor-binding site: L74, G75, A76, G77, N78, G79, 80, V81, V82, A95, K97, V127, M143, E144, H145, M146, D147, G149, S150, D152, Q153, D190, K192, S194, N195, L197, C207, D208, V224, G225 of SEQ ID NO: 2.

### Example 15: Analysis of the crystal structures of the MEK2: ligand: cofactor complexes

The crystal structure of the ternary complex was obtained in the PDB file format and analyzed using software that performed the functions performed by SYBYL® and REDUCE® software. Initially, the structural data were processed by modifying the atom types of the ligand and cofactor and any waters that were present. The software was then used to titrate the protein by adding all of the hydrogens in the most favorable protonation state, as well as by rotating all the water molecules into orientations that gave the optimal interactions with the protein. After this modification, the protein complexes were ready for binding site characterization and docking simulation. The ligand and cofactor binding sites were characterized using GRIN/GRID® and MOLCAD® contouring. In this procedure, a 4 Å and 5 Å radius was traced around each atom of the ligand and the cofactor and all the amino acid residues that fell within that radius were identified.

After analysis of the binding site characterization and the docking studies using the X-ray crystallographic structural data, it was discovered that the MEK2 peptide comprises a ligand-binding pocket that is defined by the structural coordinates of the following amino acid residues within 4 Å of the MEK2 inhibitors located in the ligand-binding site: G81, N82, G83, G84, K101, I103, L119, L122, V131, F133, I145, M147, C211, D212, F213, G214, V215, S216, L219, I220, M223 of SEQ ID NO: 4. Further, it was discovered that the MEK2 peptide has a ligand-binding pocket that is defined by structural coordinates of the following amino acid residues within 5 Å of the MEK2 inhibitors located in the ligand-binding site: G81, N82, G83, G84, K101, I103, L119, L122, I130, V131, G132, F133, I145, M147, D194, N199, L210, C211, D212, F213, G214, V215, S216, L219, I220, M223, F227 of SEQ ID NO: 4.

It was also discovered that the MEK2 peptide comprises a cofactor-binding pocket that is defined by the structural coordinates of the following residues within 4 Å of the ATP molecule in the cofactor-binding site: L78, G79, A80, G81, N82, G84, V85, V86, A99, K101, V131, M147, E148, H149, M150, G153, S154, D156, Q157, K196, S198, N199, L201, D212, V228 of SEQ ID NO: 4. Further, it was discovered that the MEK2 peptide comprises a cofactor-binding pocket that is defined by structural coordinates of the following residues within 5 Å of the ATP molecule in the cofactor-binding site: L78, G79, A80, G81, N82, G83, G84, V85, V86, A99, K101, V131, M147, E148, H149, M150, D151, G153, S154, D156, Q157, D194, K196, S198, N199, L201, C211, D212, V228, G229 of SEQ ID NO: 4.

After analysis of the binding site characterization and the docking studies using the X-ray crystallographic structural data, it was discovered that certain features of MEK1 and MEK2 are responsible for the high affinity binding of the ligand to the MEK1 or MEK2 ligand binding site. With the inhibitors described above, a binding mode was observed that involves the orientation of the ligand into the hydrophobic prime pocket with a hydrogen bond donor and acceptor interaction with several residues of the activation loop. However, it is expected that continued crystallization of other classes of compounds may reveal novel binding modes that are not currently seen. For example, modeling and docking simulations have revealed alternative binding modes for other more novel classes of compounds. This information can be used, for example, in the evaluation, screening and/or design and modification of a chemical entity that may associate with MEK1 or MEK2 and thus may inhibit MEK1 or MEK2 activity.

The binding sites according to the invention can serve as a basis for screening a virtual library using various screening techniques known in the art, including, for example, UNITY® (Tripos, Inc.) and any other 3D database screening software. For example, the X-ray crystallographic data found in Table 1 has allowed for the detailed identification of MEK1's three-dimensional structure for the first time. Likewise, the X-ray crystallographic data found in Table 2 has allowed for the detailed identification of MEK2's three-dimensional structure for the first time.

### Example 16: Calculation of RMSD's between the MEK1 and MEK2 structures

The RMS deviations (RMSD's) between the C alpha and/or backbone (N, C, O, and C alpha) atoms of MEK1 and MEK2 structures were calculated herein using the Superimpose command in InsightII (Accelrys). The RMSD's can also be calculated using other modeling programs or scripts. What the Superimpose command does is to perform a minimum RMS alignment of two molecules on selected sets of atoms from each molecule and output the RMSD value between the selected atoms of the superimposed molecules.

The RMSD values between several sets of selected C alpha and/or backbone atoms of MEK1 and MEK2 structures have been calculated and listed in Table 8. The RMSD for the C alpha and/or backbone atoms of the overall MEK1 and MEK2 structures was calculated using the following residues: E62-N221, V224-P266, and P307-L381 of MEK1; and E66-N225, V228-P270, and P315-L389 of MEK2. The RMSD for the C alpha and/or backbone atoms of the kinase domain of MEK1 and MEK2 was calculated using the following residues: F68-N221, V224-P266, and P307-I361 of MEK1; and F72-N225, V228-P270, and P315-I369 of MEK2.

The RMSD for the C alpha and/or backbone atoms of the 4 Å inhibitor binding site residues of MEK1 and MEK2 was calculated using G77, N78, G79, G80, K97, I99, L115, L118, V127, F129, I141, M143, C207, D208, F209, G210, V211, S212, L215, I216 and M219 of SEQ ID NO: 2 (MEK1) and their corresponding residues in MEK2 (Table 2). The RMSD for the C alpha and/or backbone atoms of the 5 Å inhibitor binding site residues of MEK1 and MEK2 was calculated using G77, N78, G79, G80, K97, I99, L115, L118, I126, V127, G128, F129, I141, M143, D190, N195, L206, C207, D208, F209, G210, V211, S212, L215, I216, M219 of SEQ ID NO: 2 (MEK1) and their corresponding residues in MEK2 (Table 2).

All references cited herein are incorporated by reference in their entirety.

While the invention has been described in conjunction with examples thereof, it is to be understood that the foregoing description is exemplary and explanatory in nature, and is intended to illustrate the invention and its preferred embodiments. Through routine experimentation, the artisan will recognize apparent modifications and variations that may be made without departing from the spirit of the invention. Thus, the invention is intended to be defined not by the above description, but by the following claims and their equivalents.

The following Tables are included in the application.

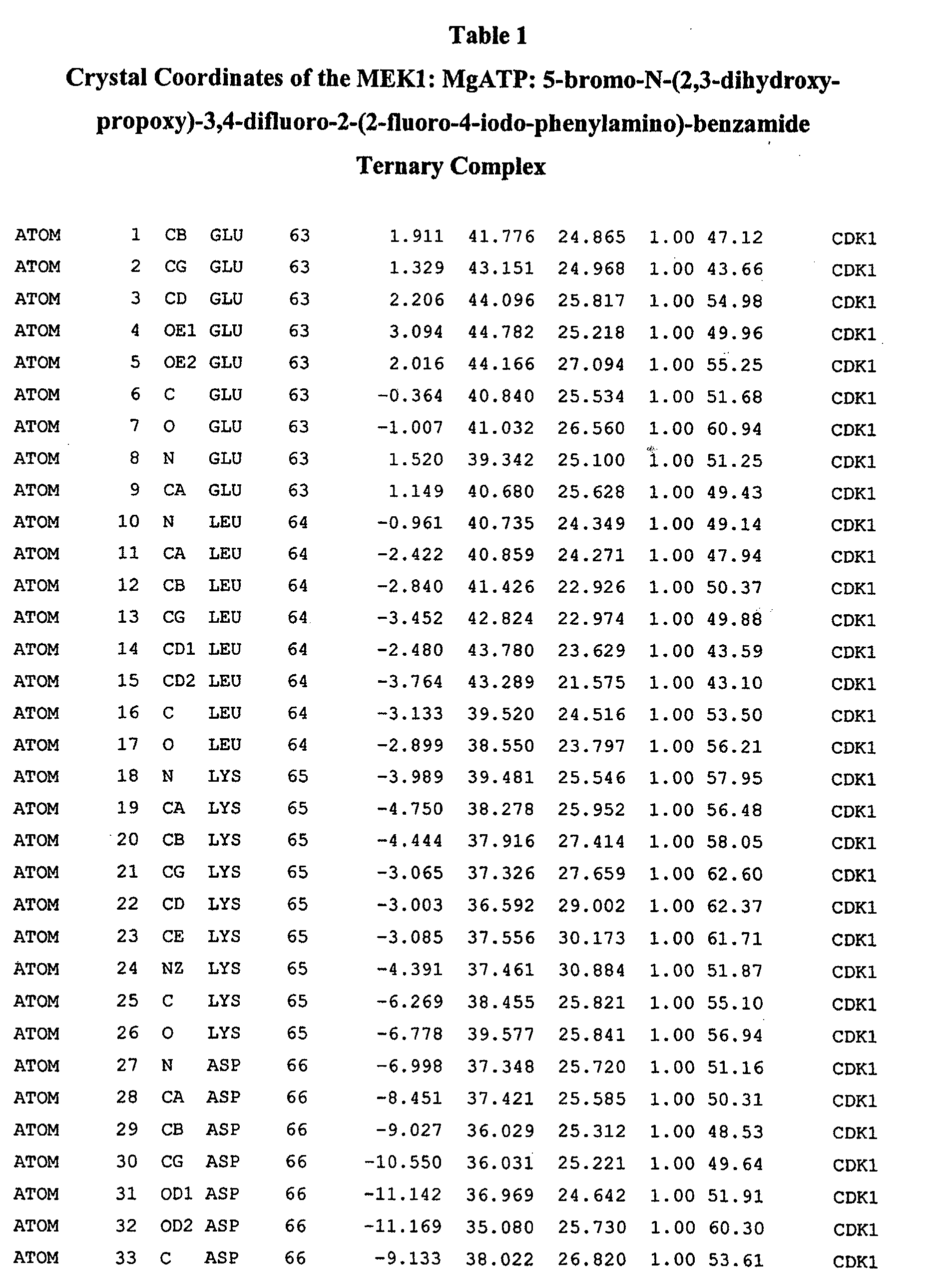

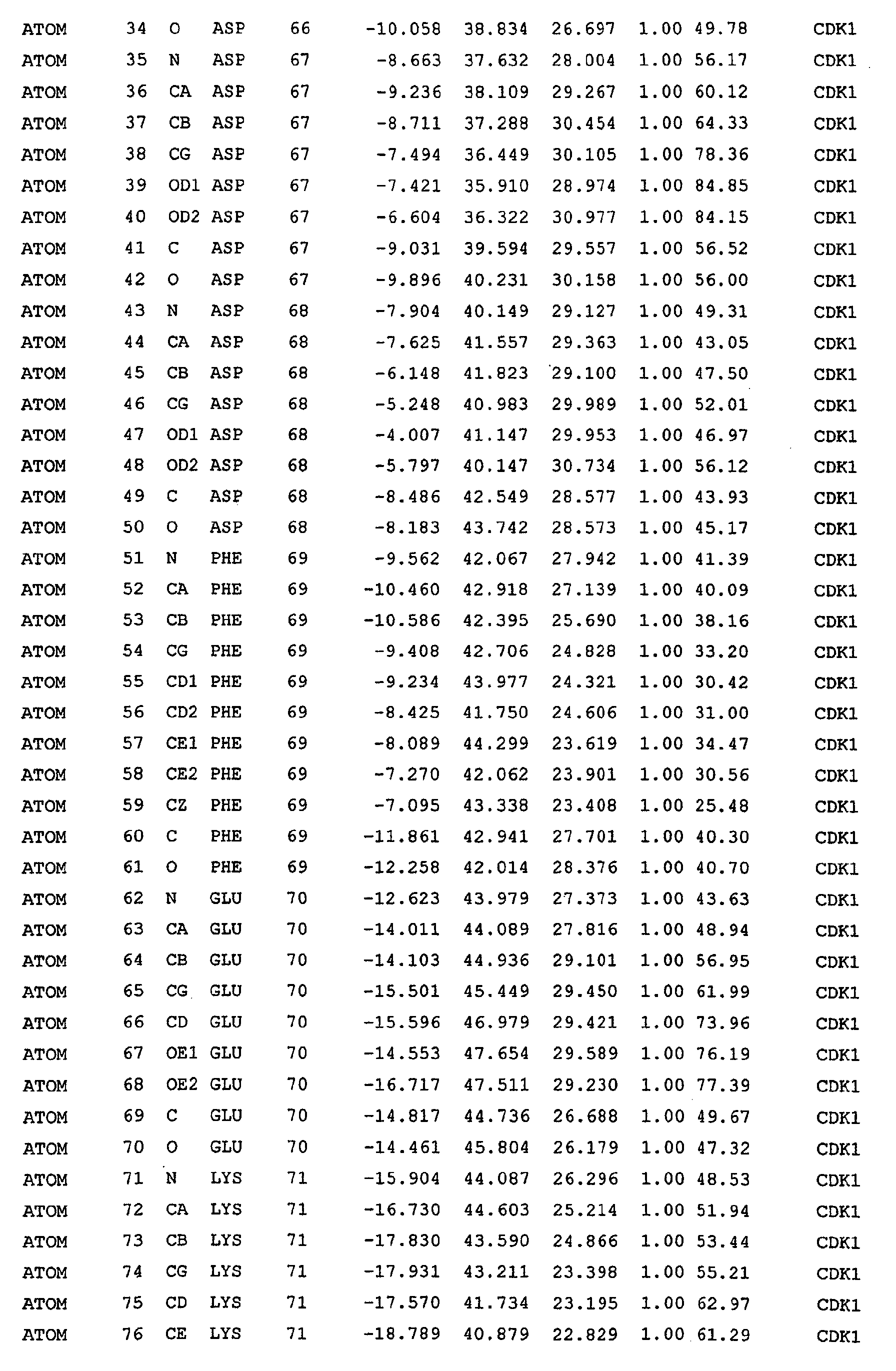

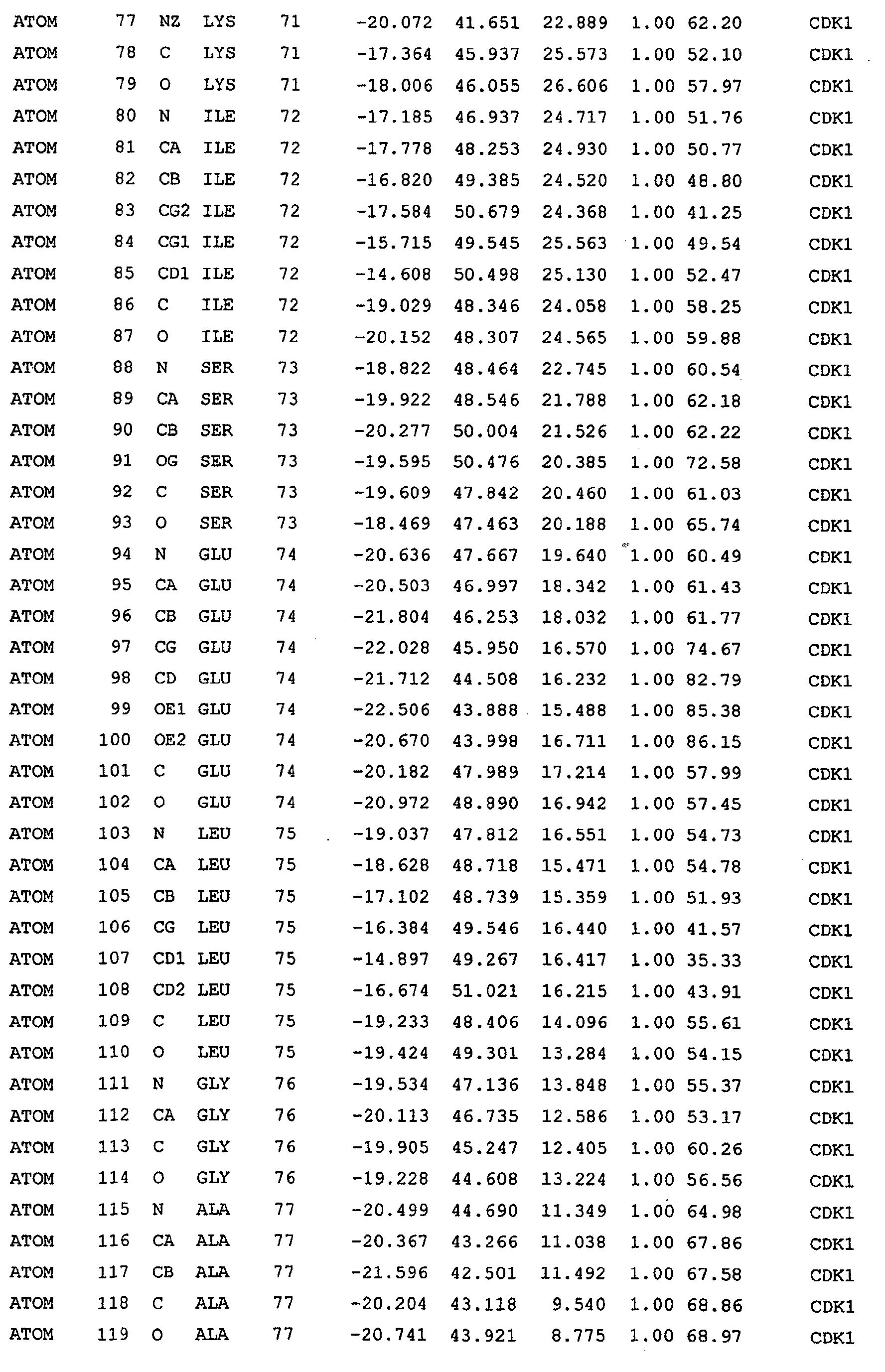

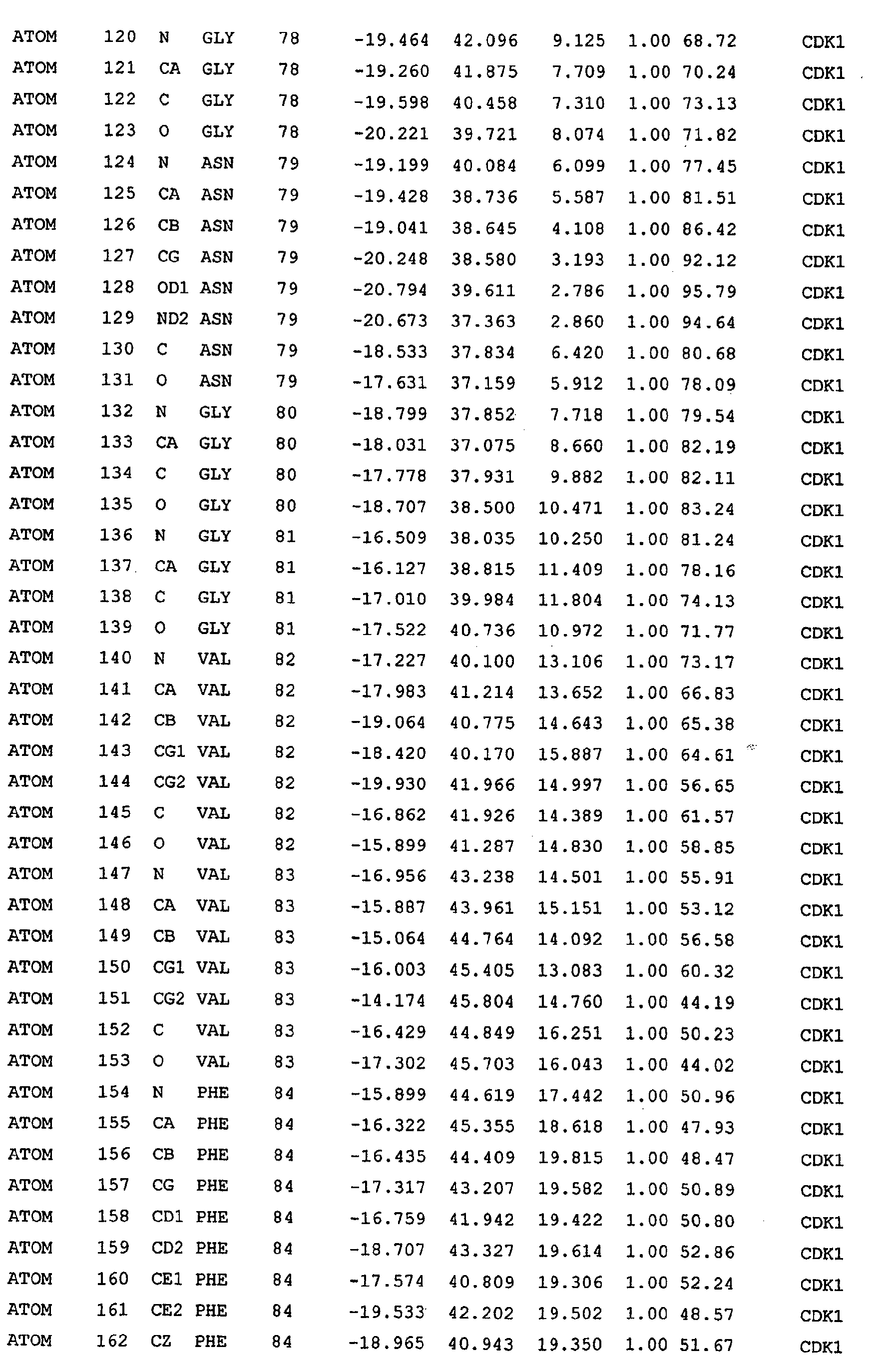

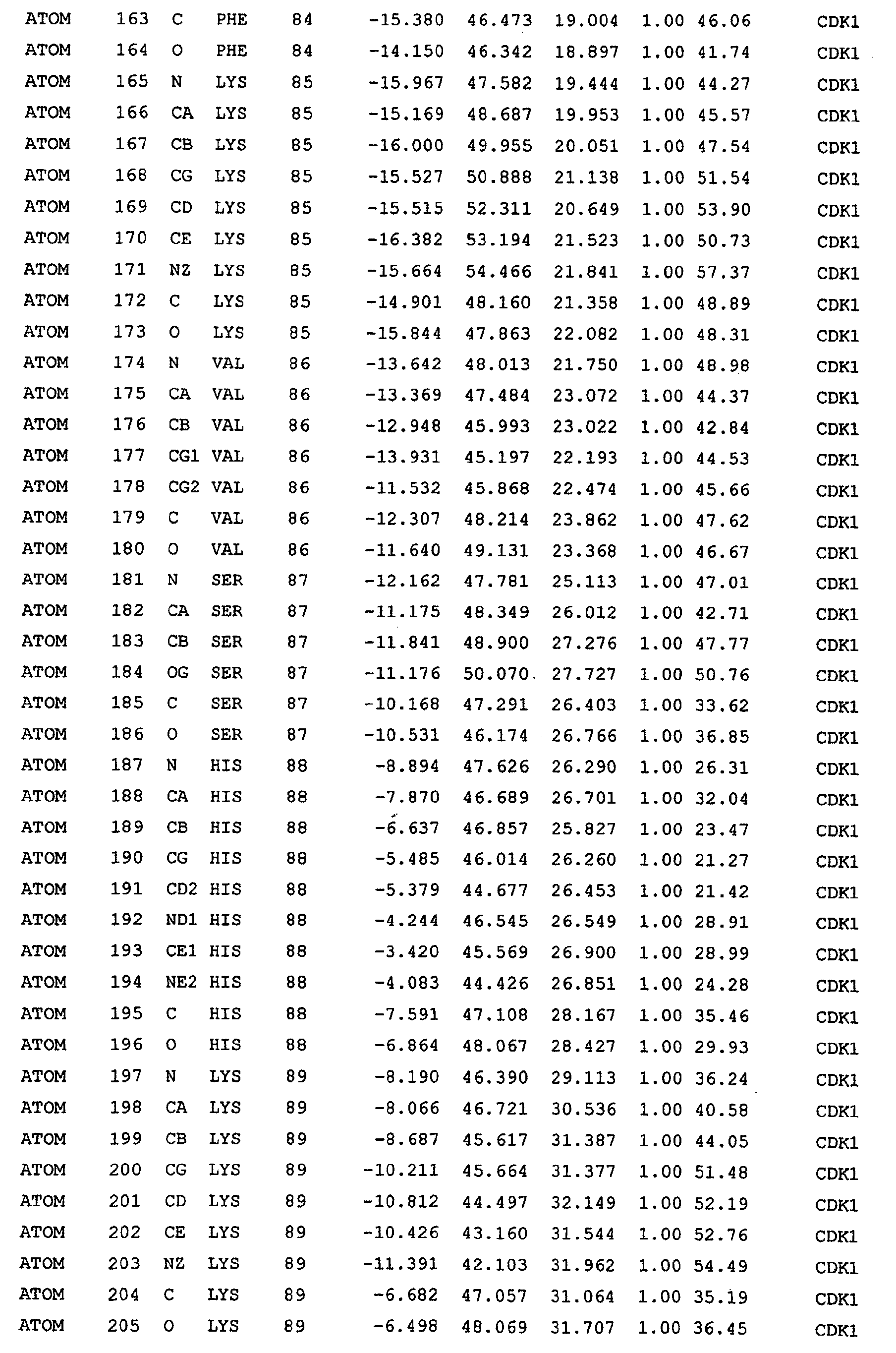

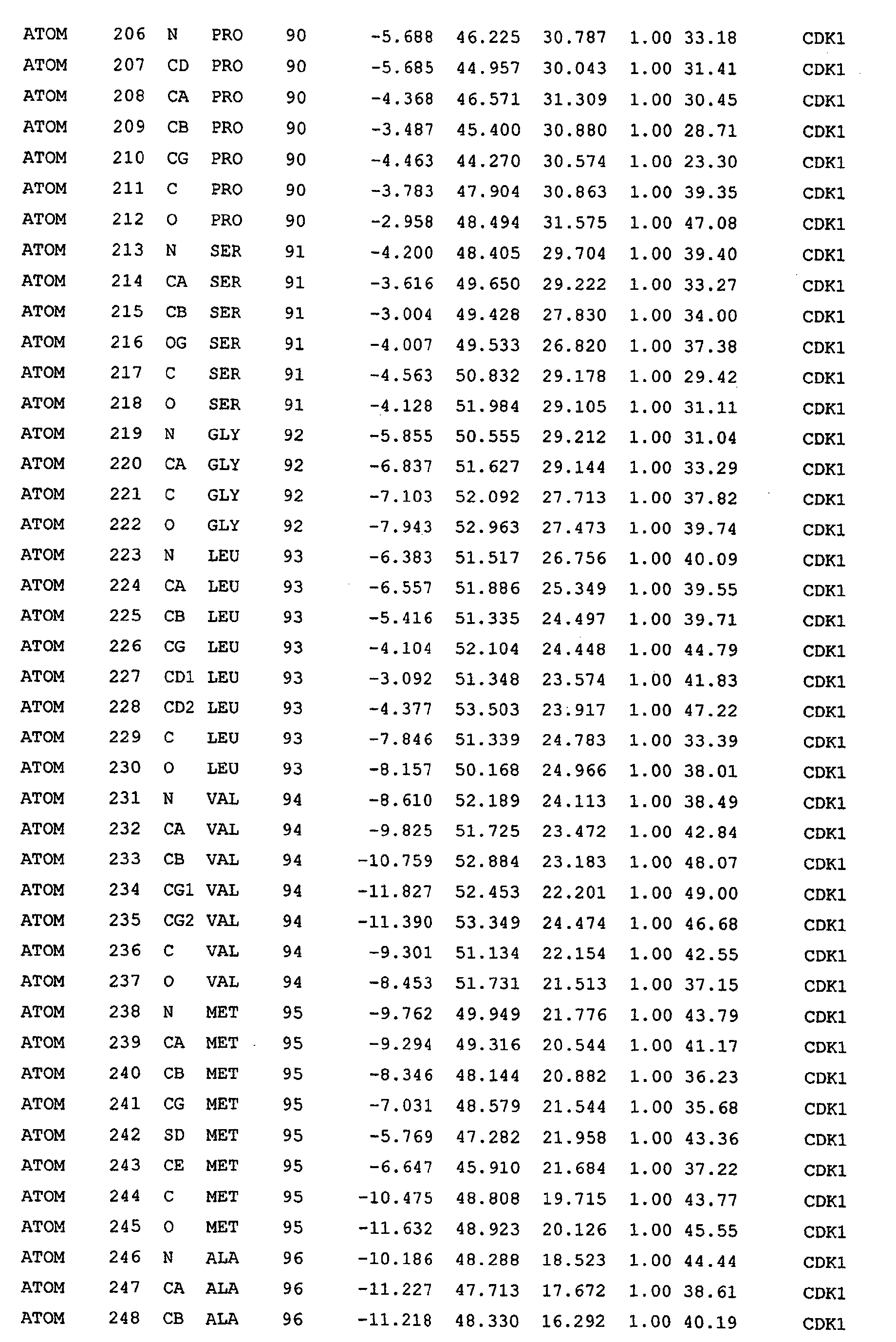

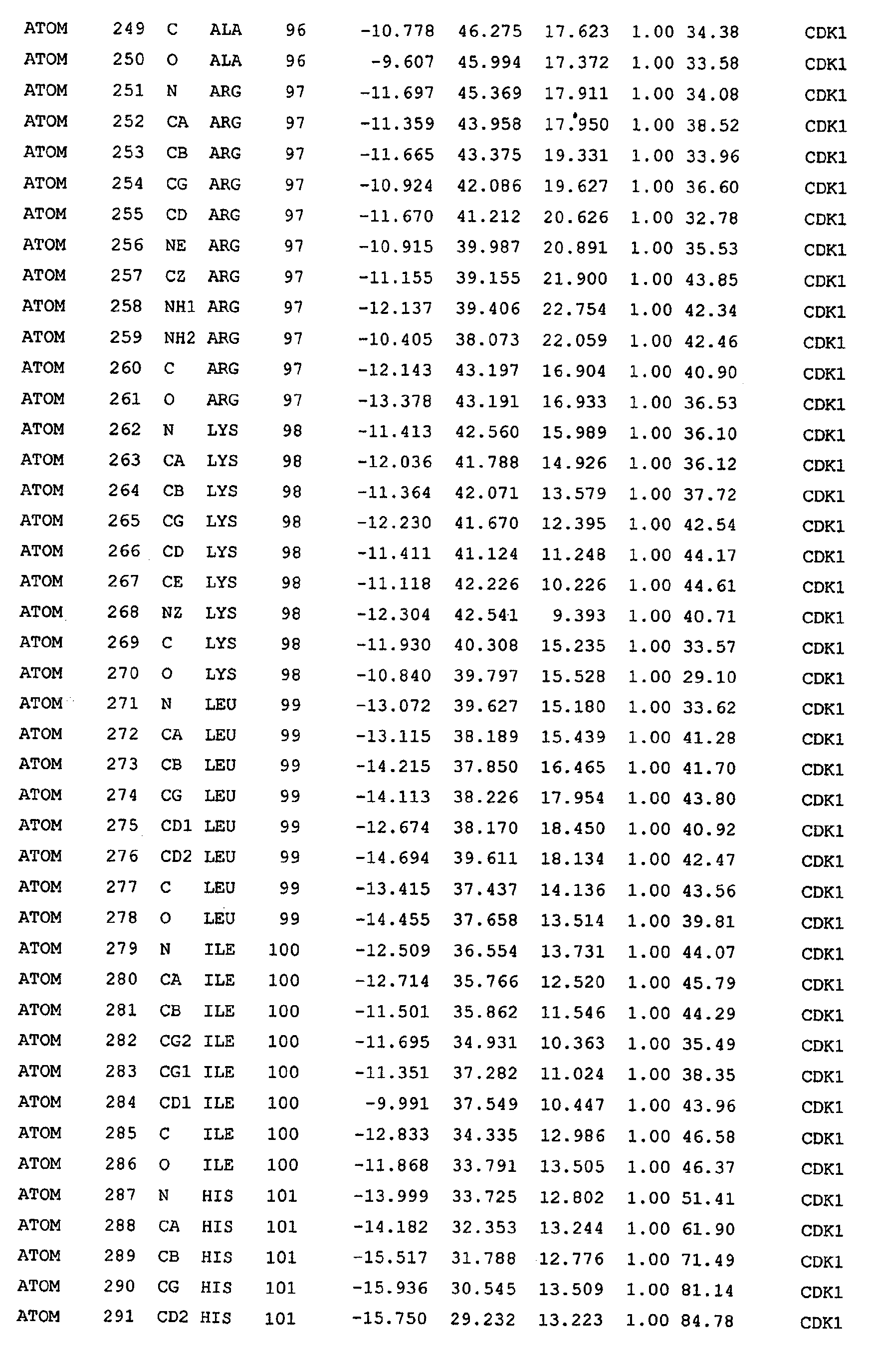

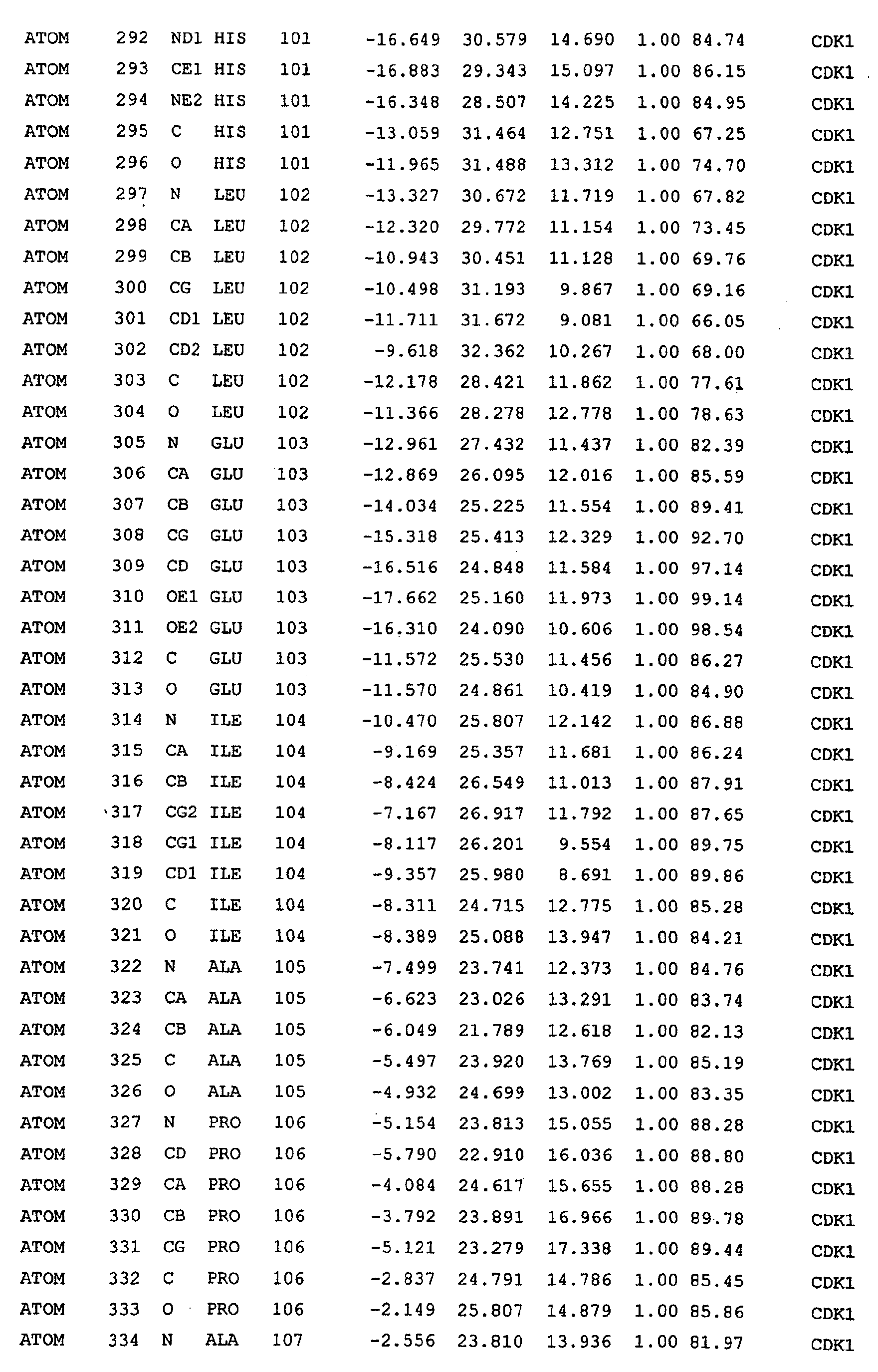

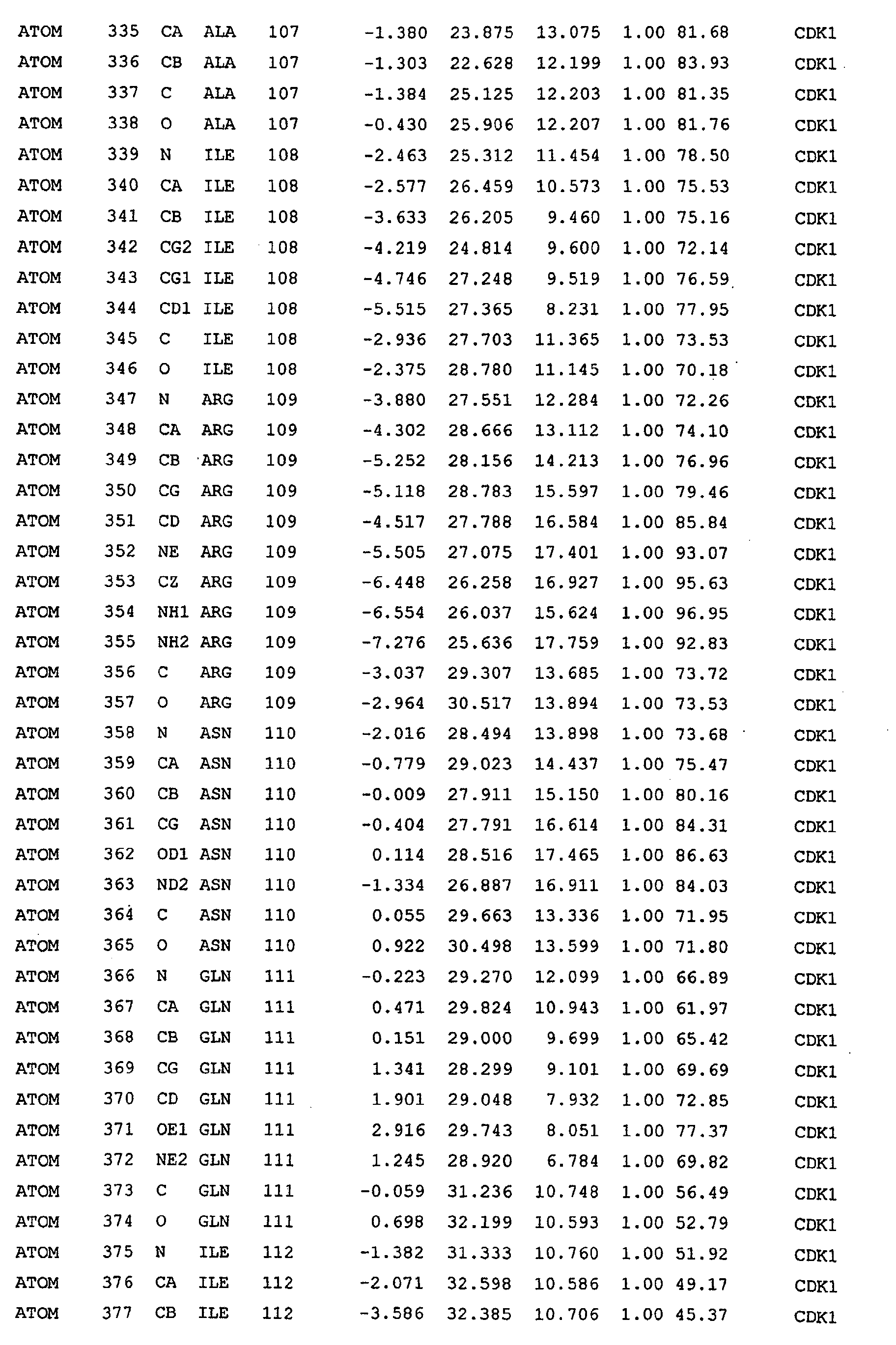

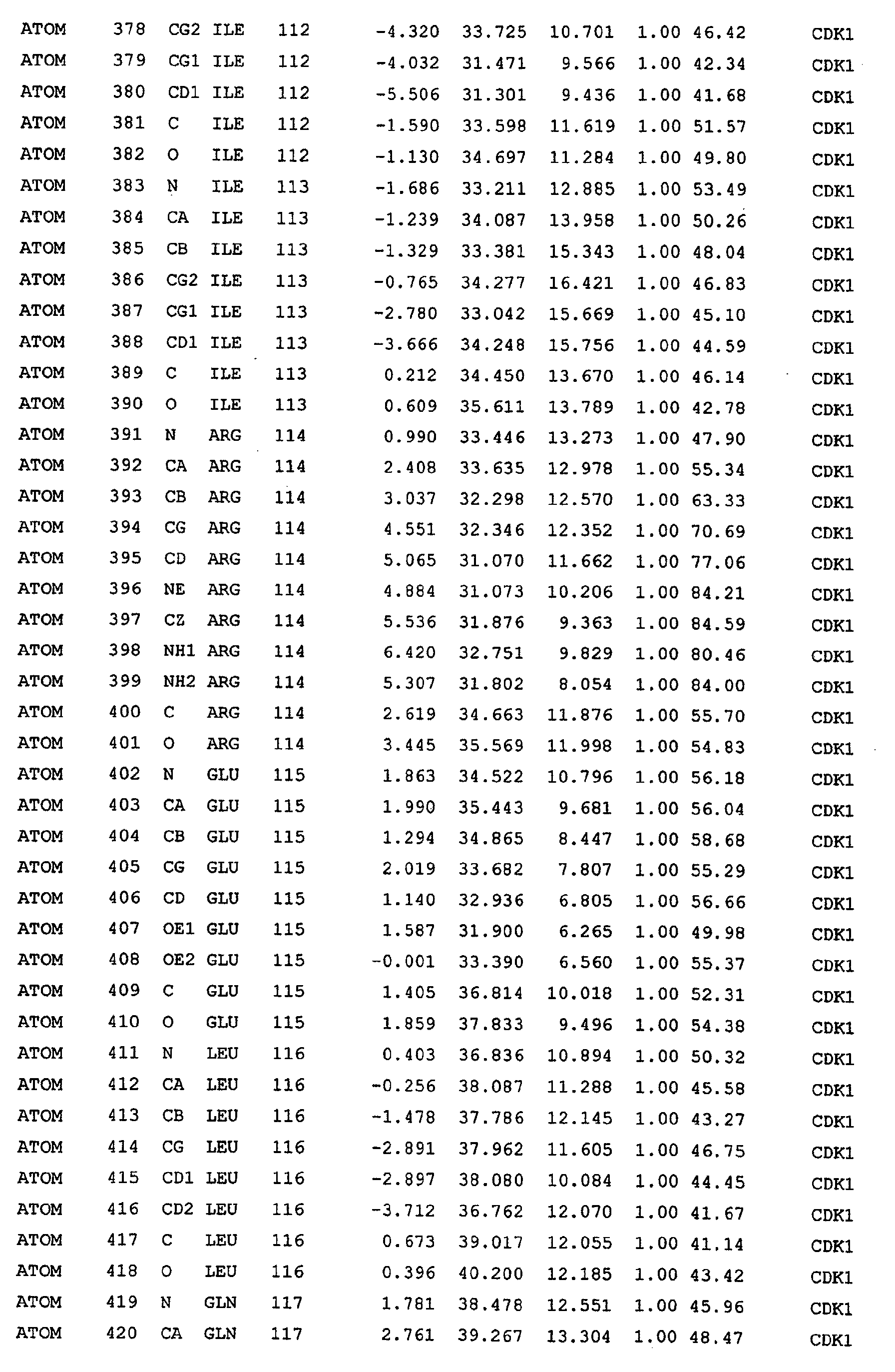

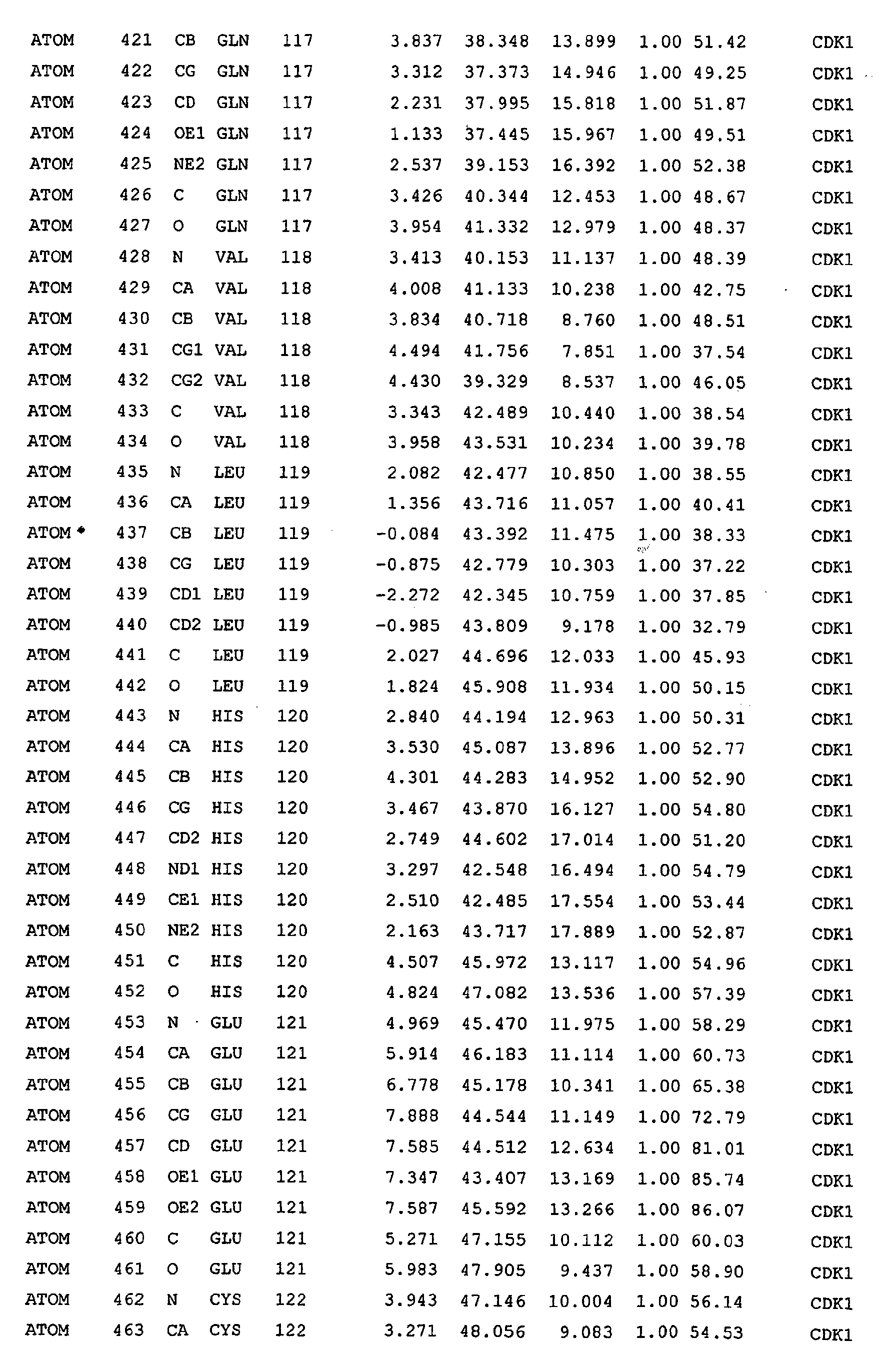

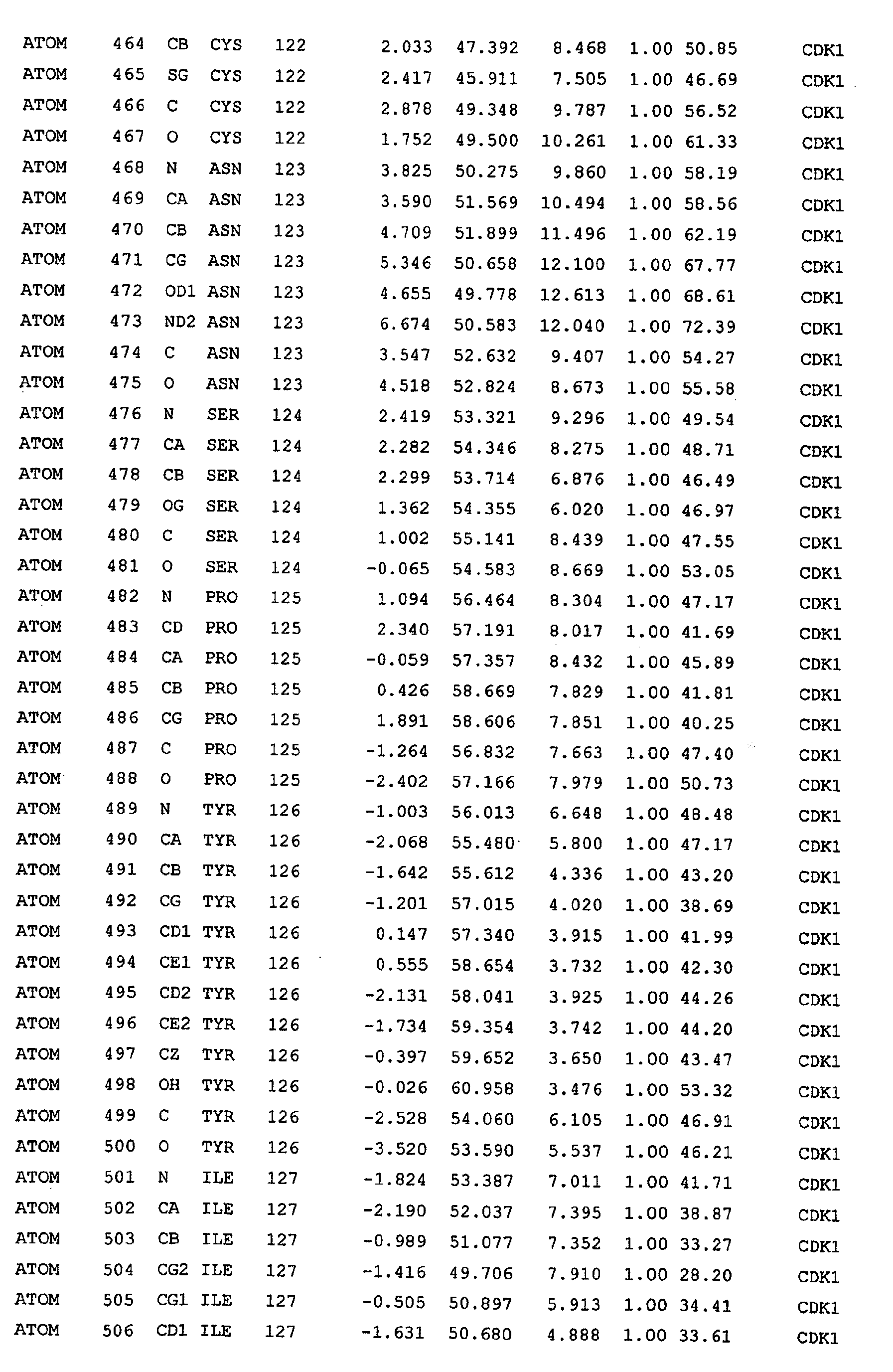

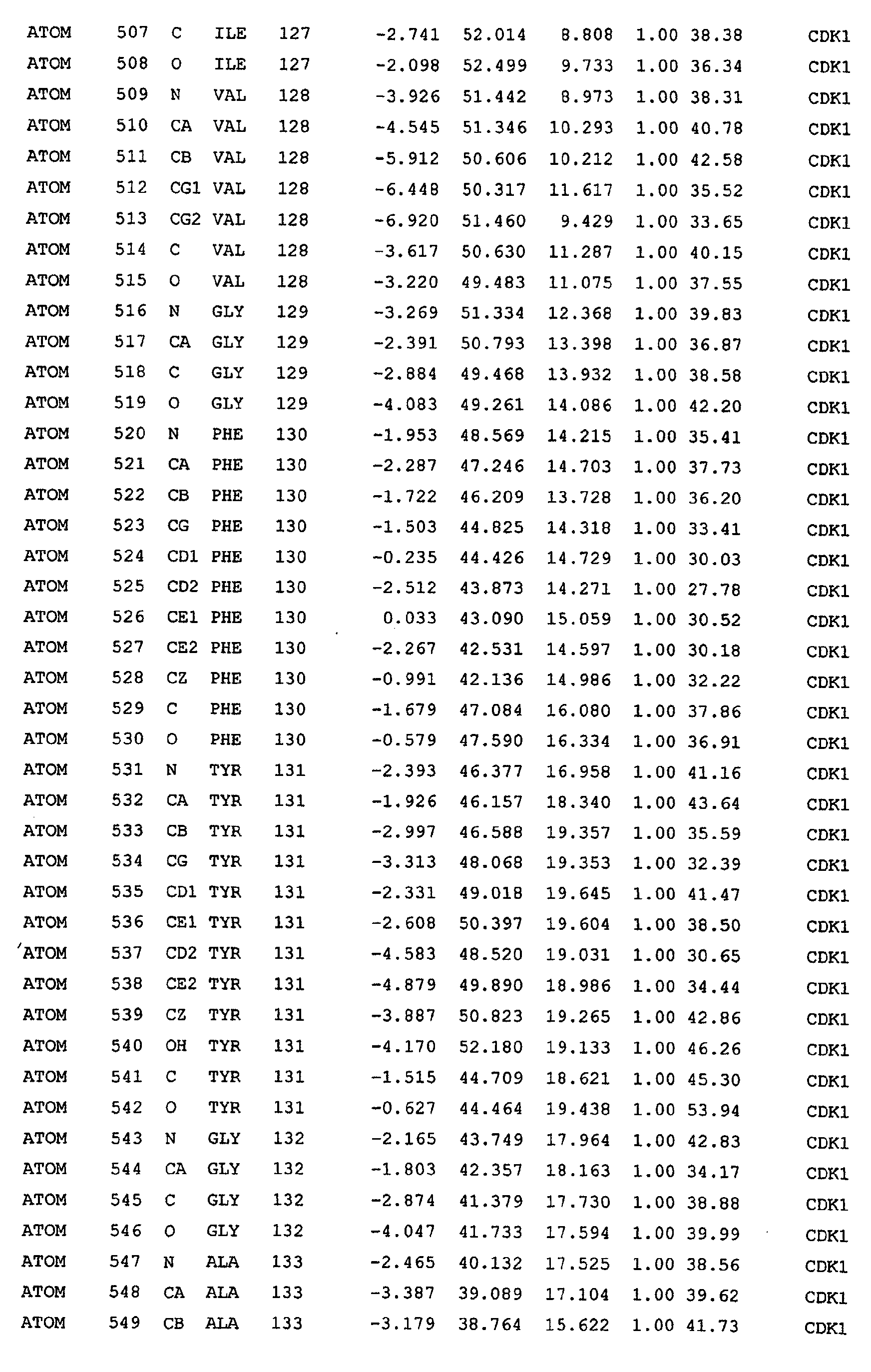

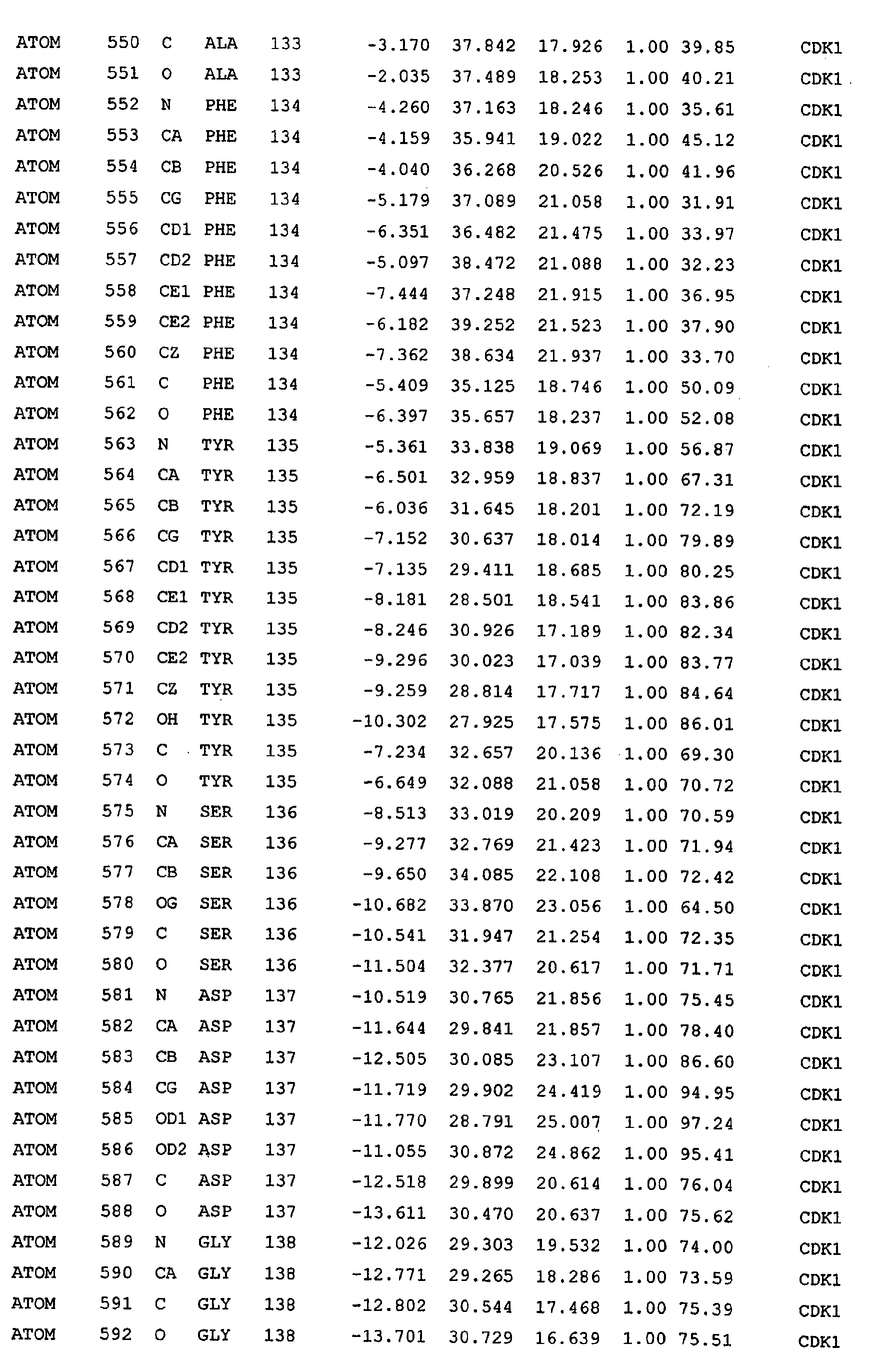

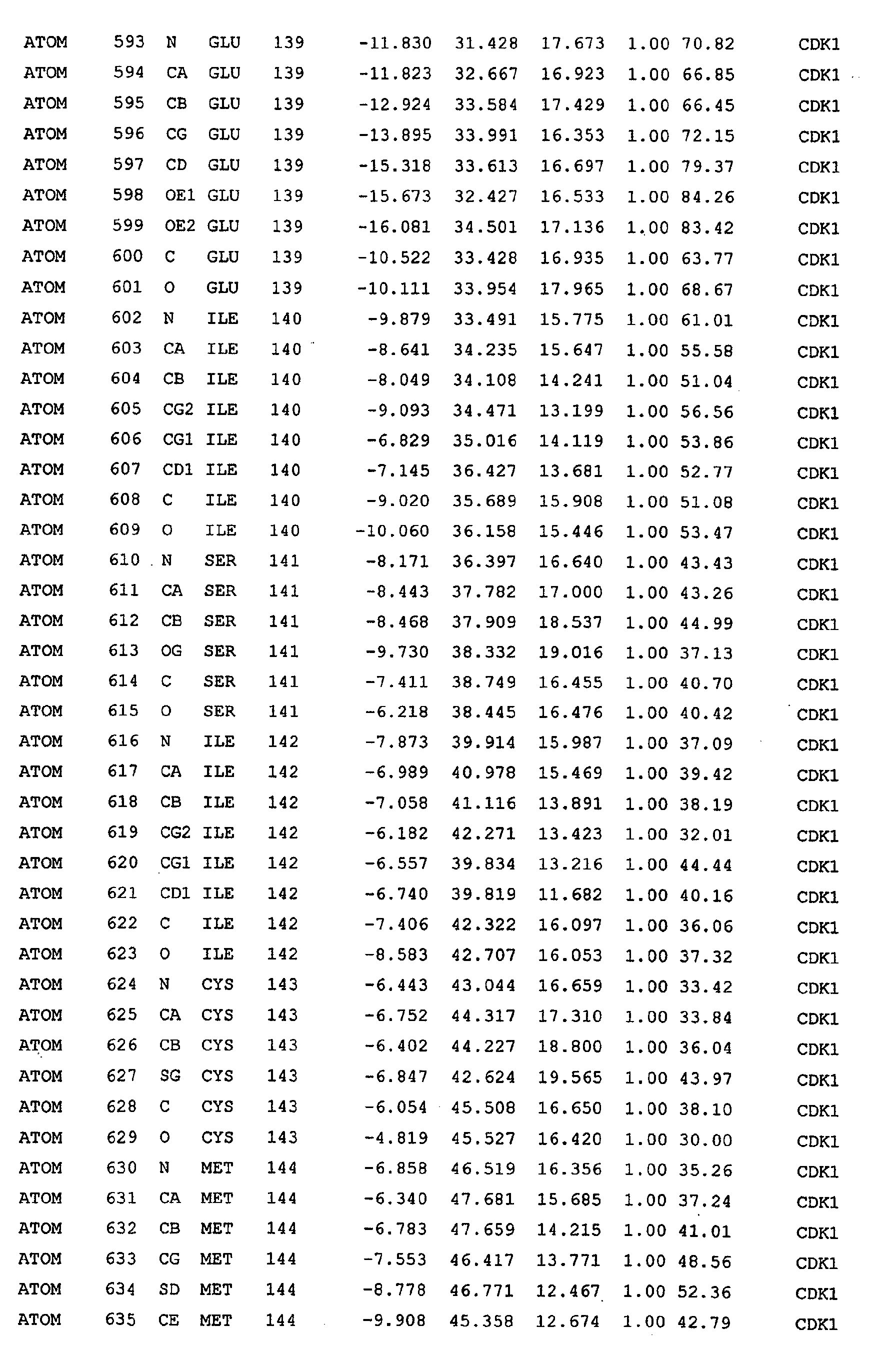

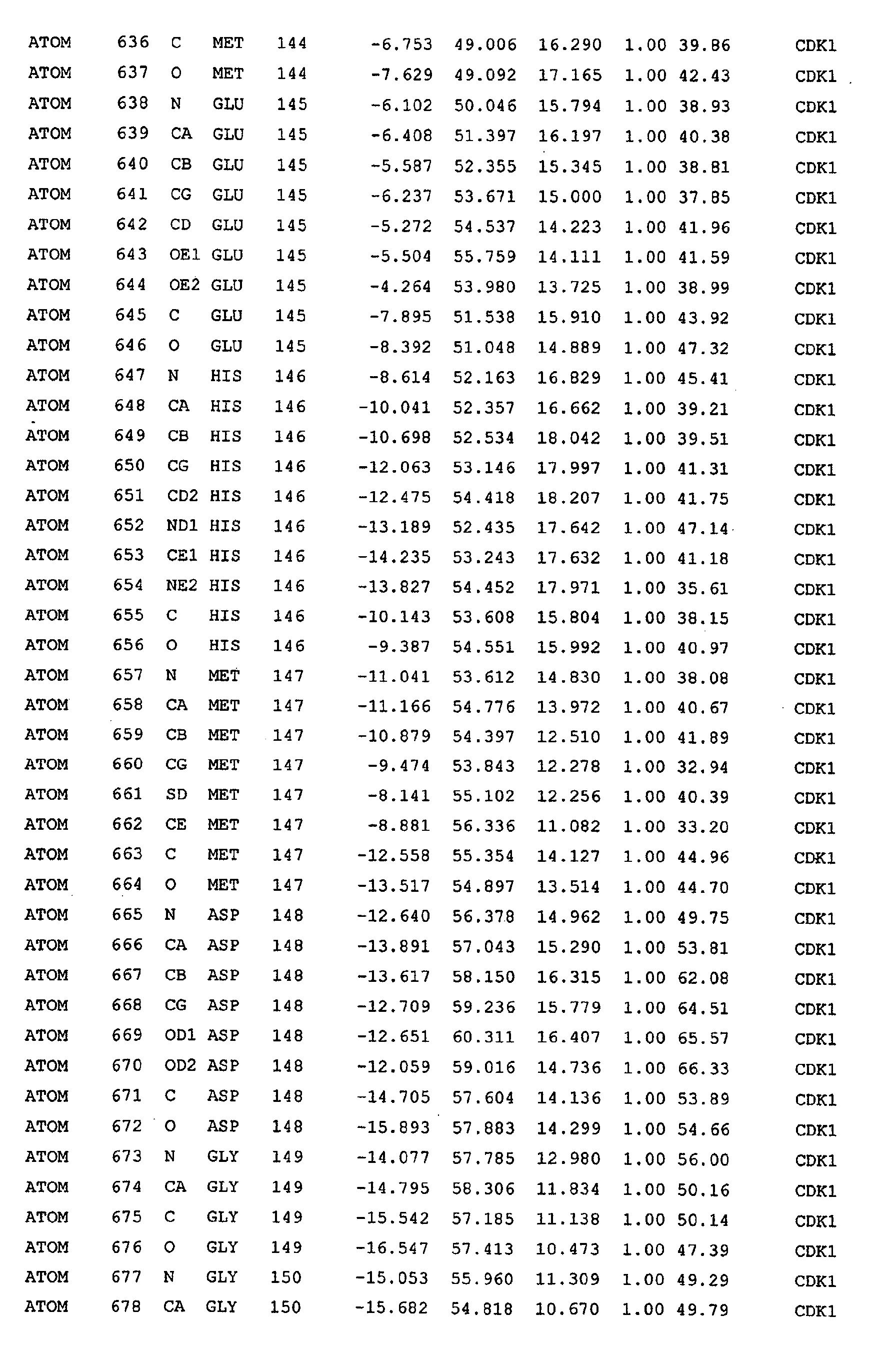

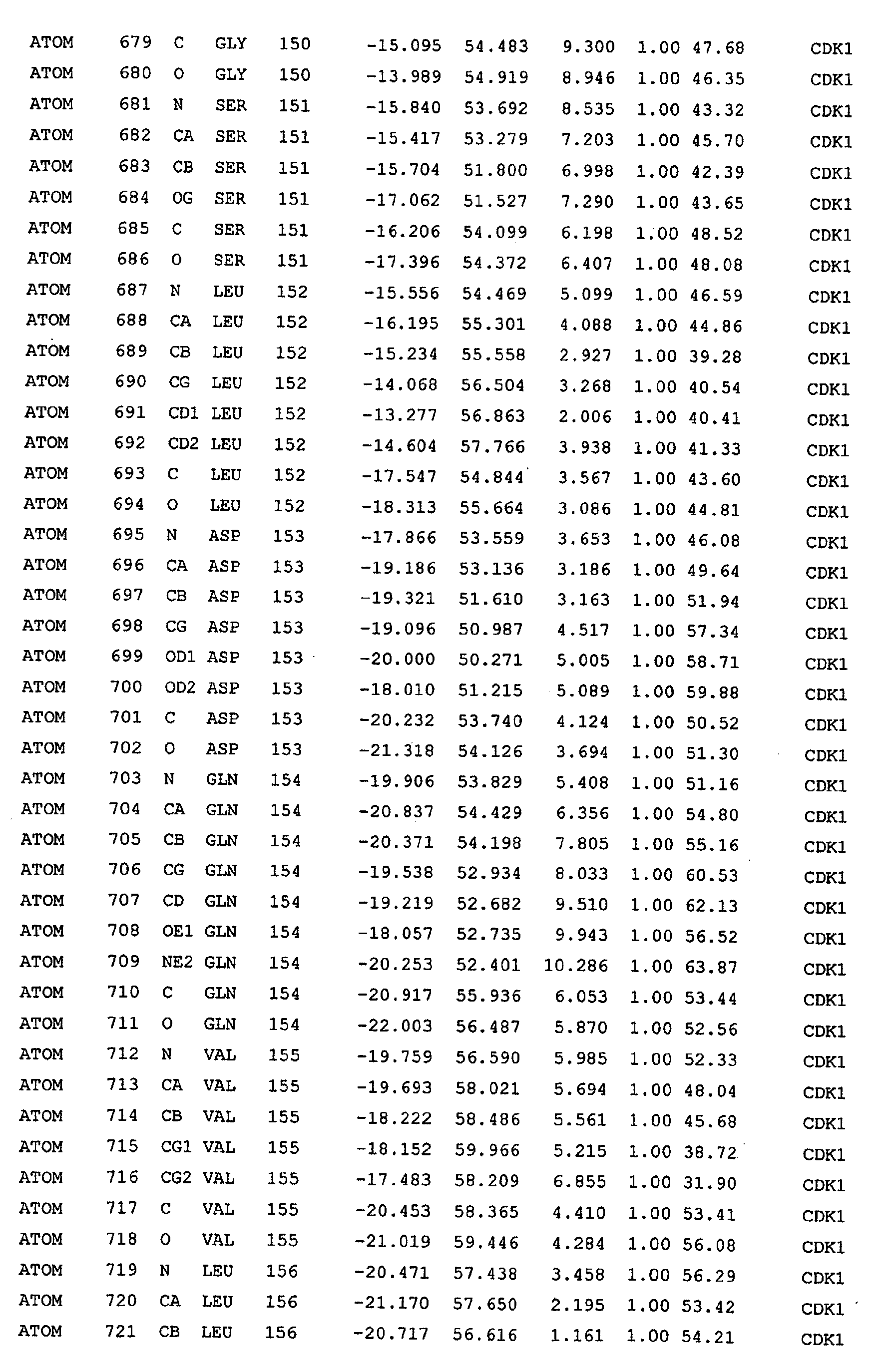

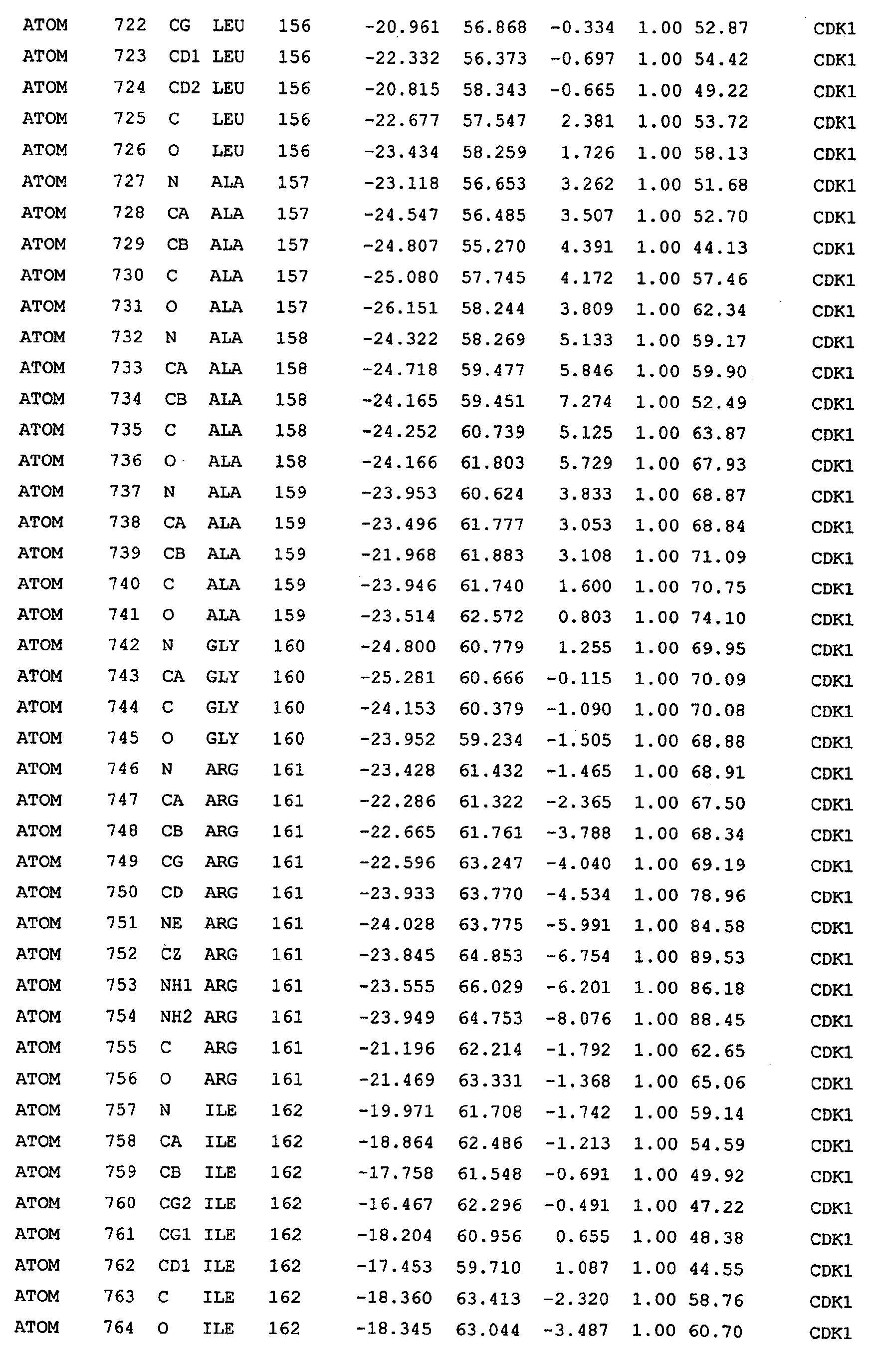

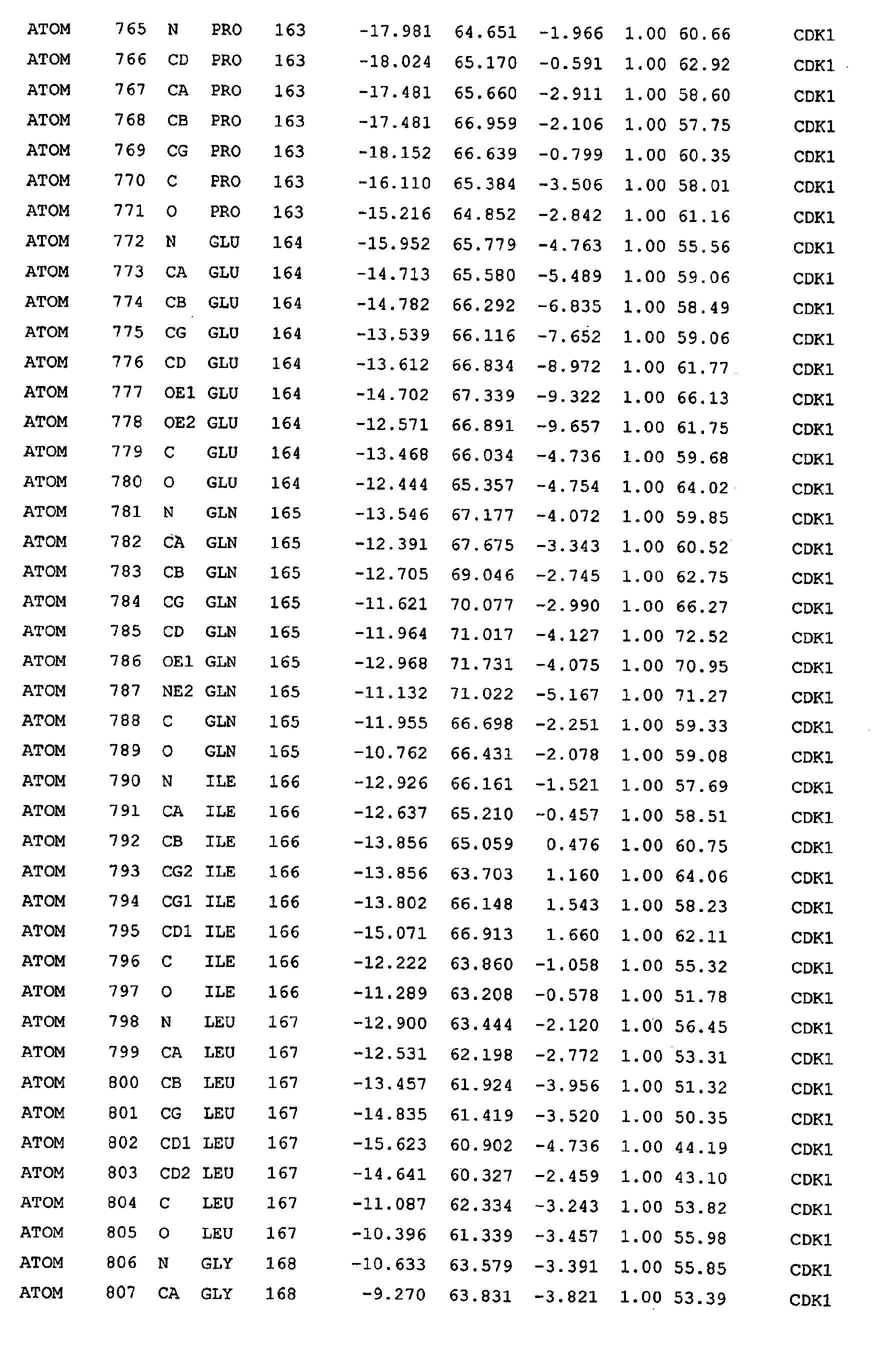

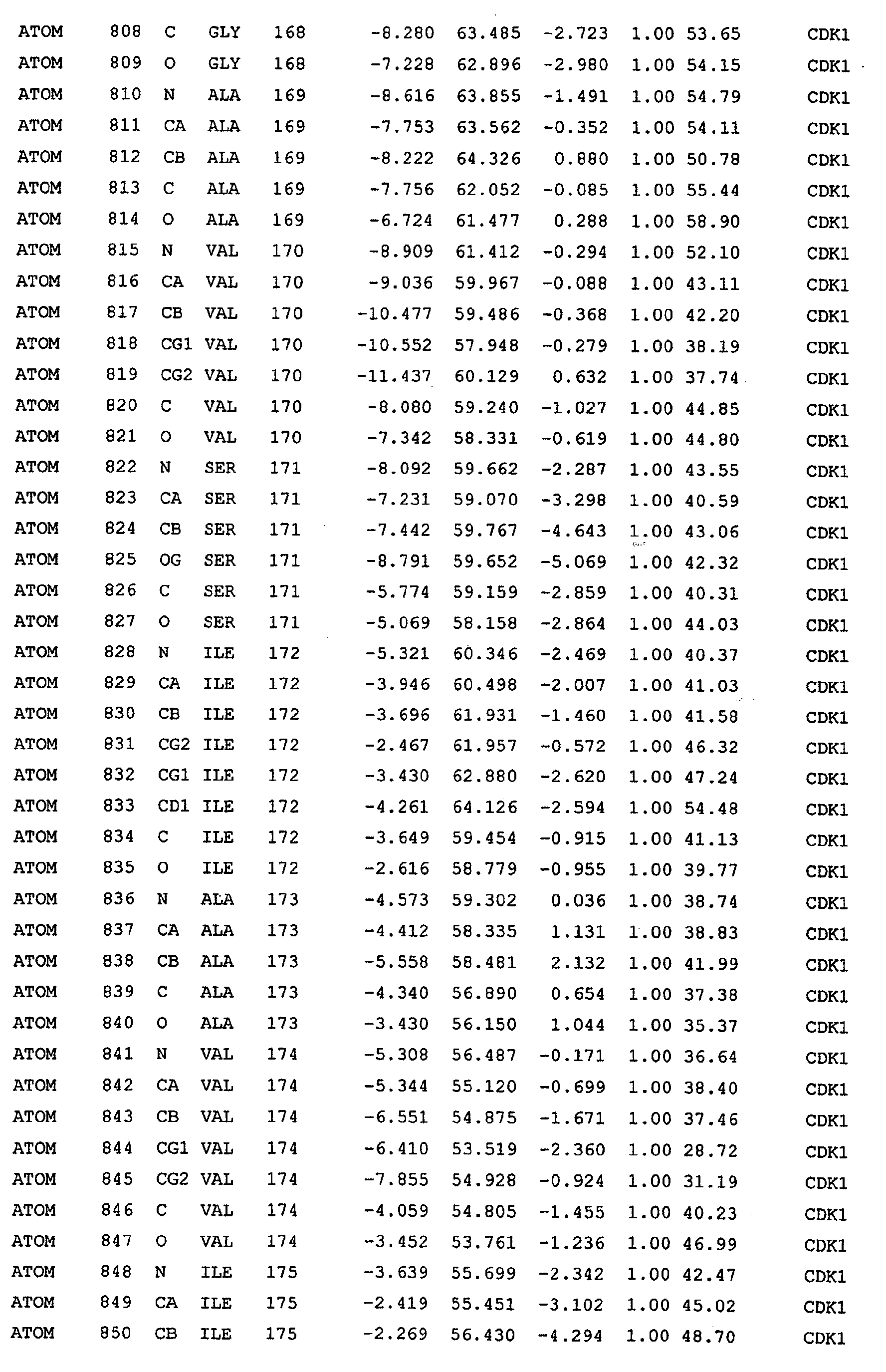

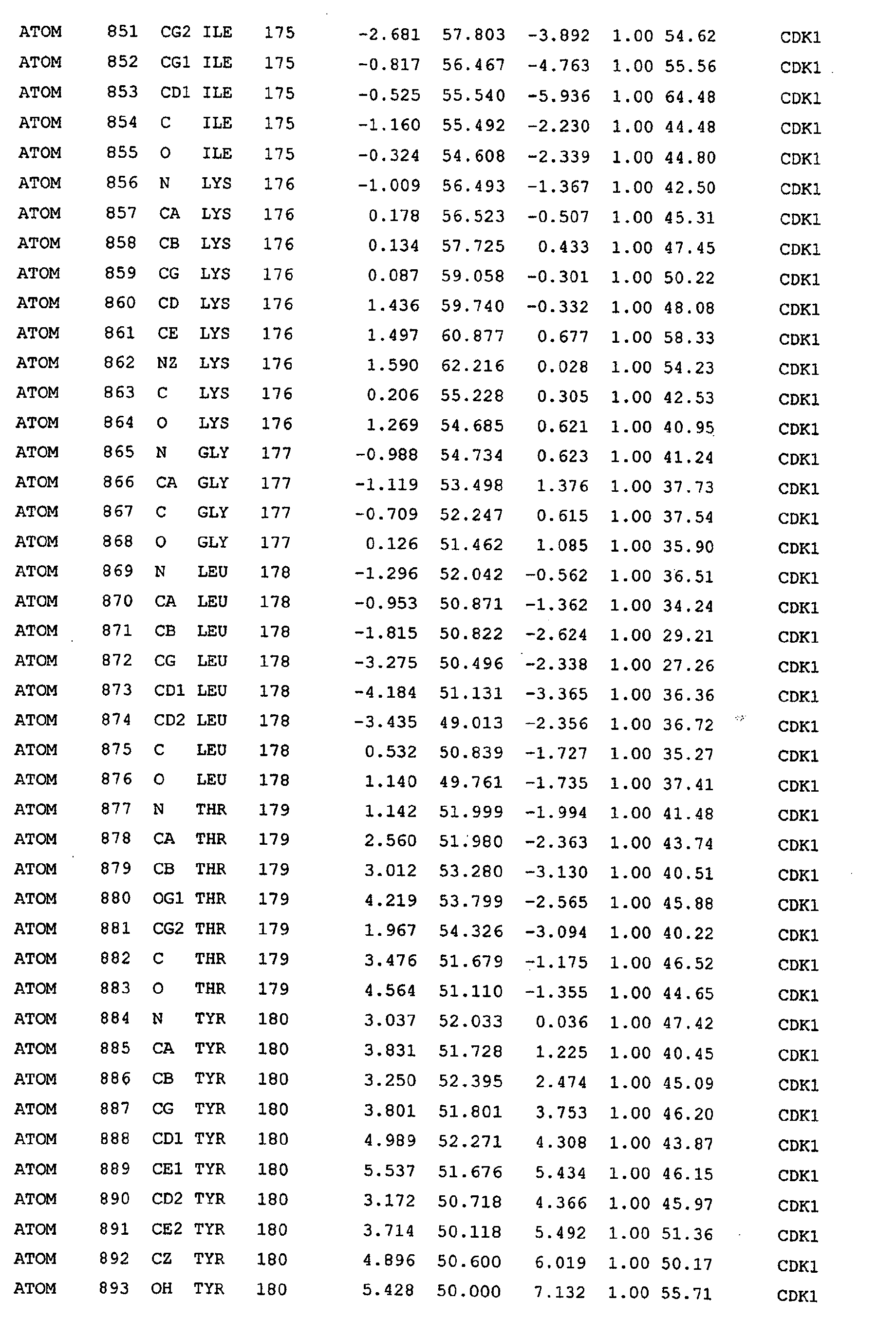

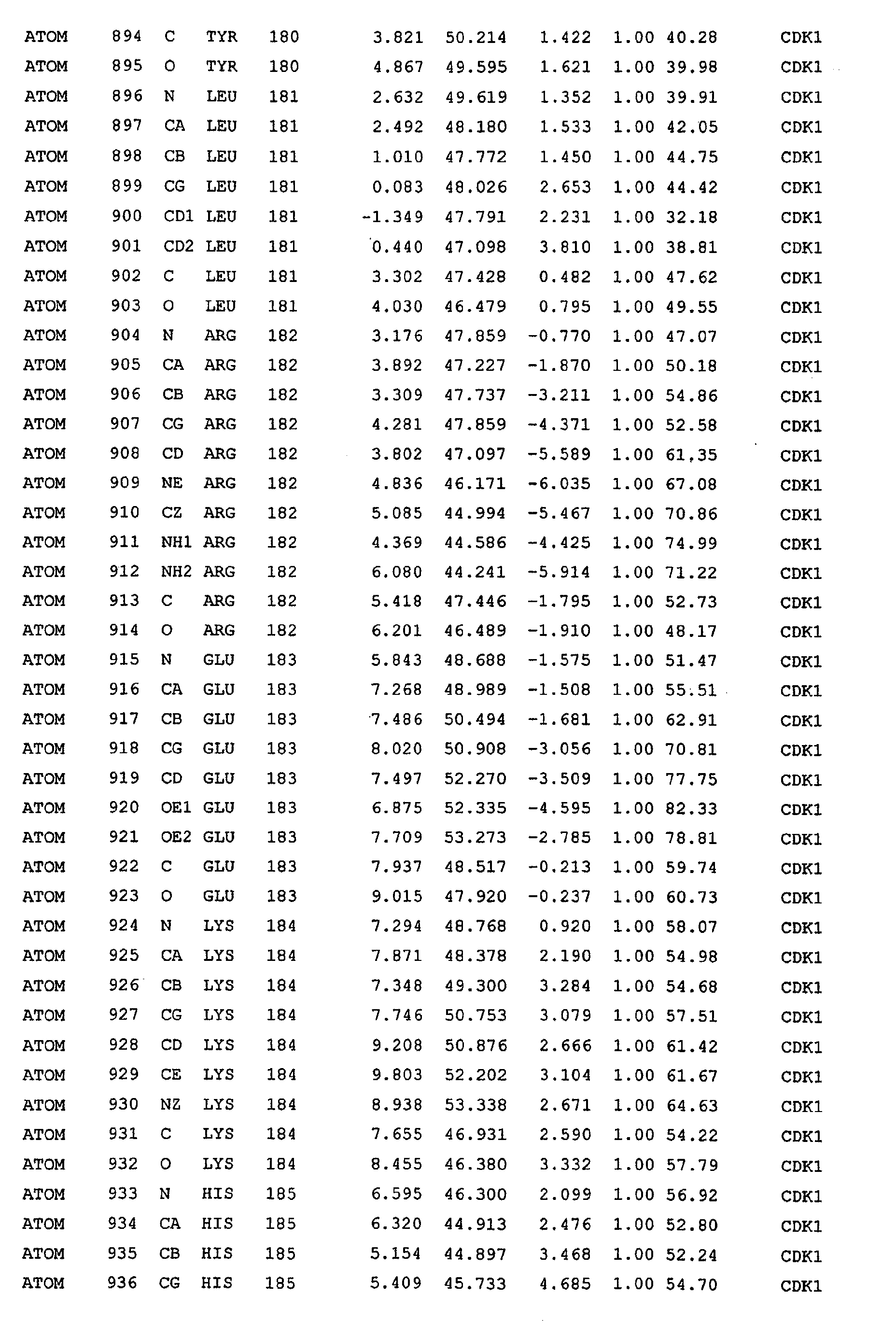

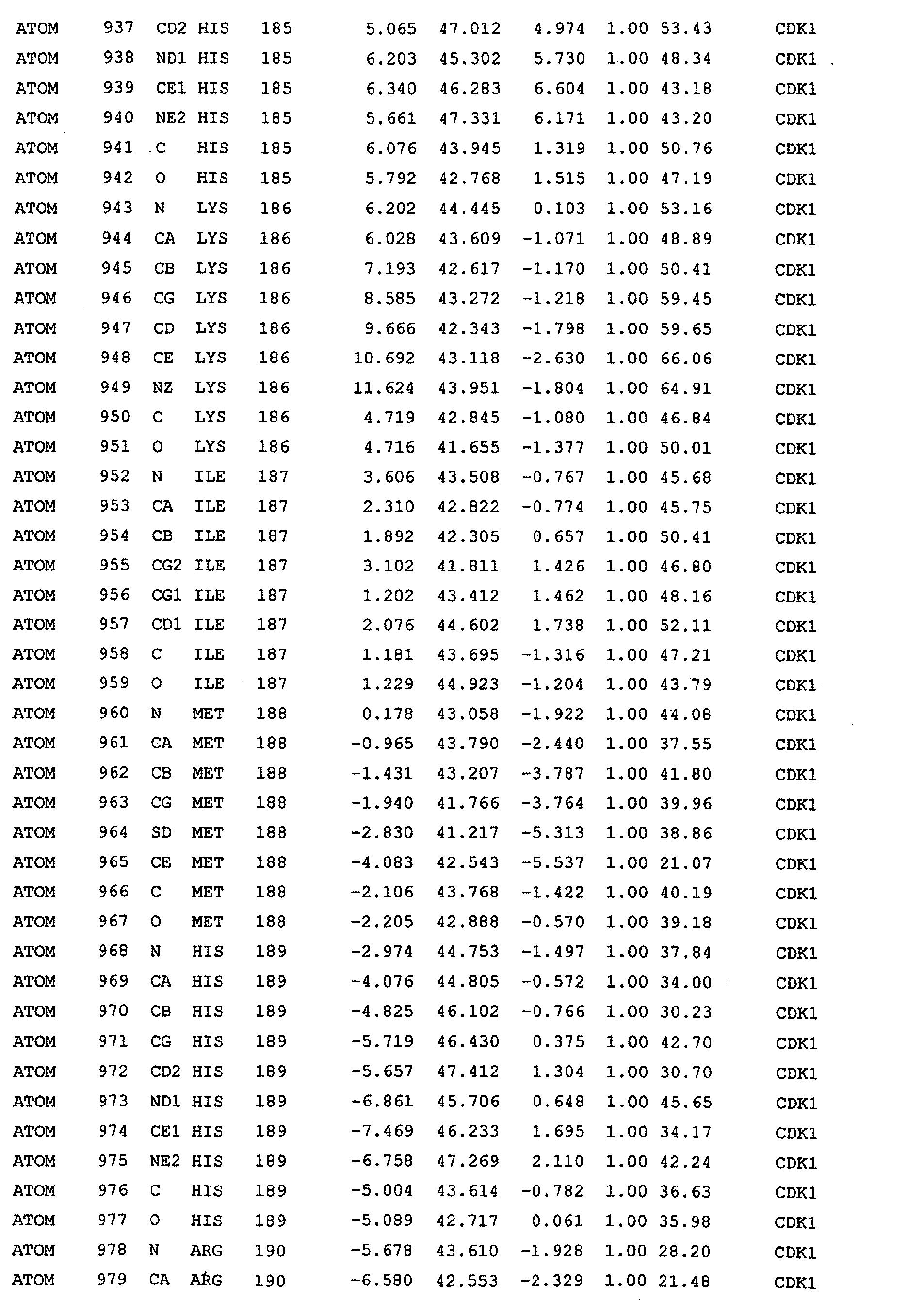

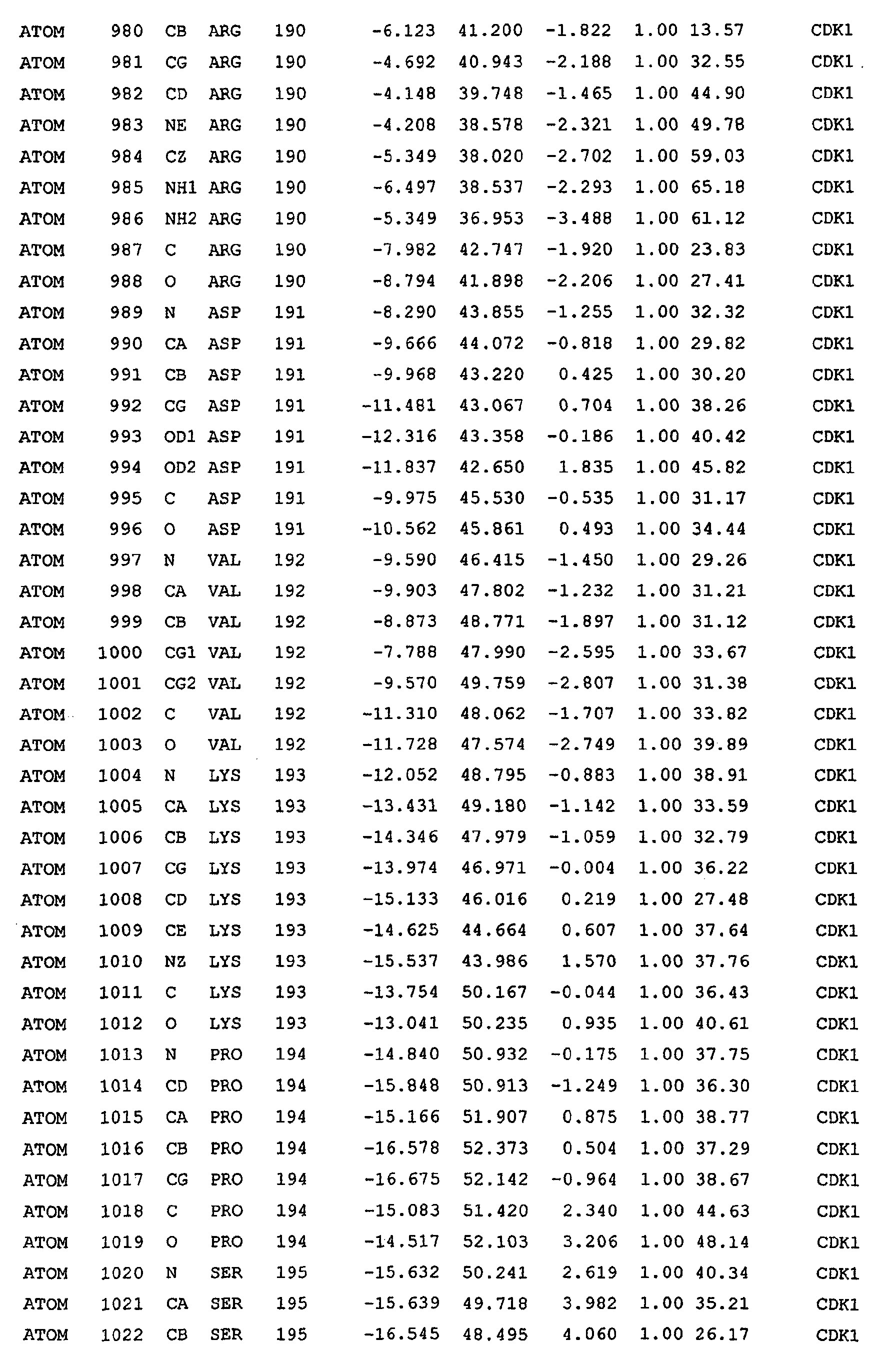

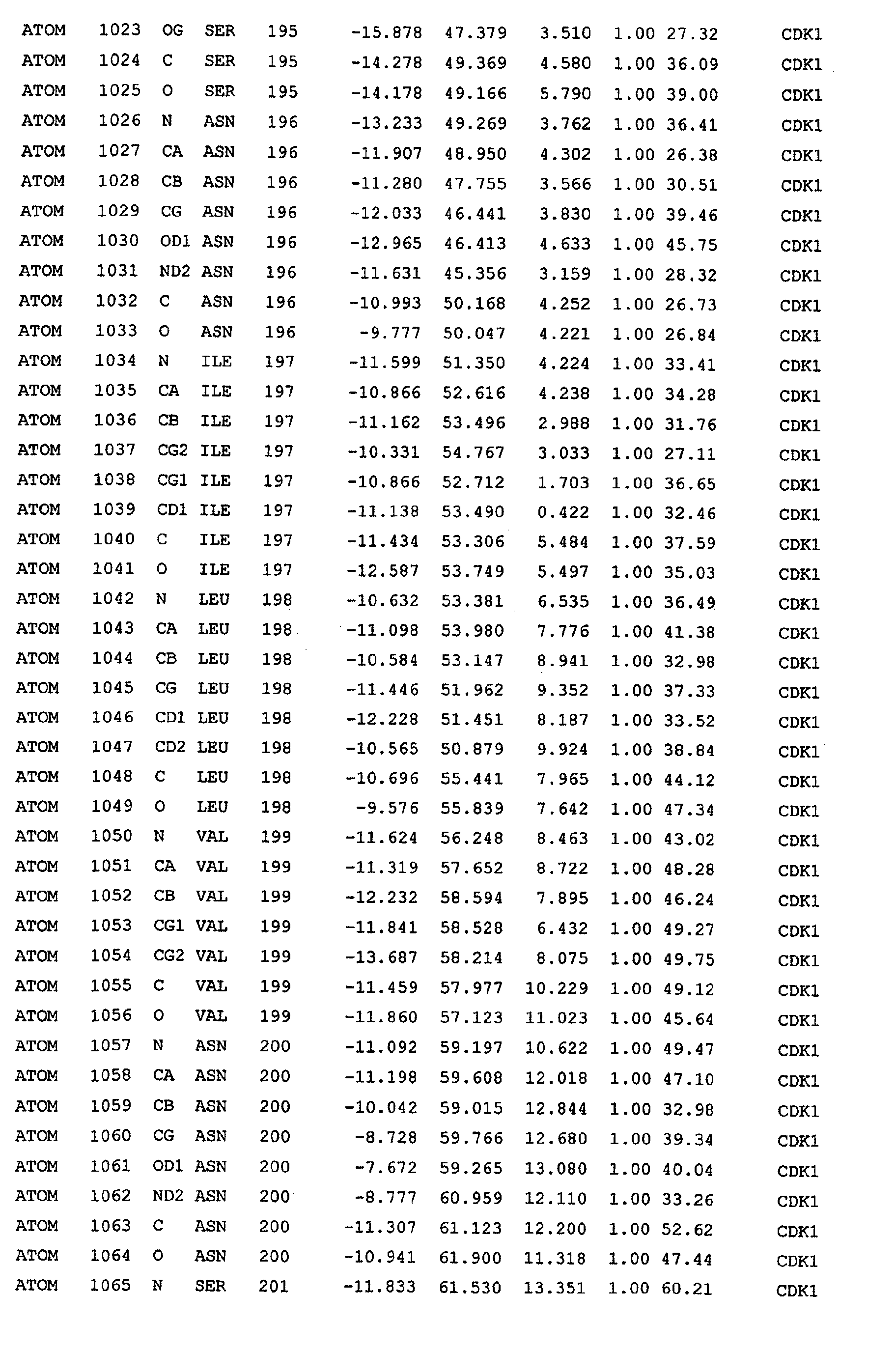

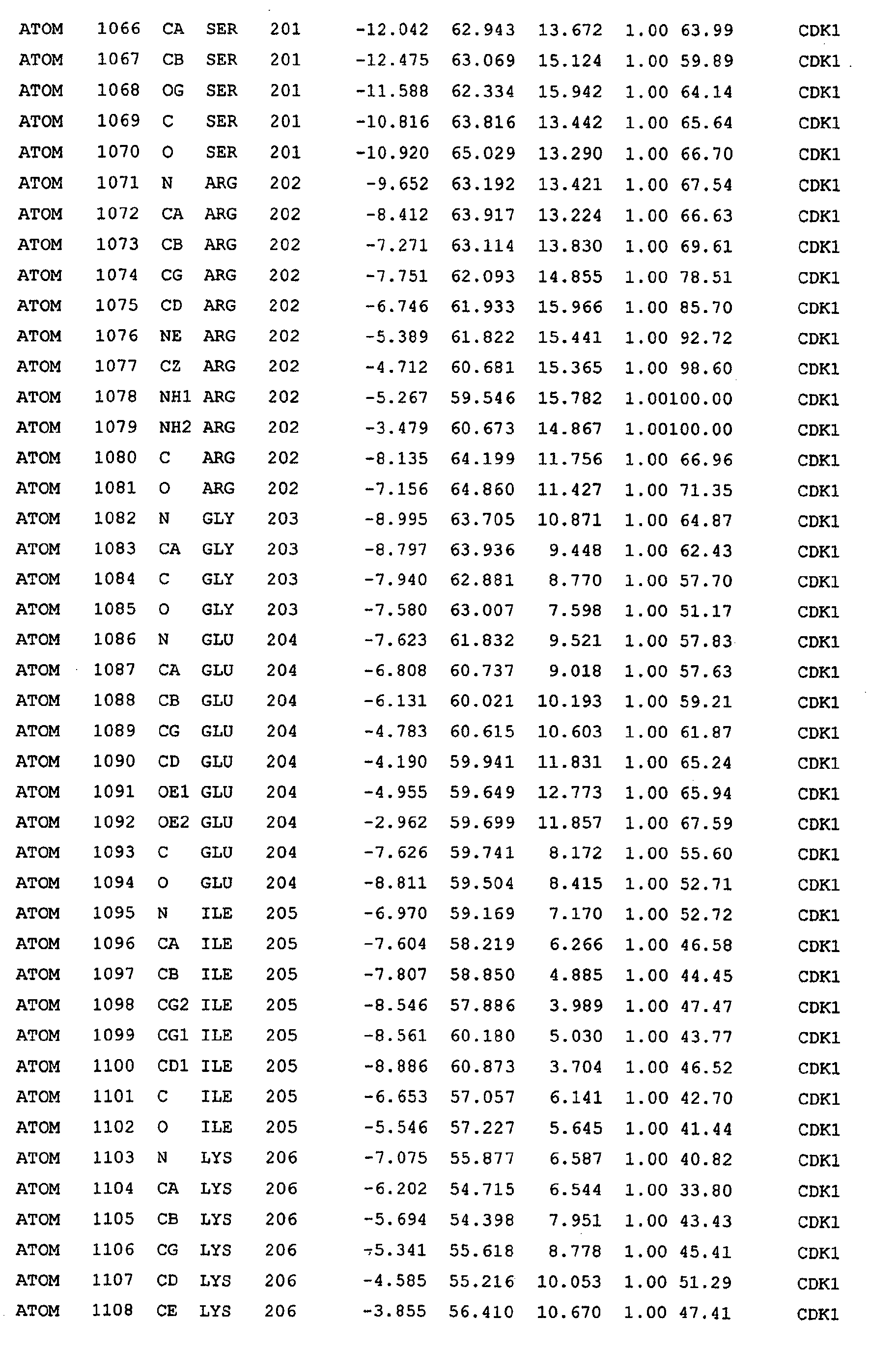

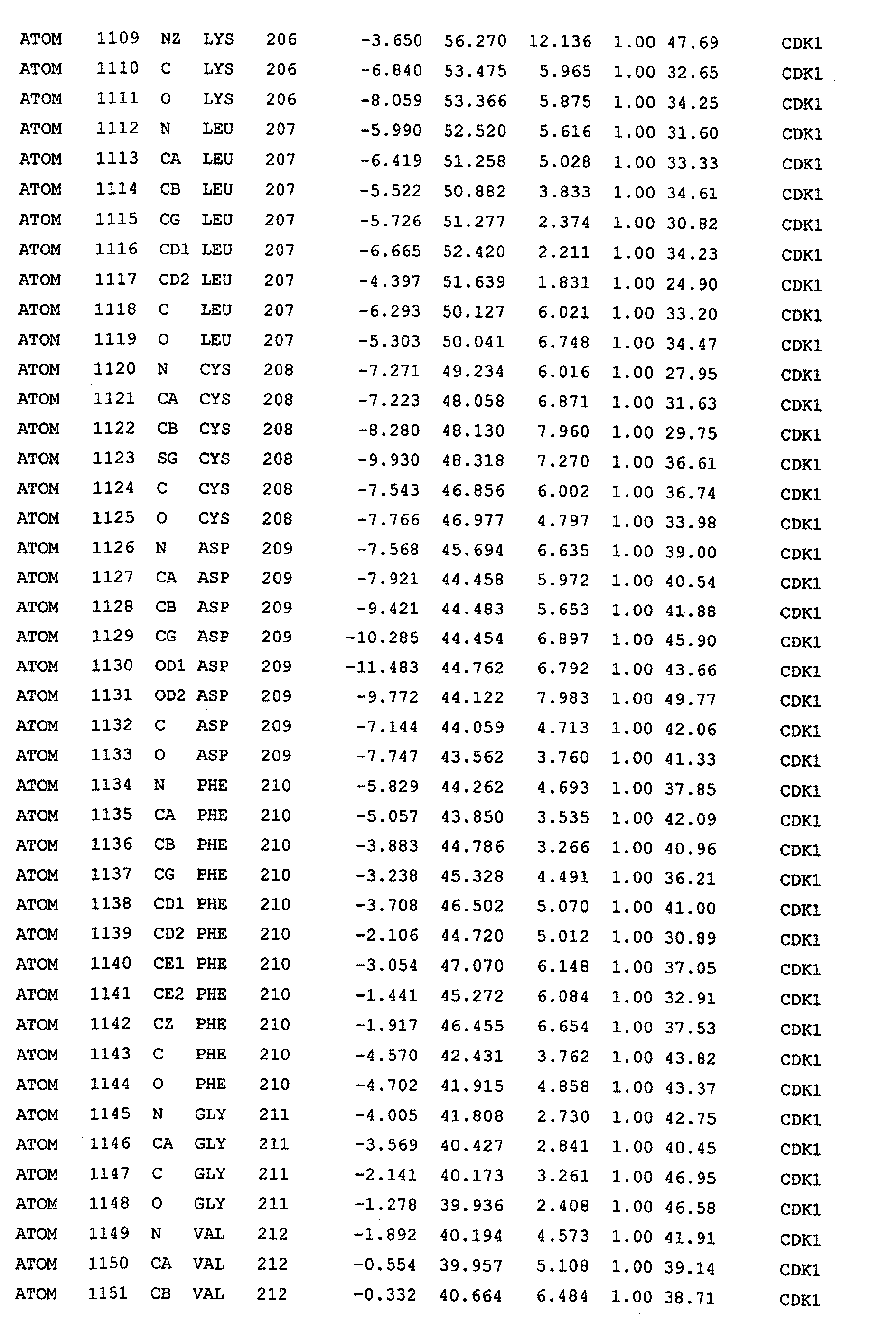

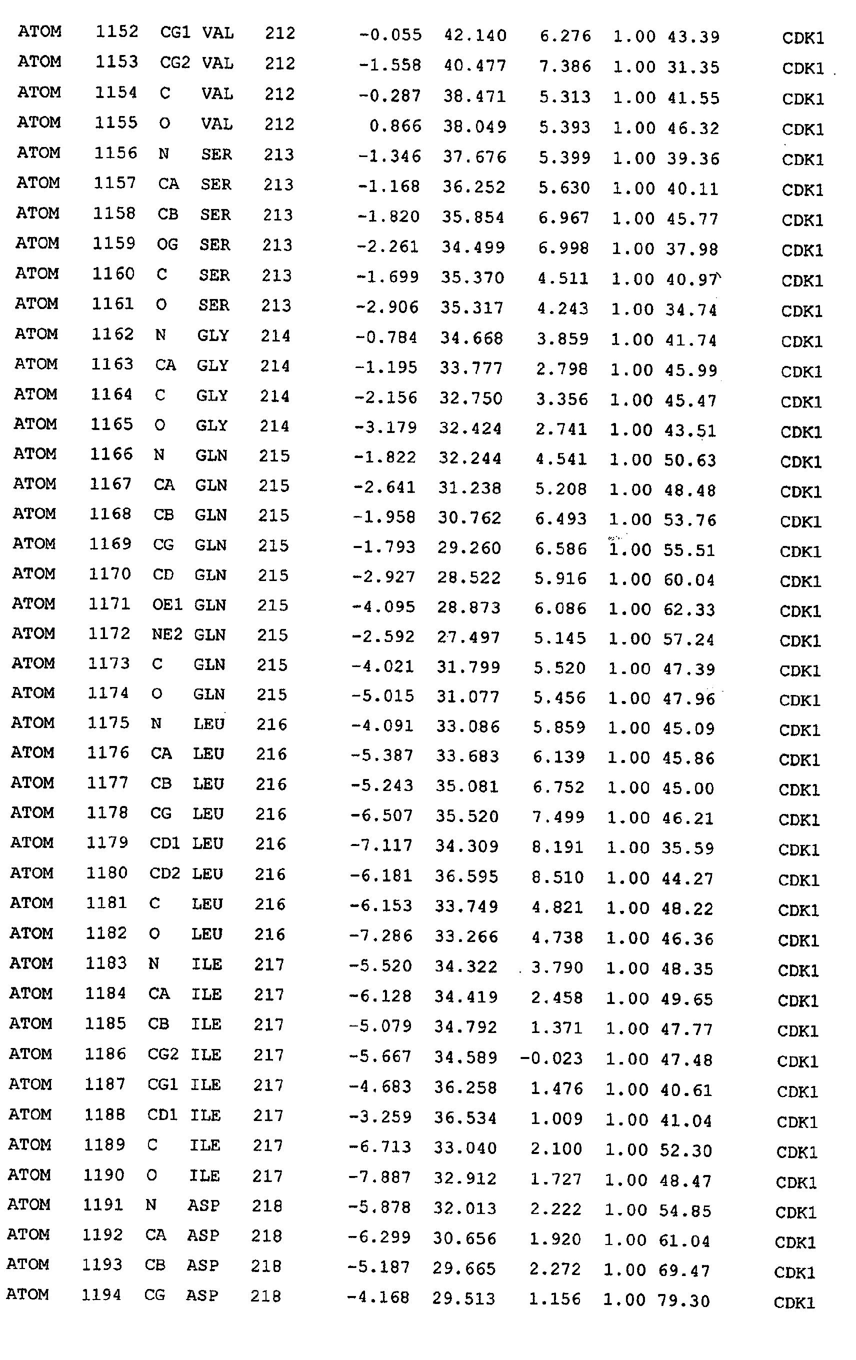

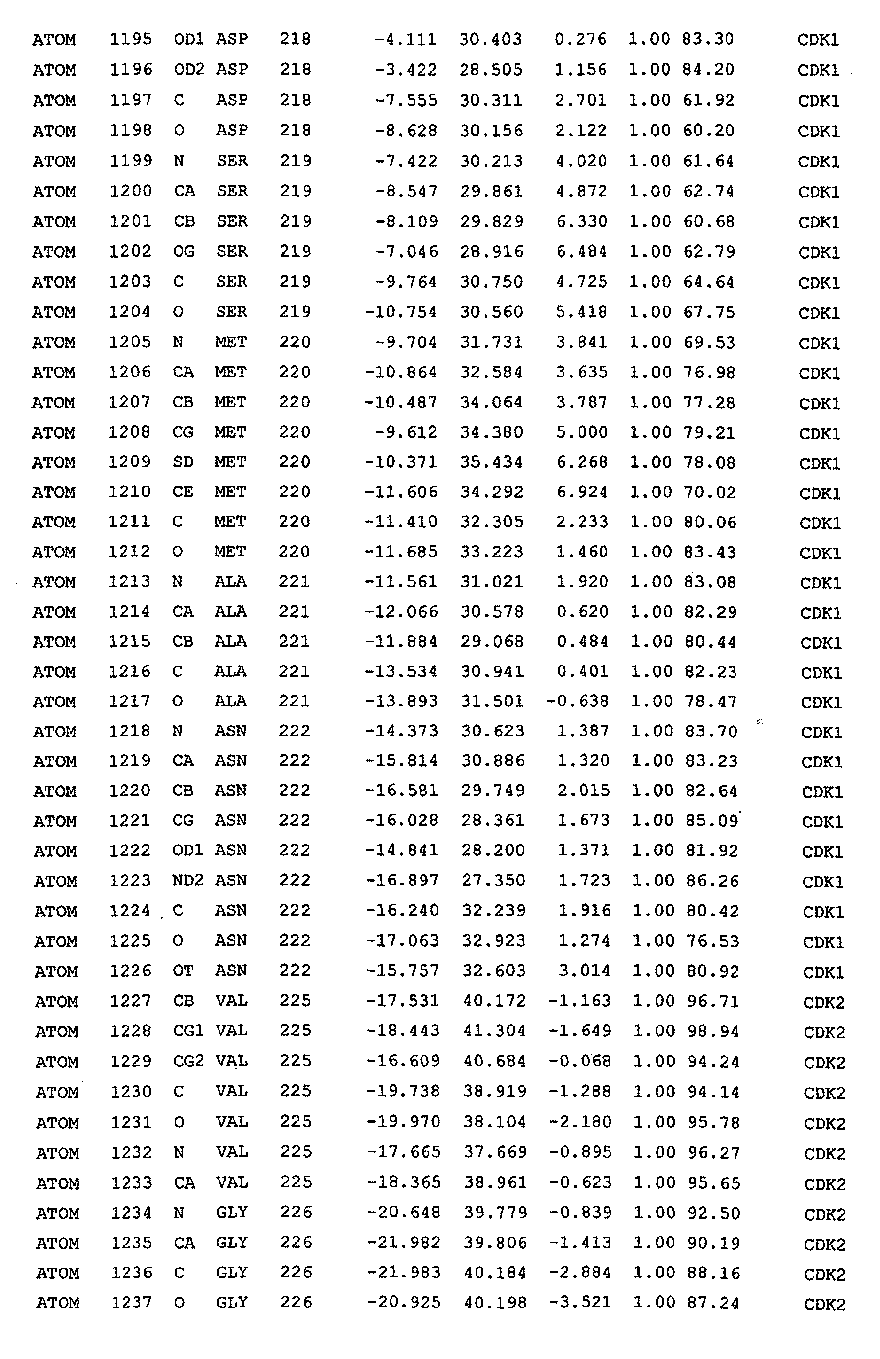

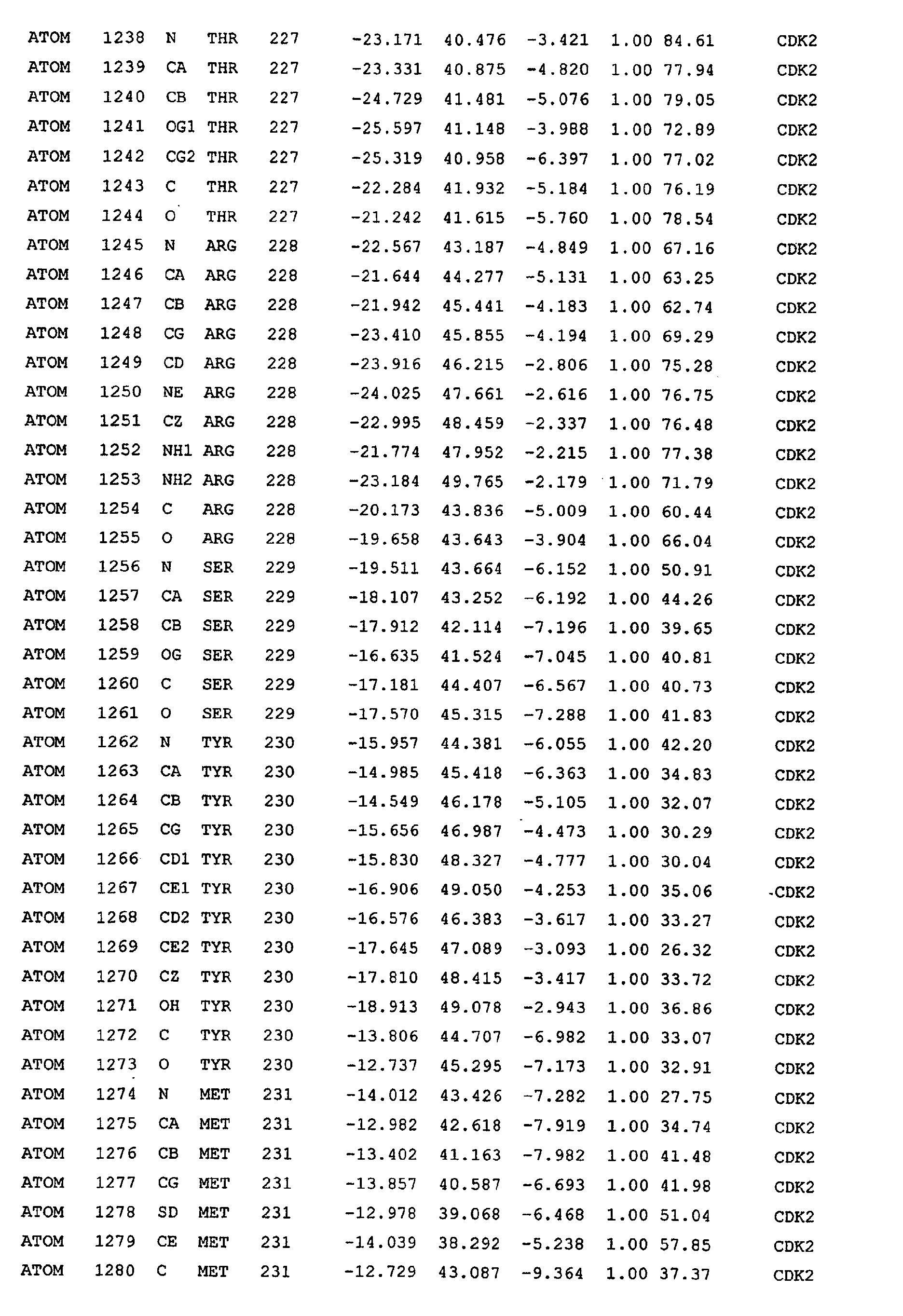

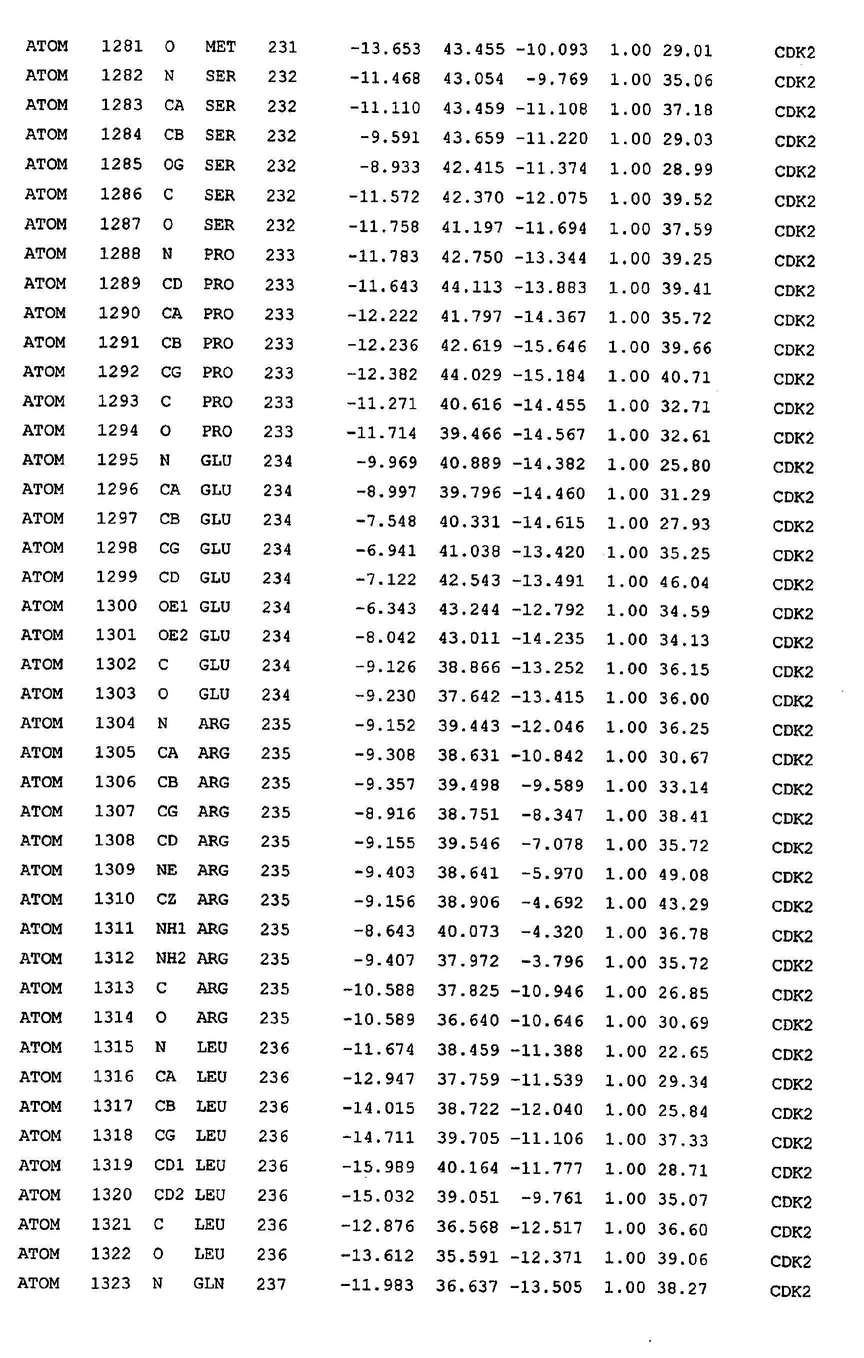

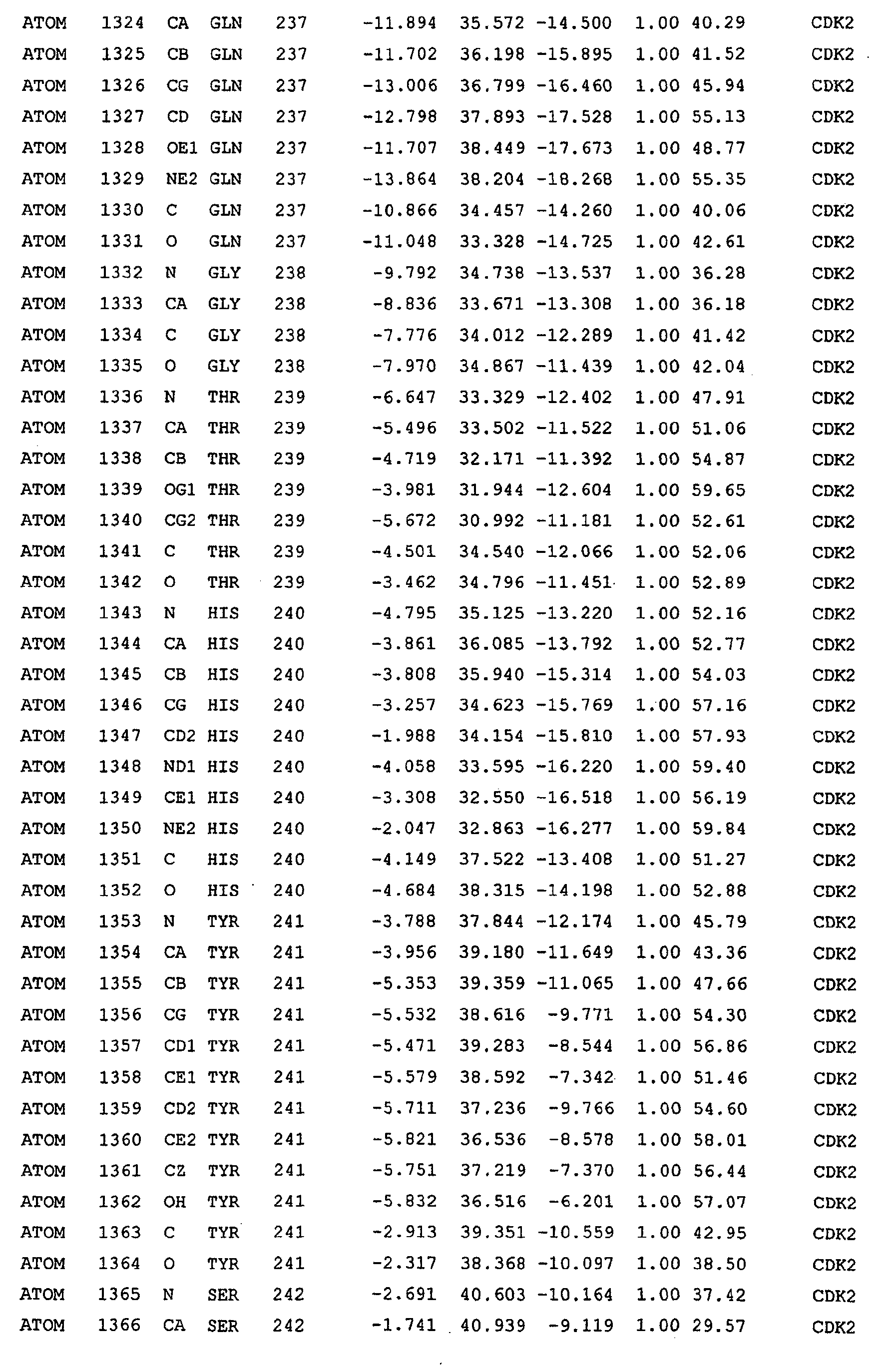

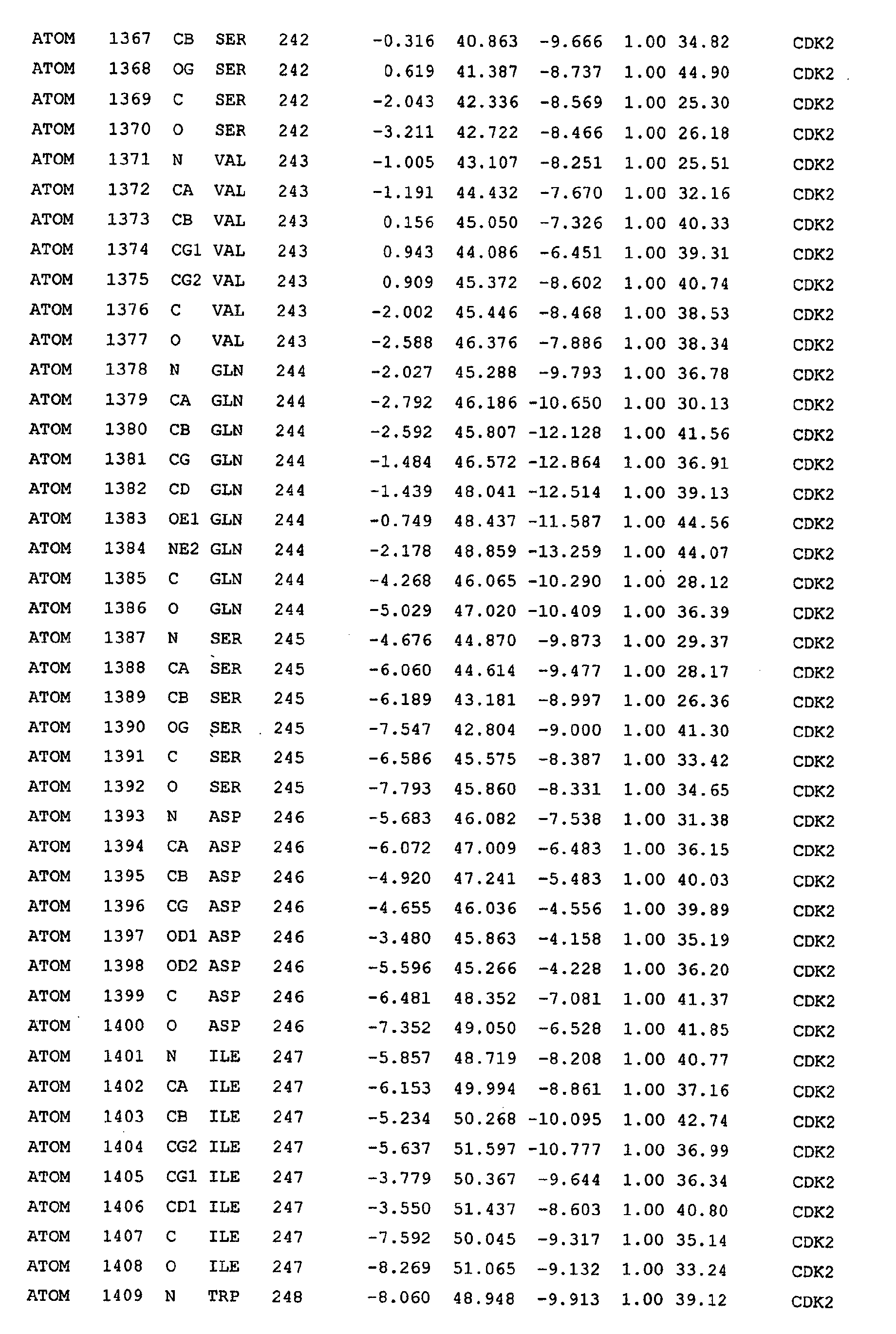

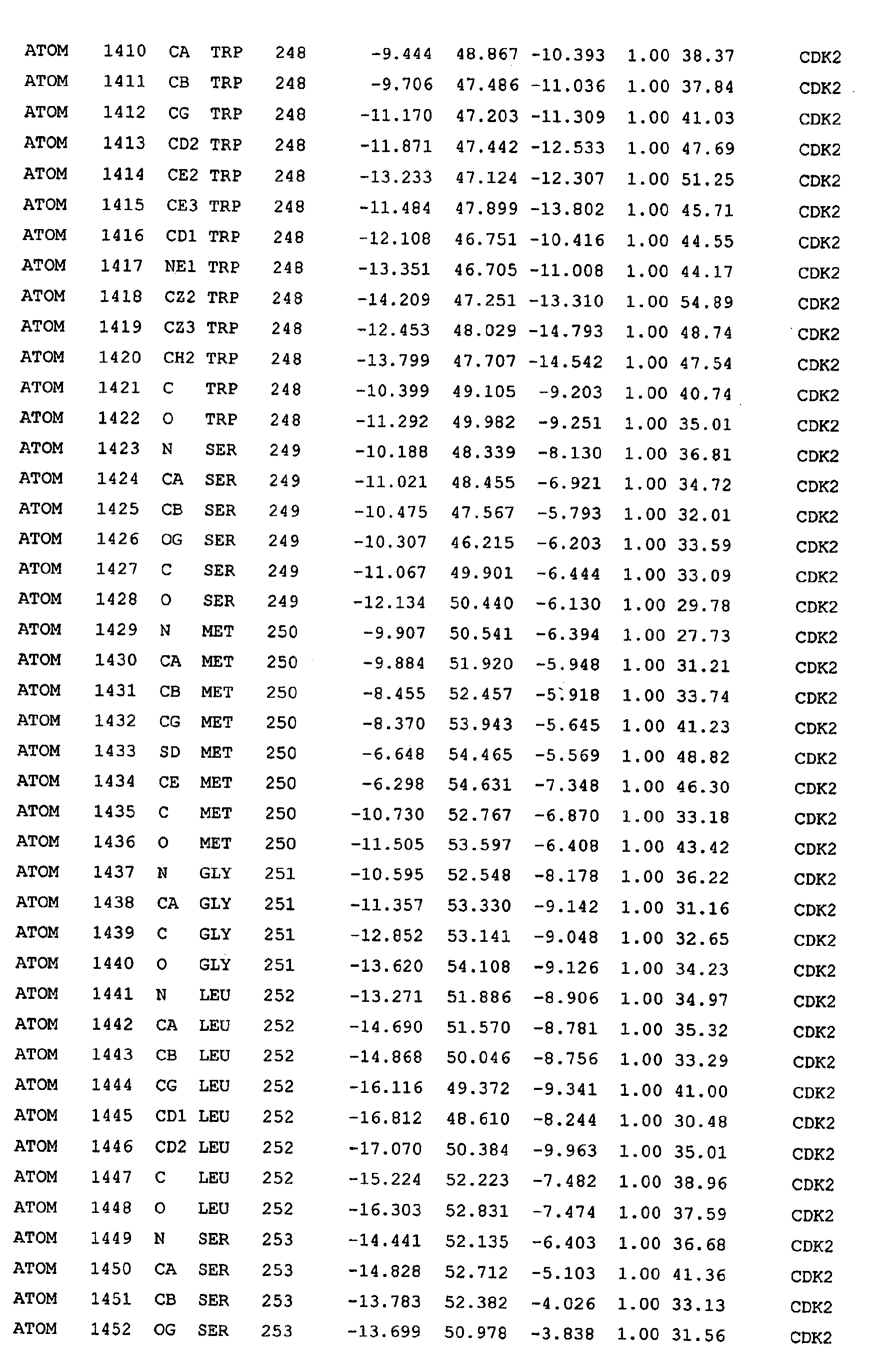

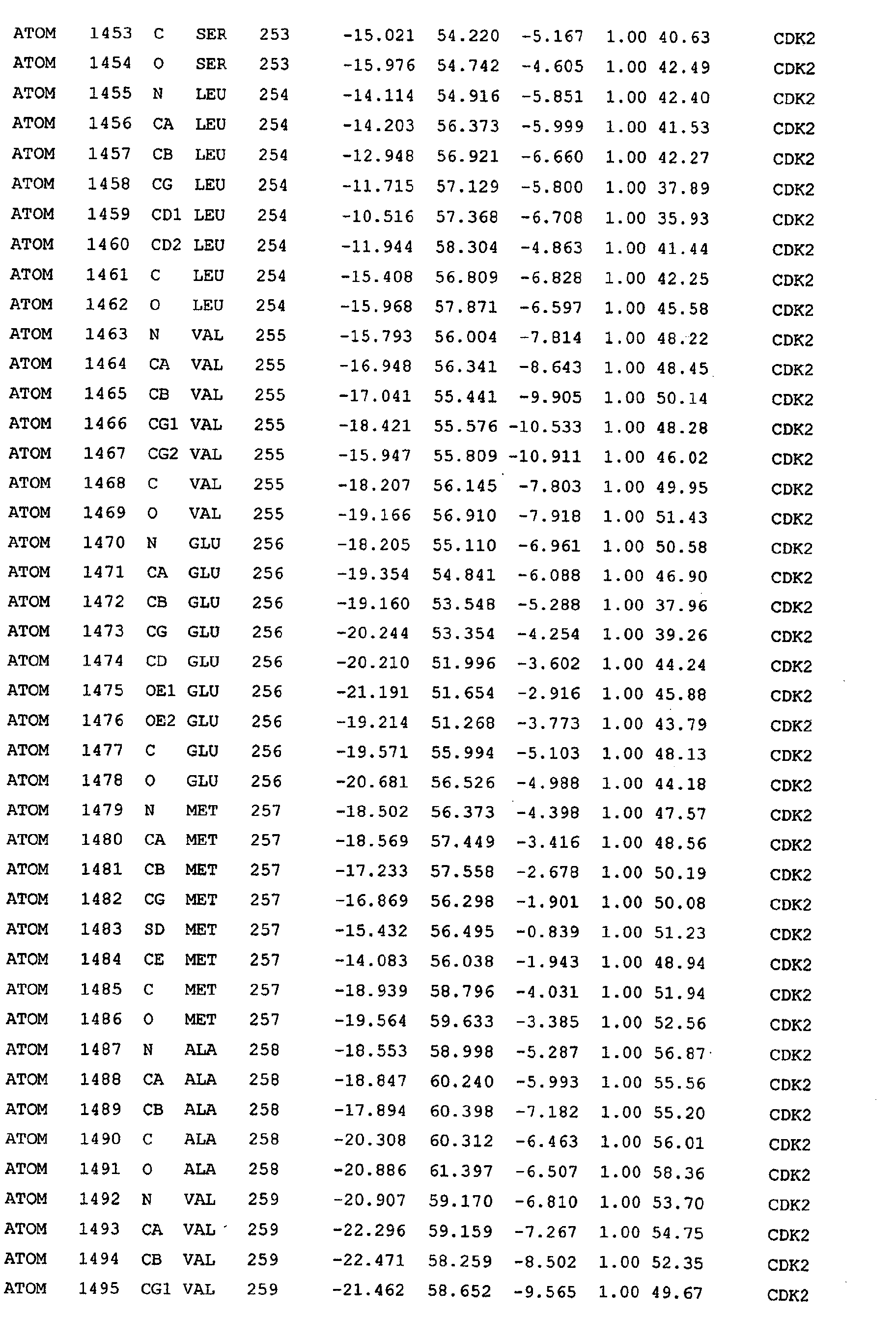

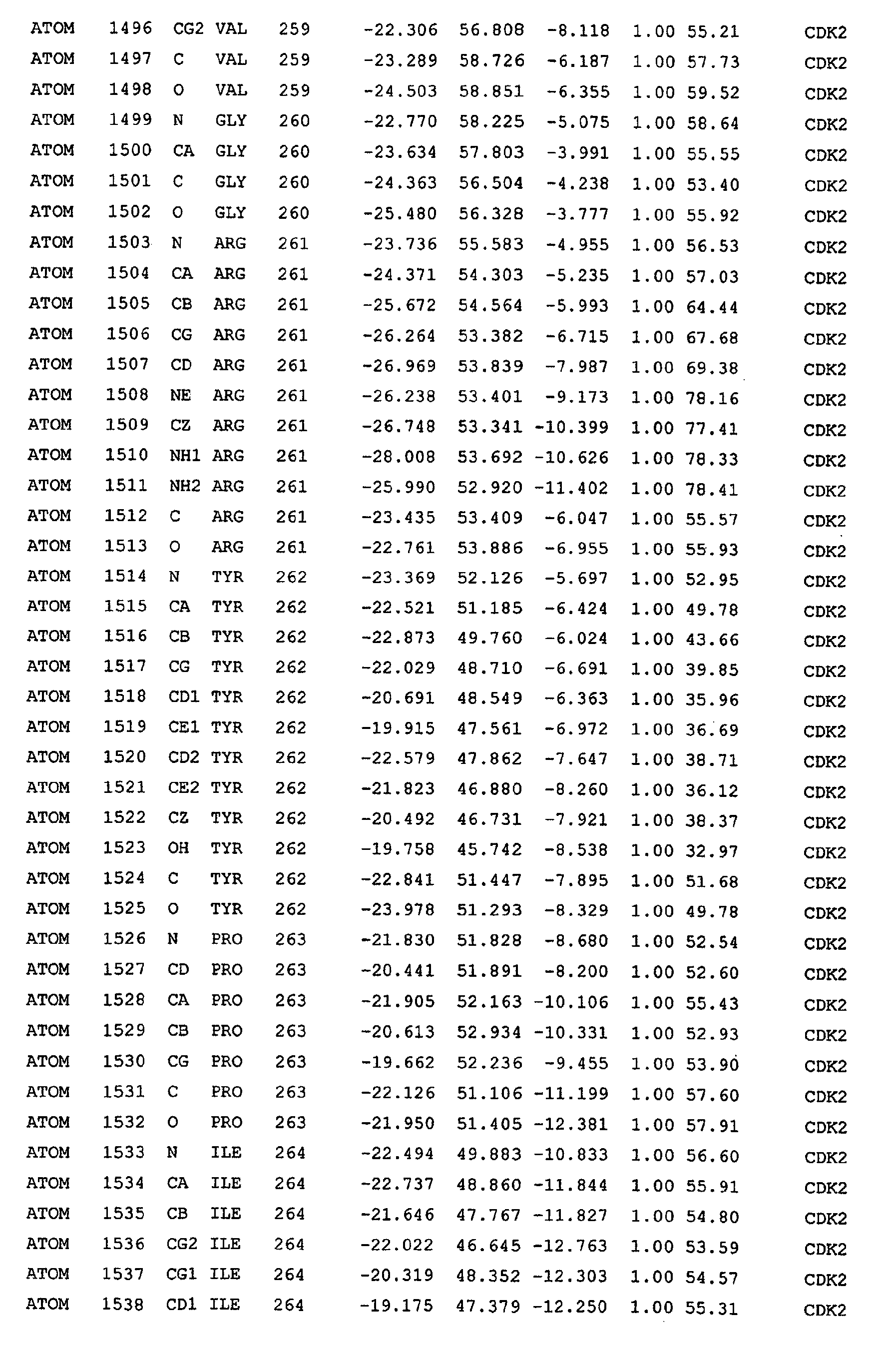

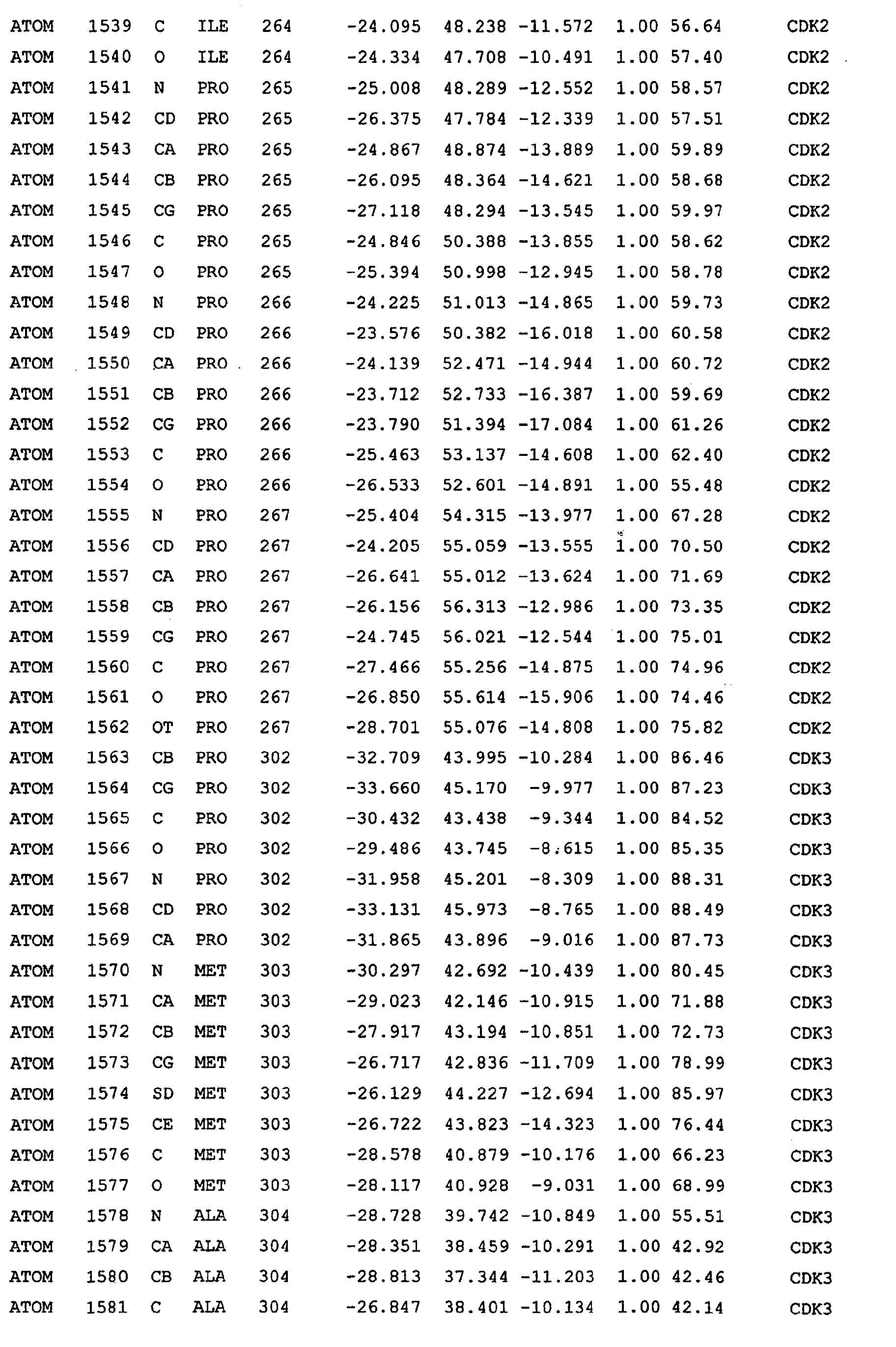

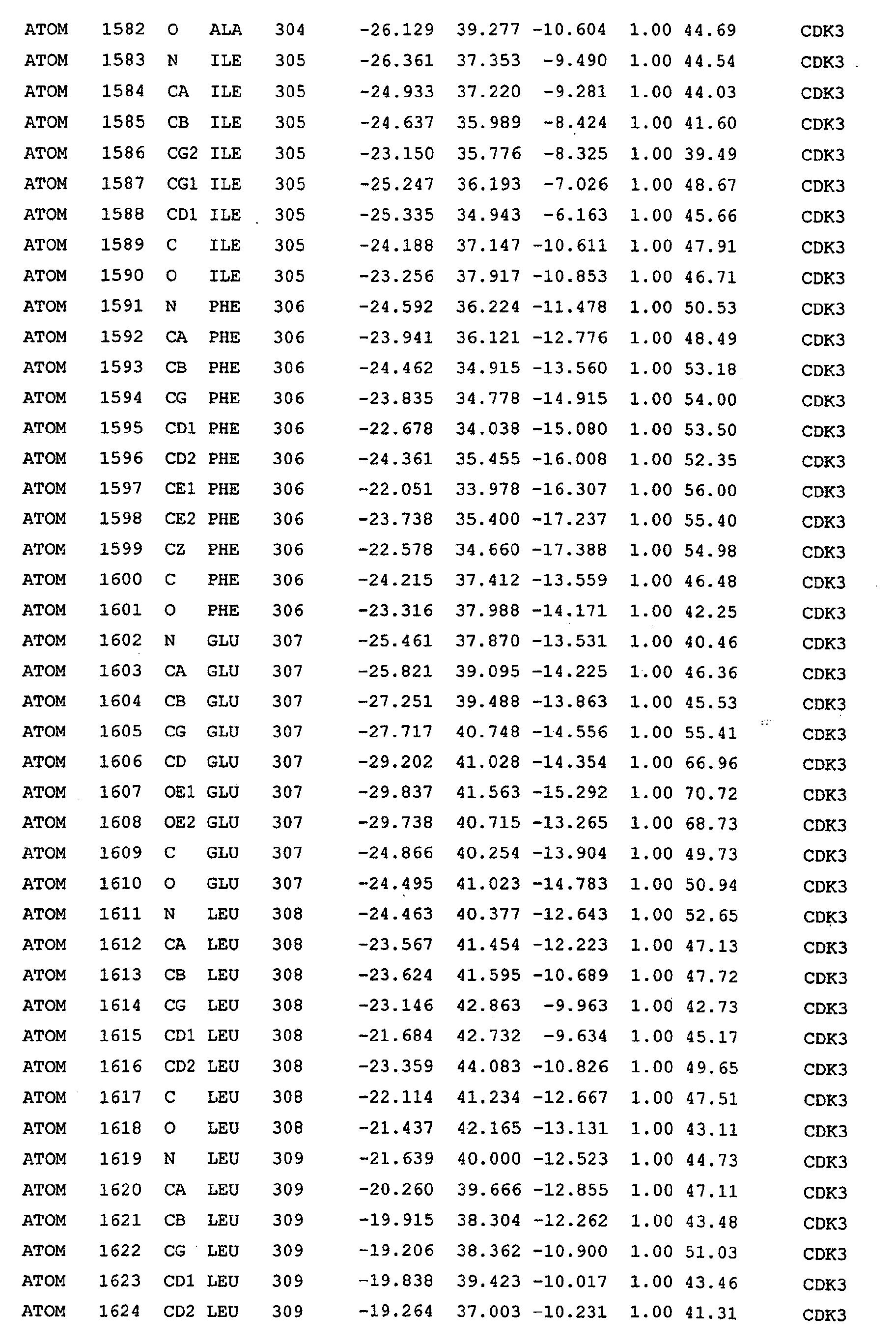

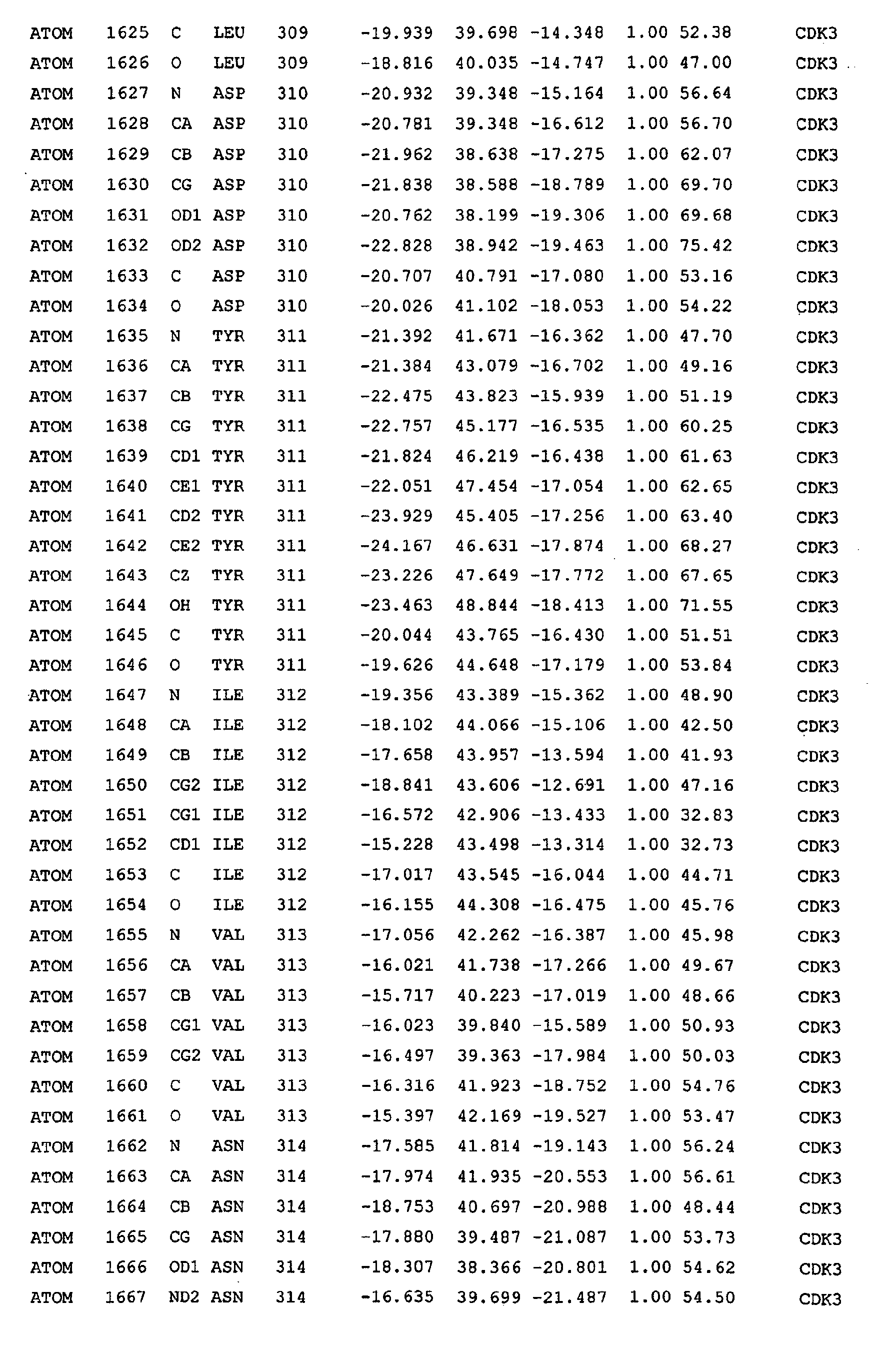

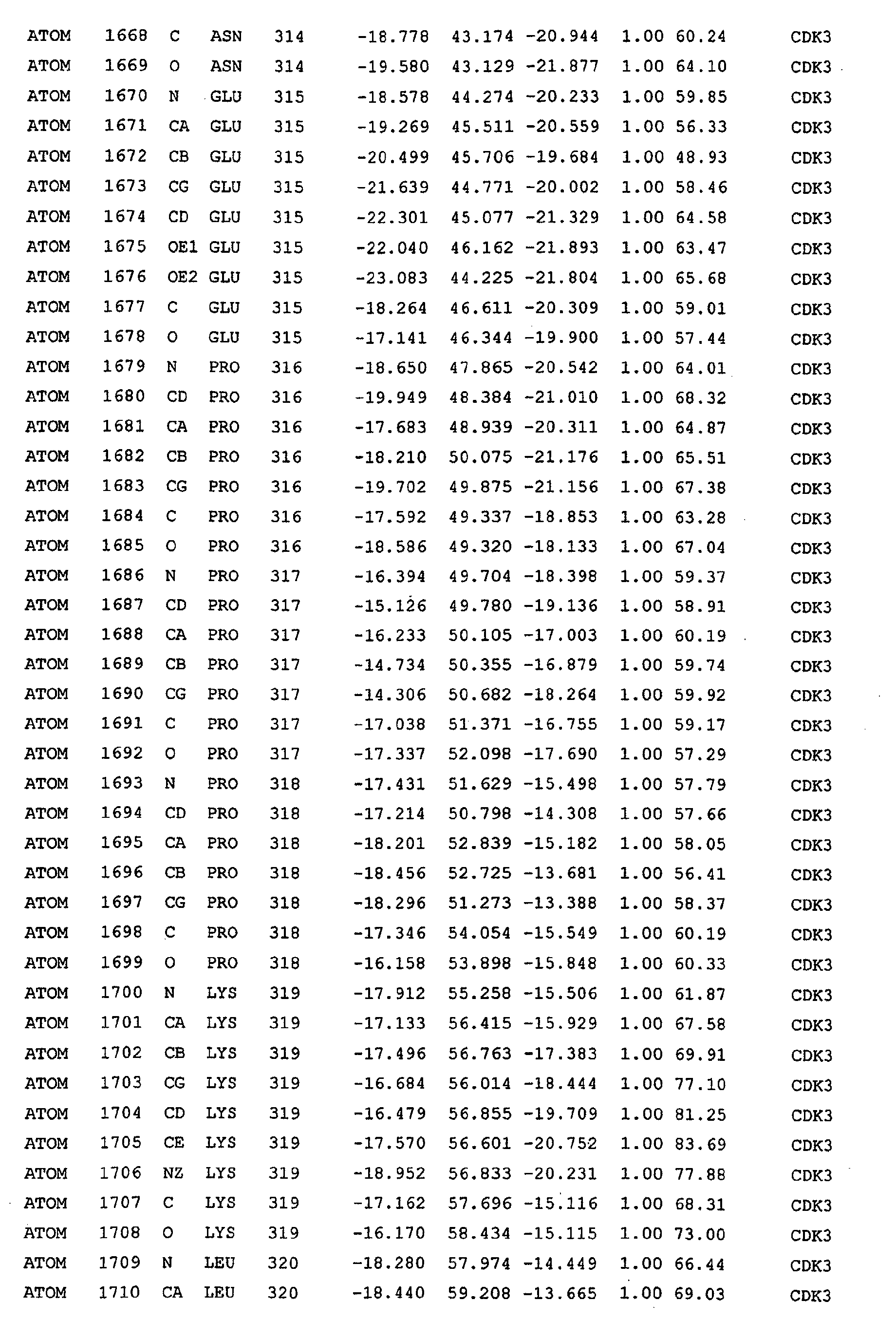

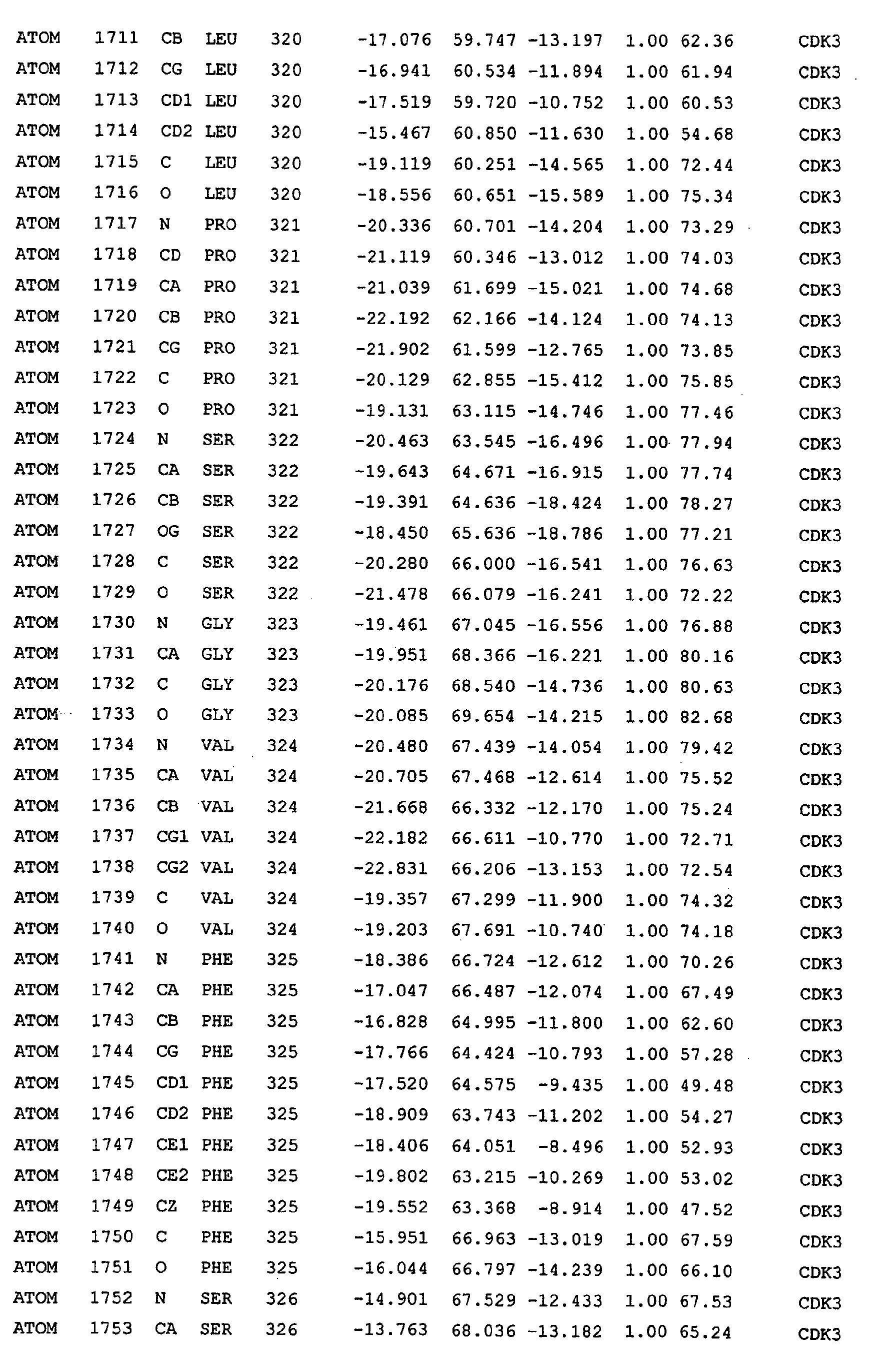

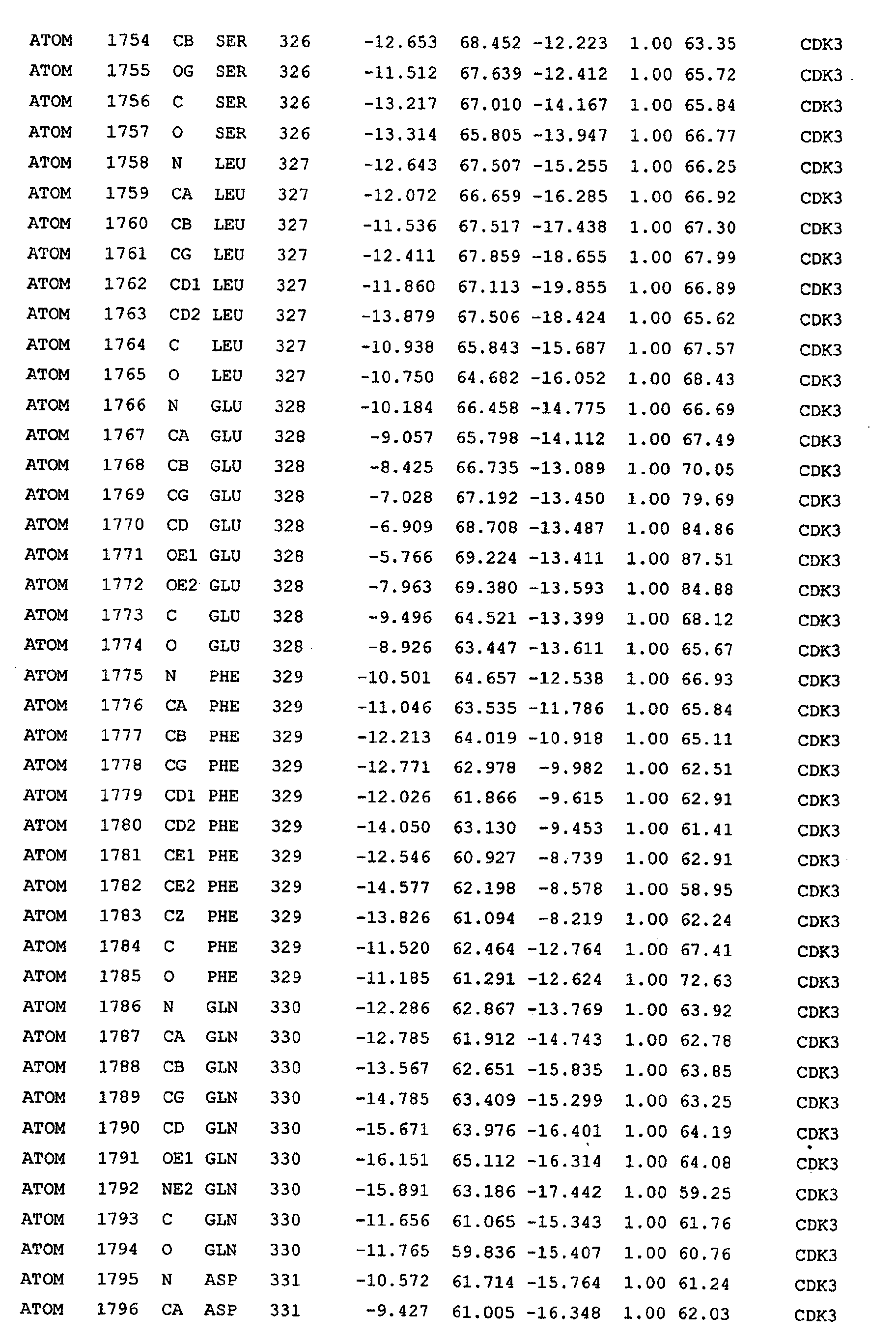

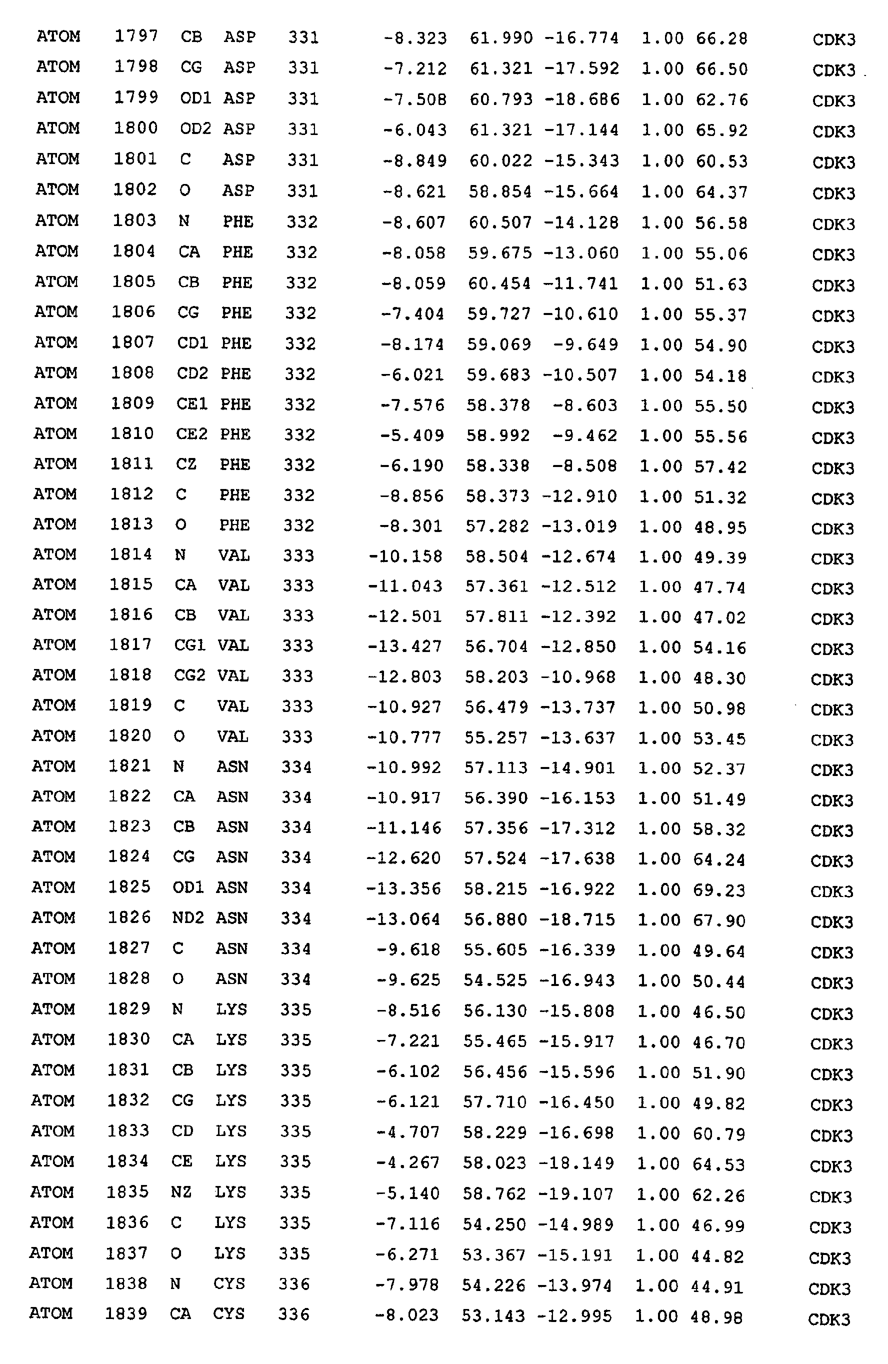

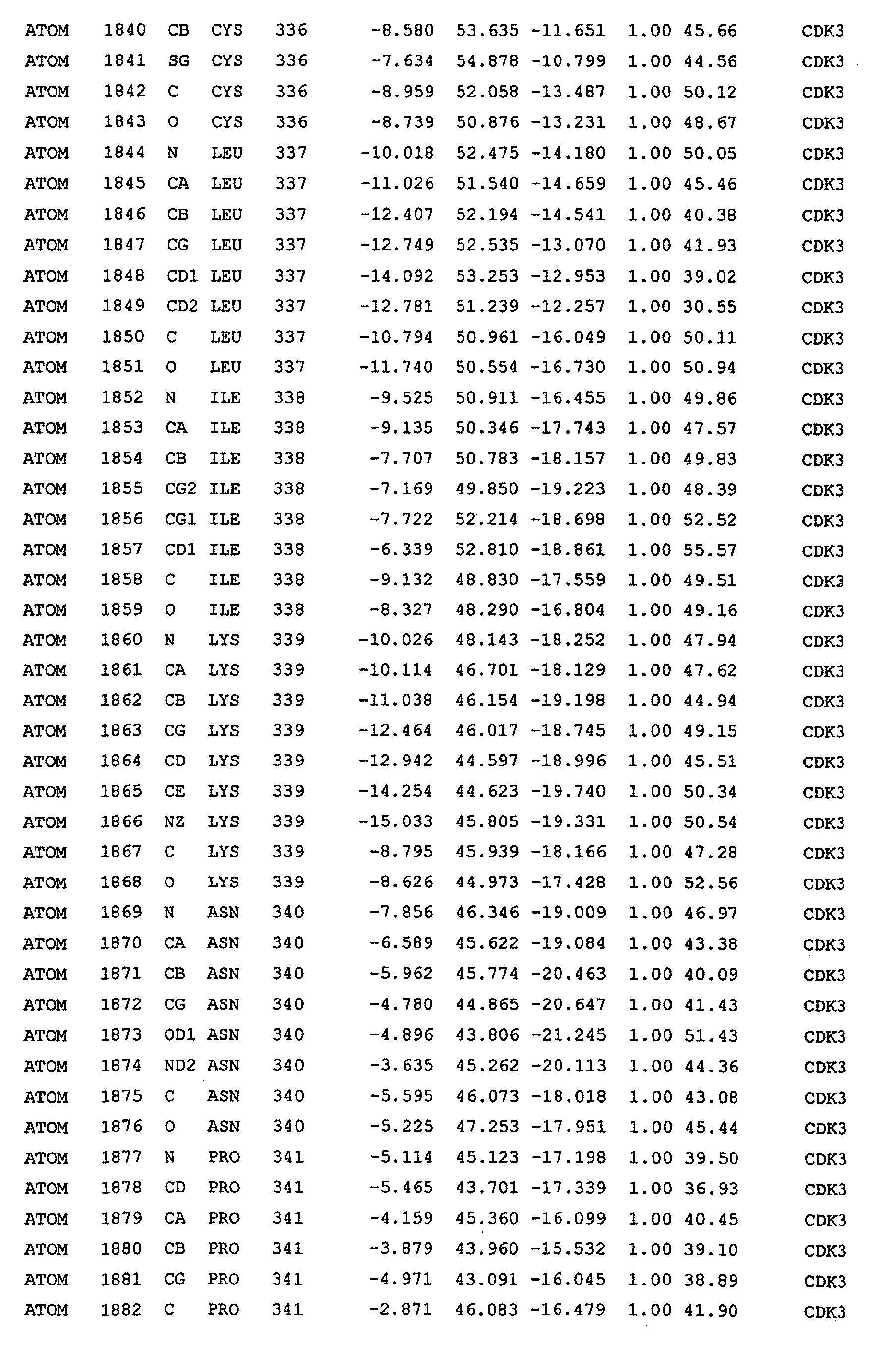

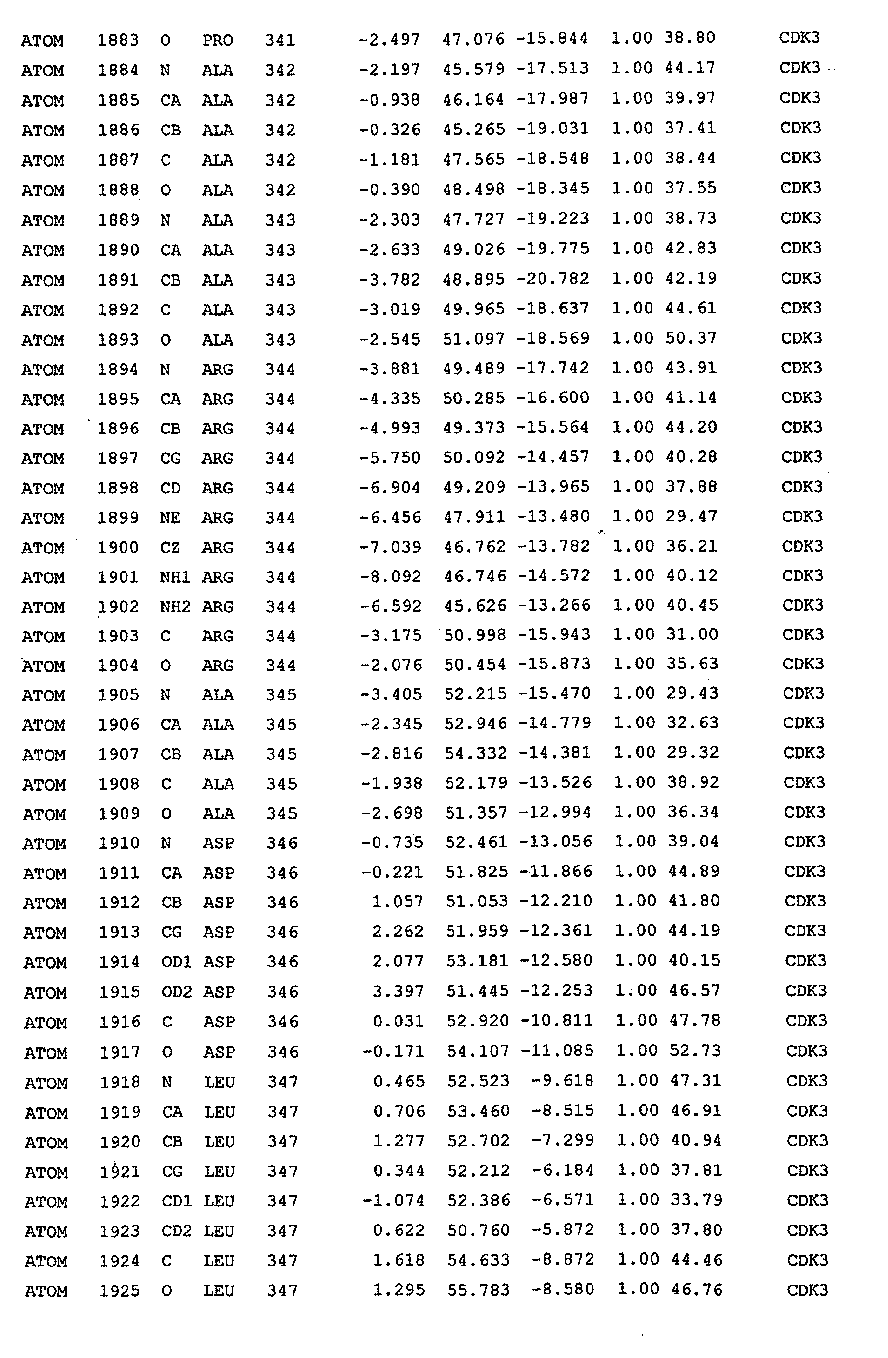

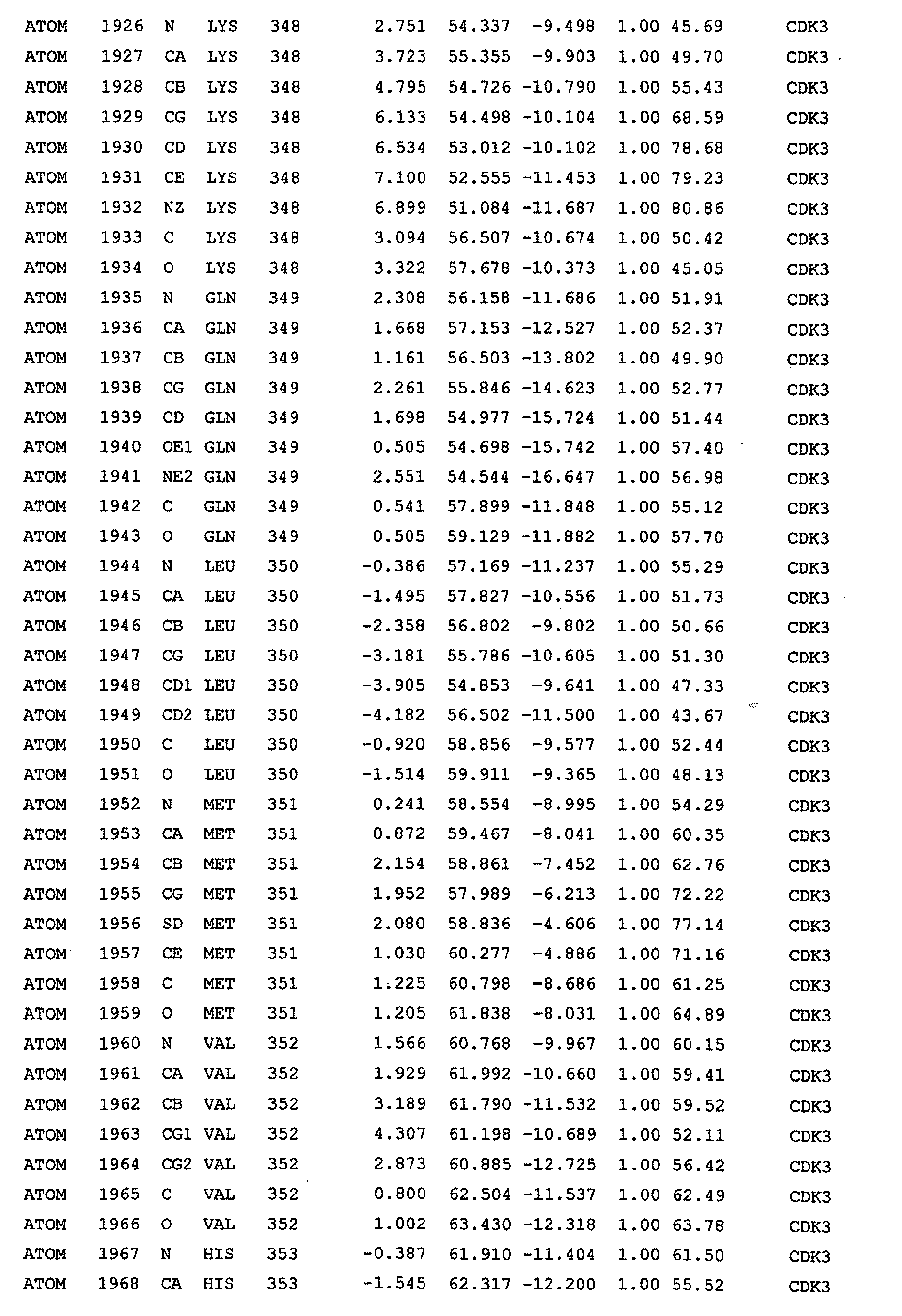

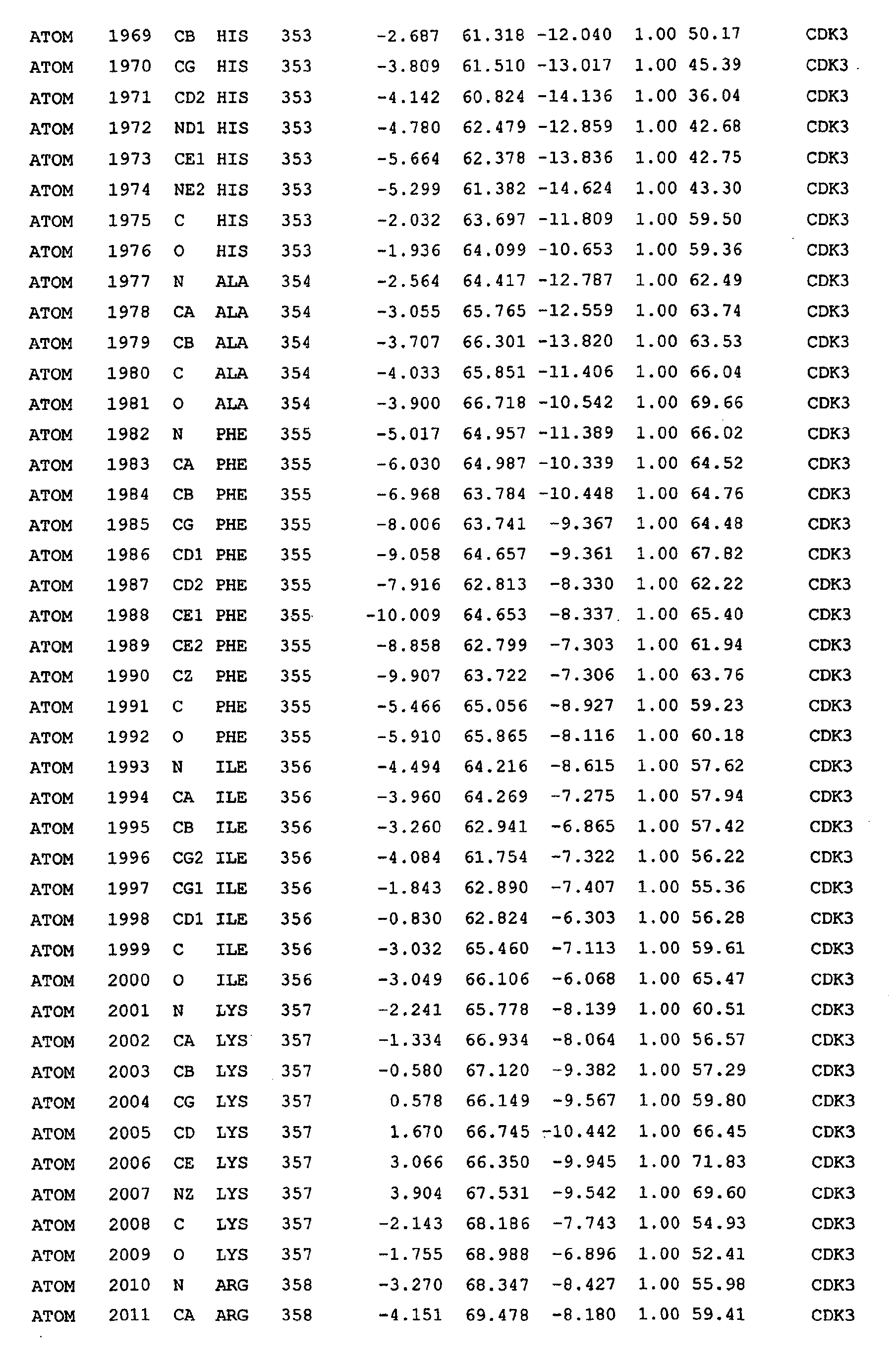

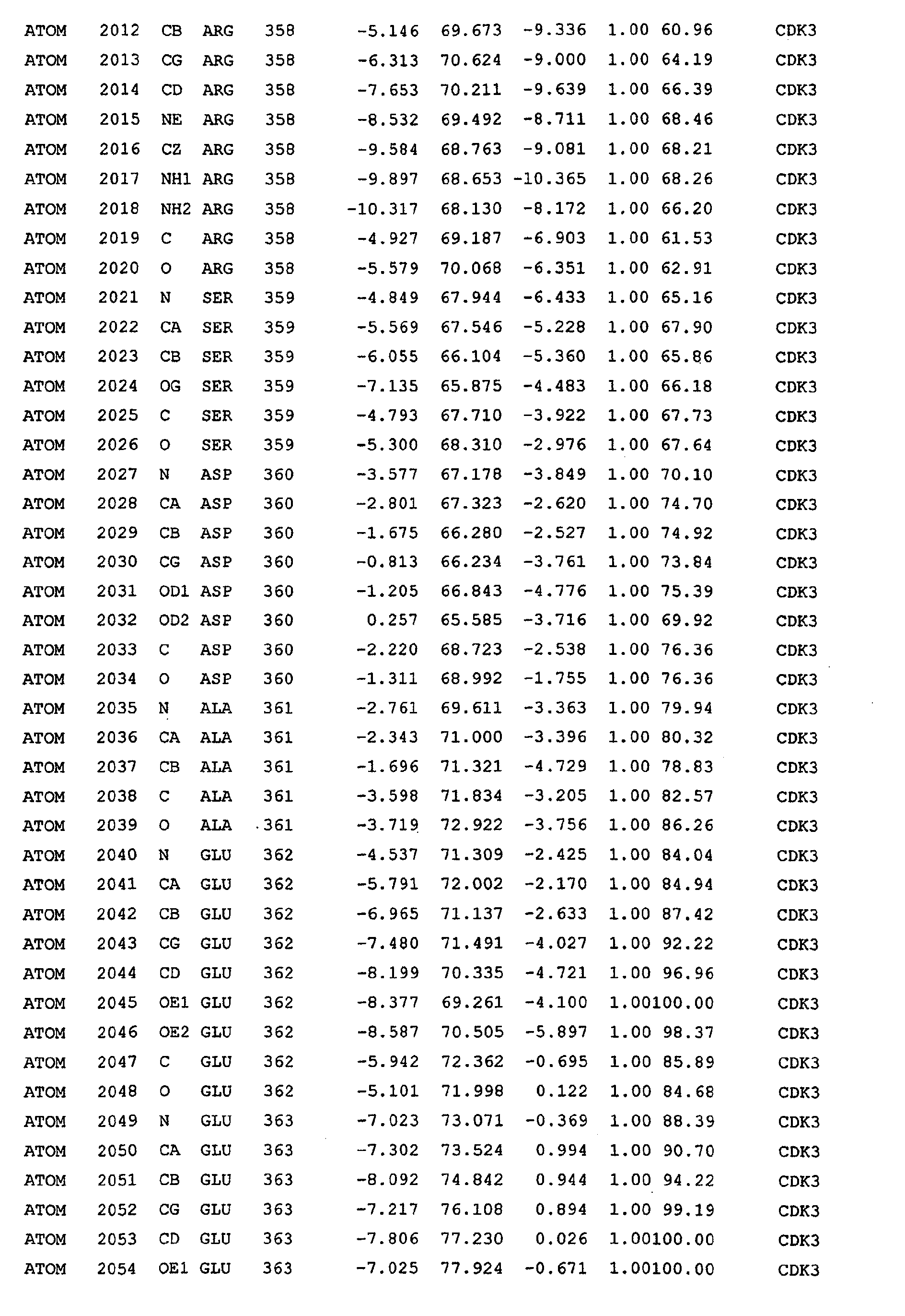

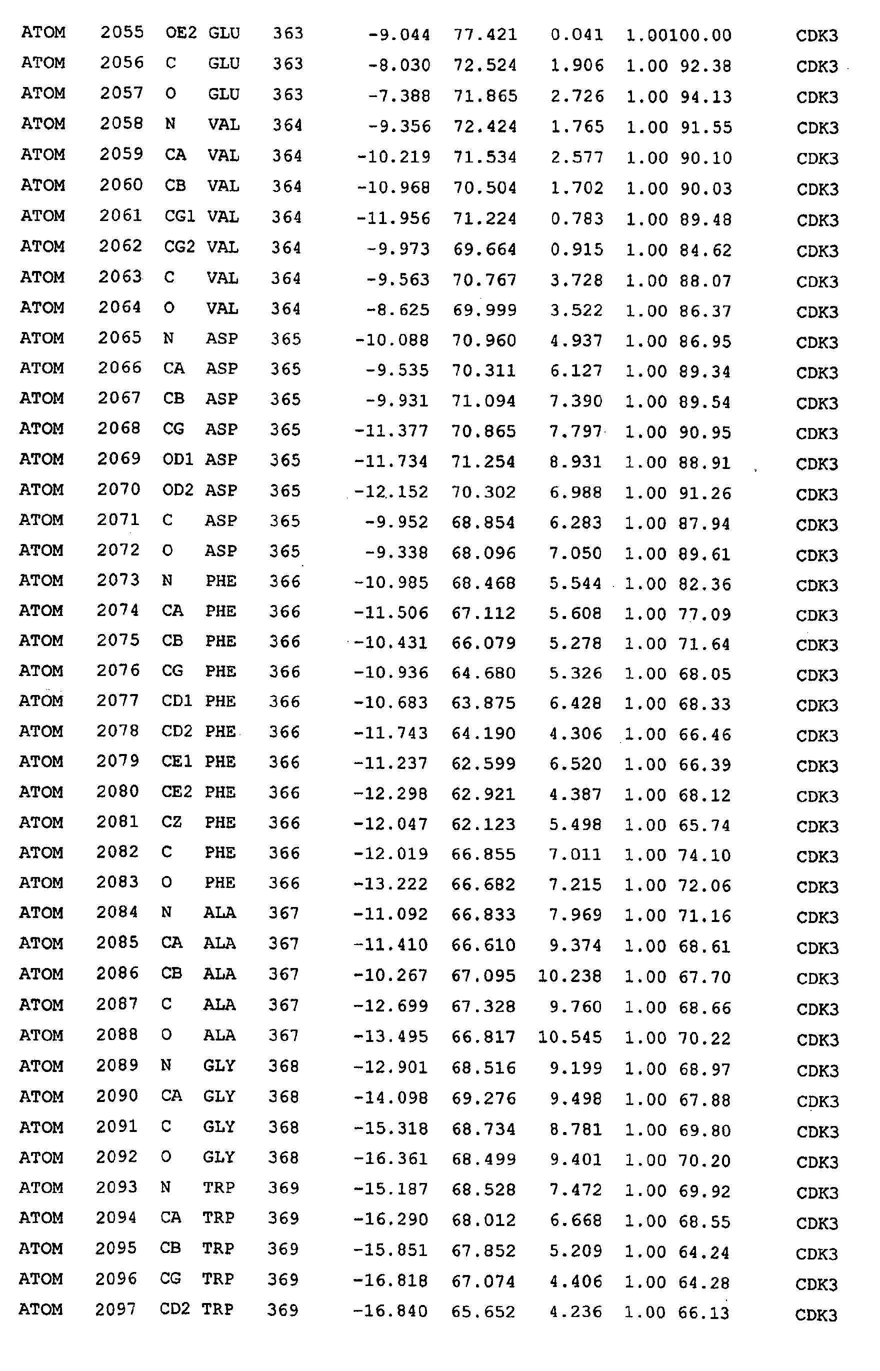

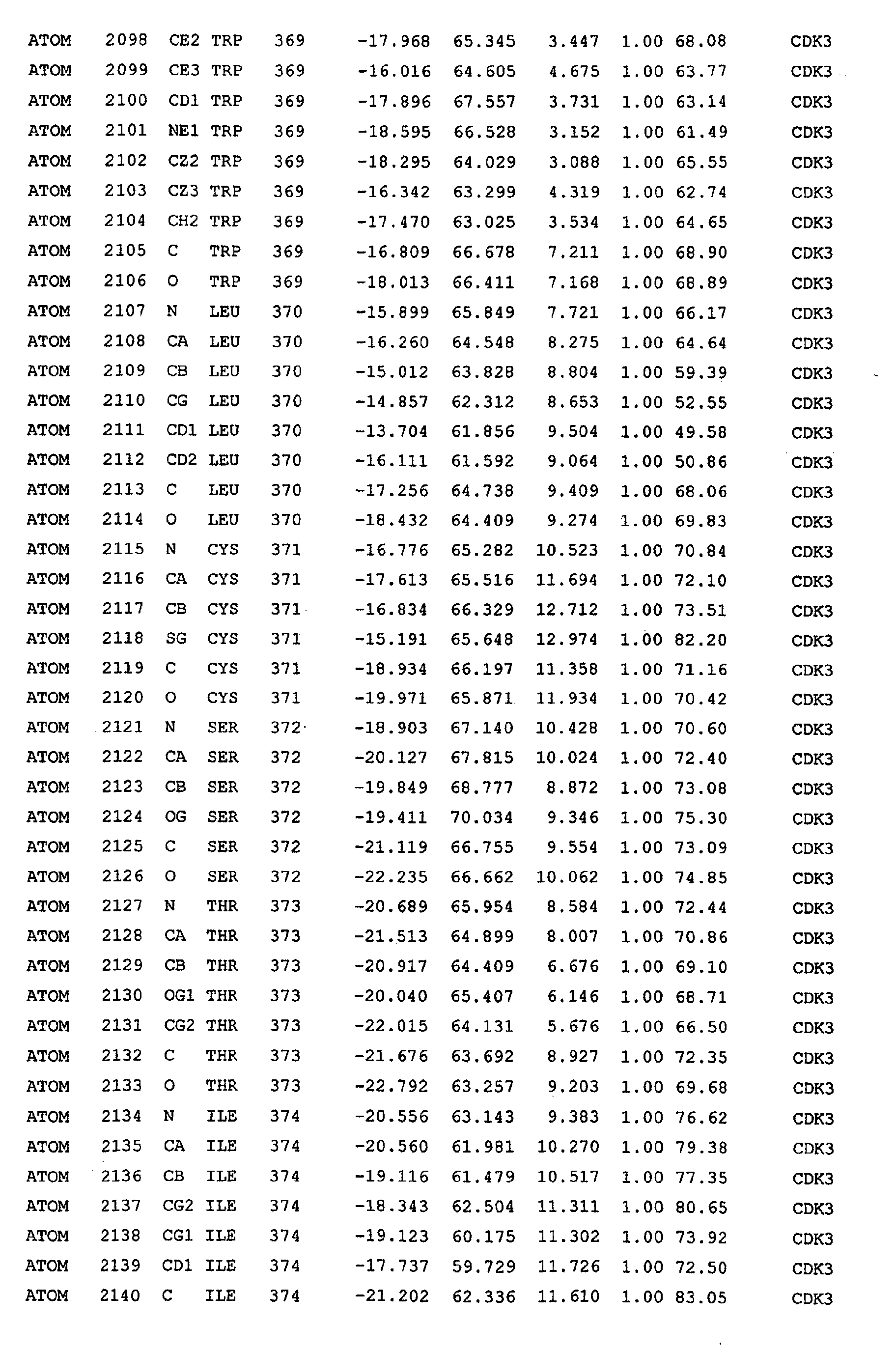

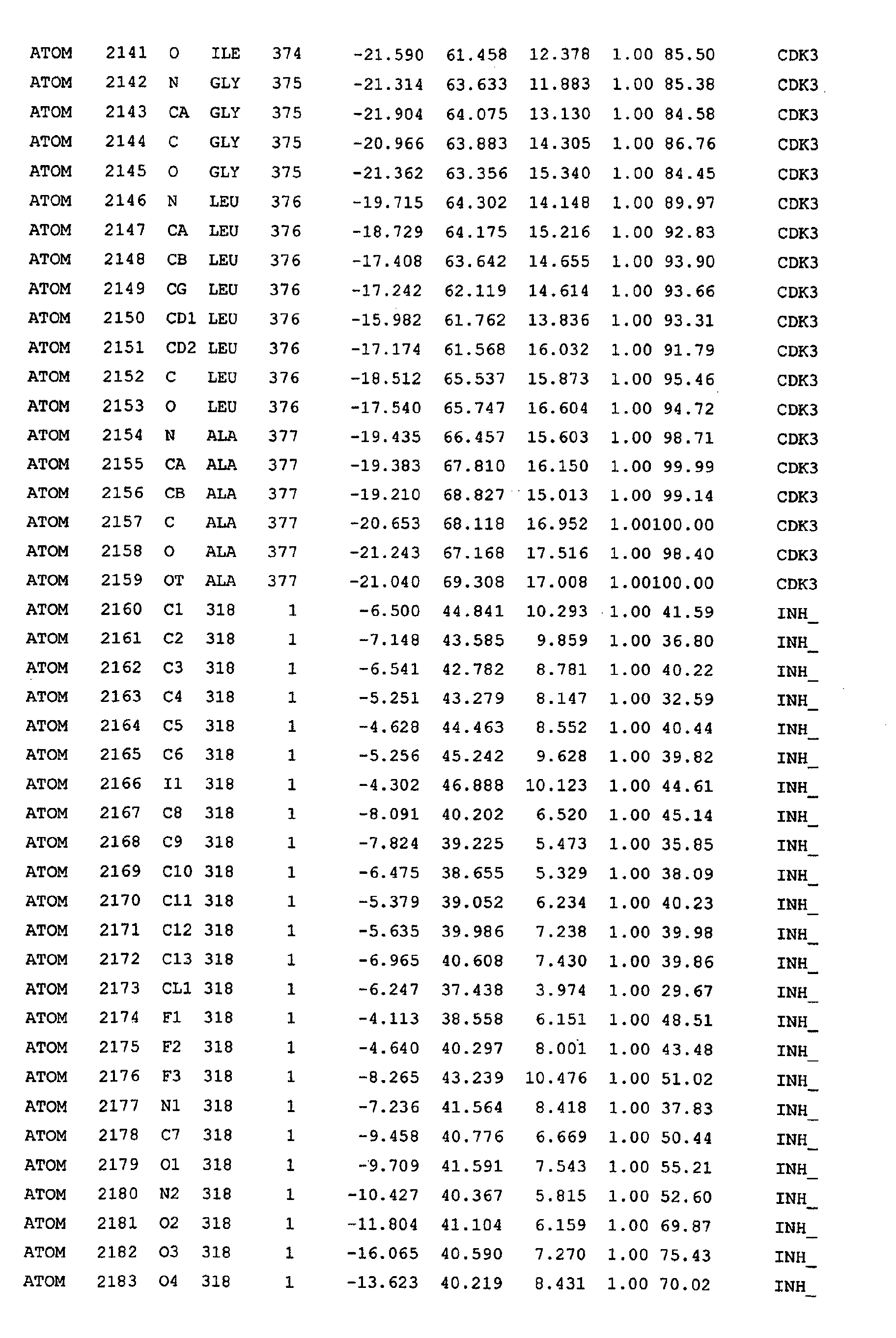

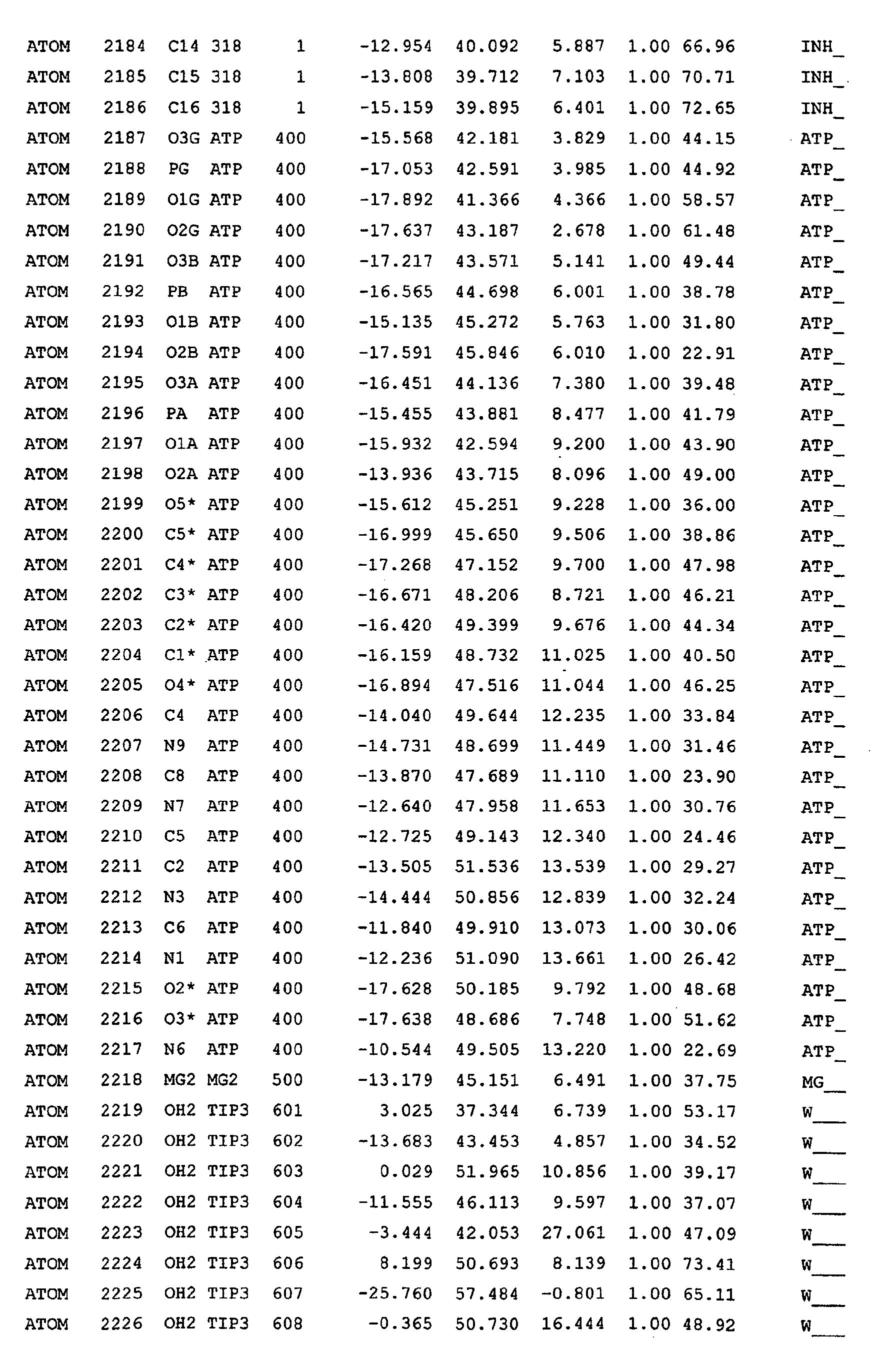

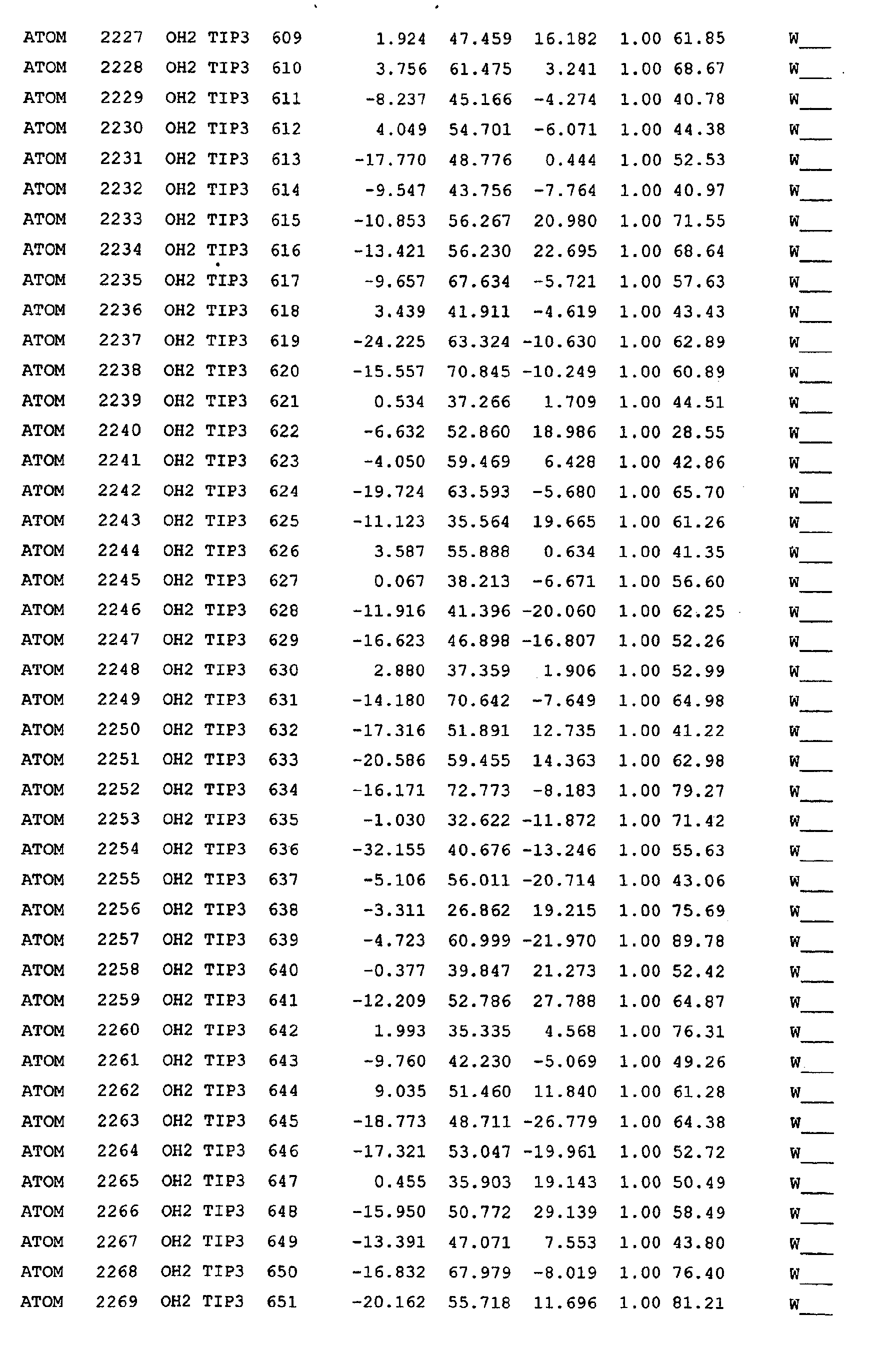

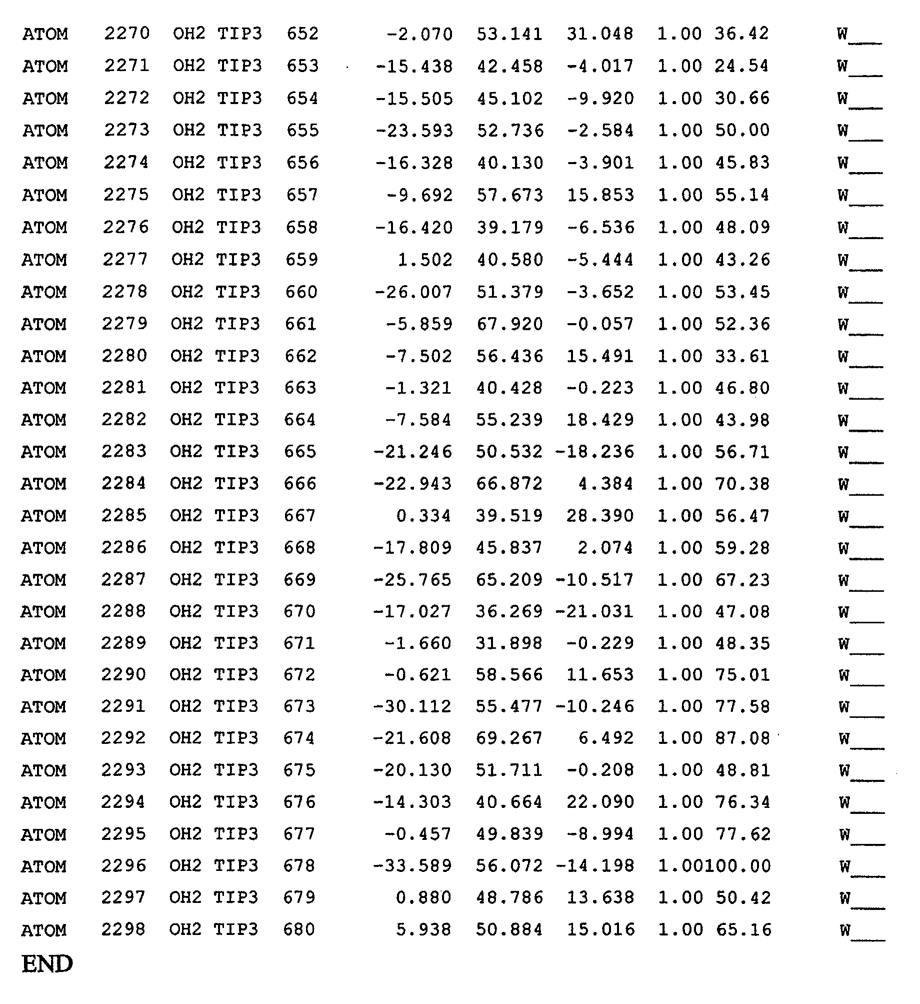

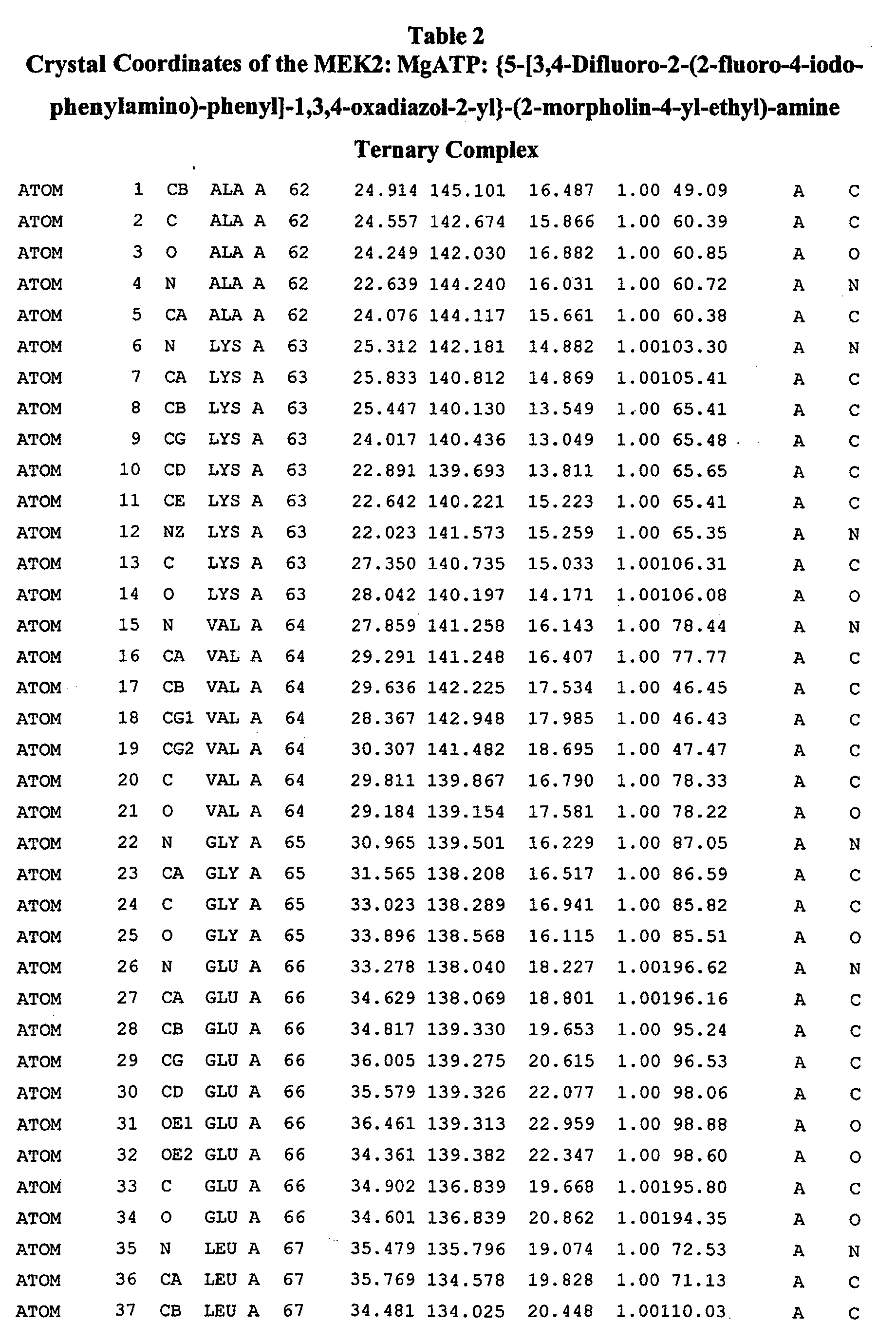

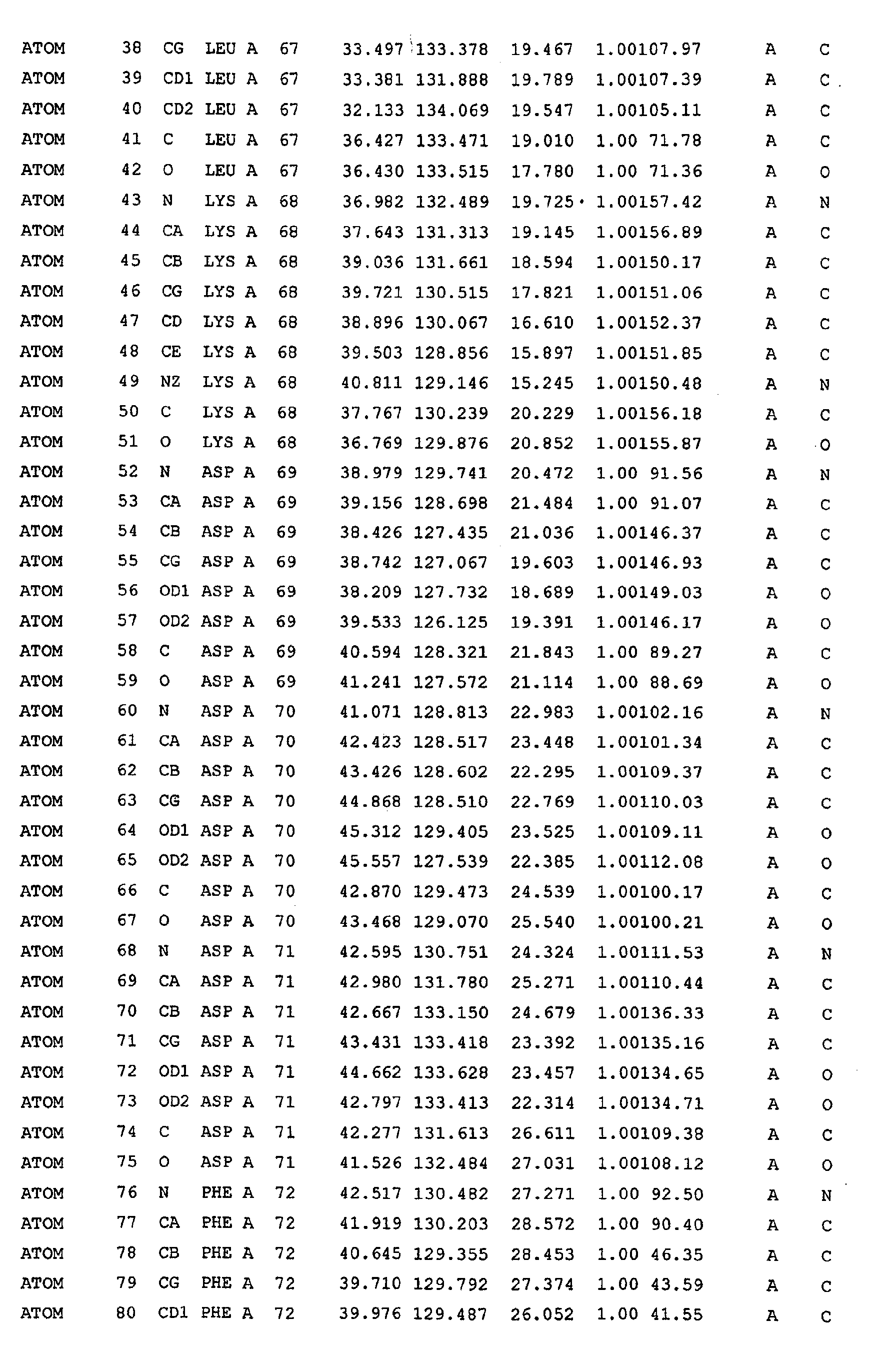

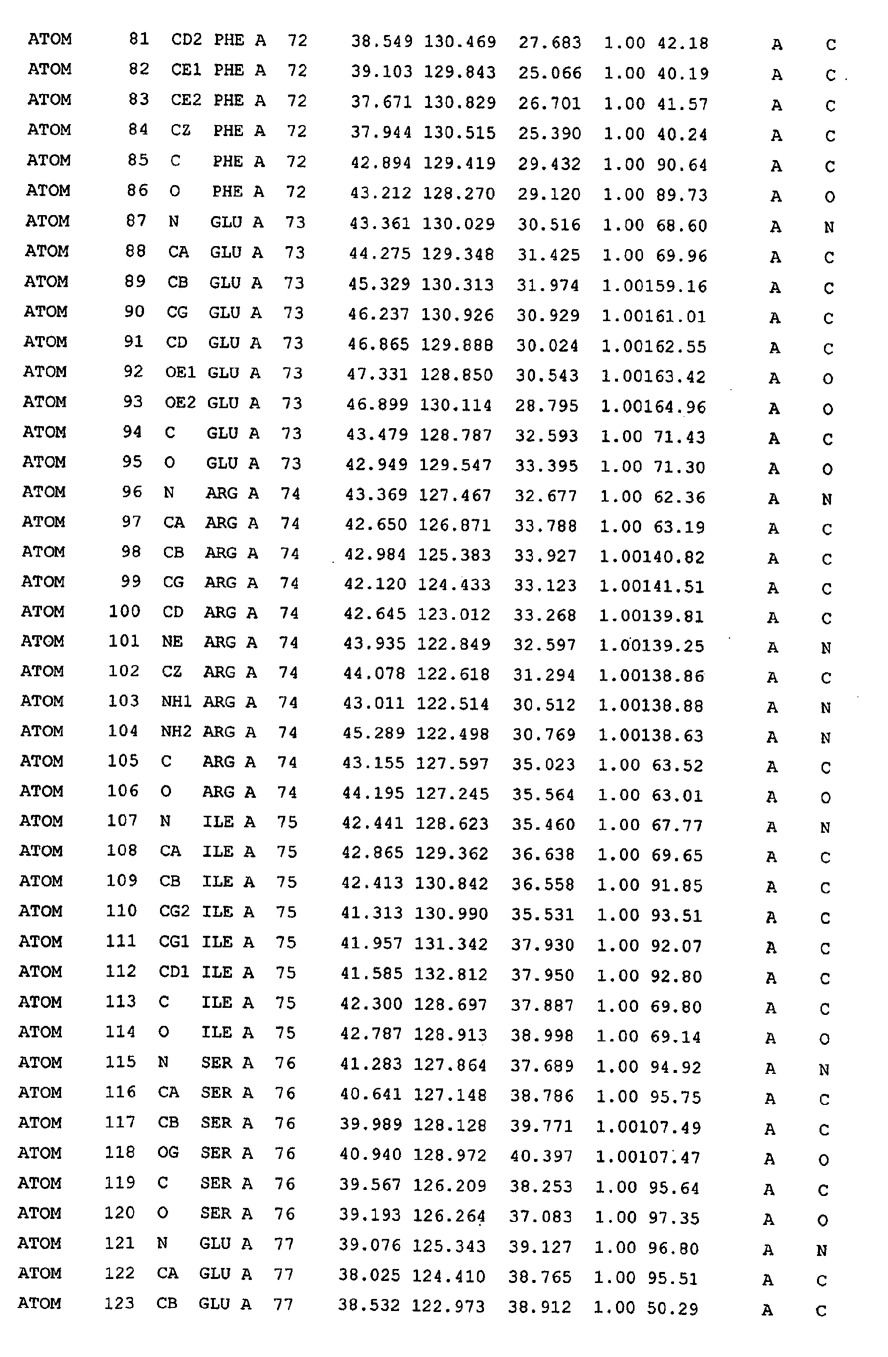

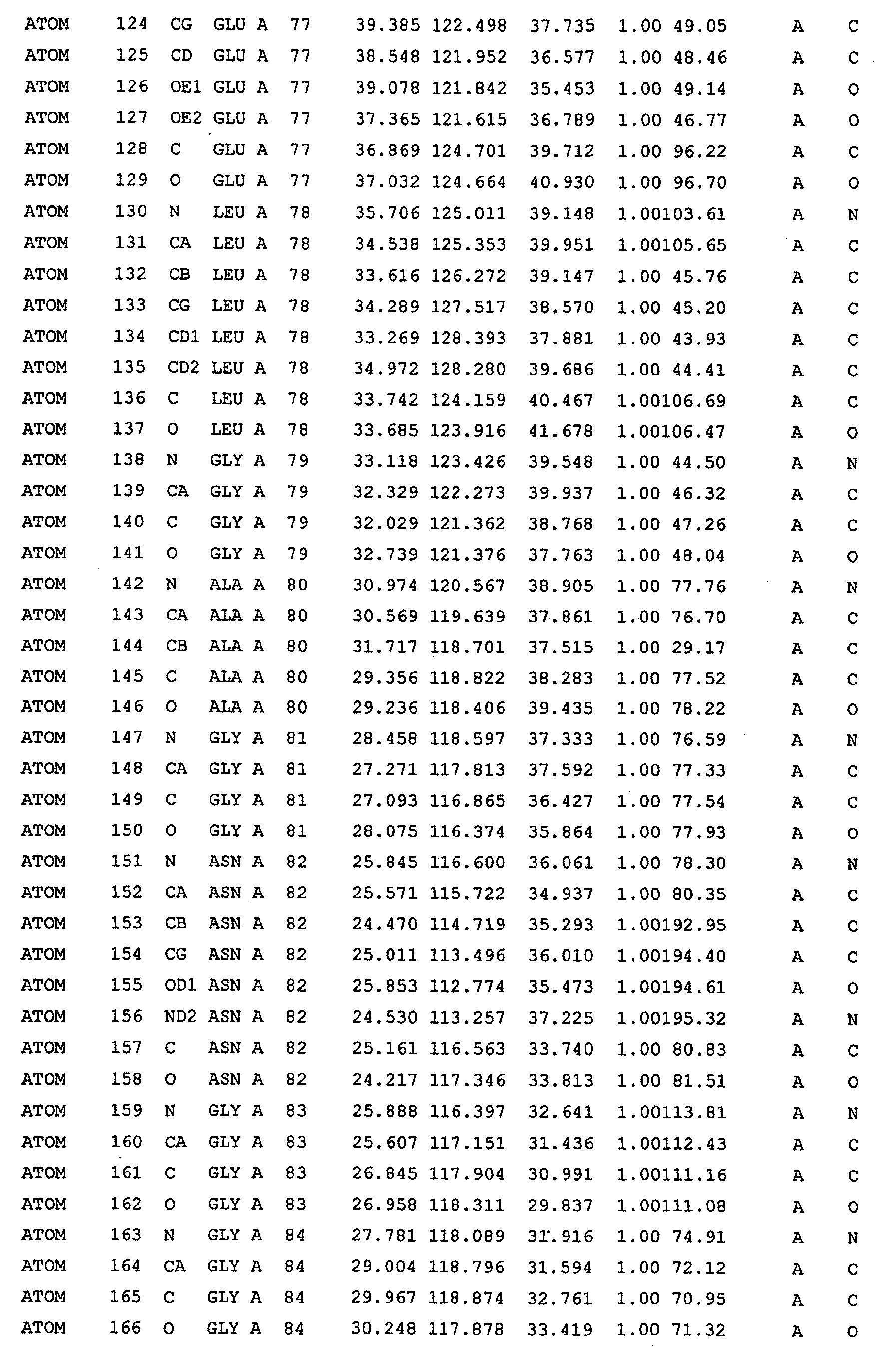

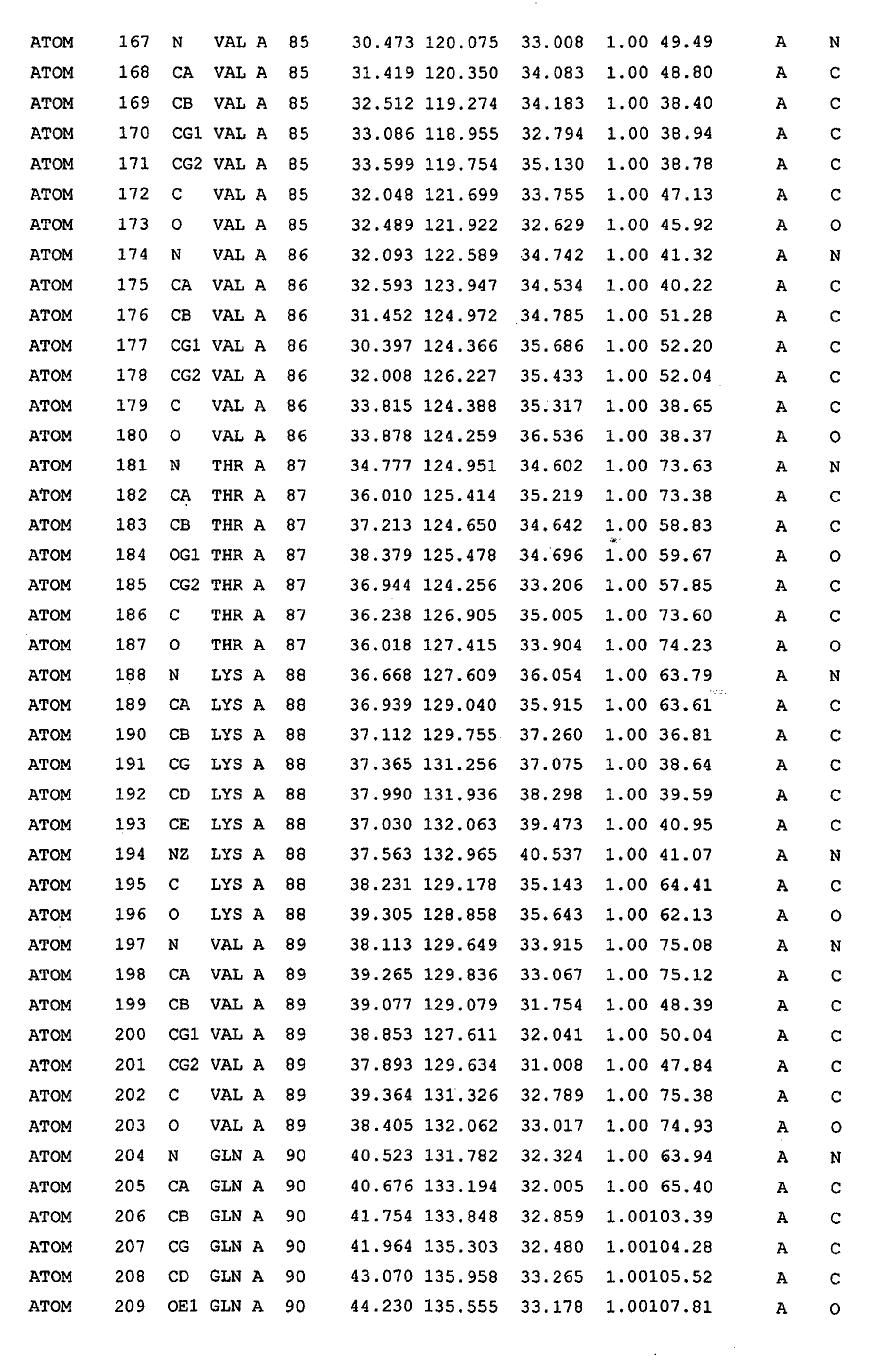

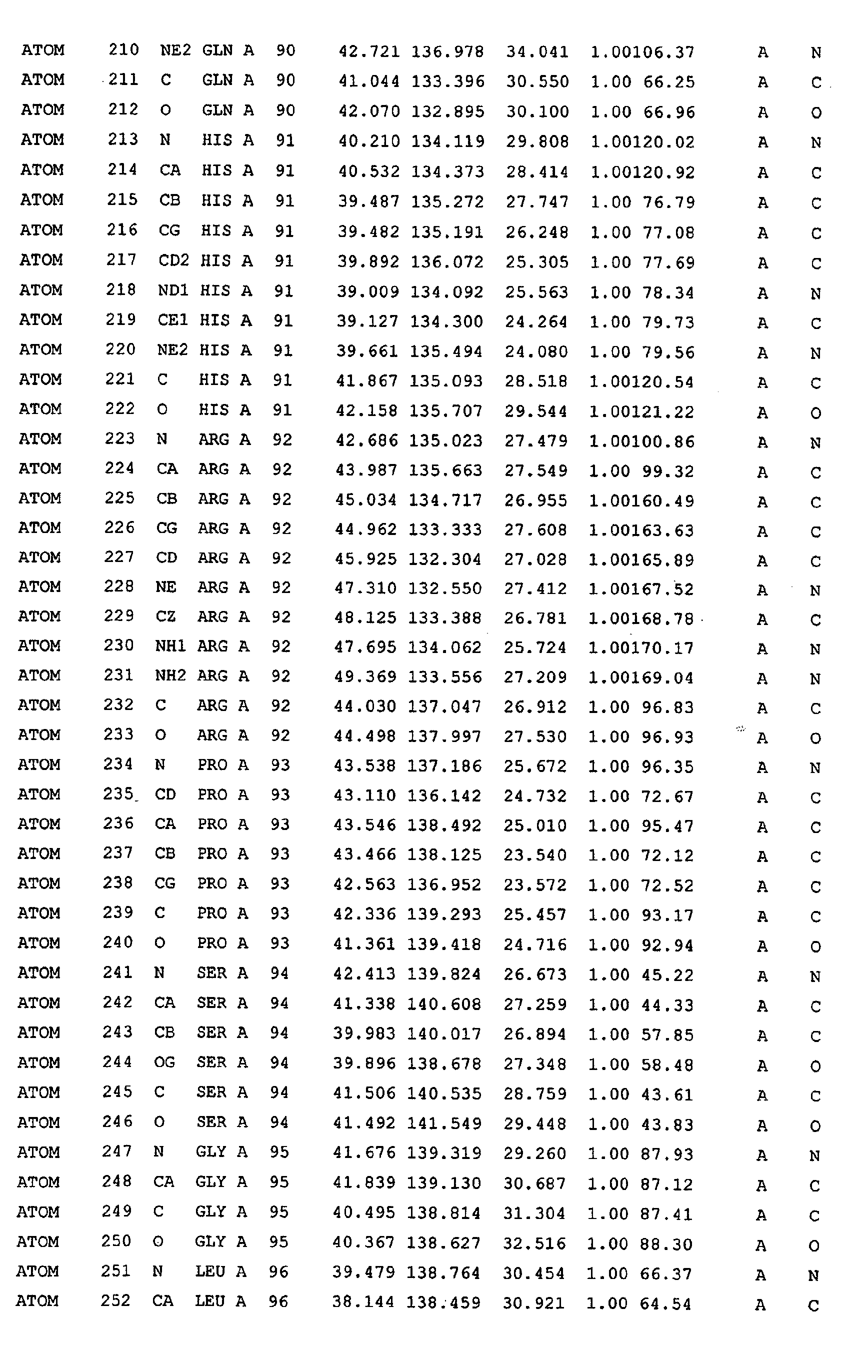

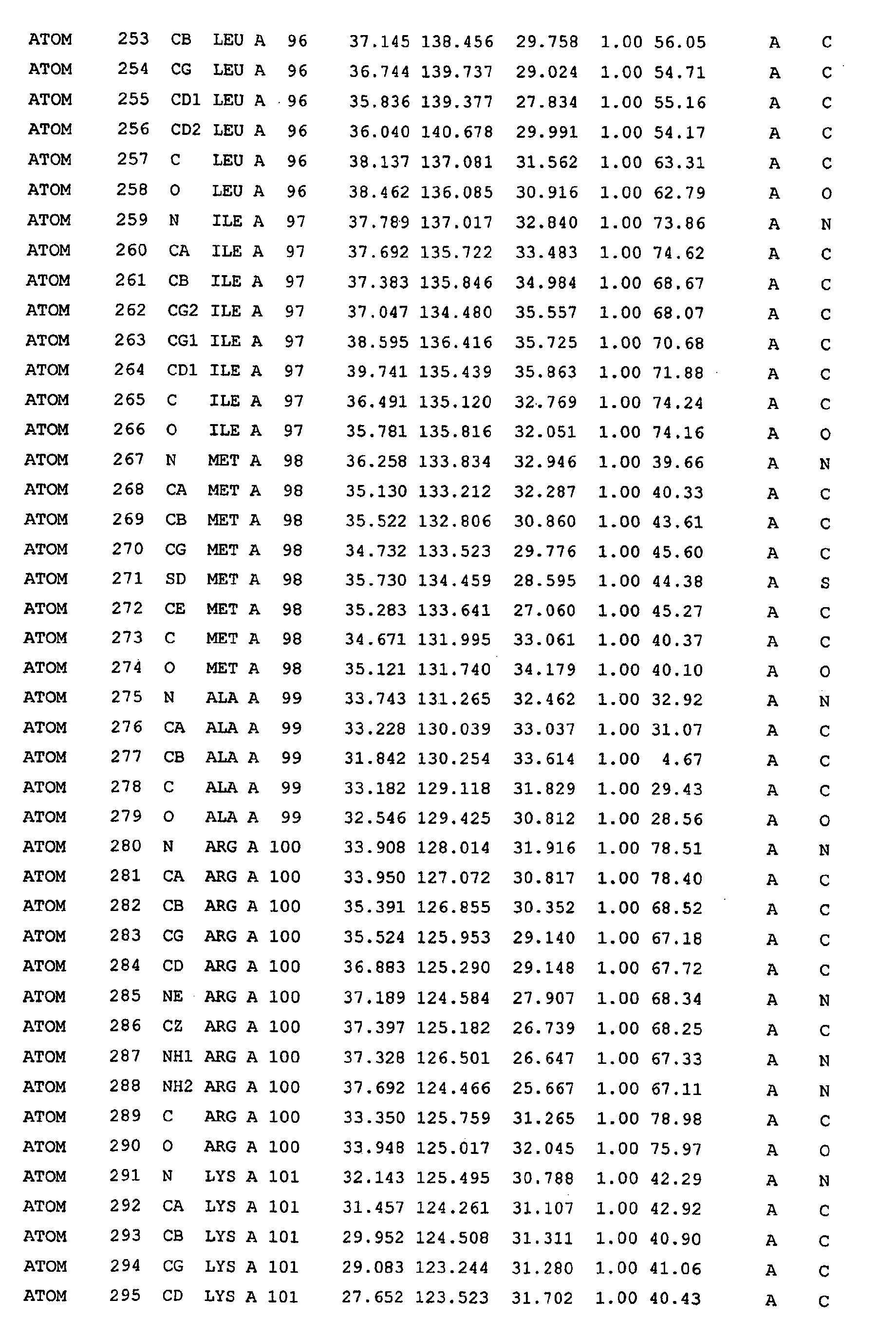

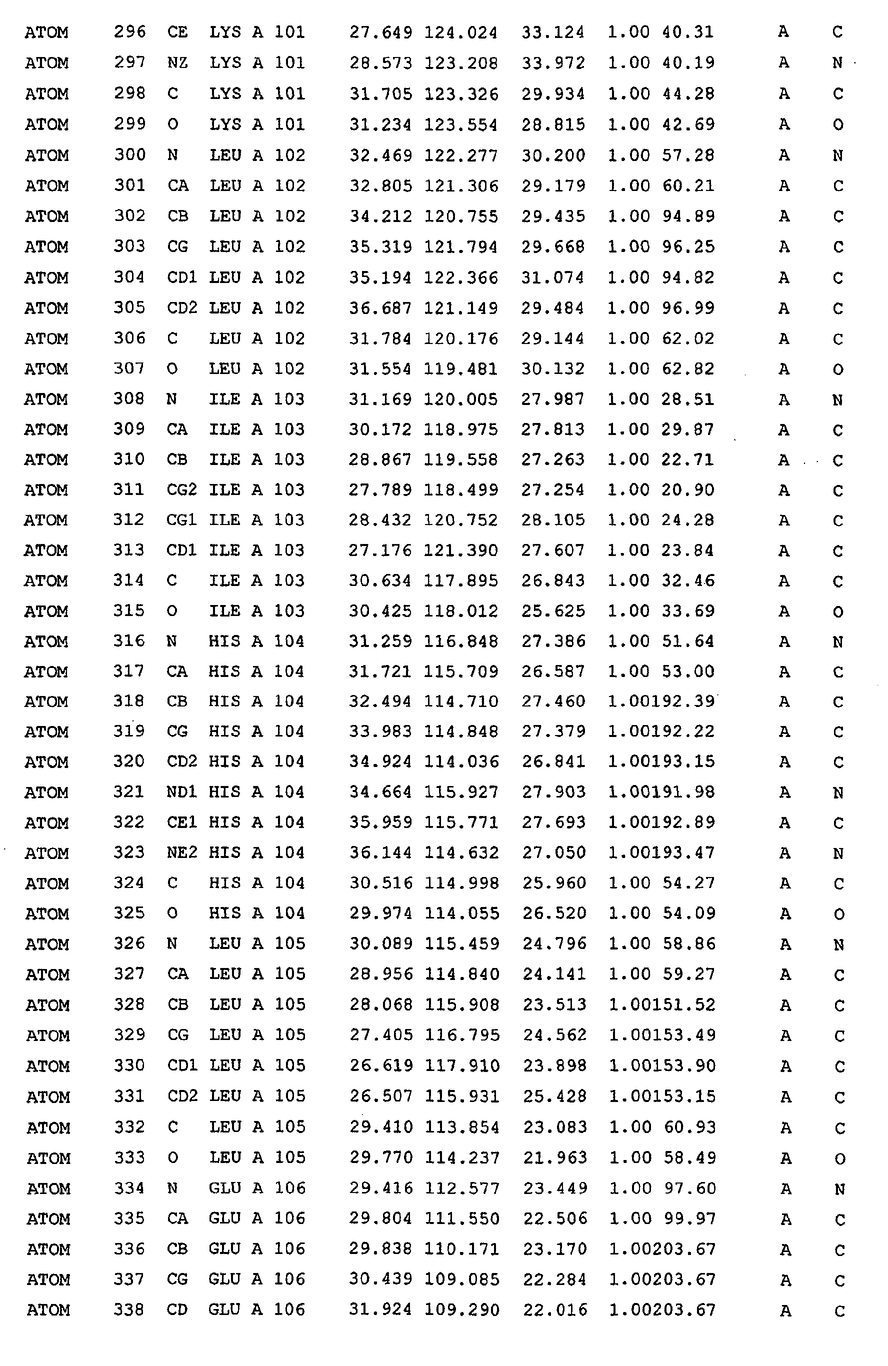

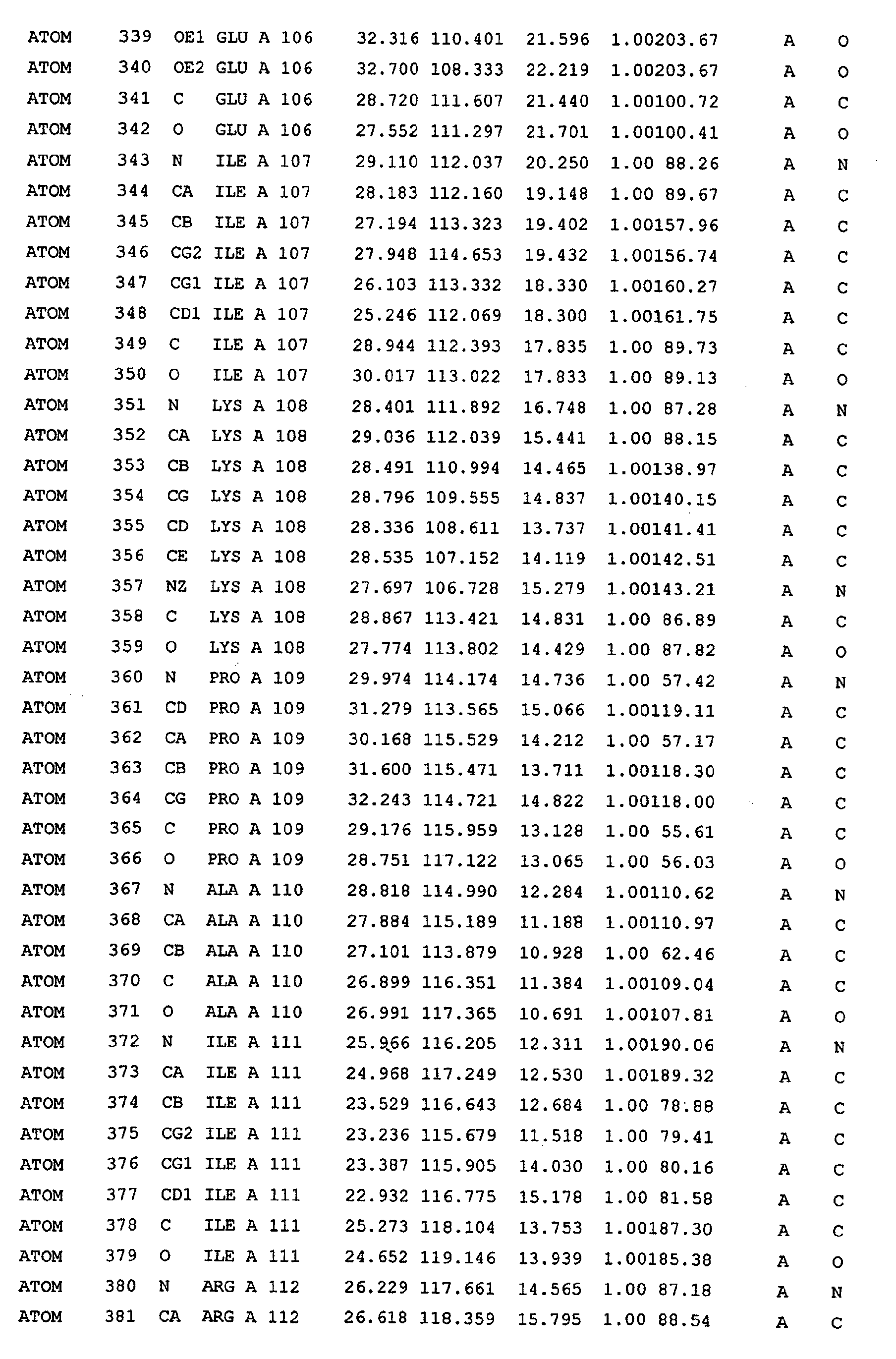

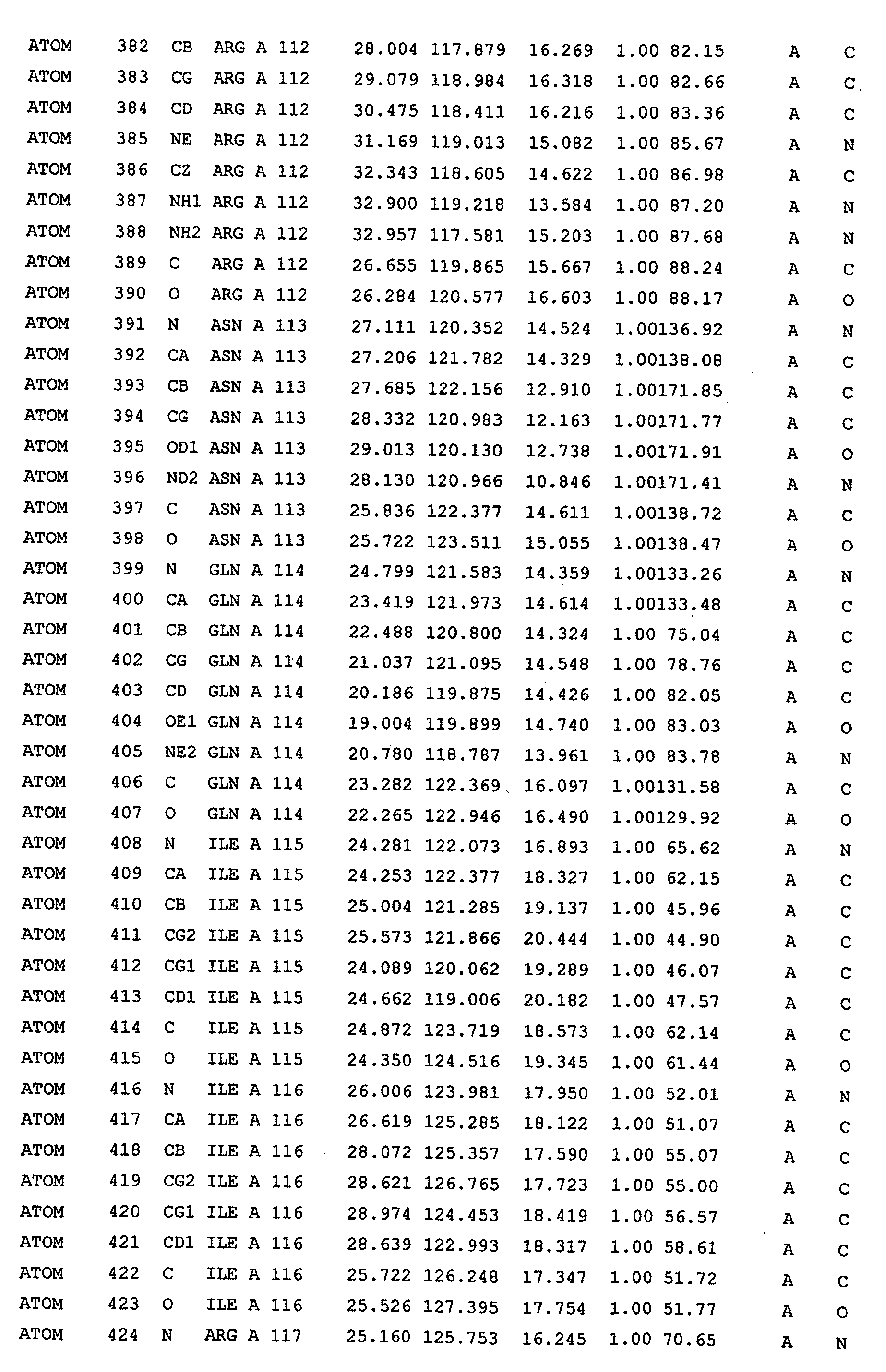

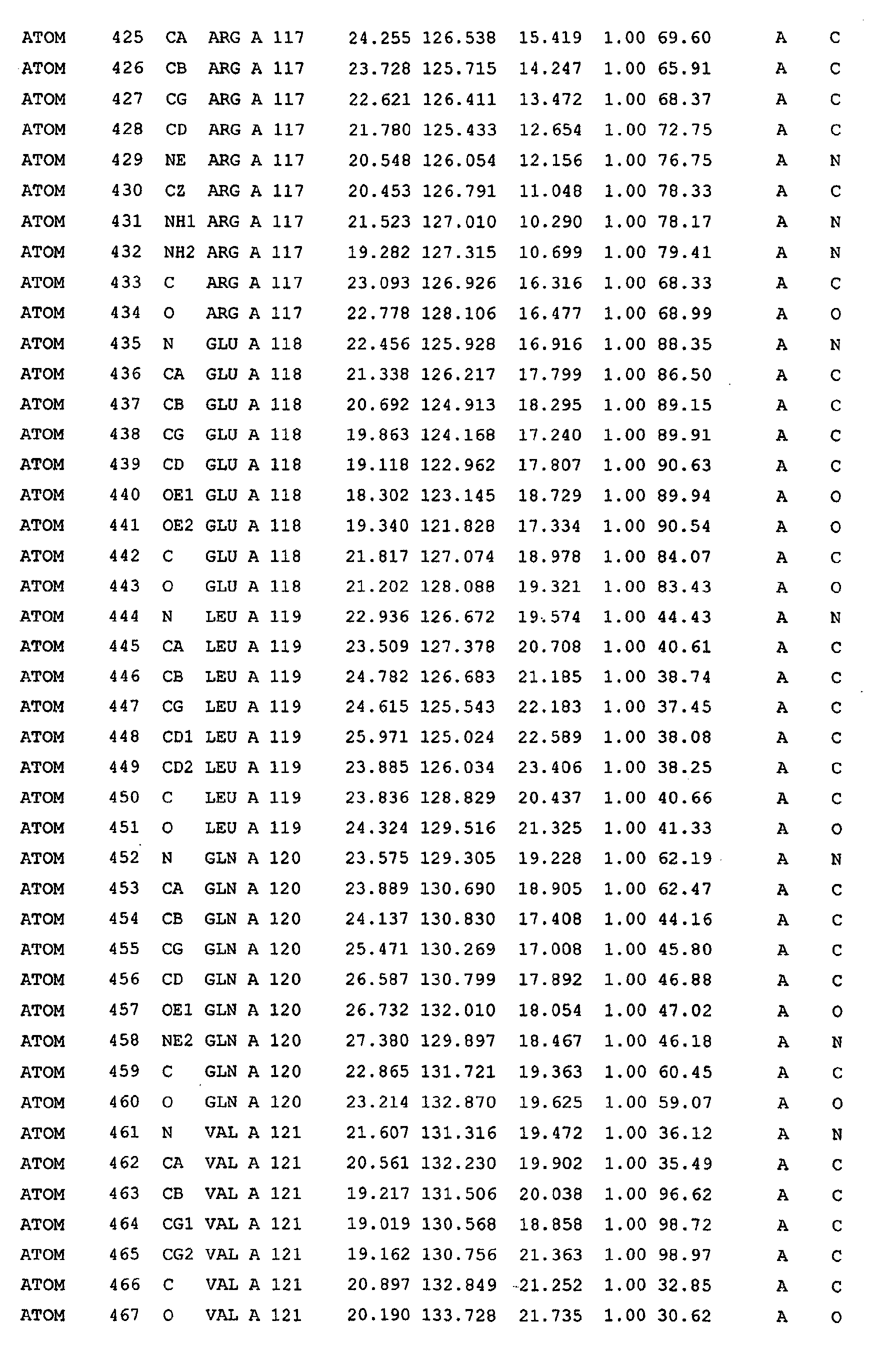

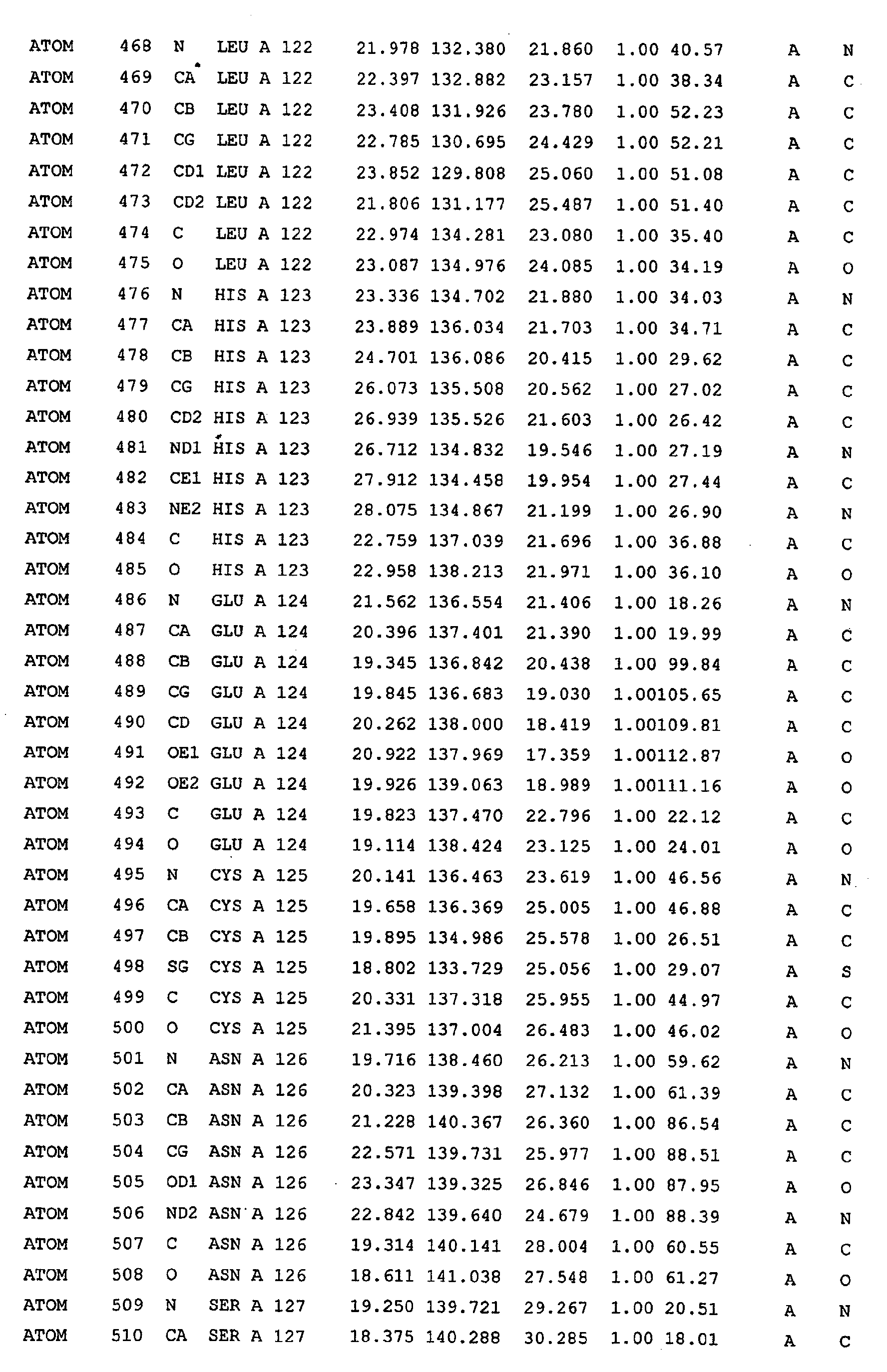

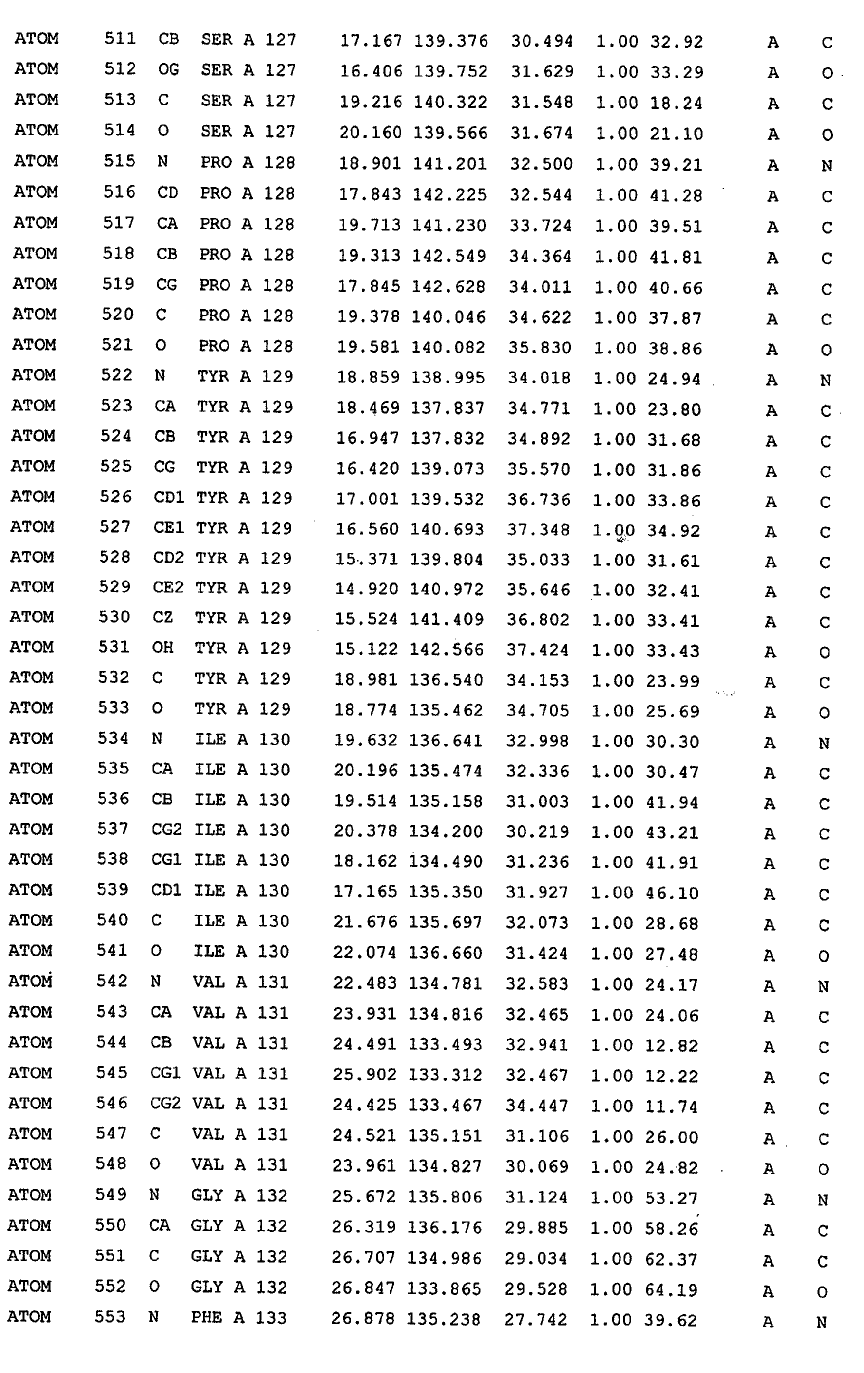

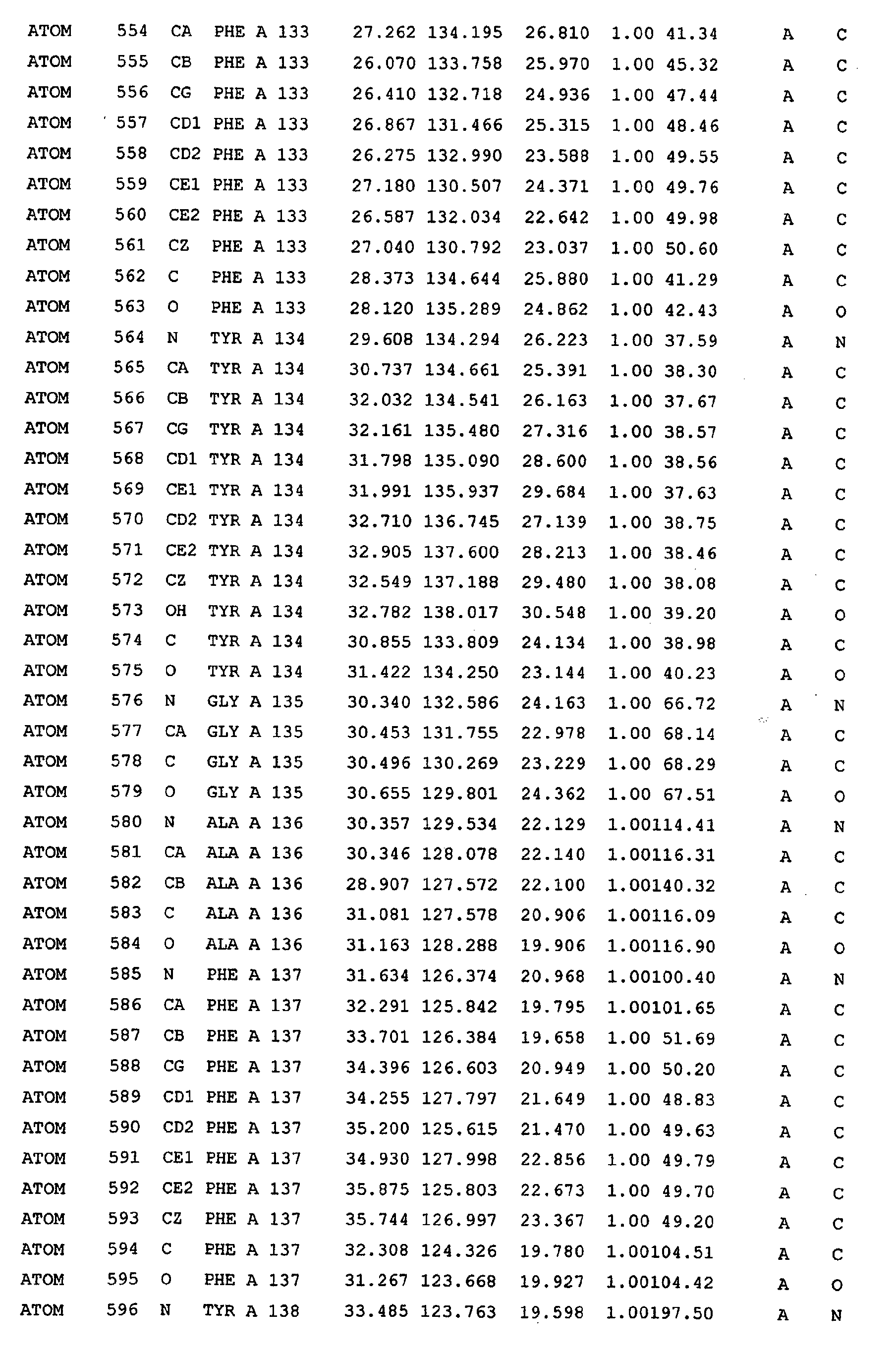

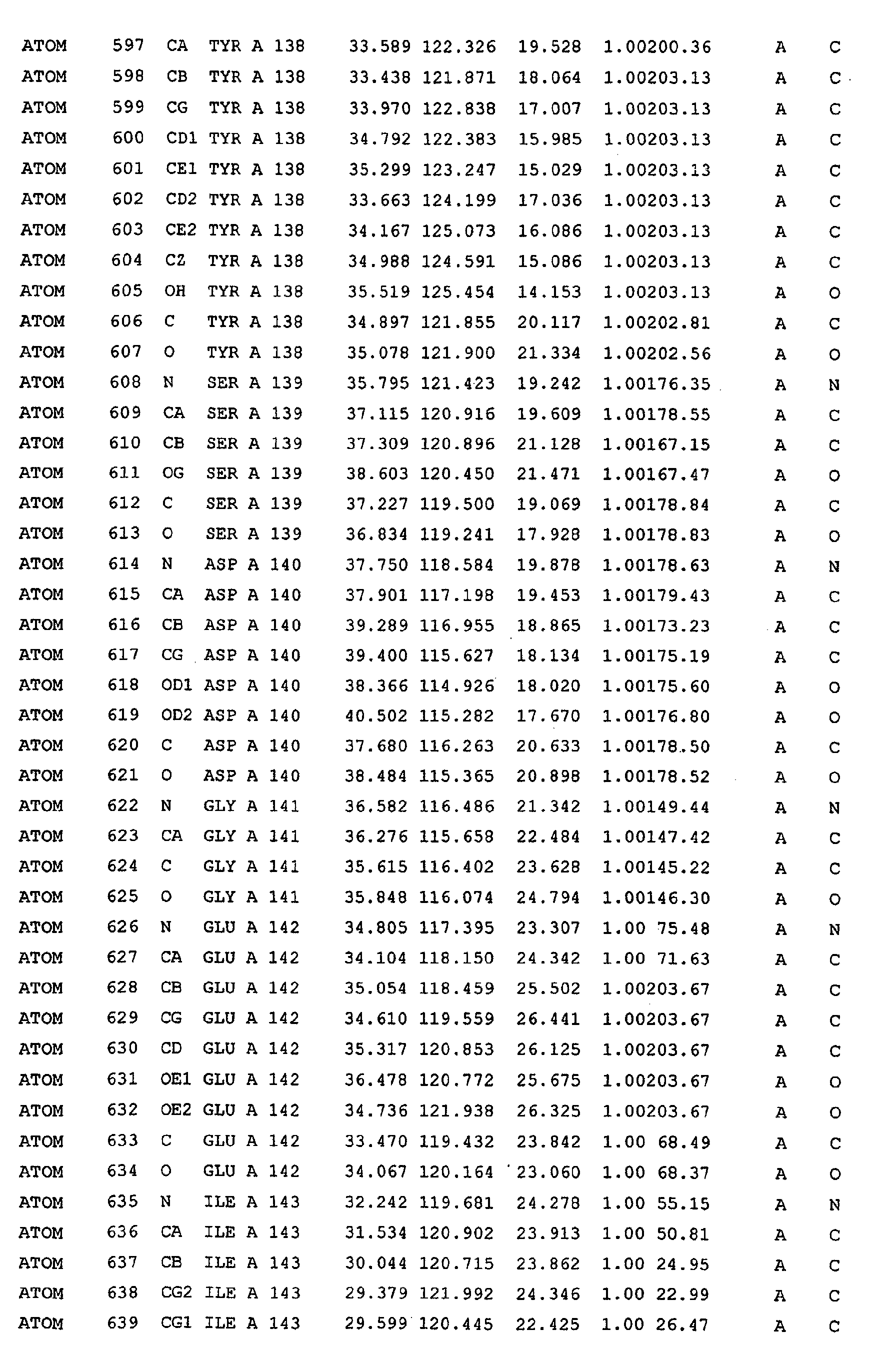

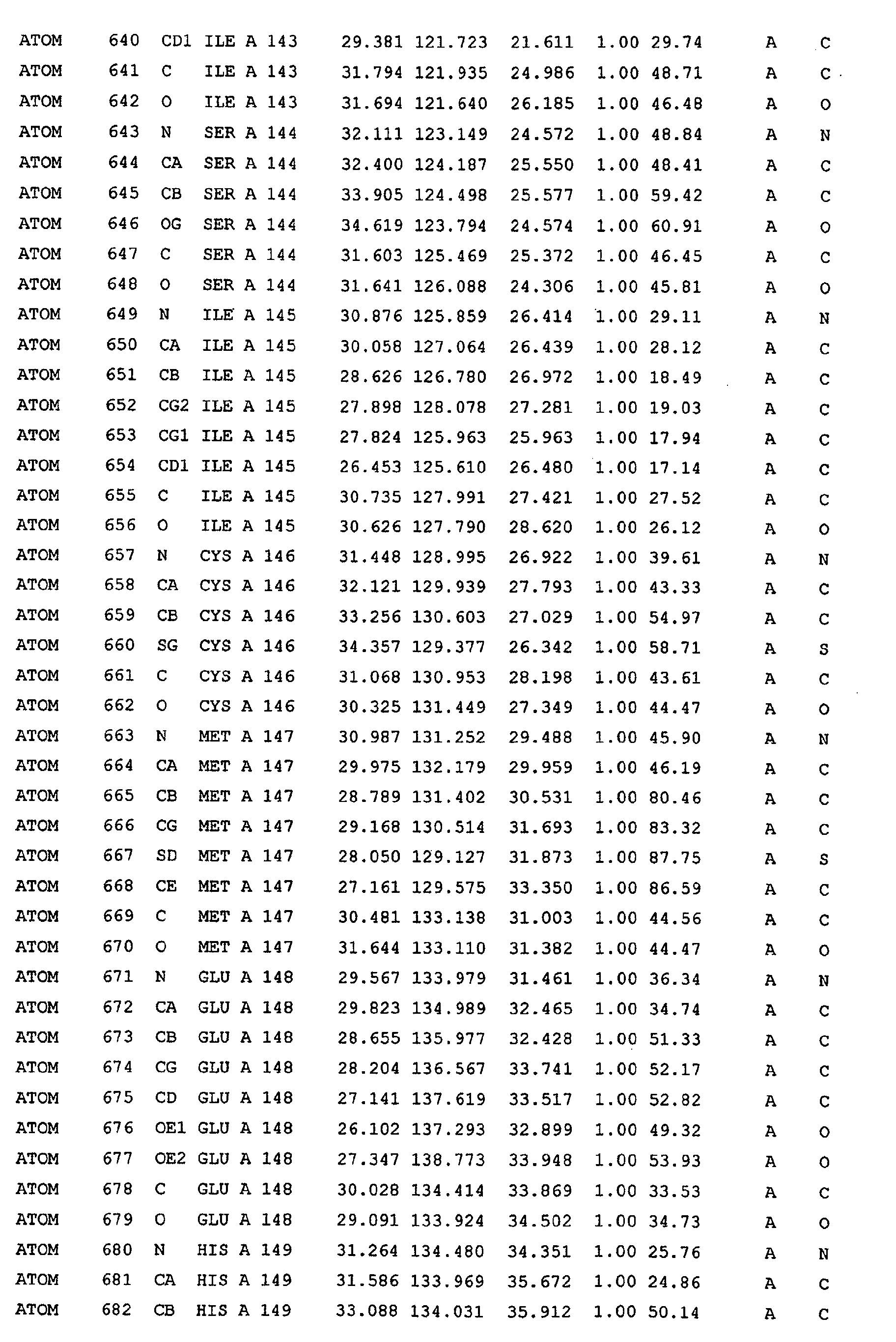

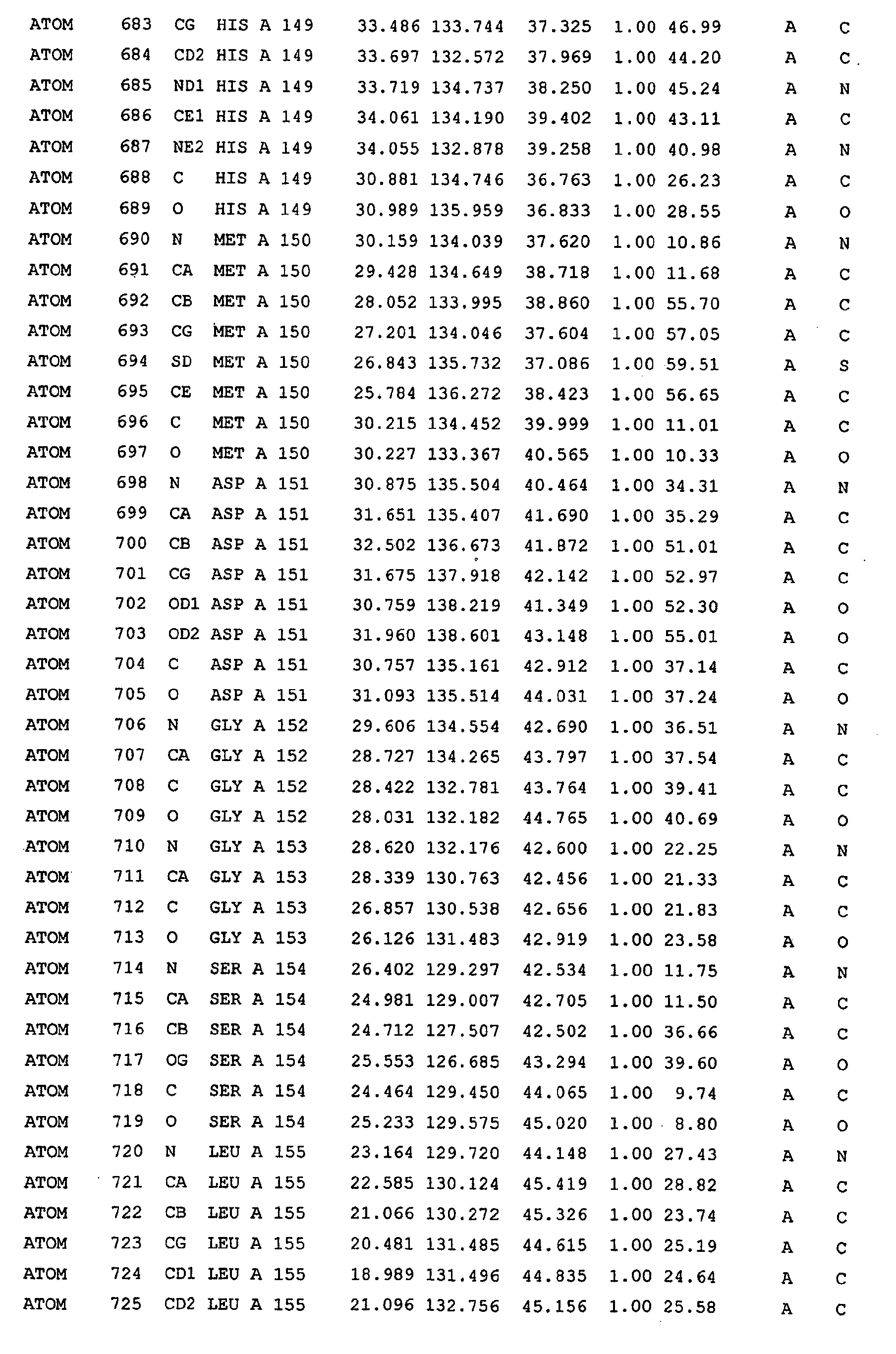

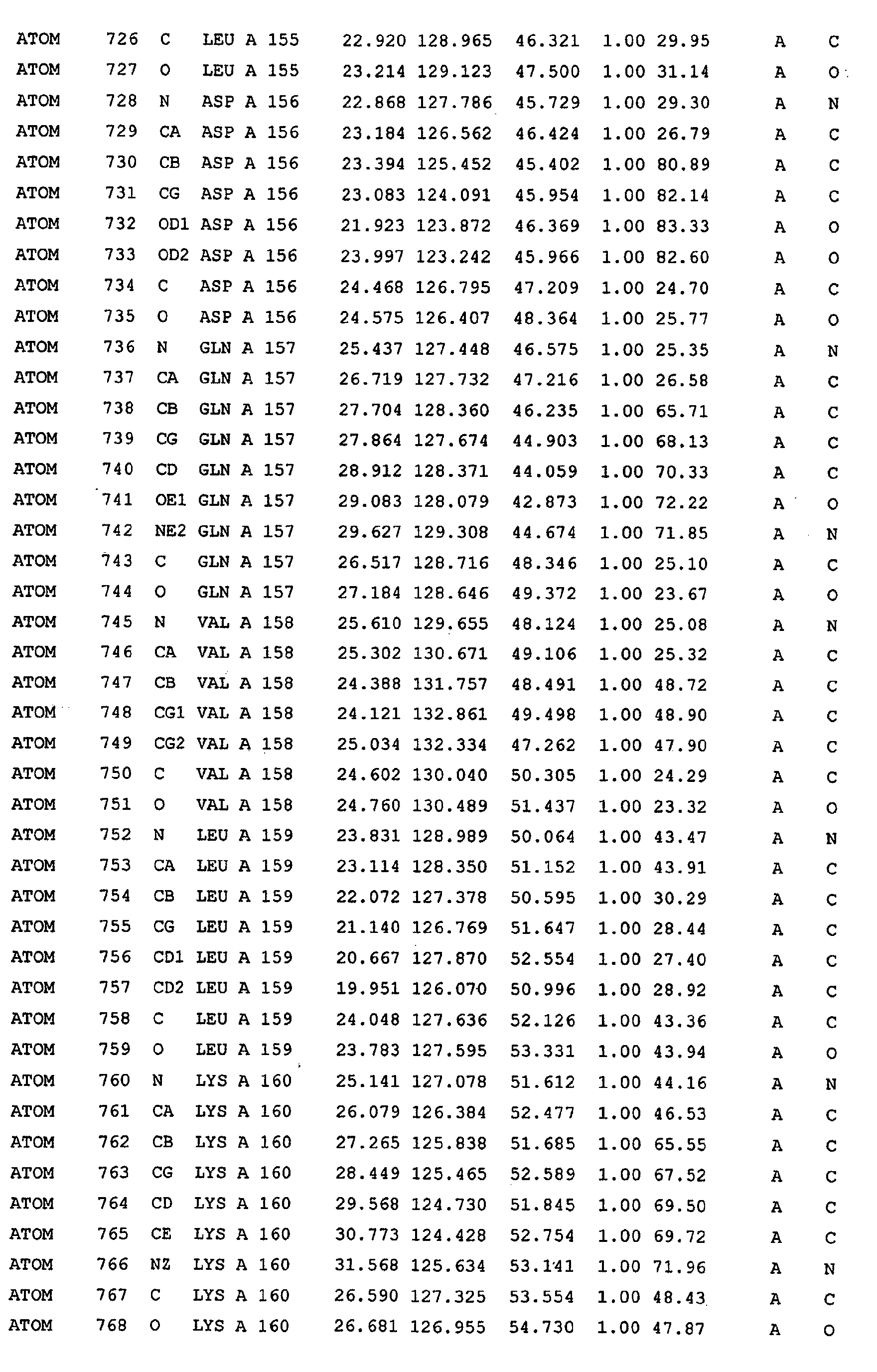

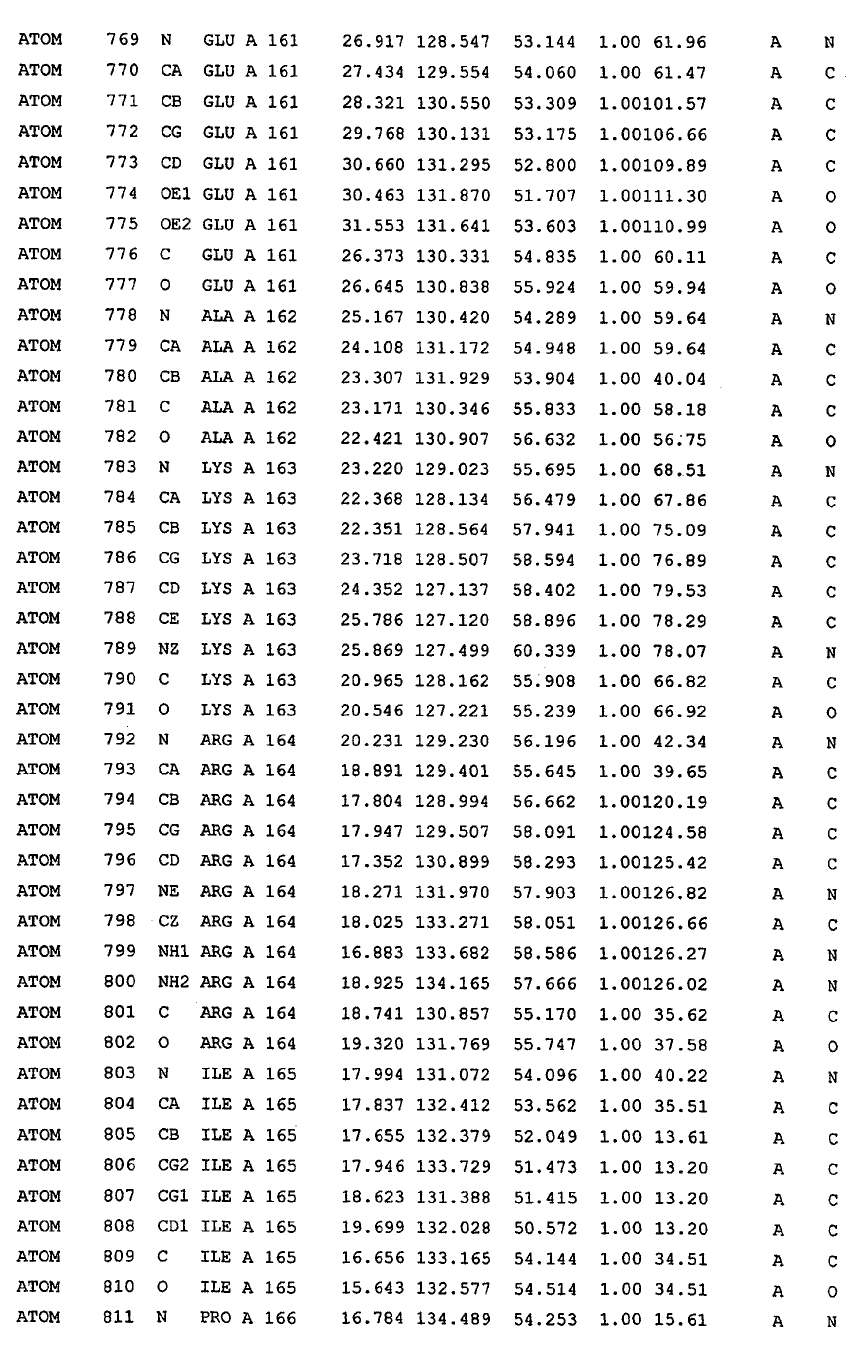

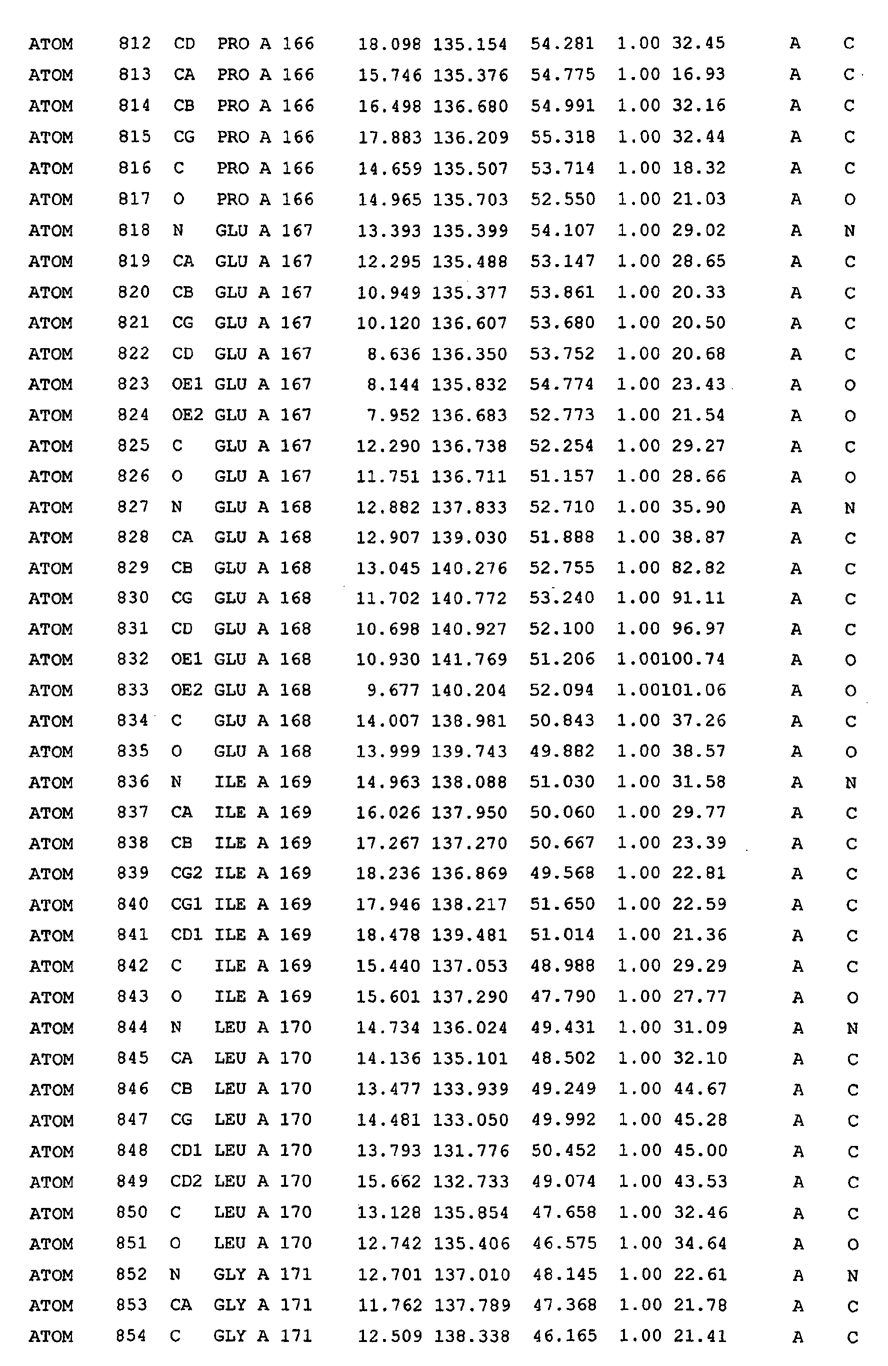

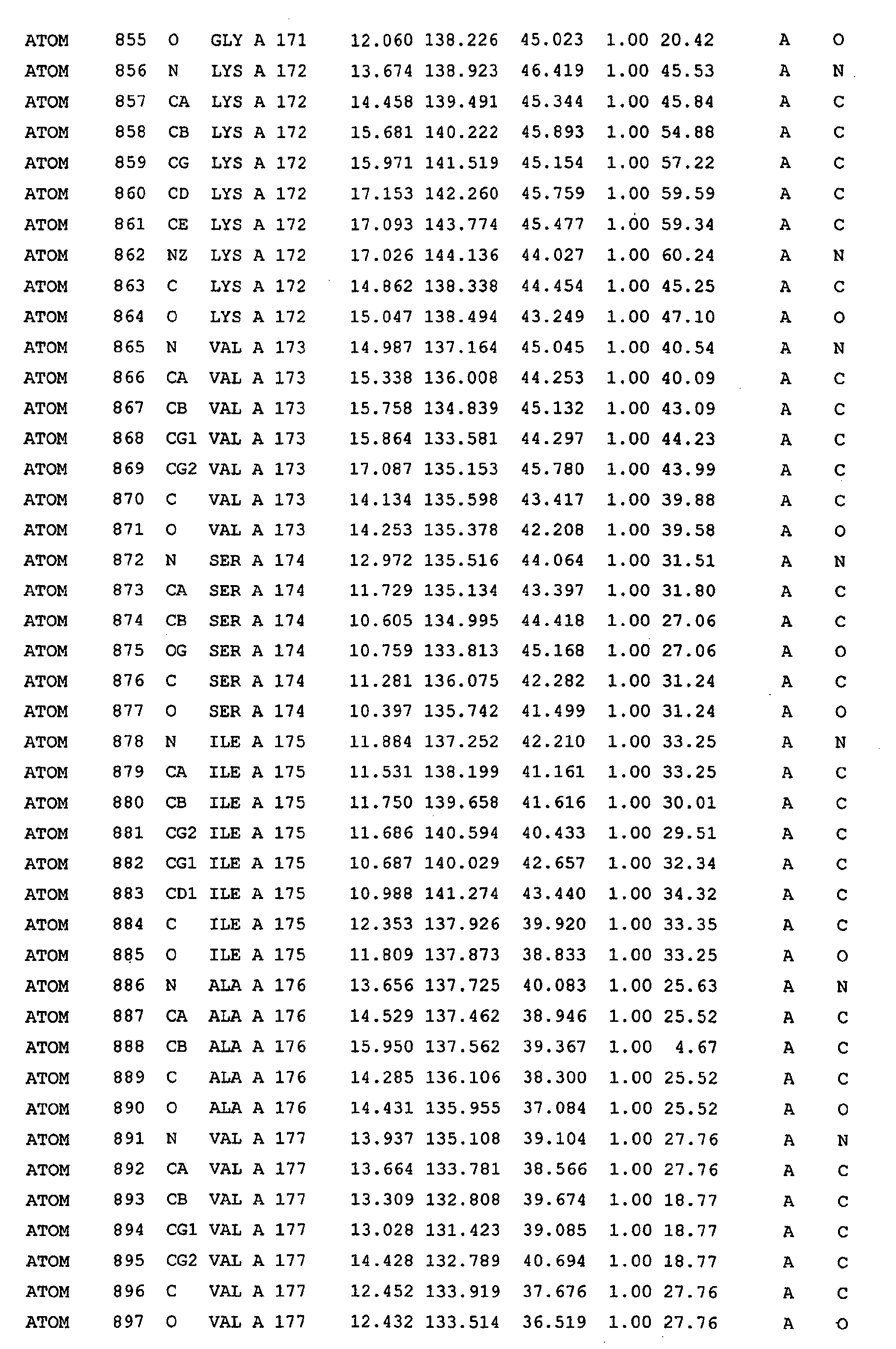

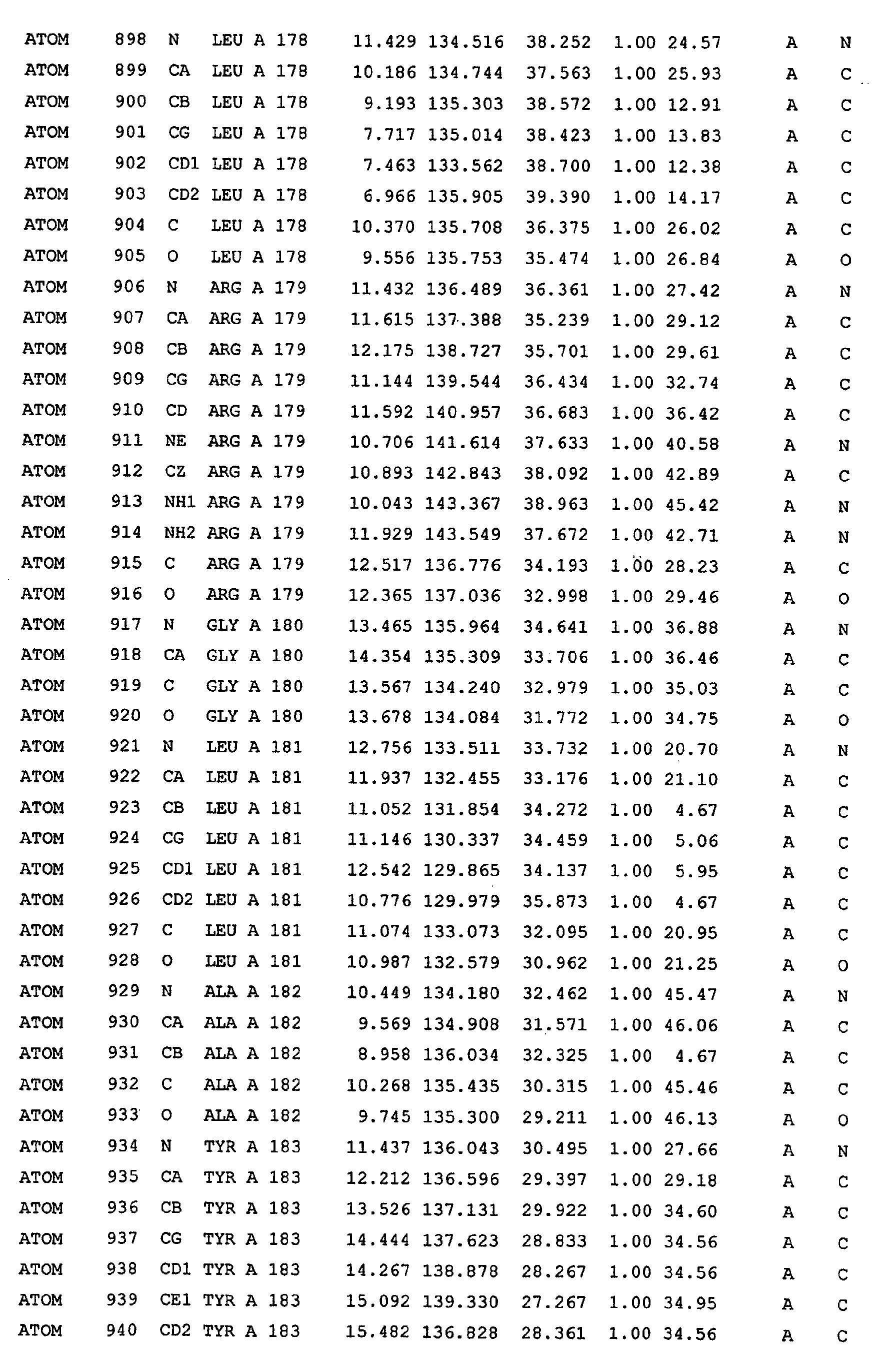

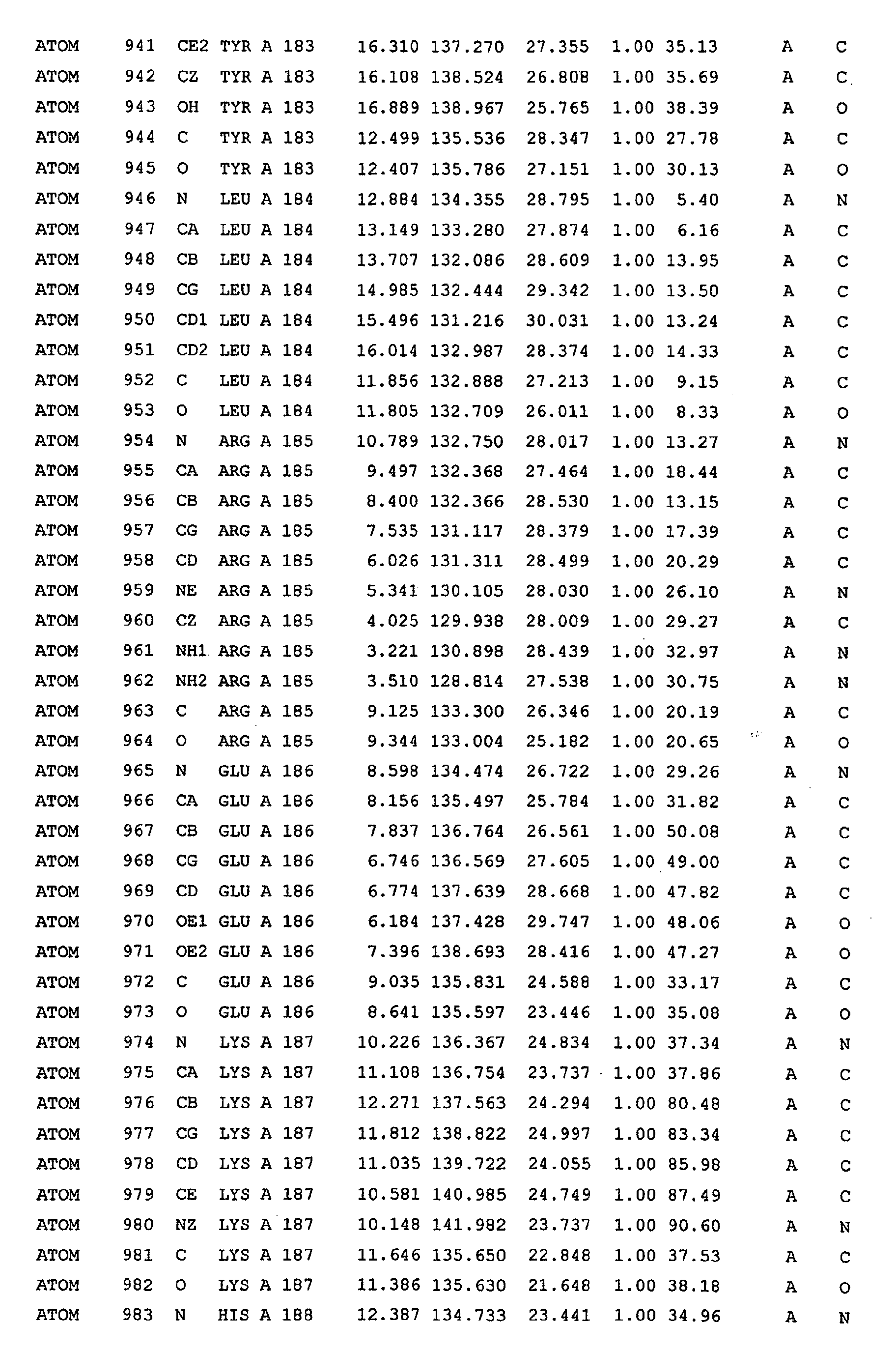

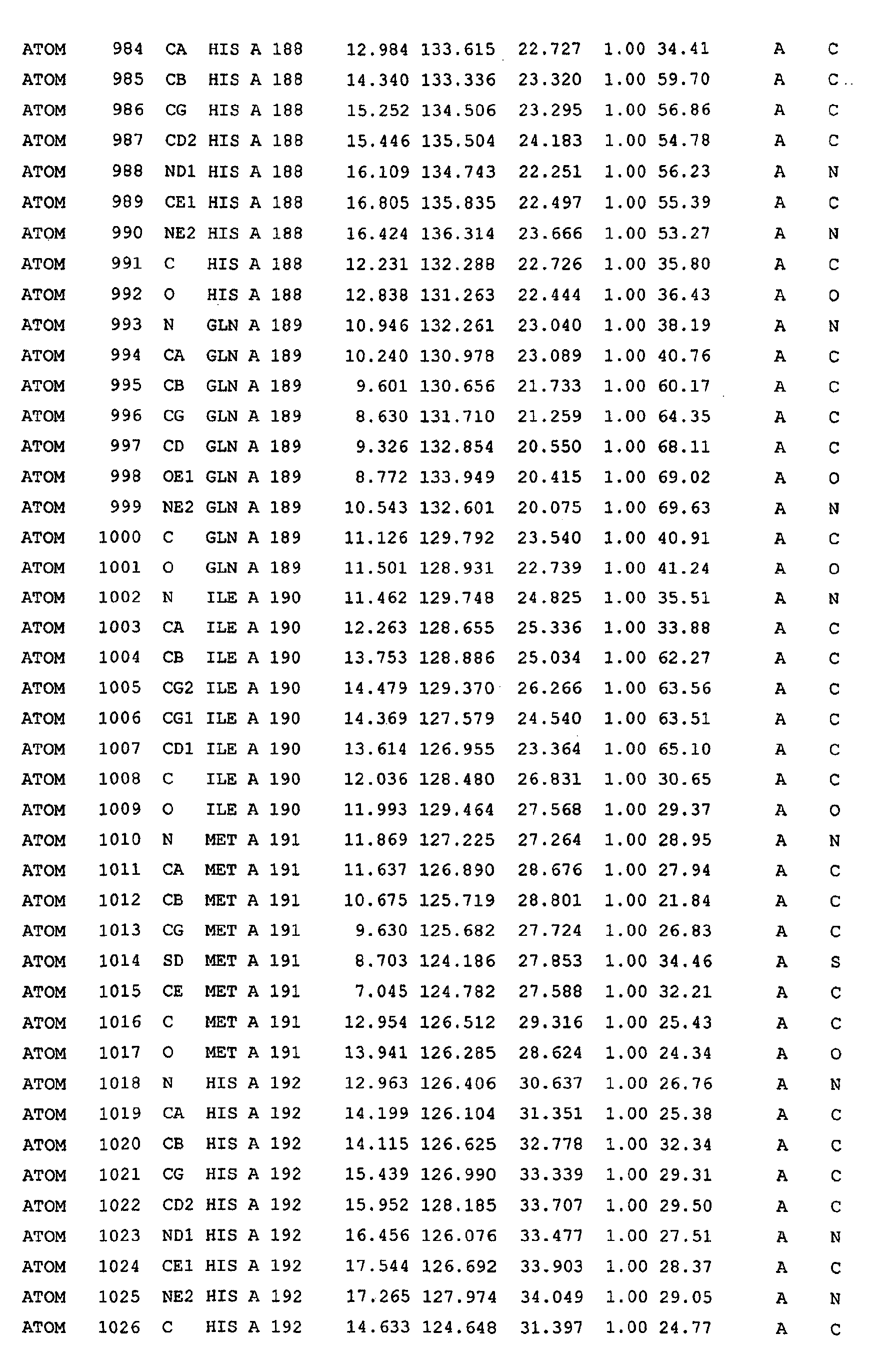

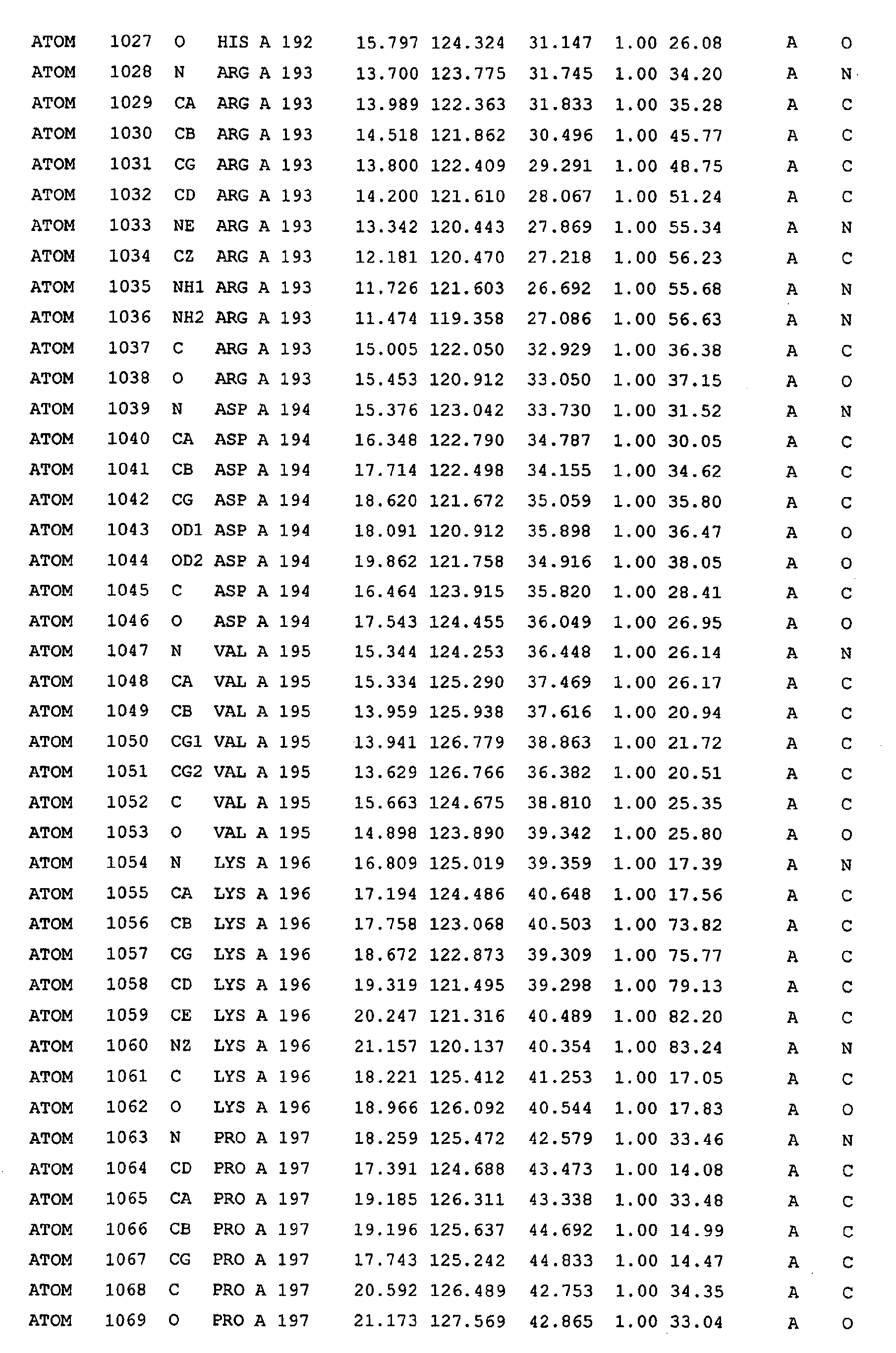

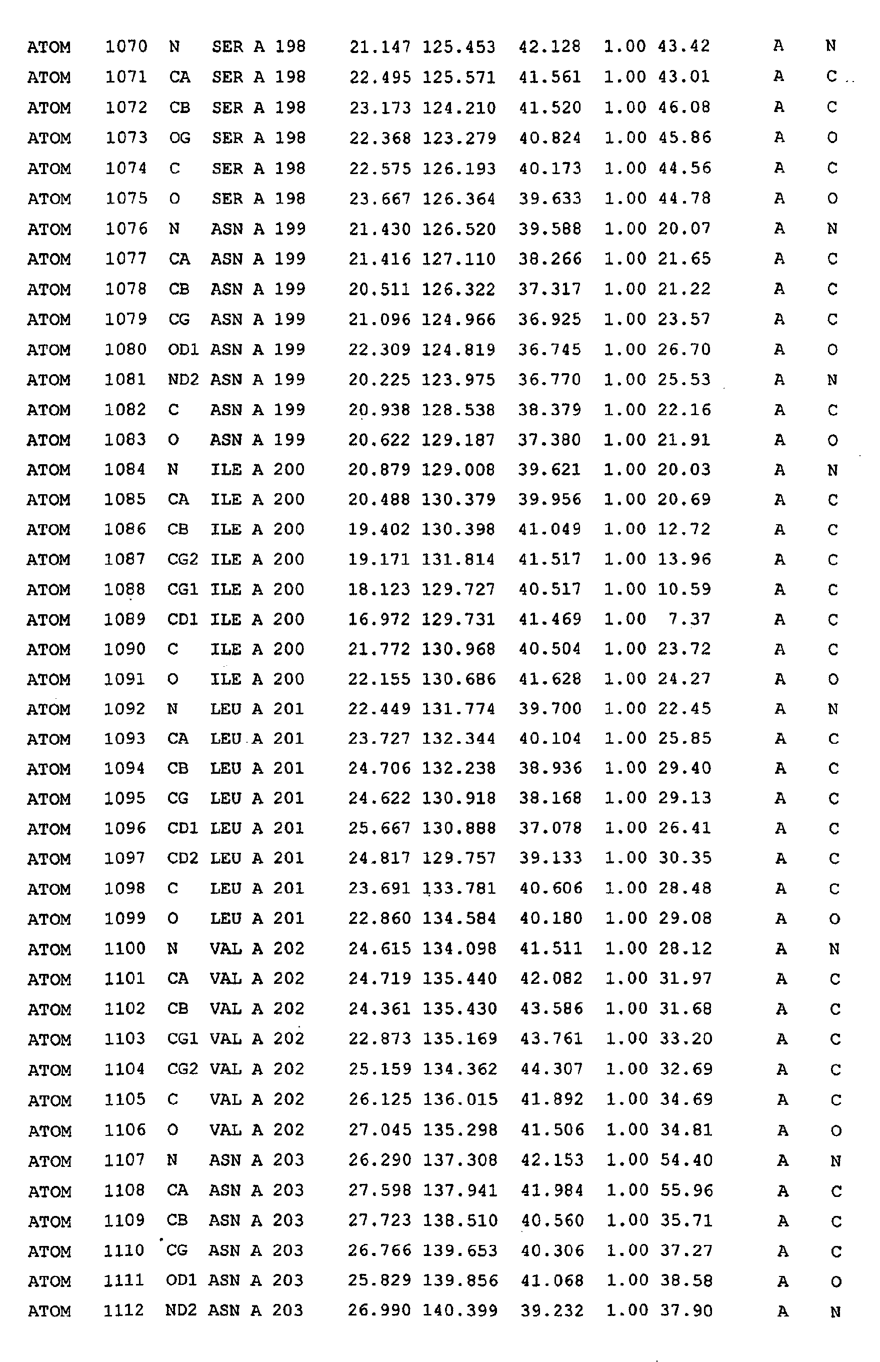

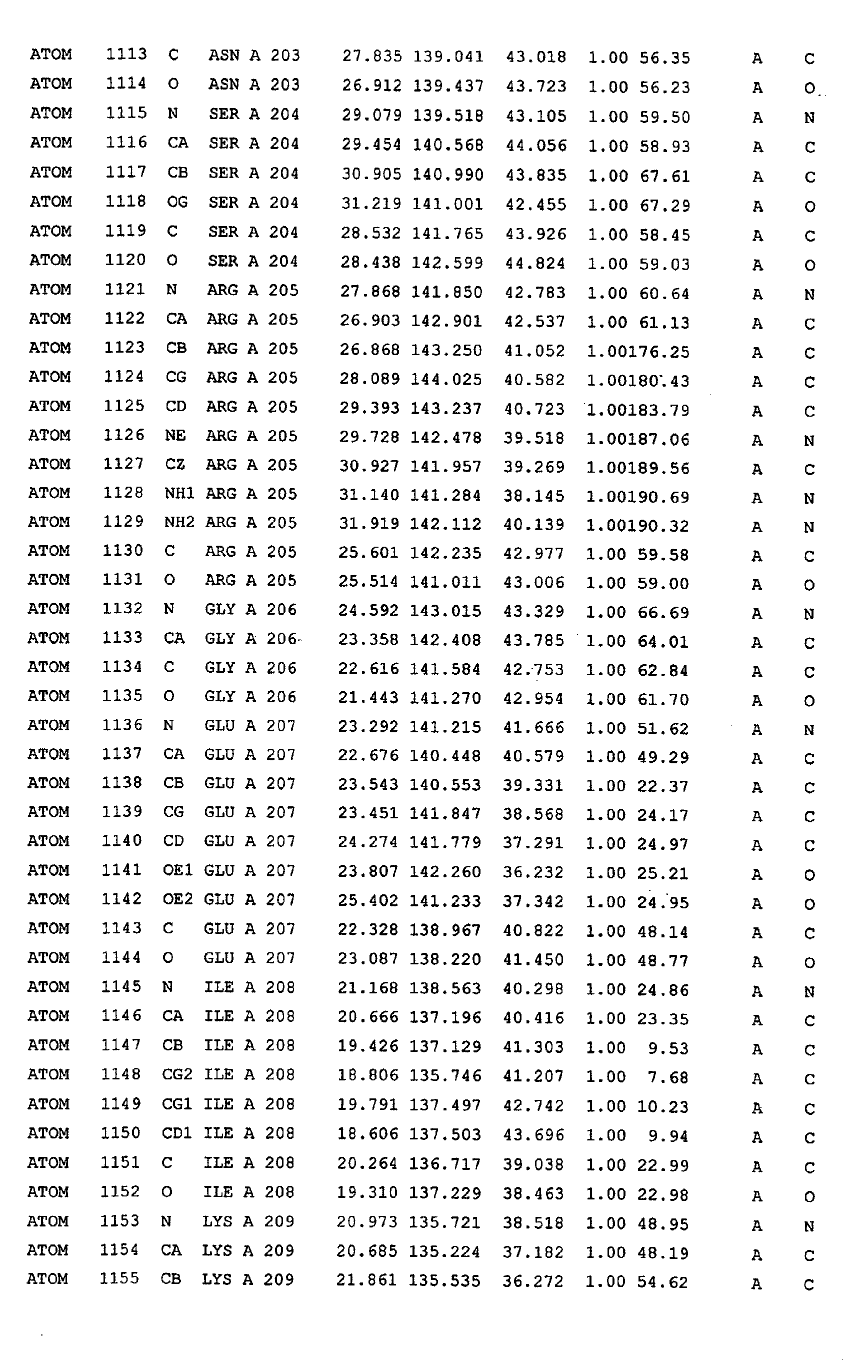

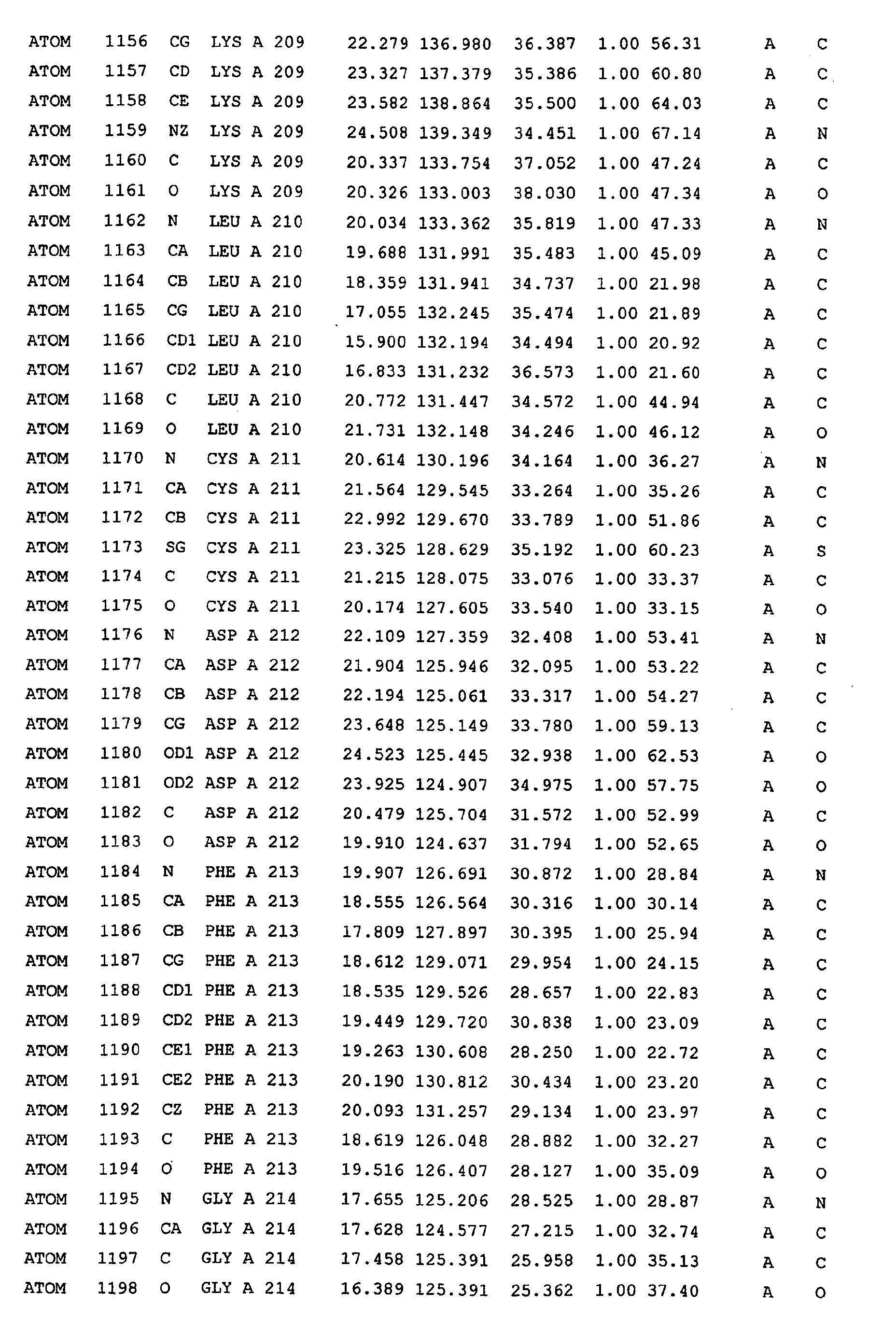

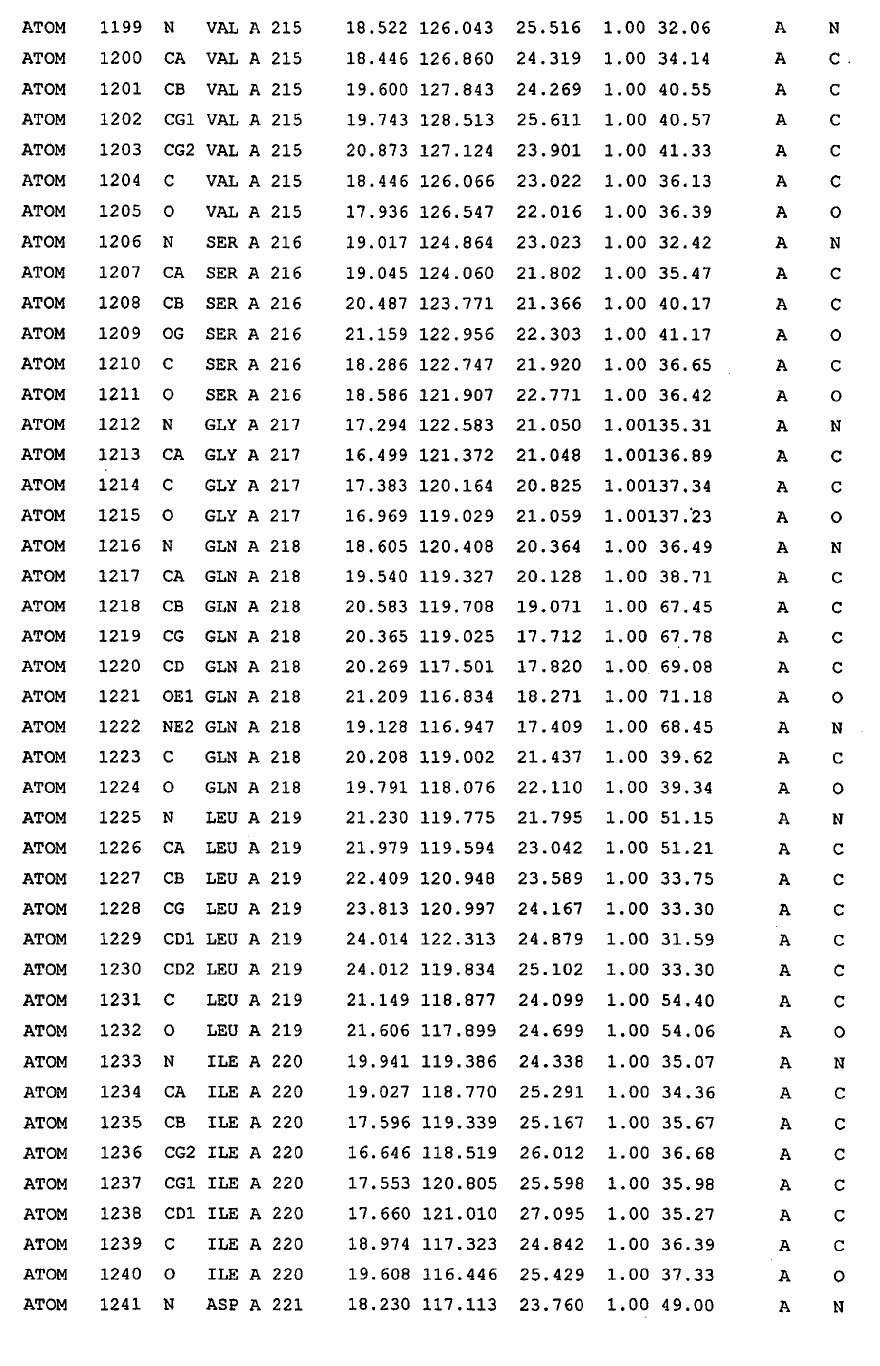

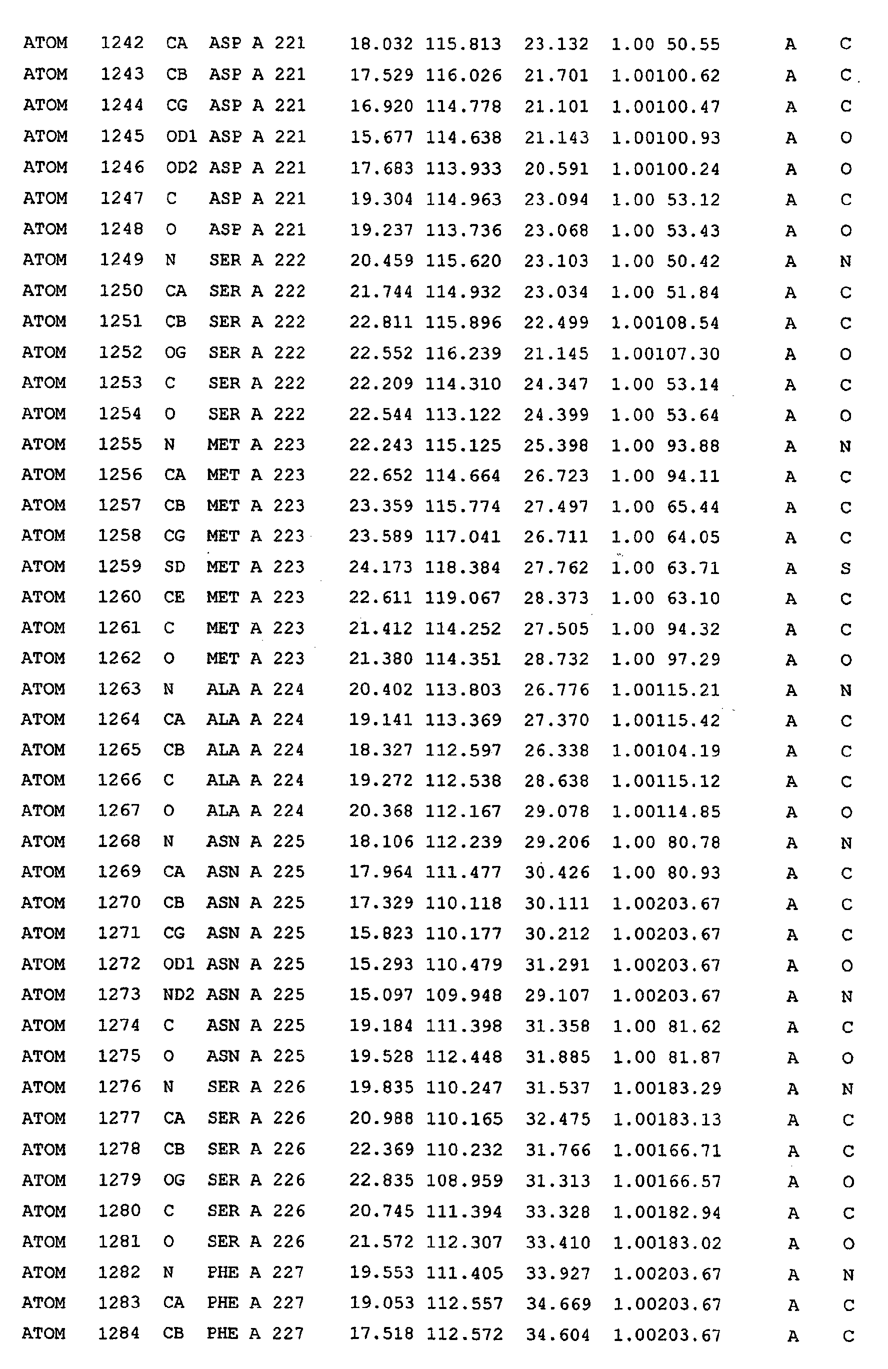

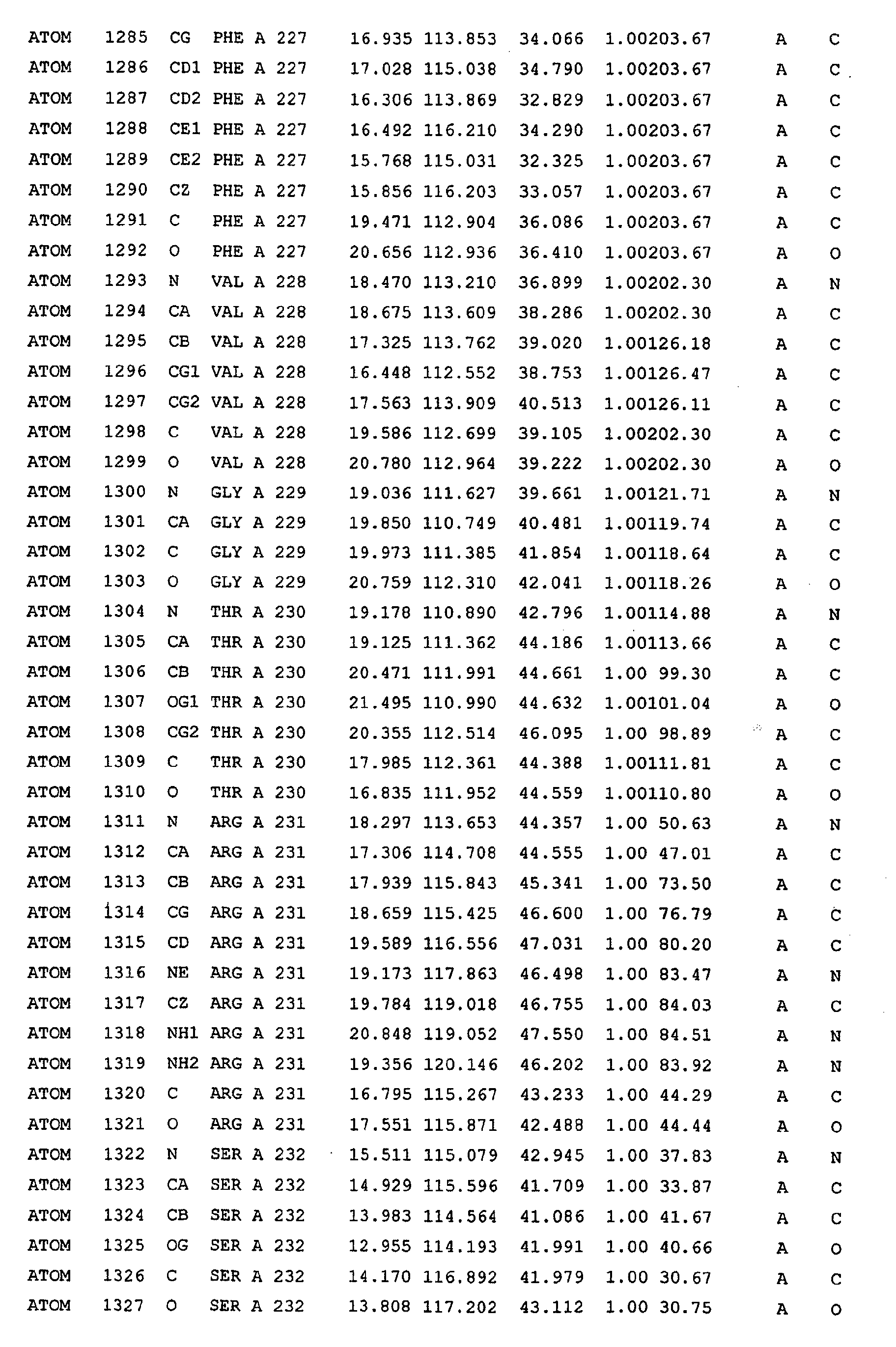

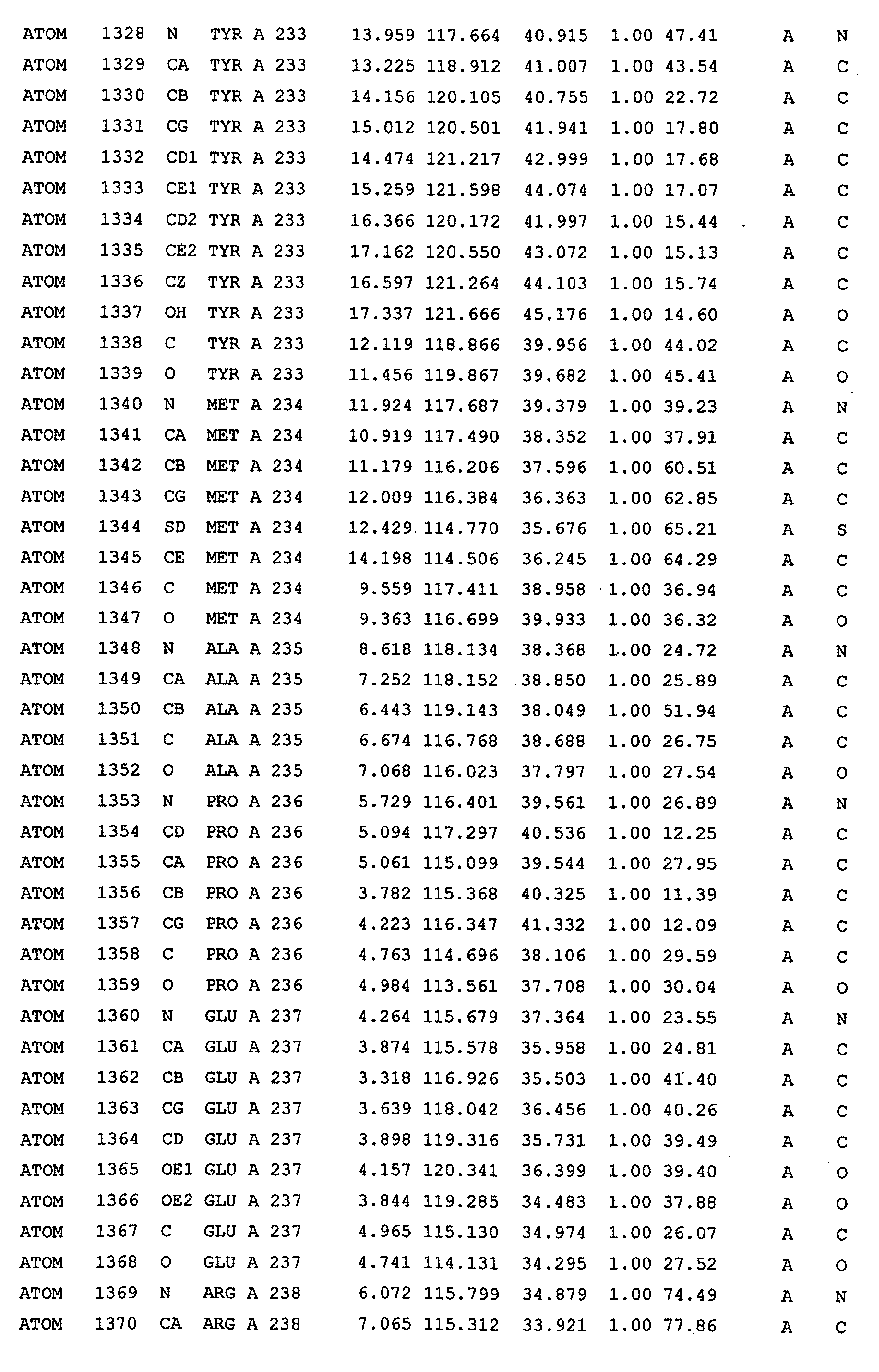

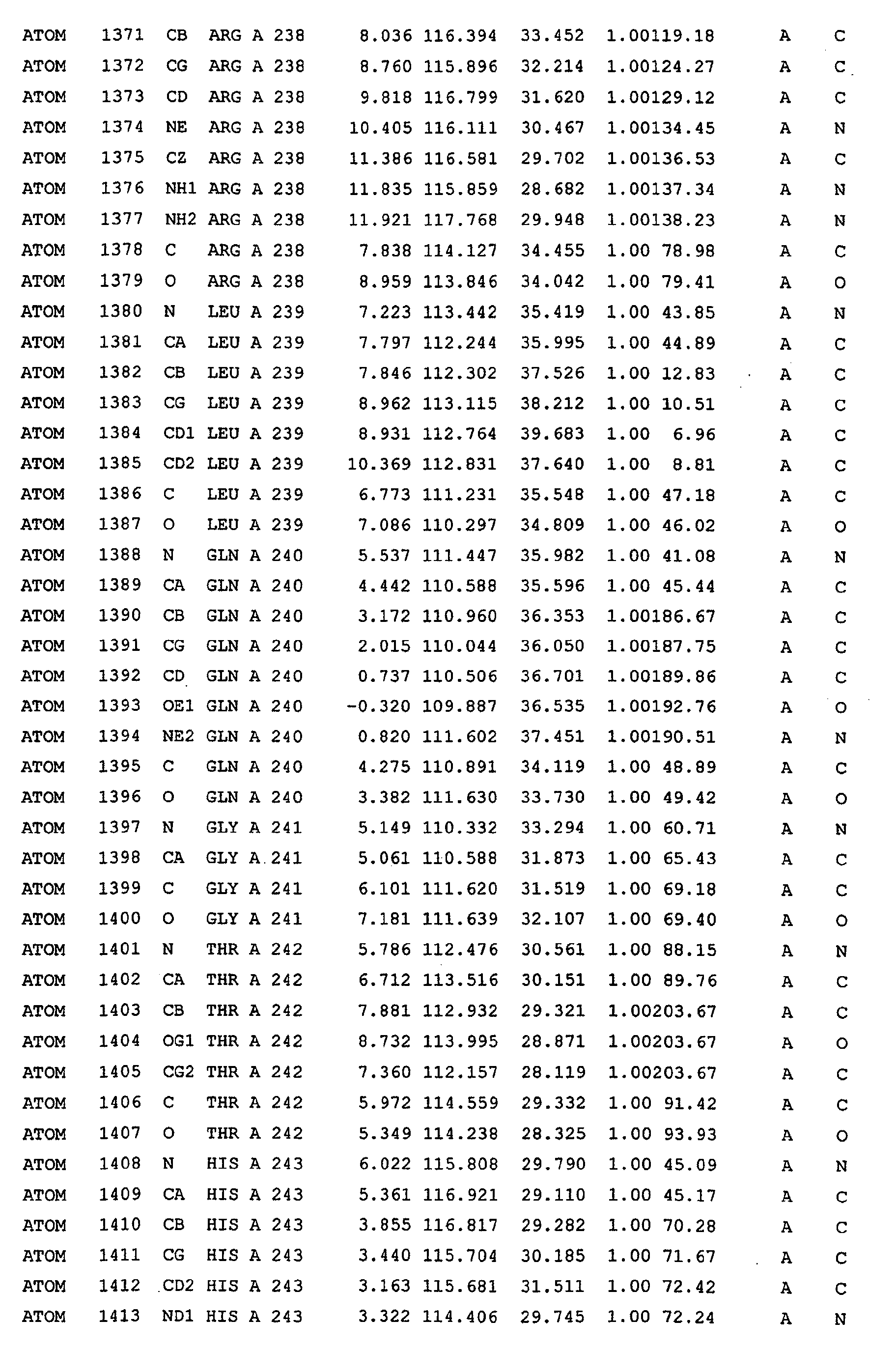

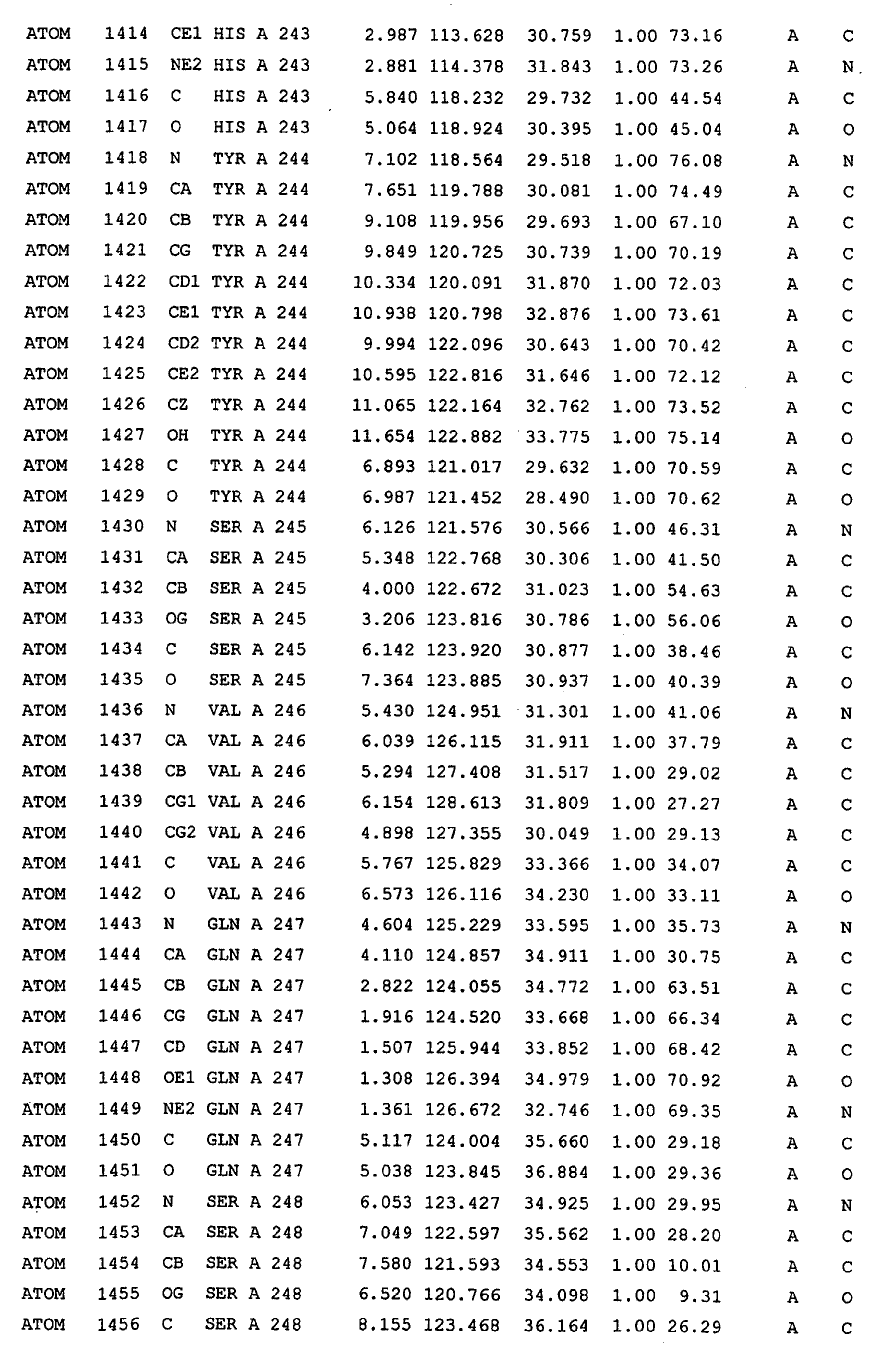

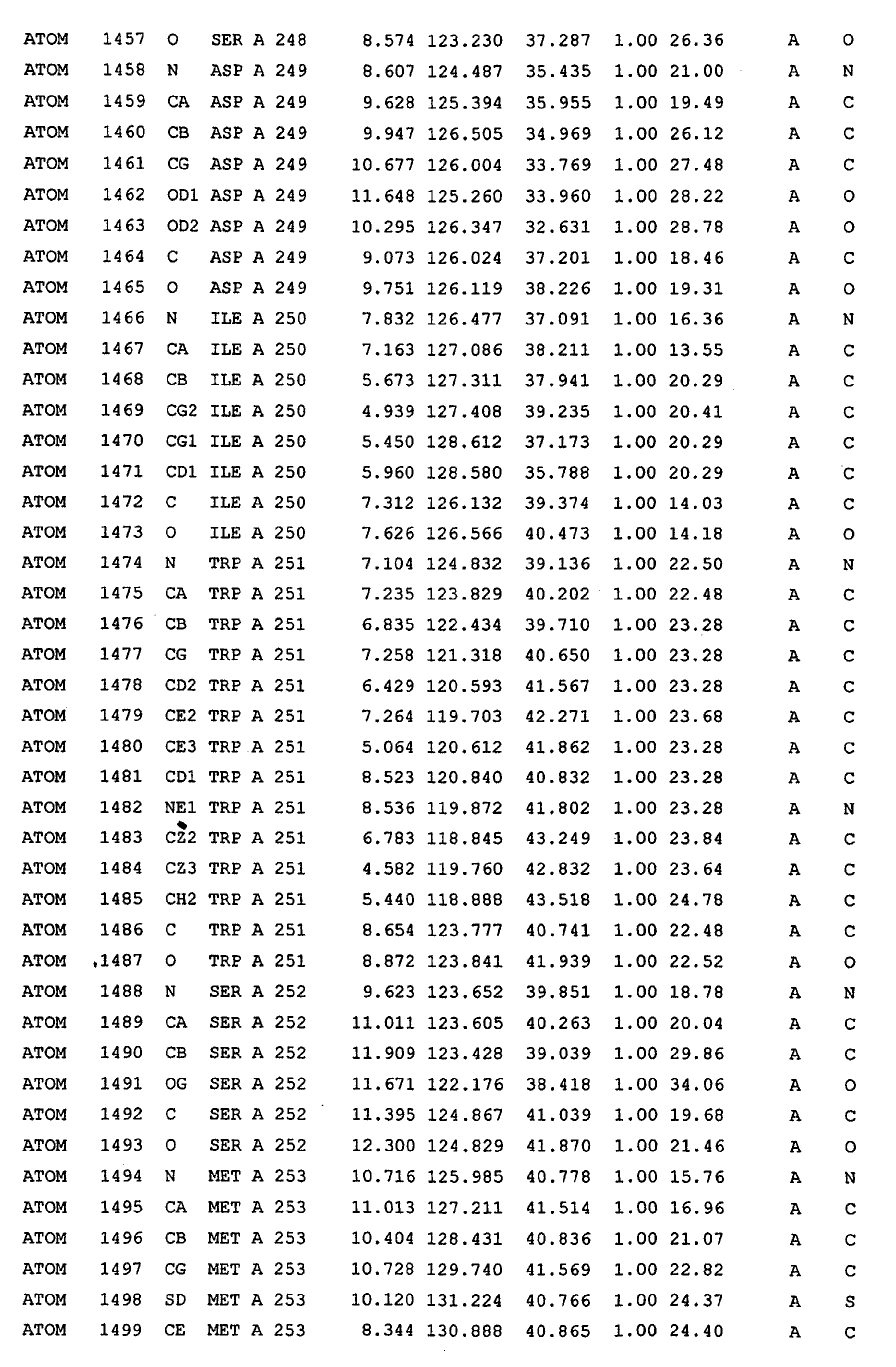

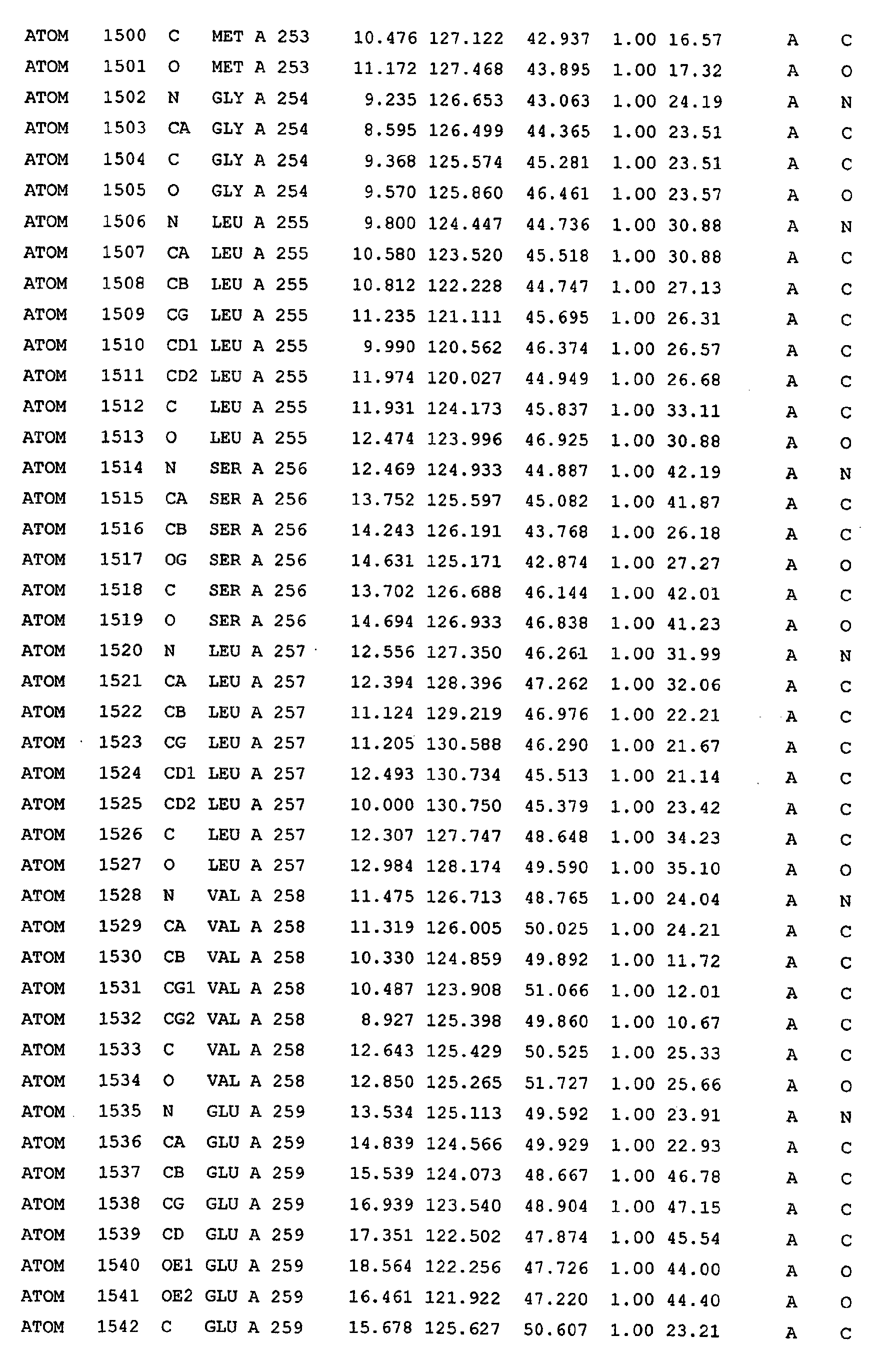

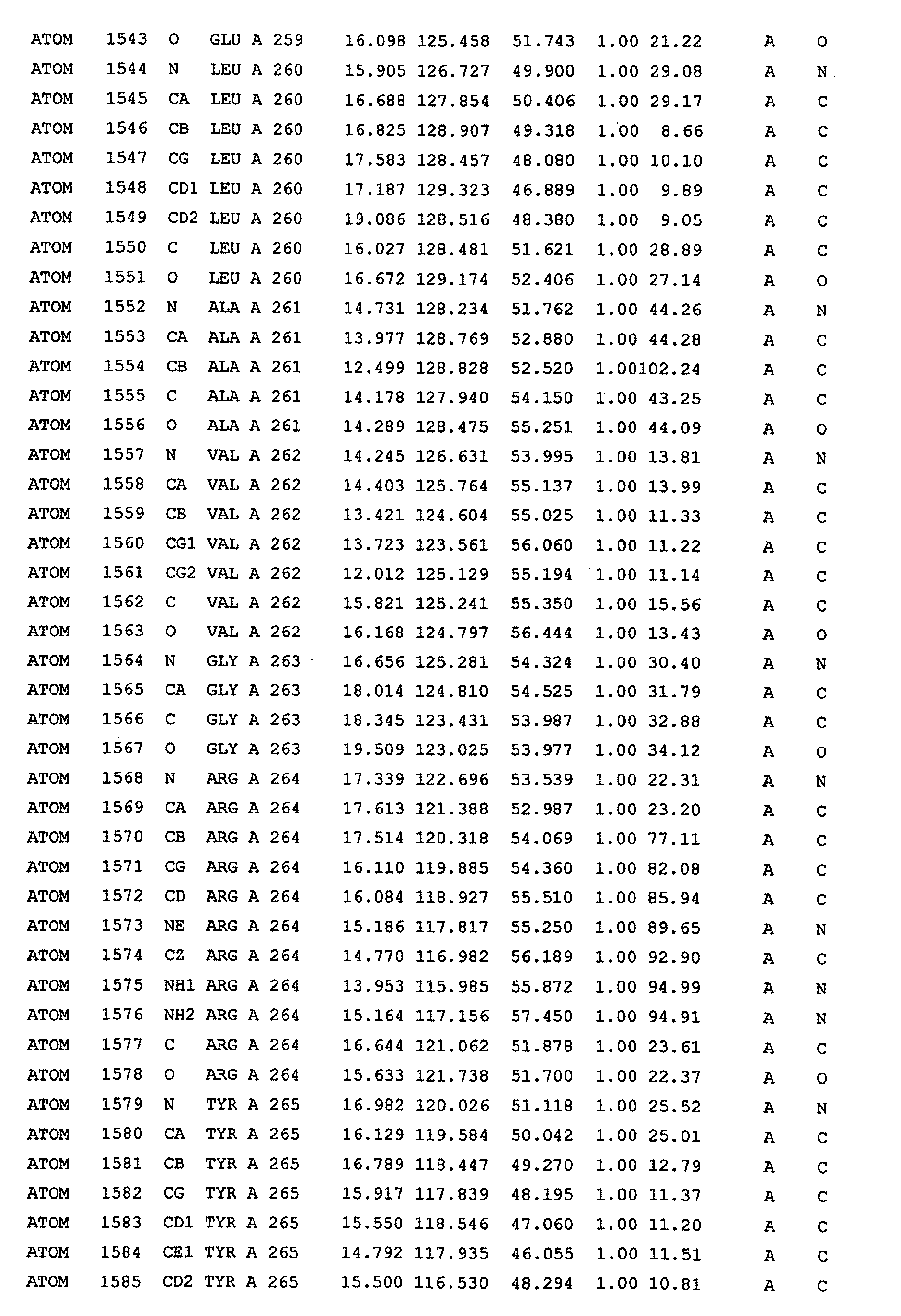

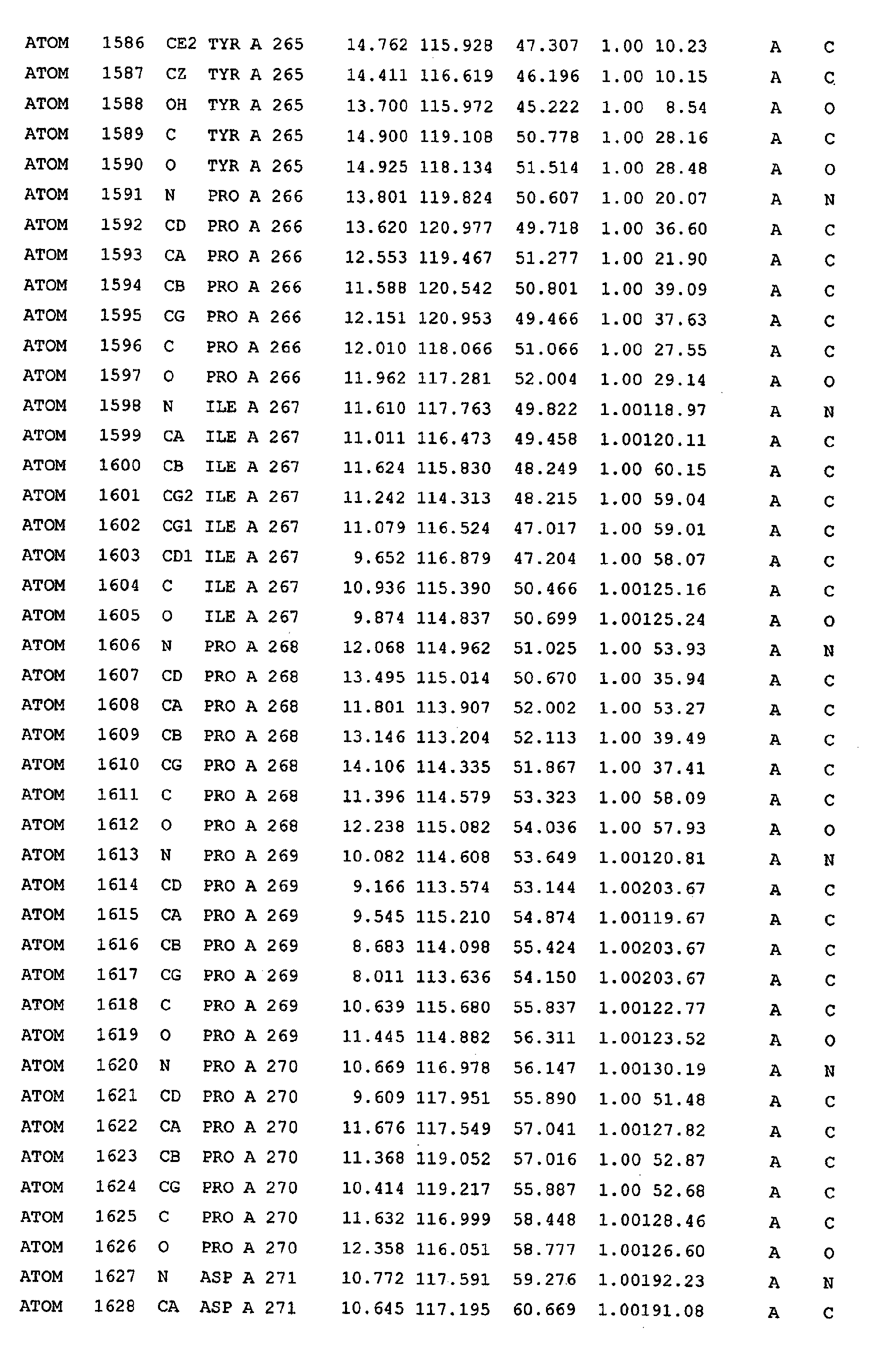

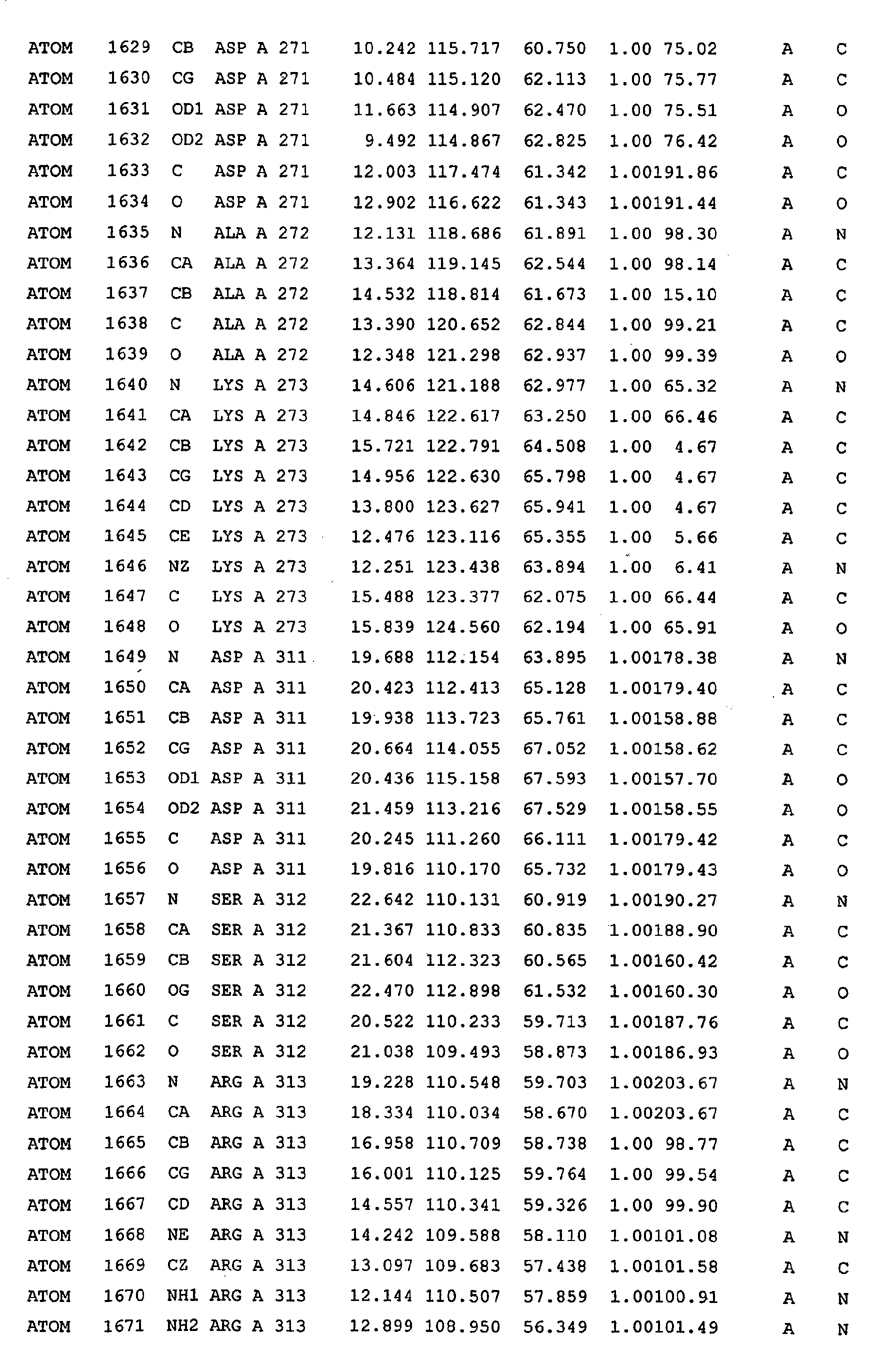

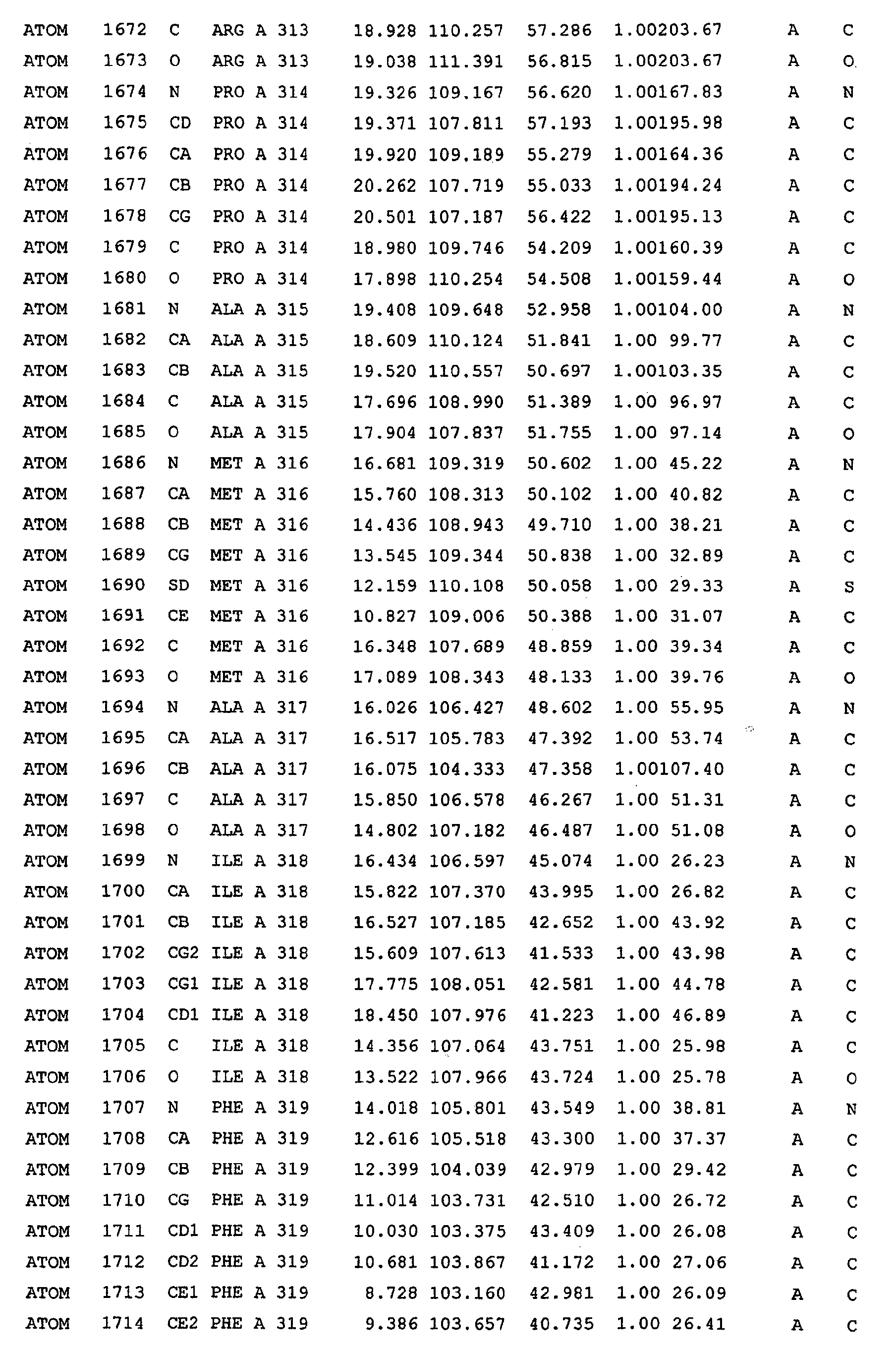

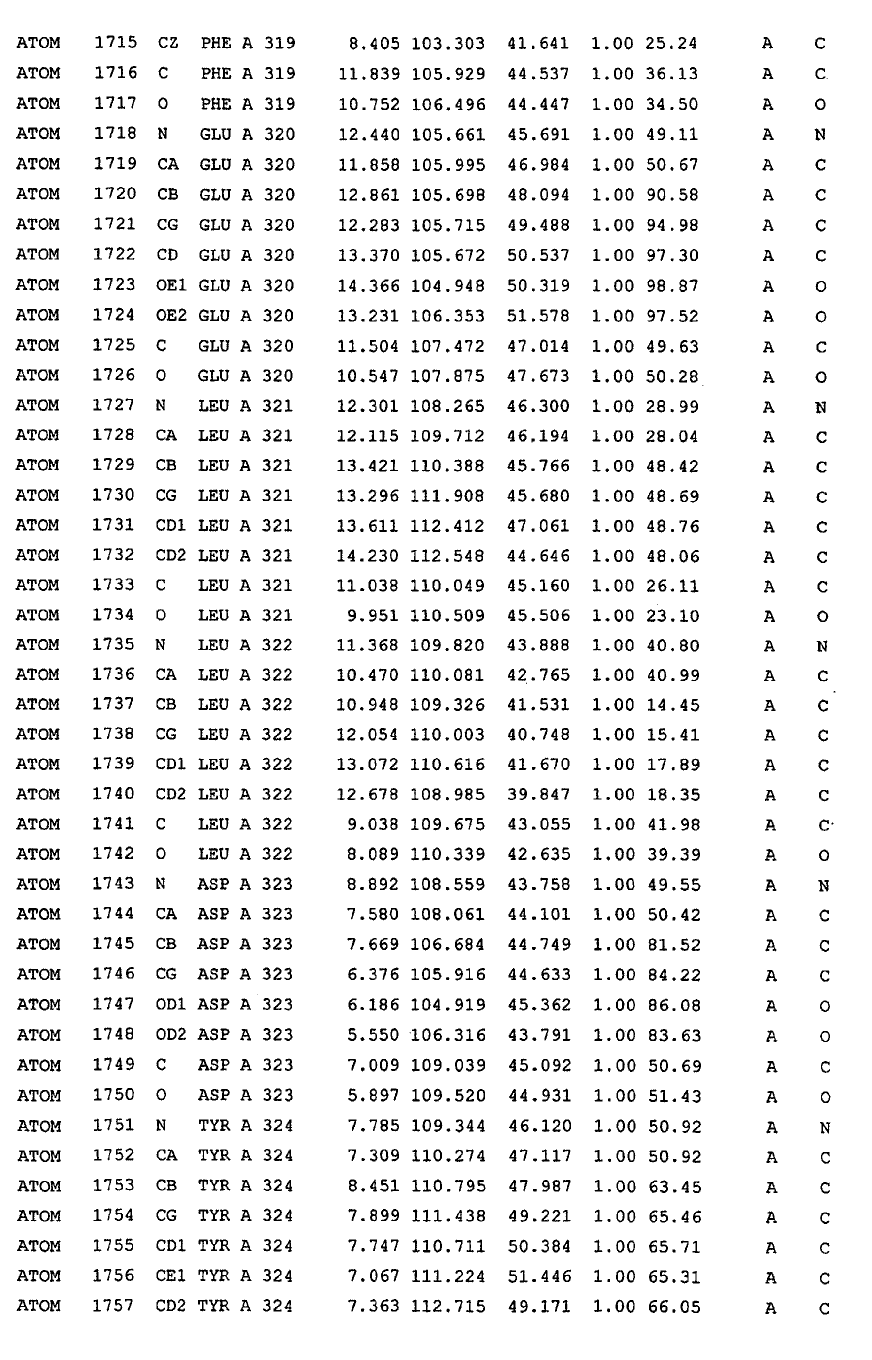

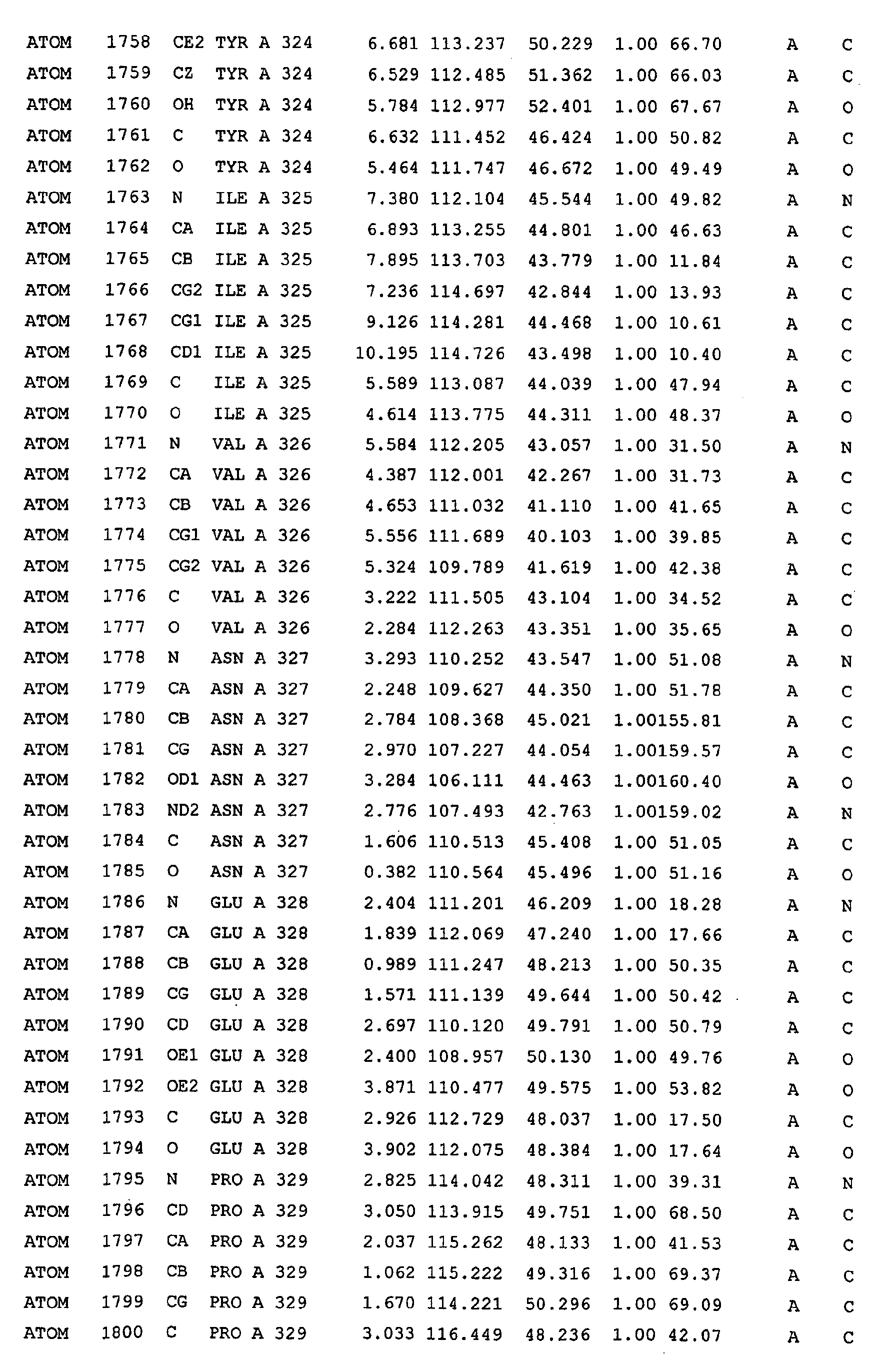

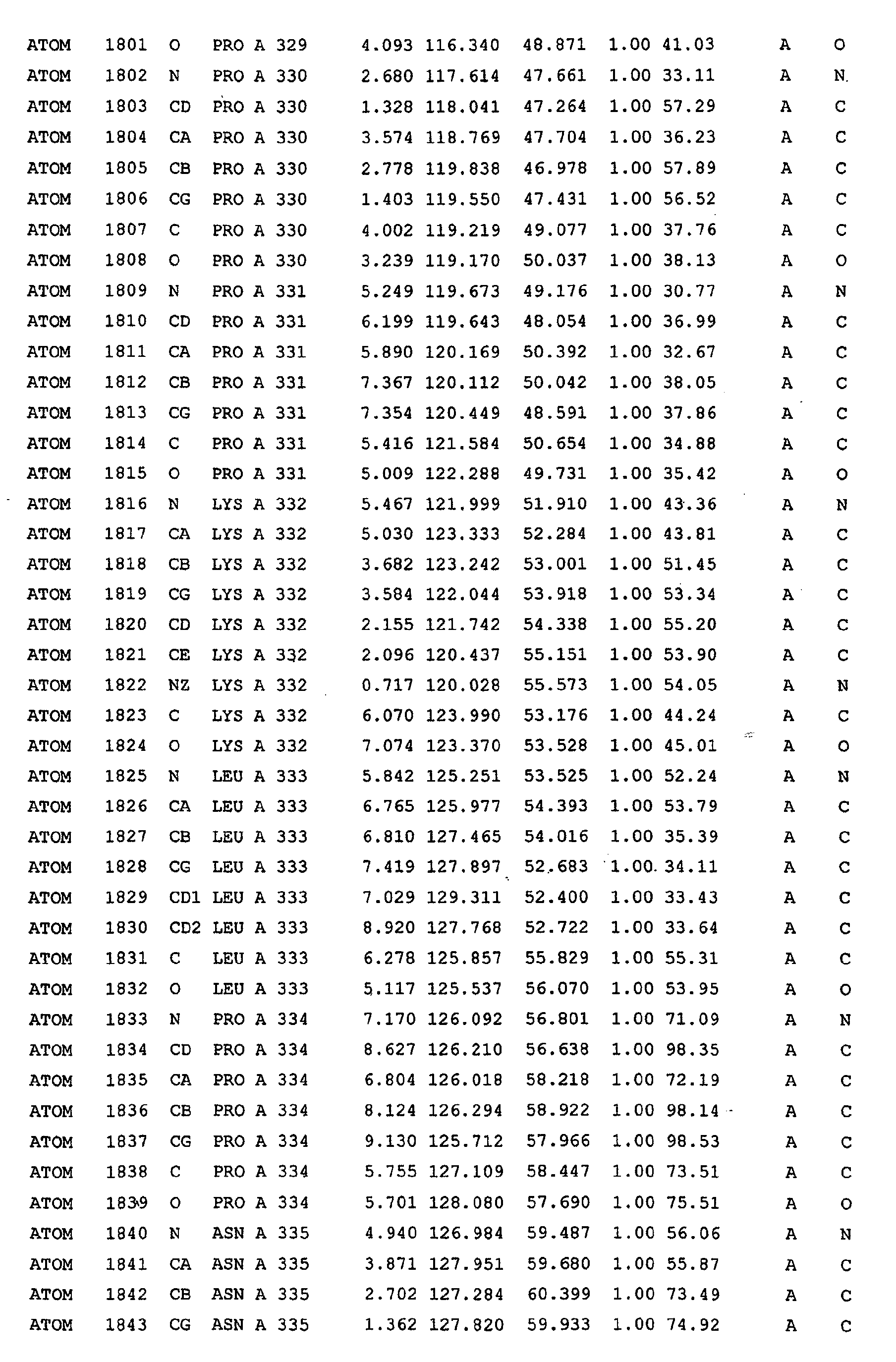

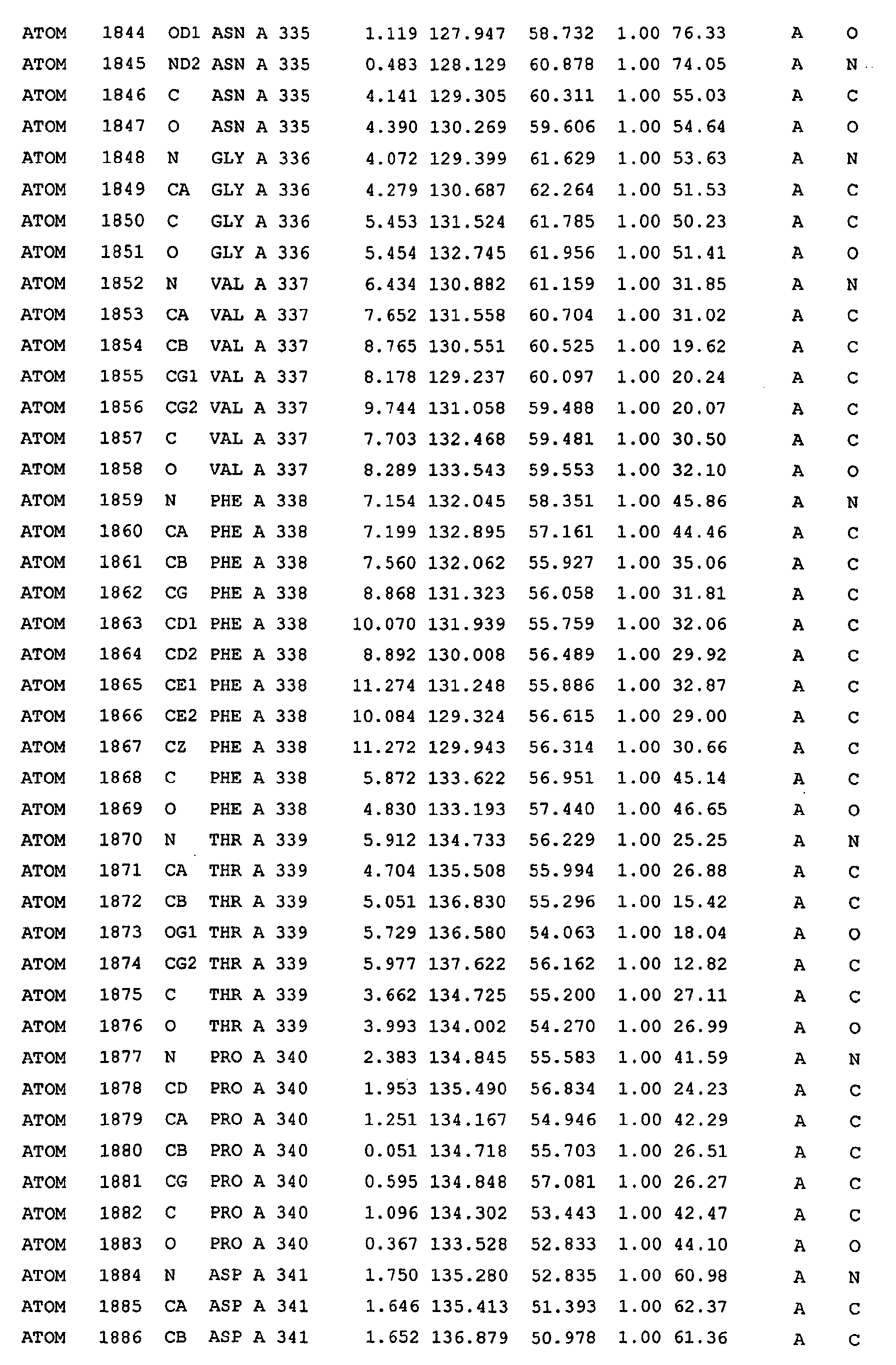

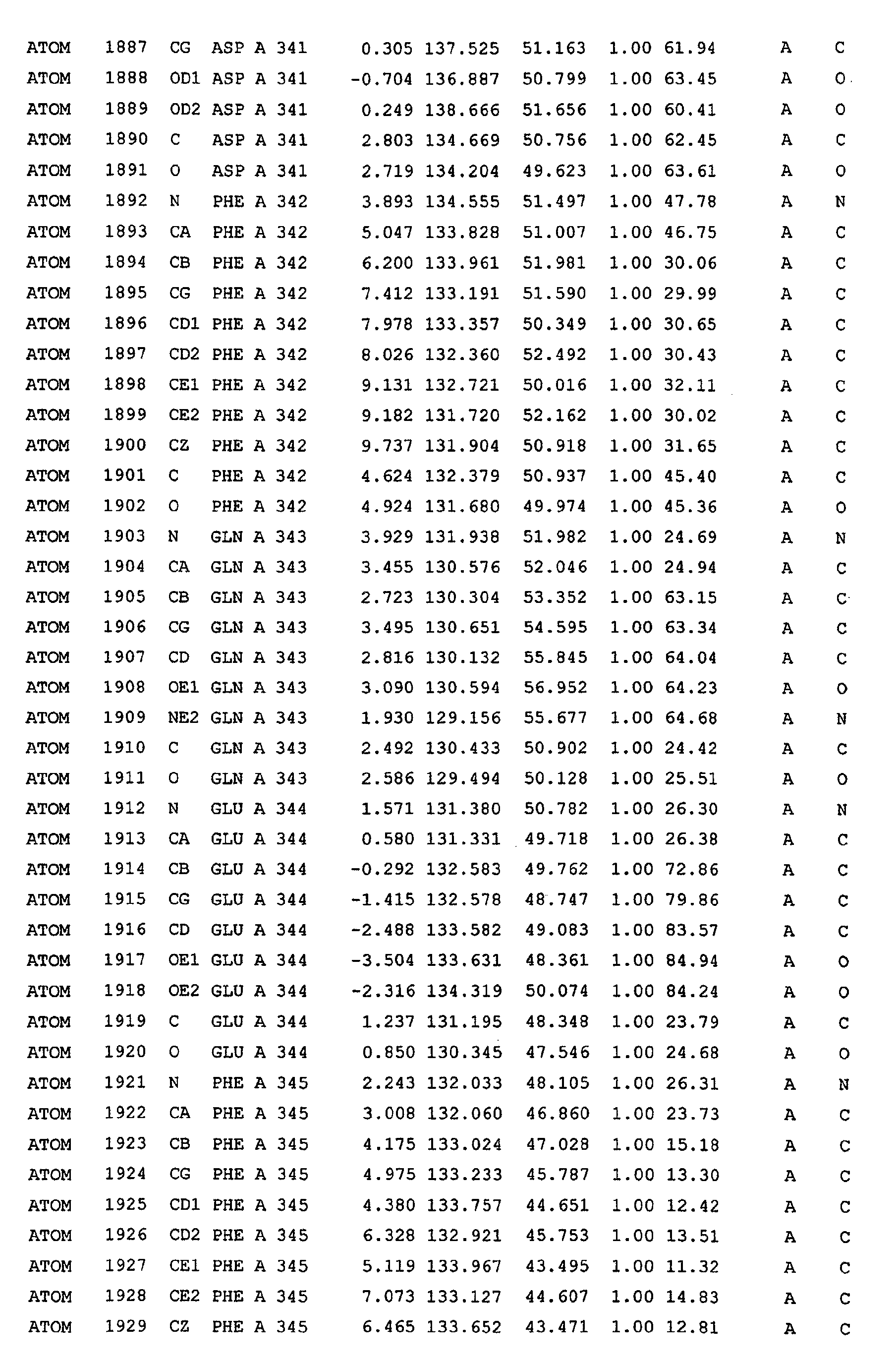

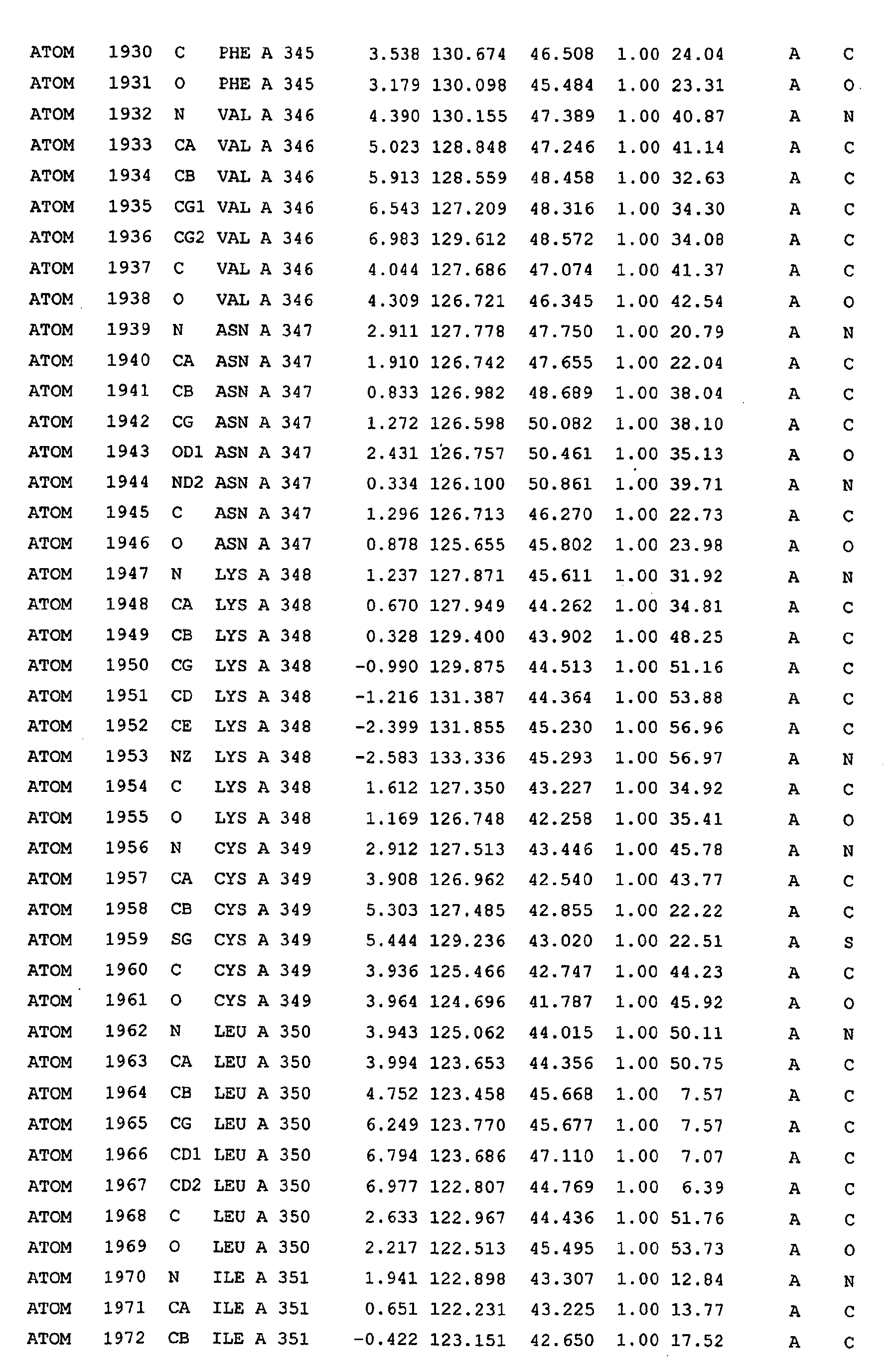

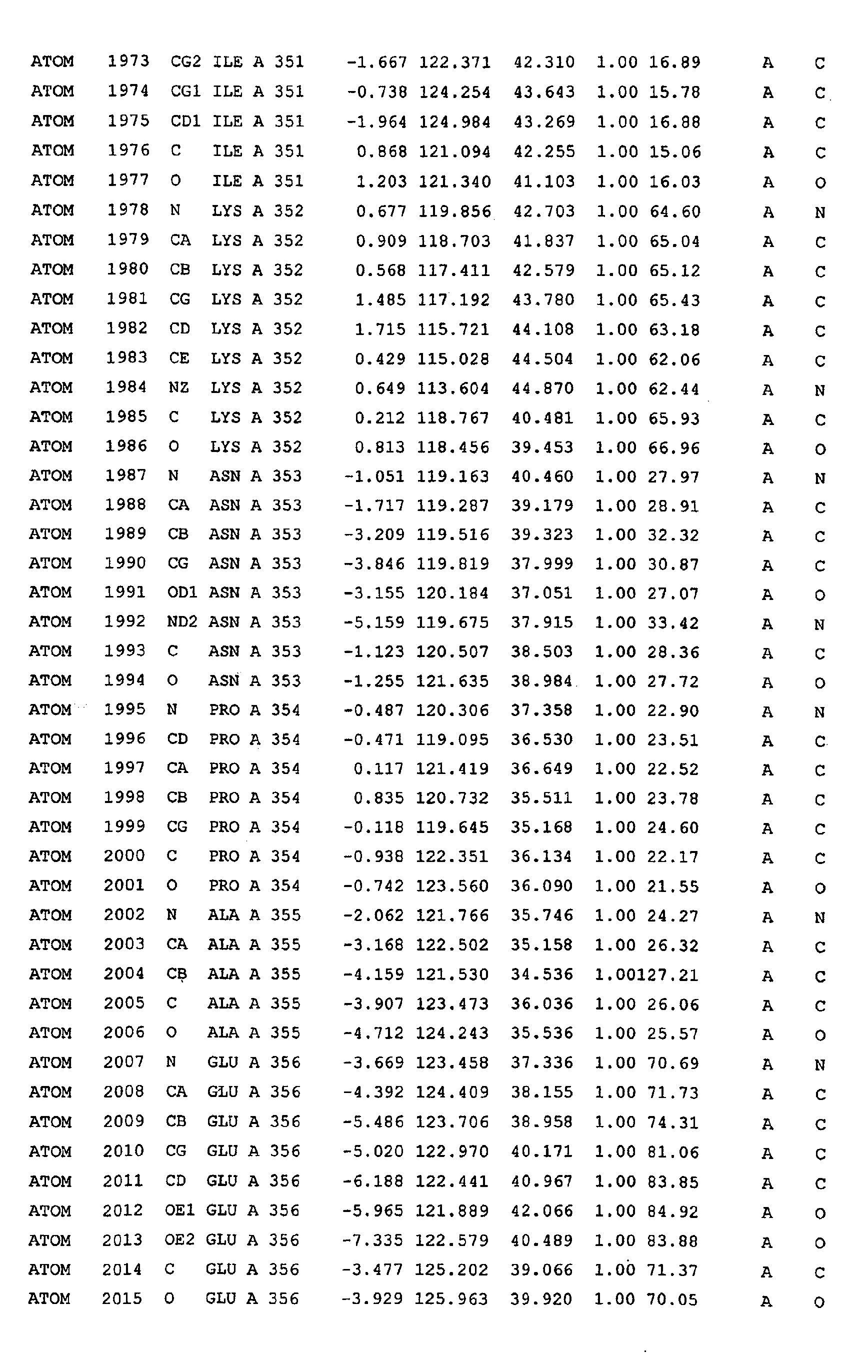

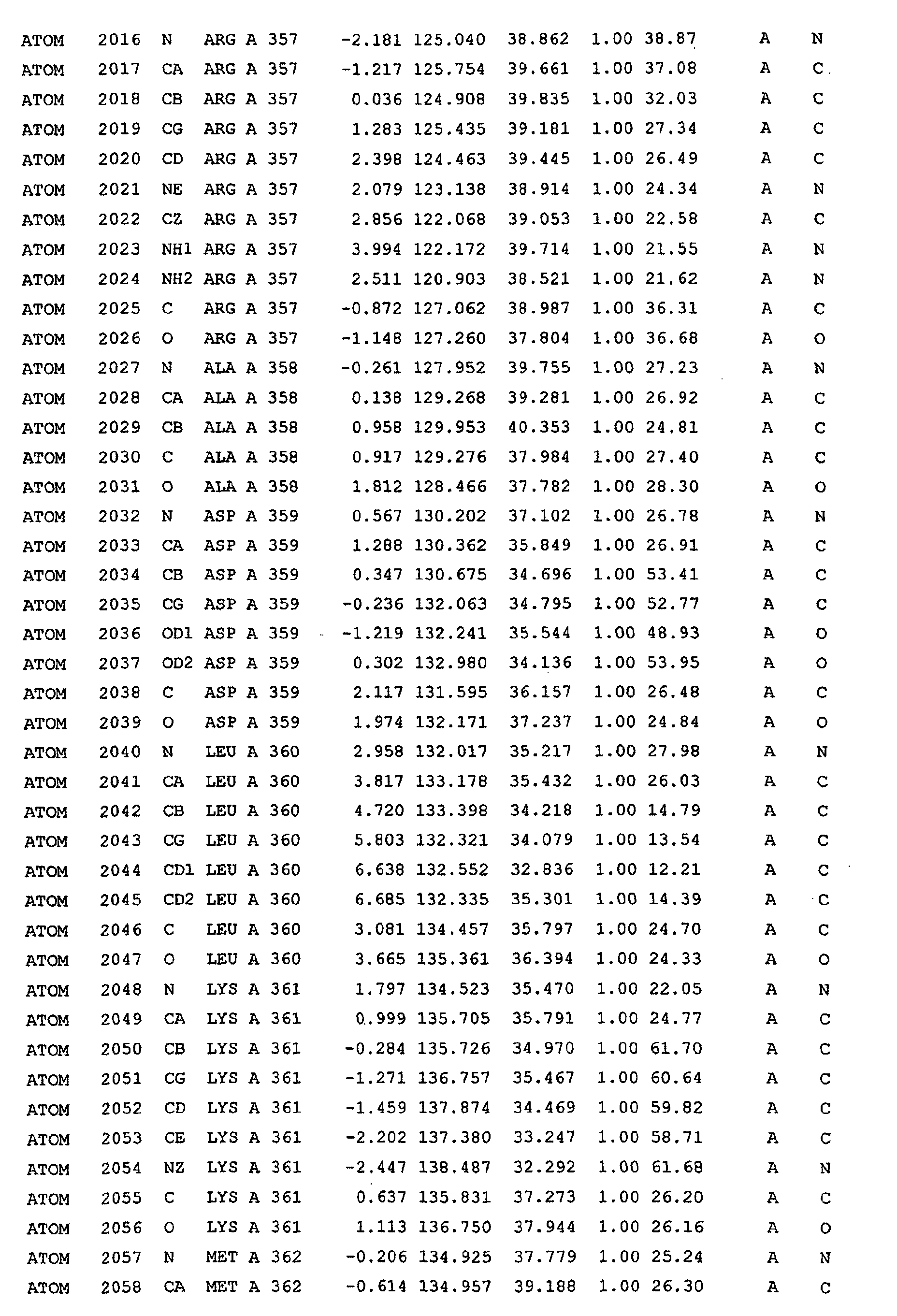

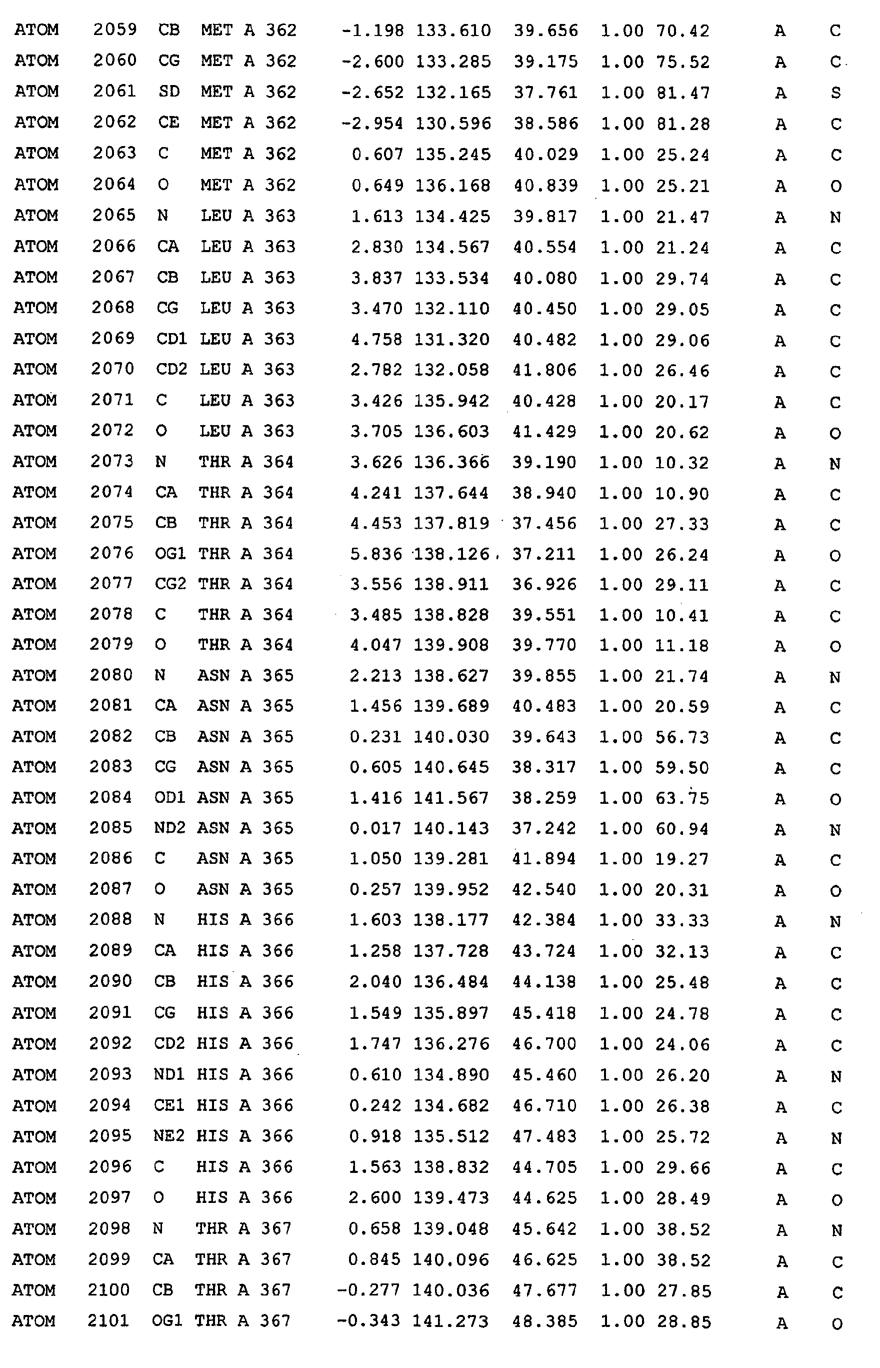

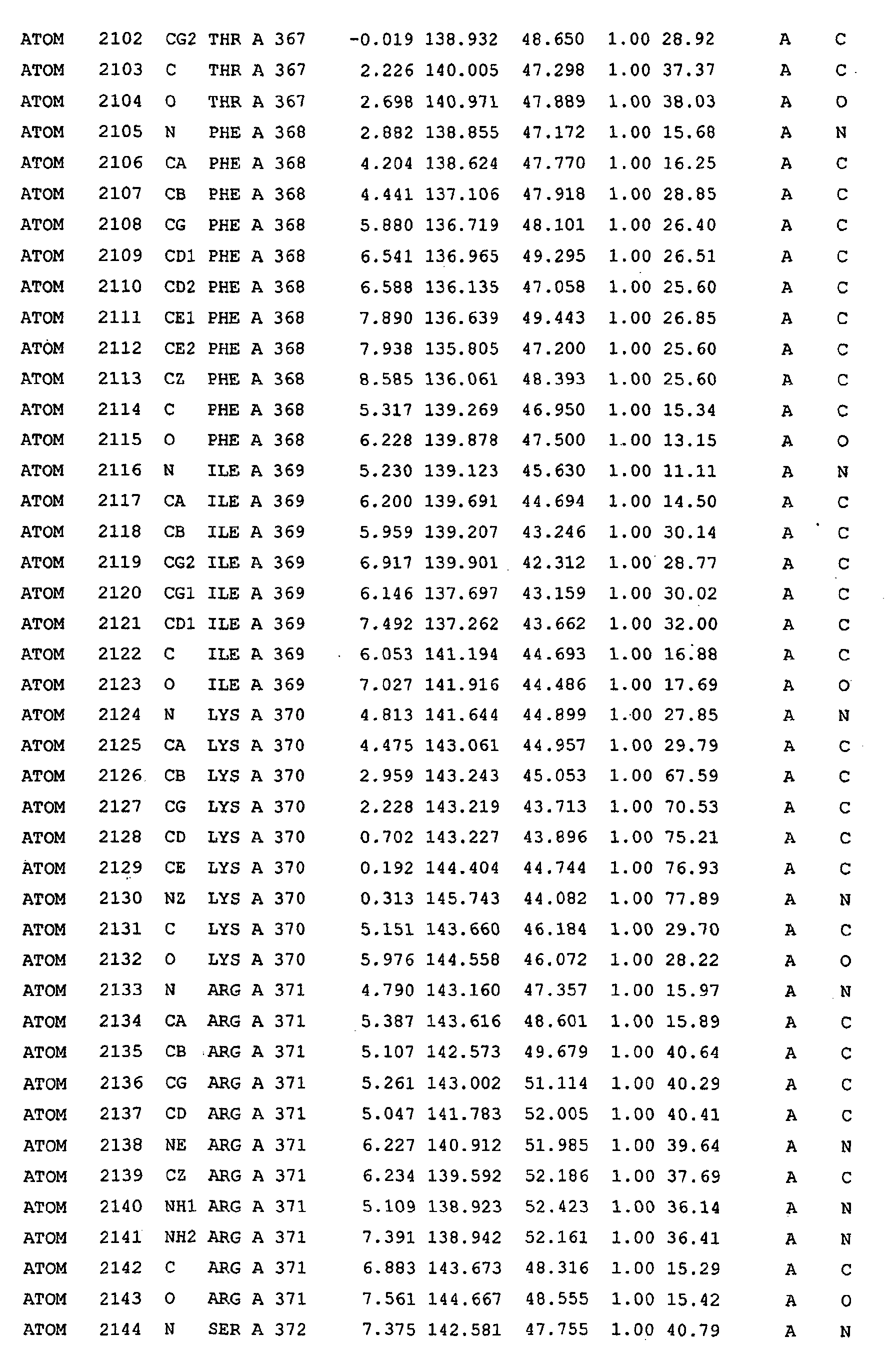

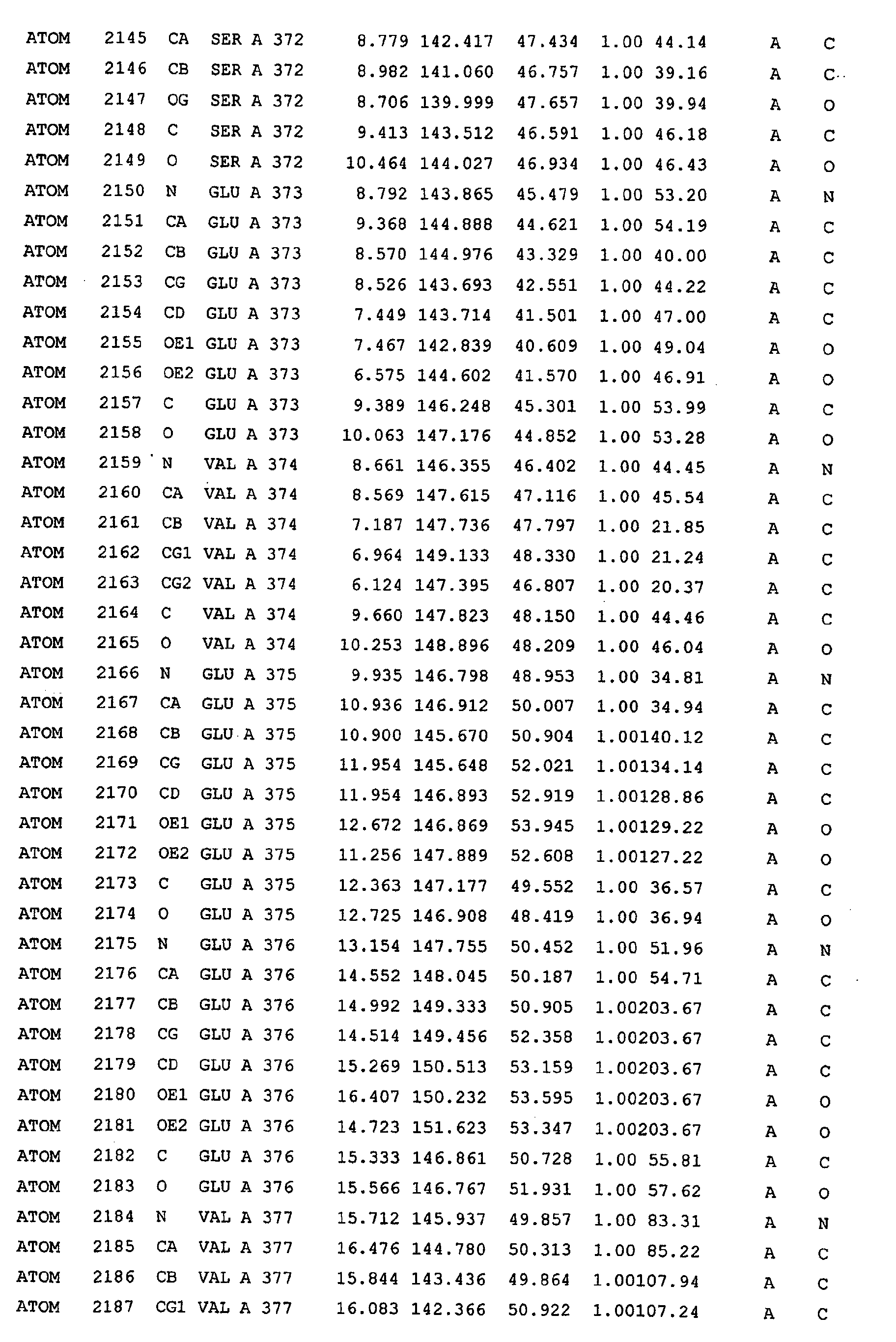

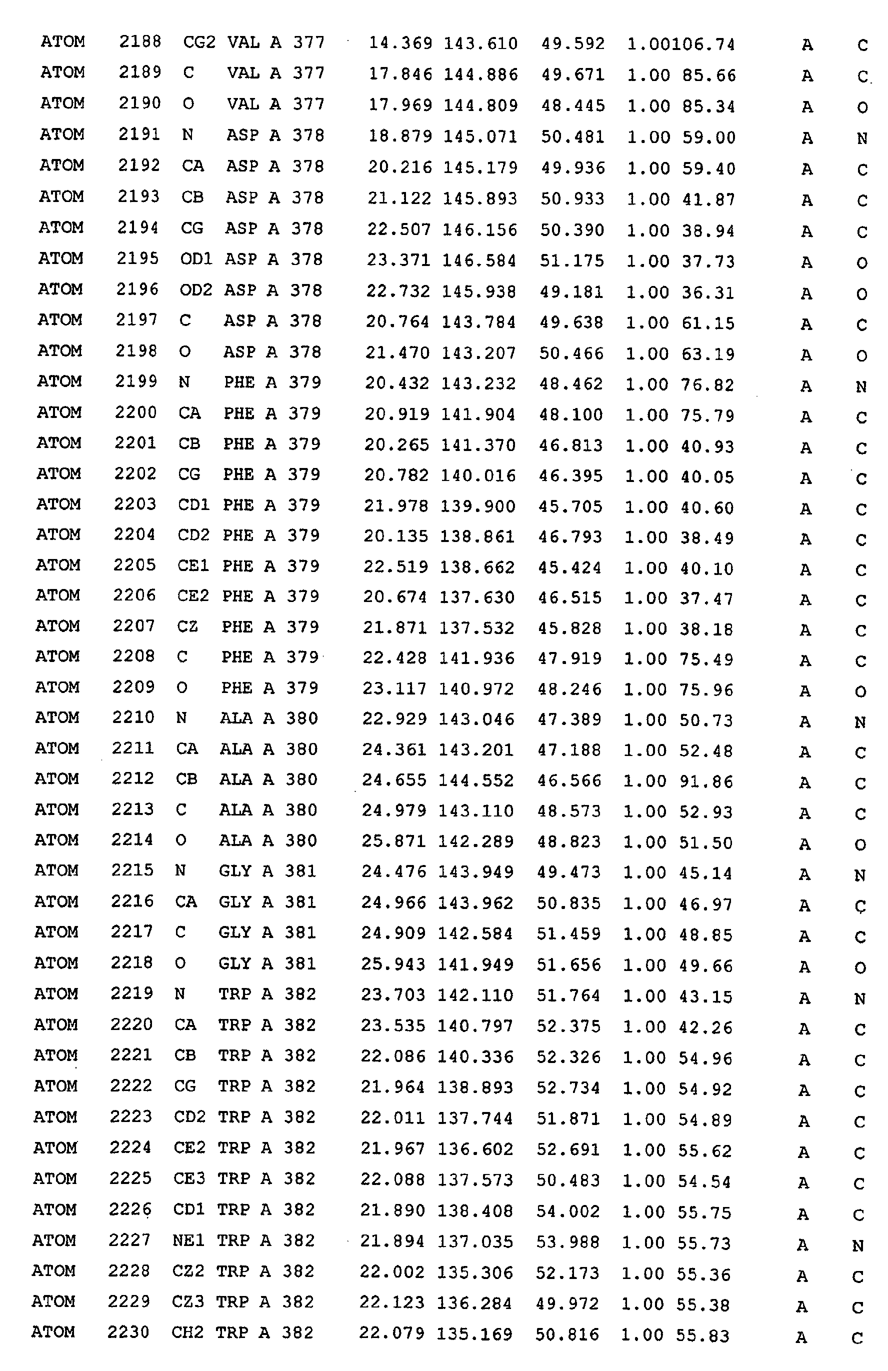

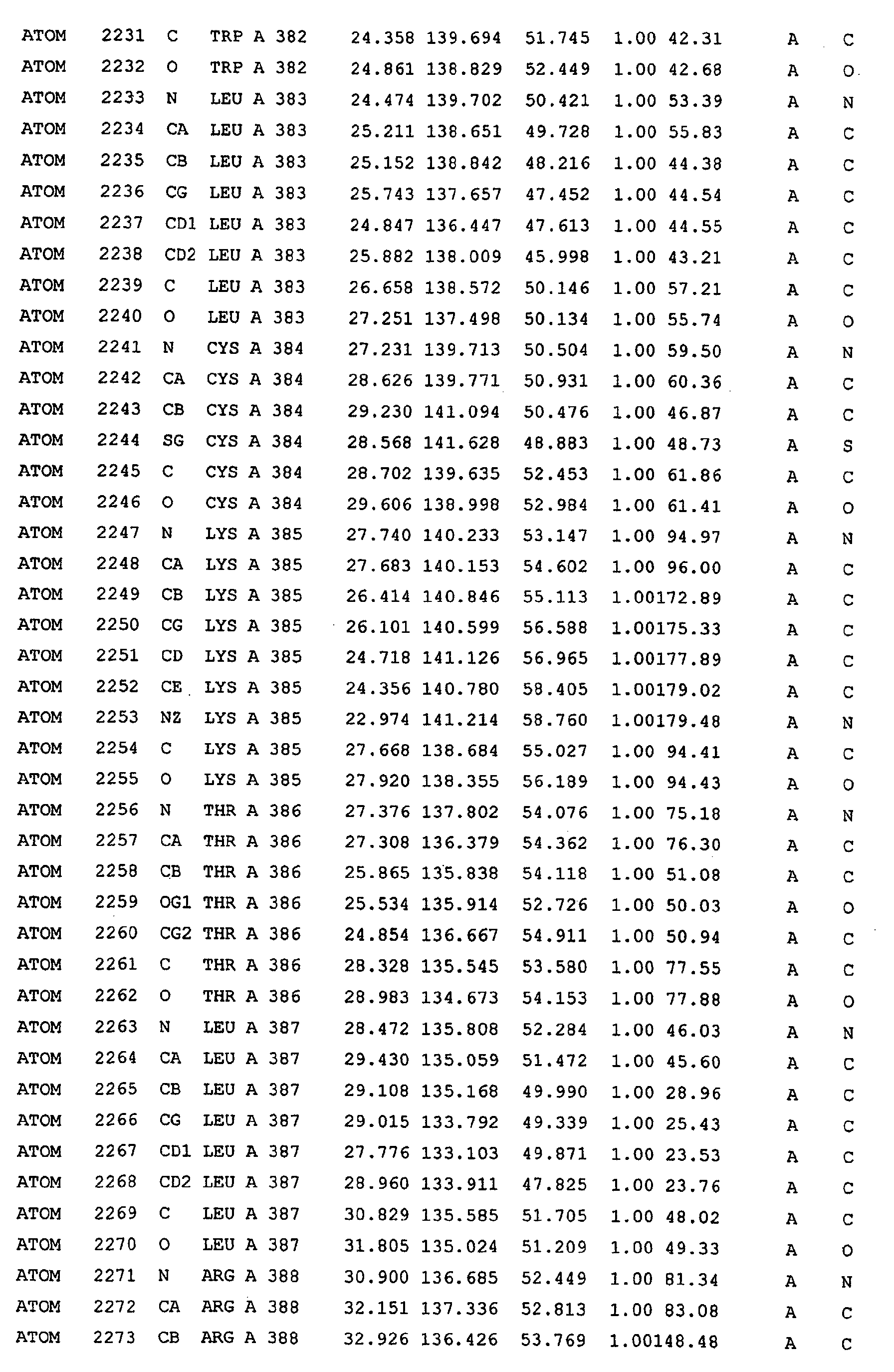

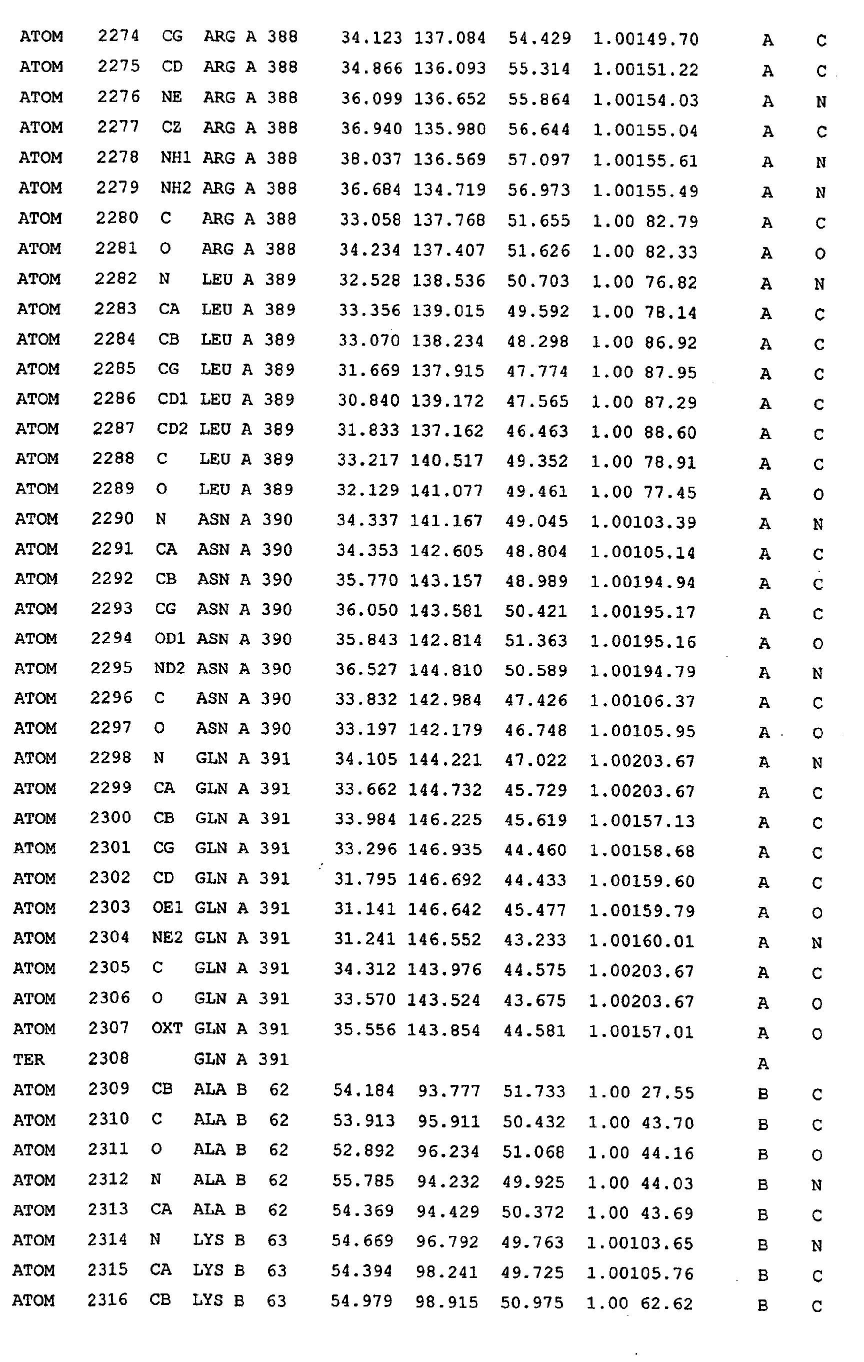

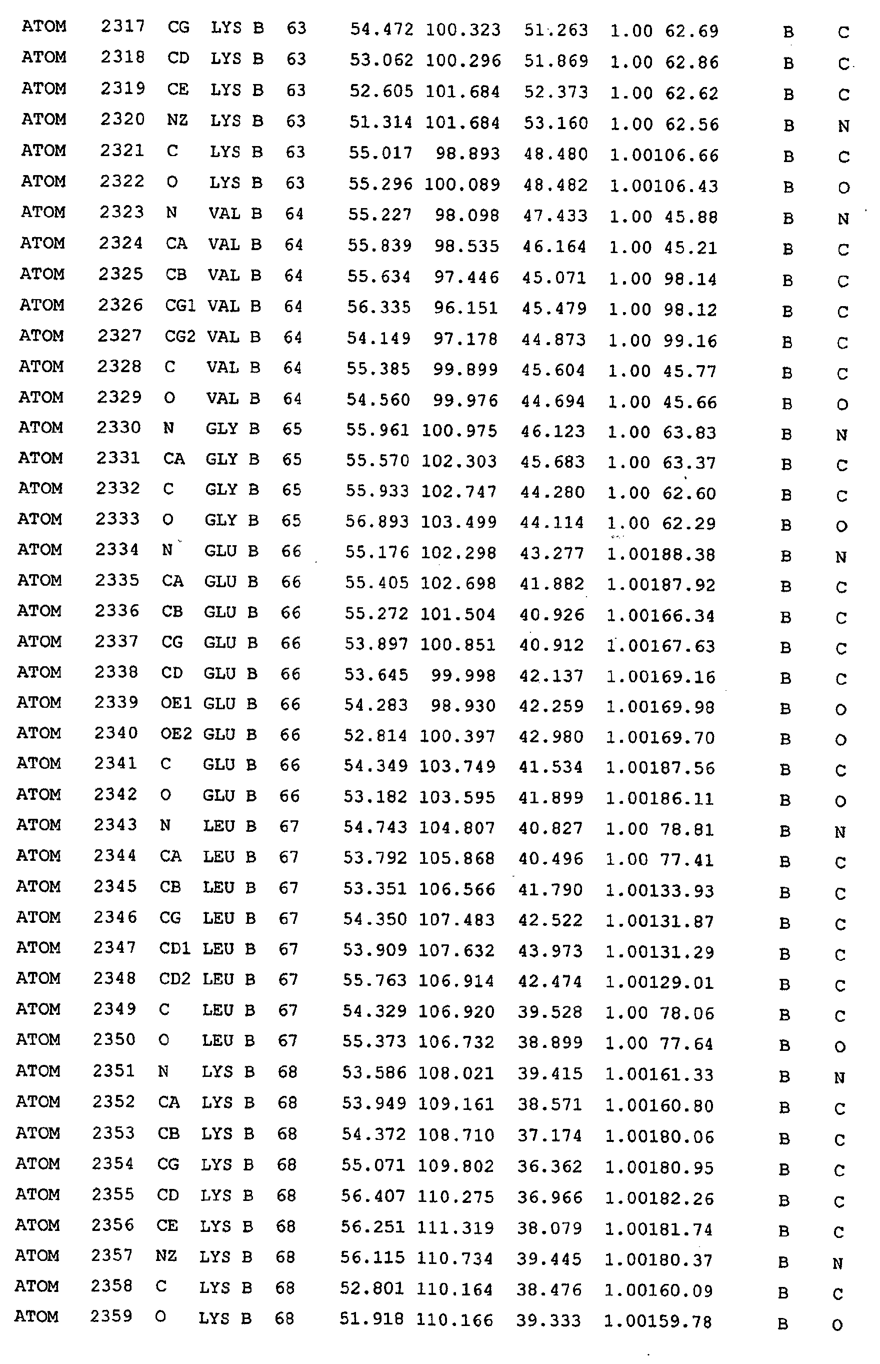

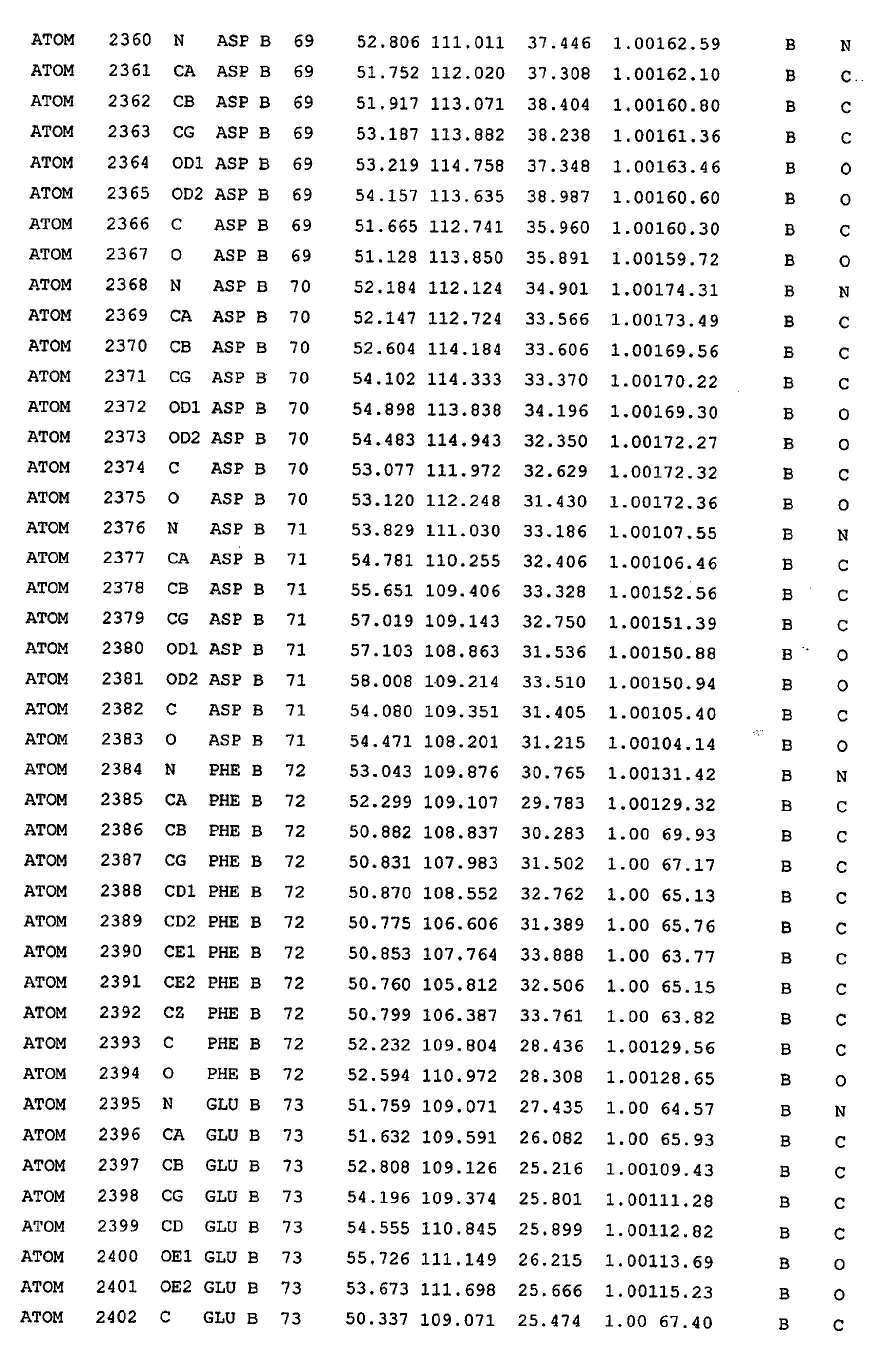

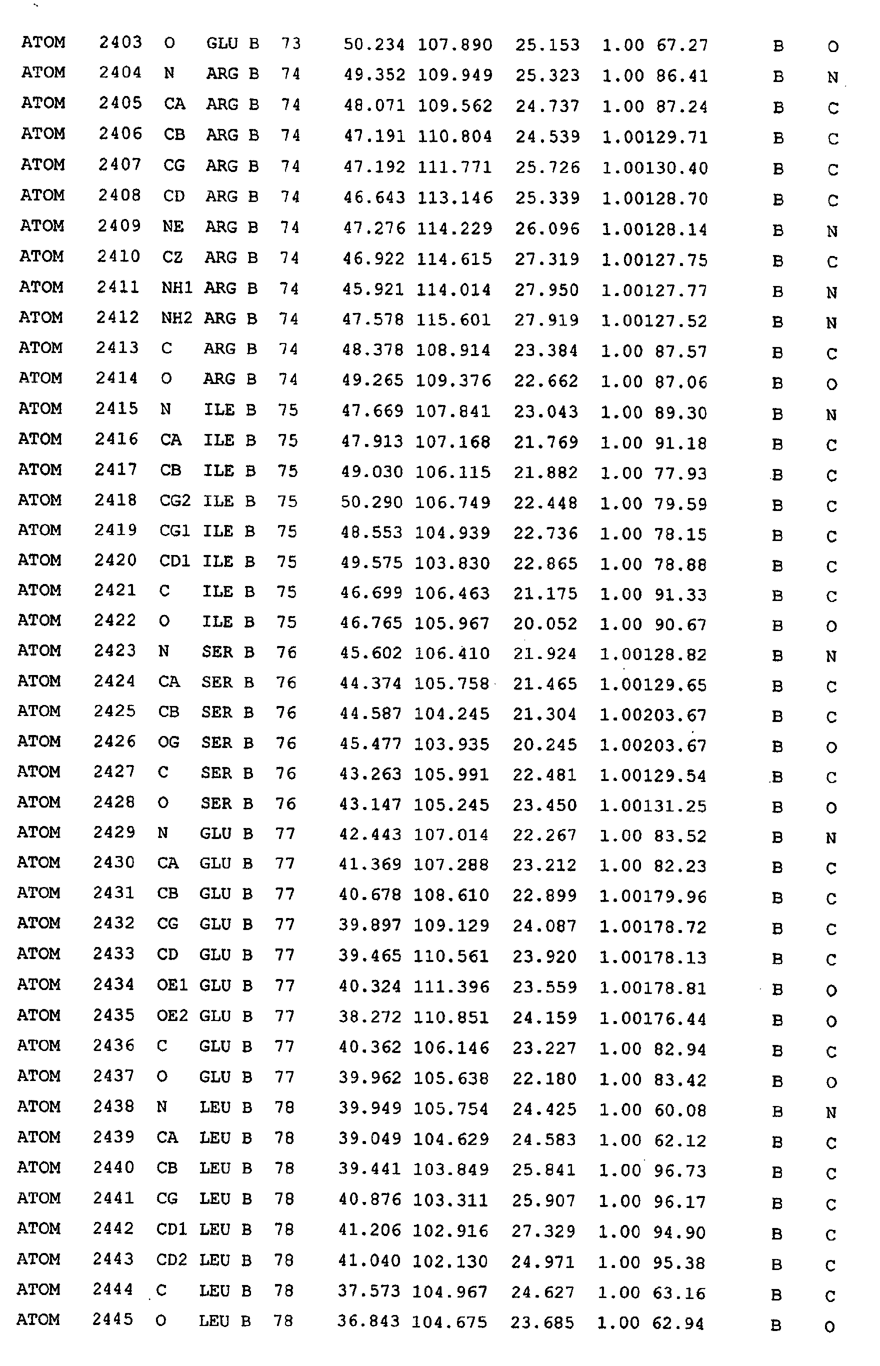

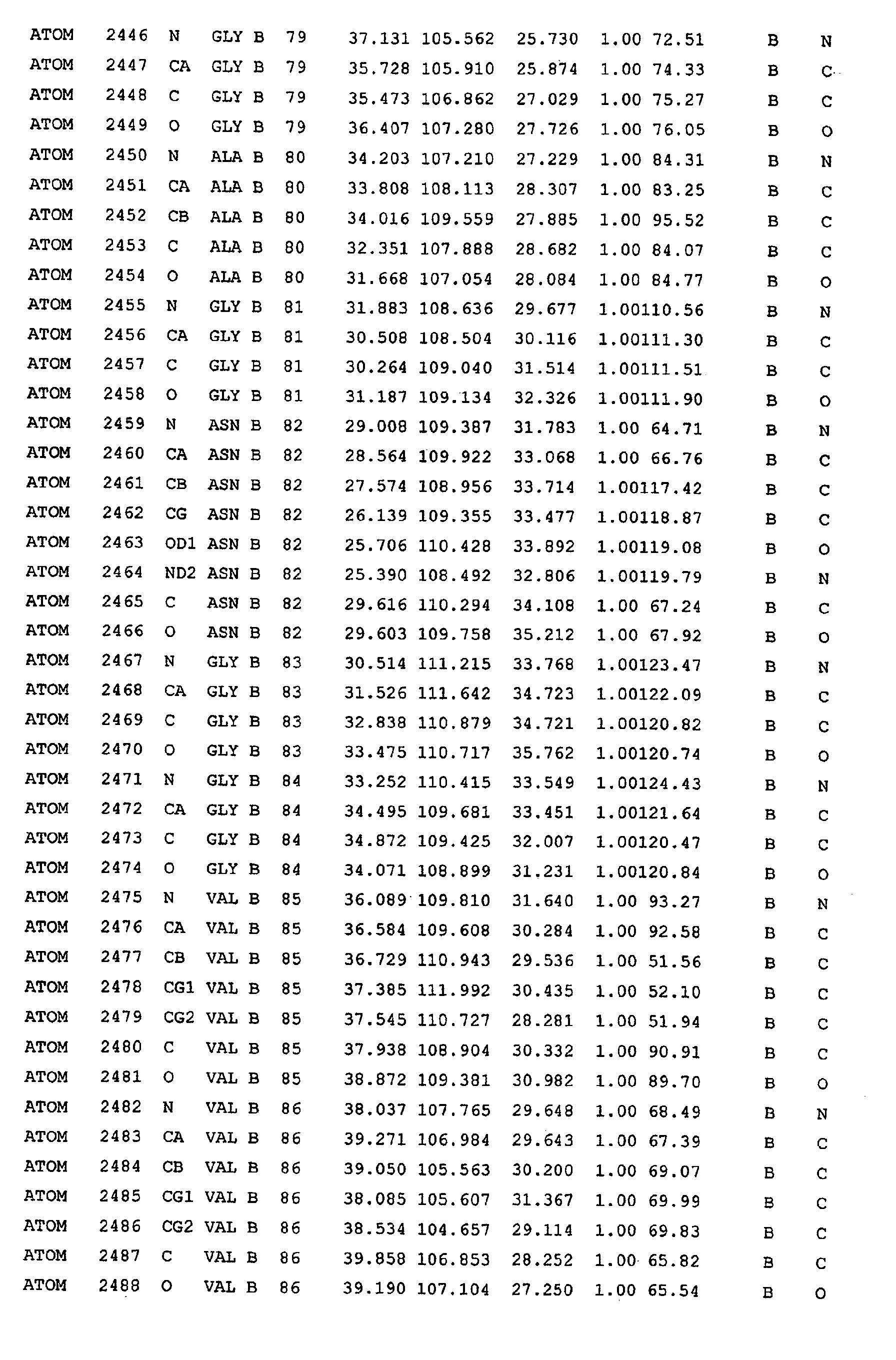

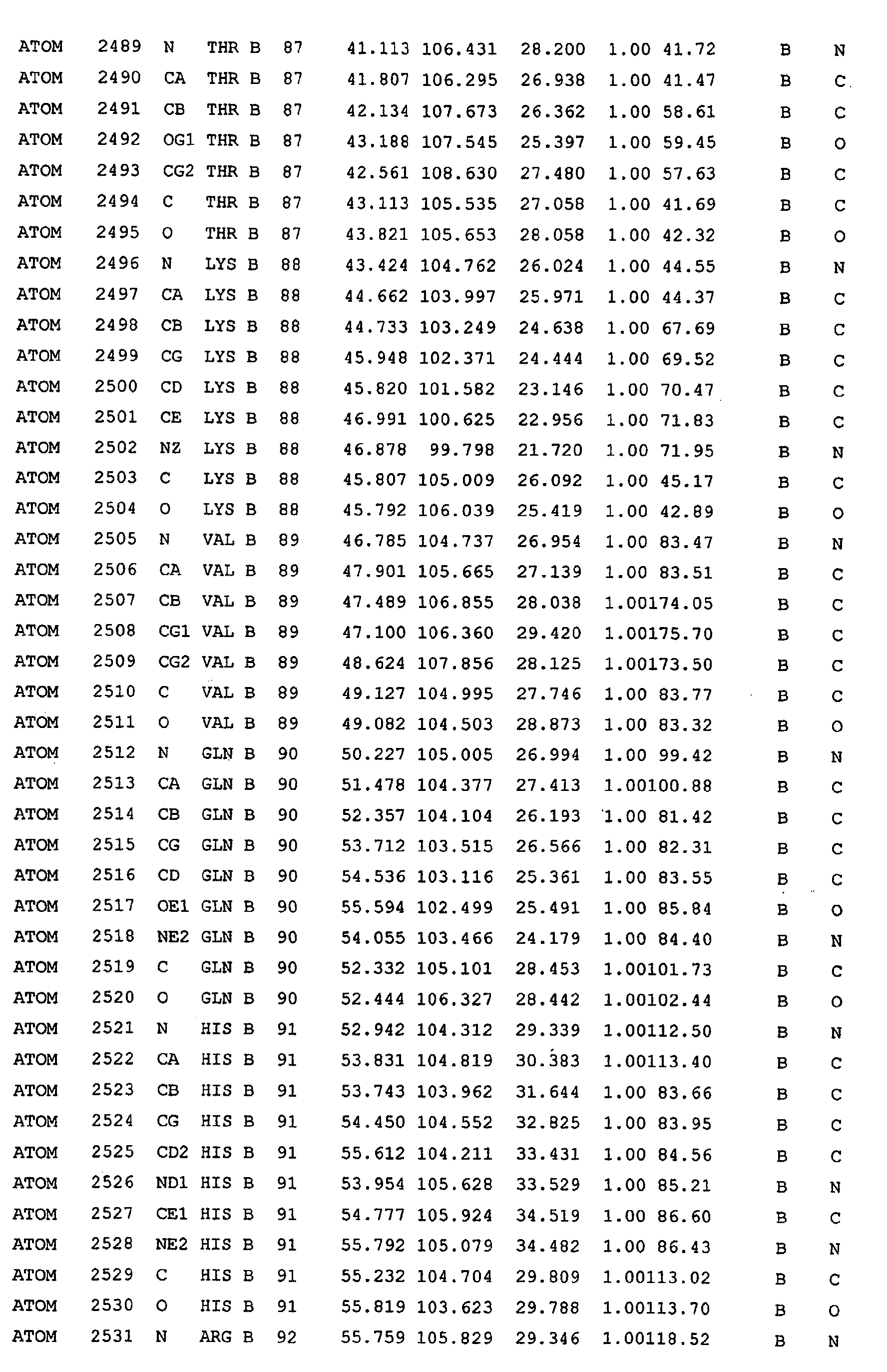

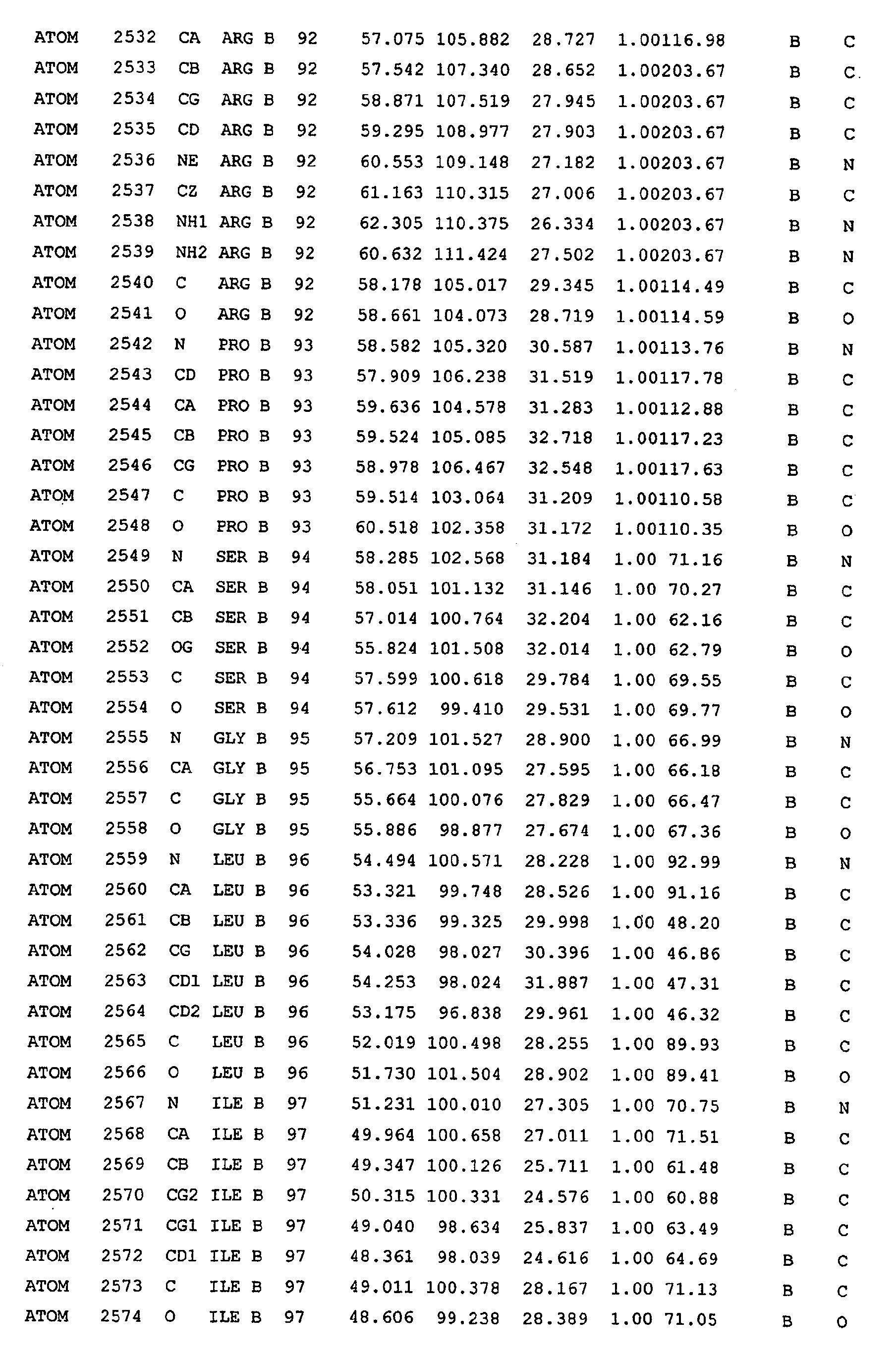

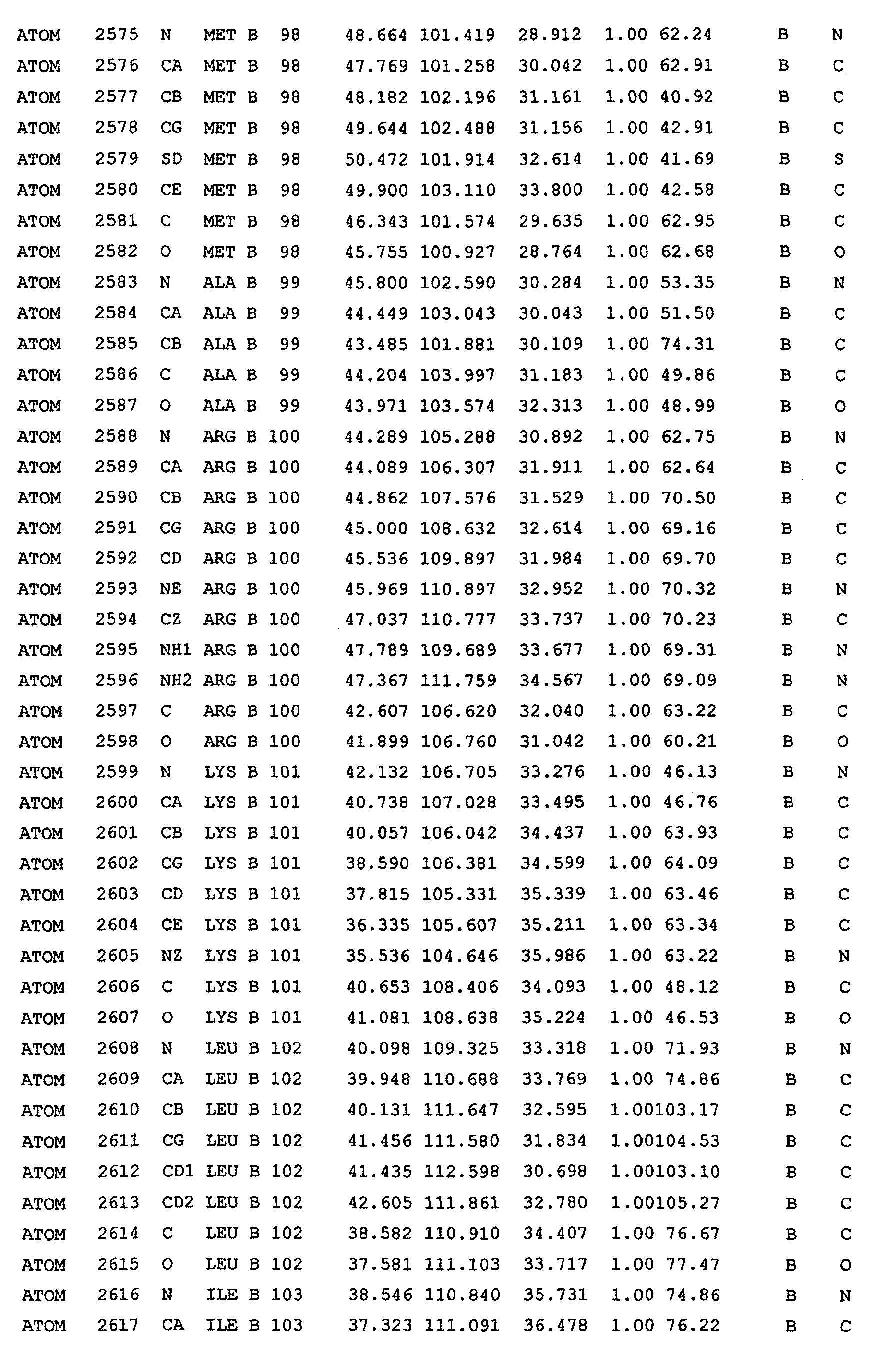

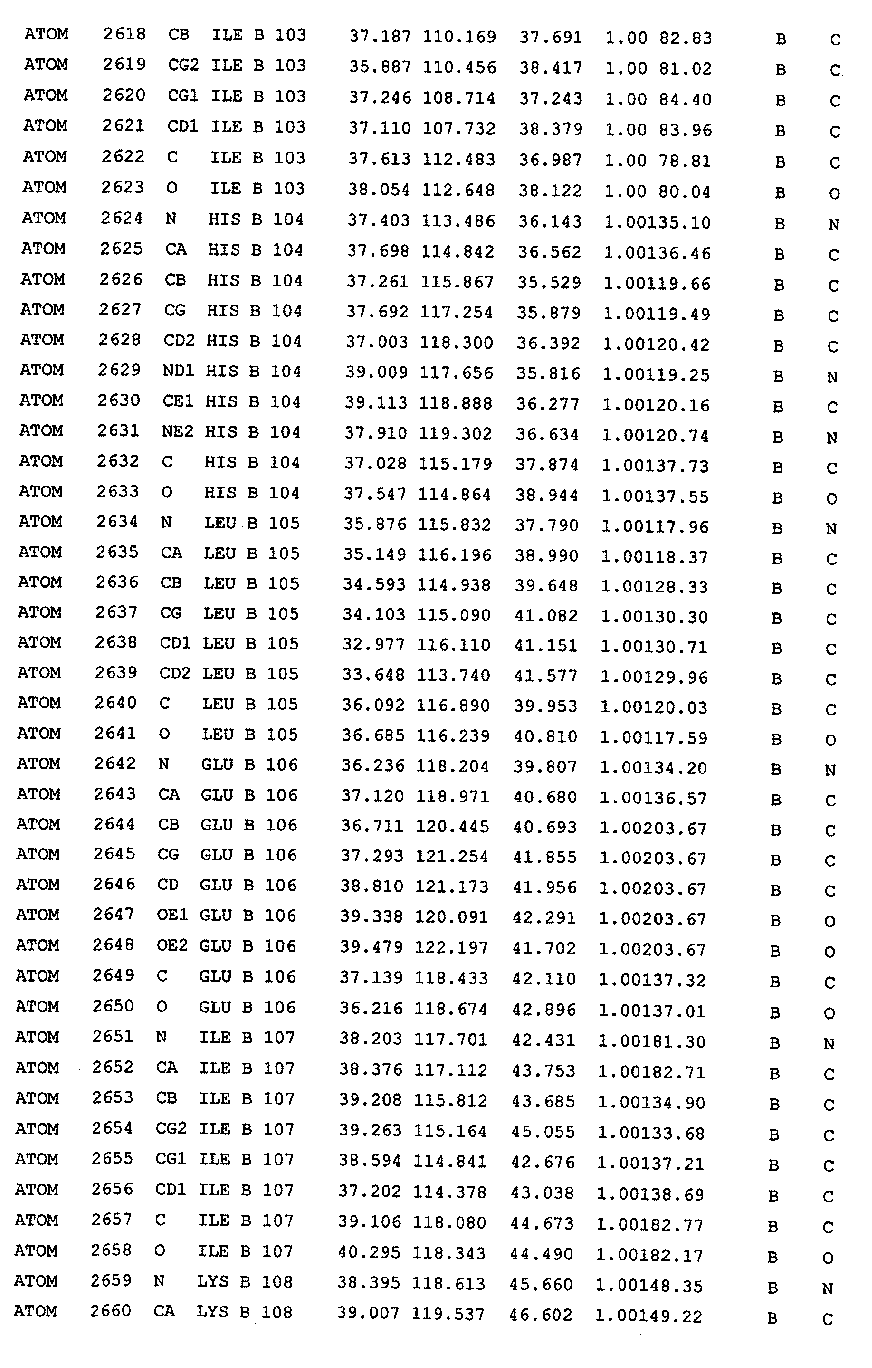

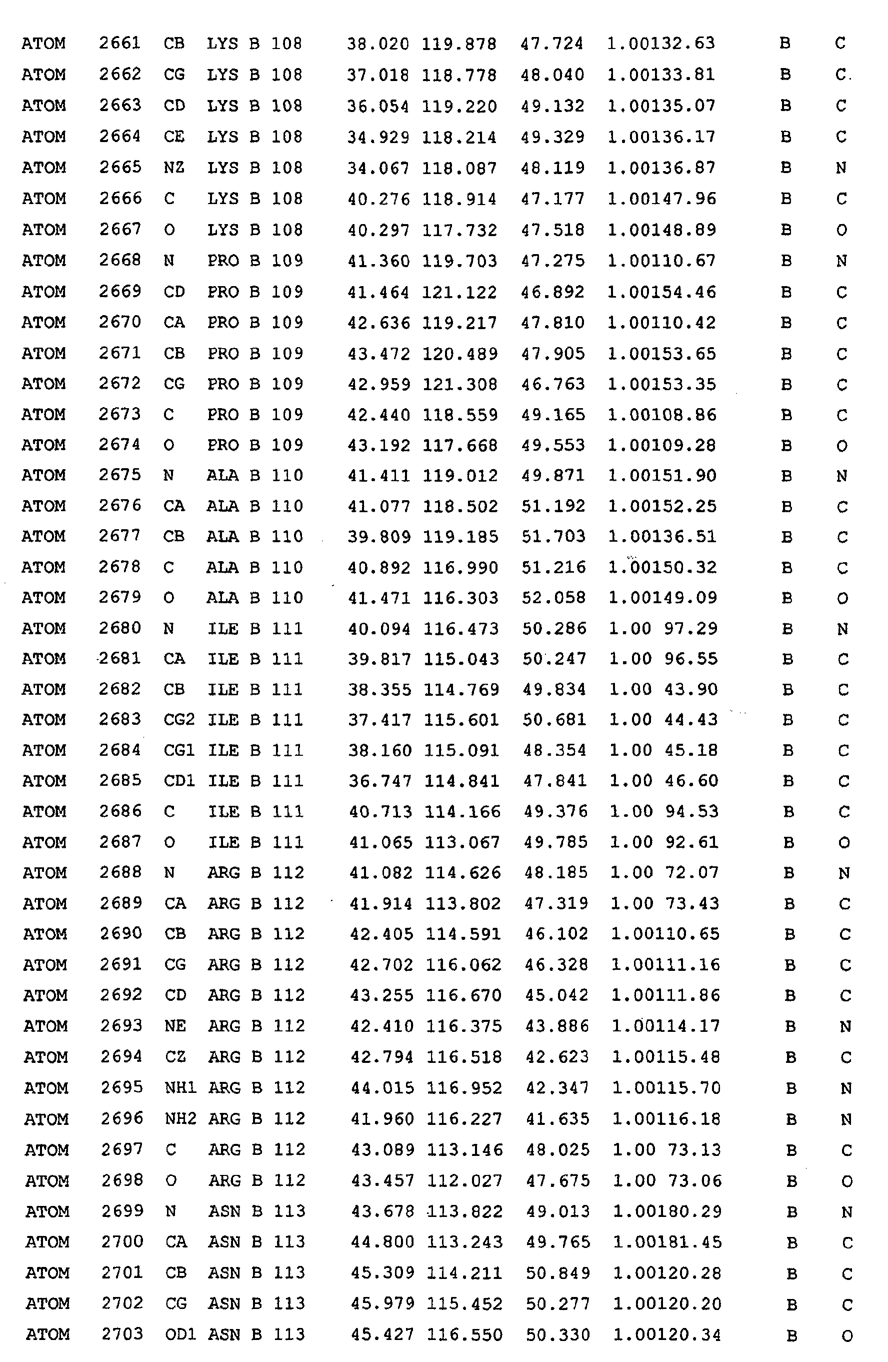

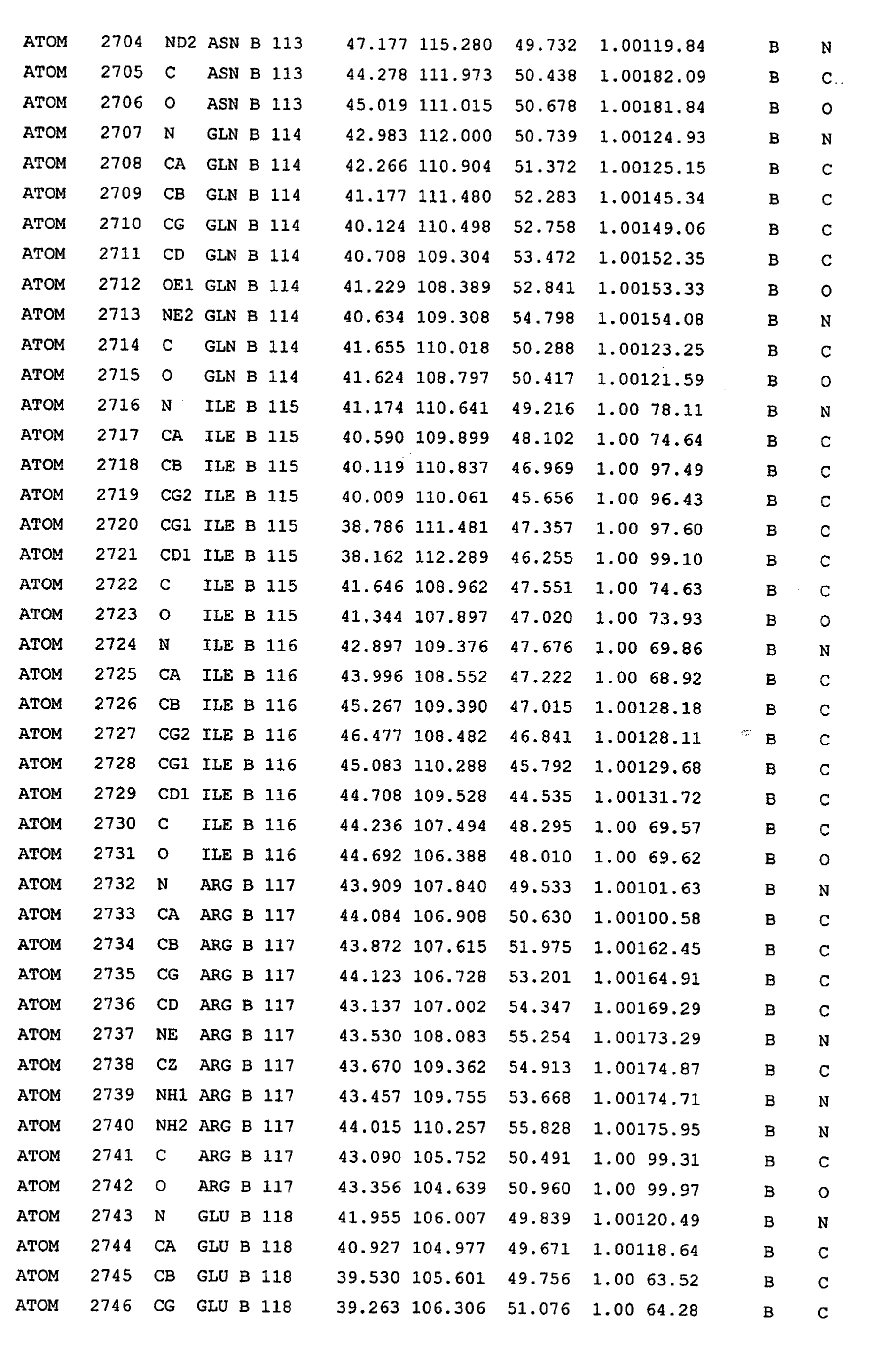

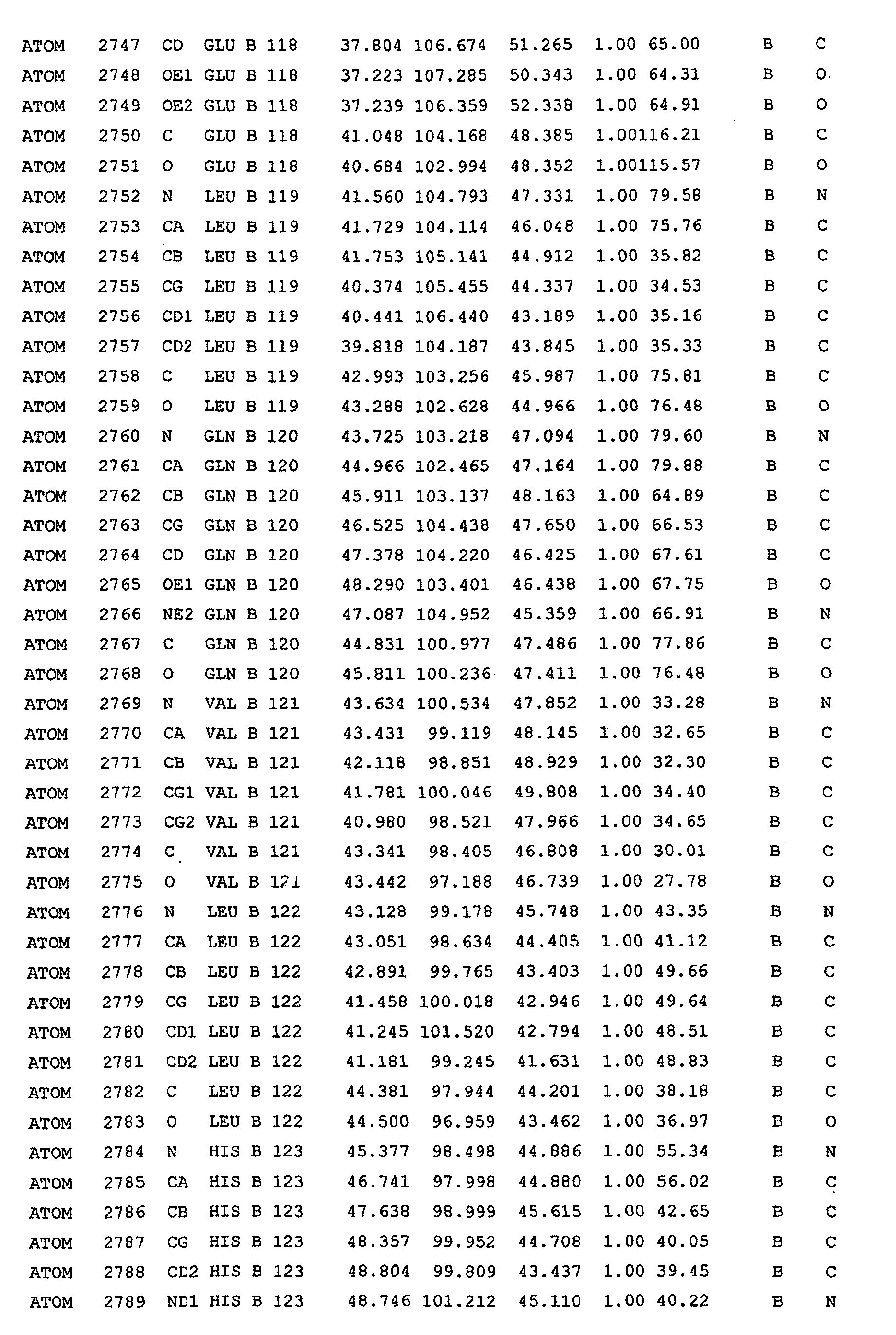

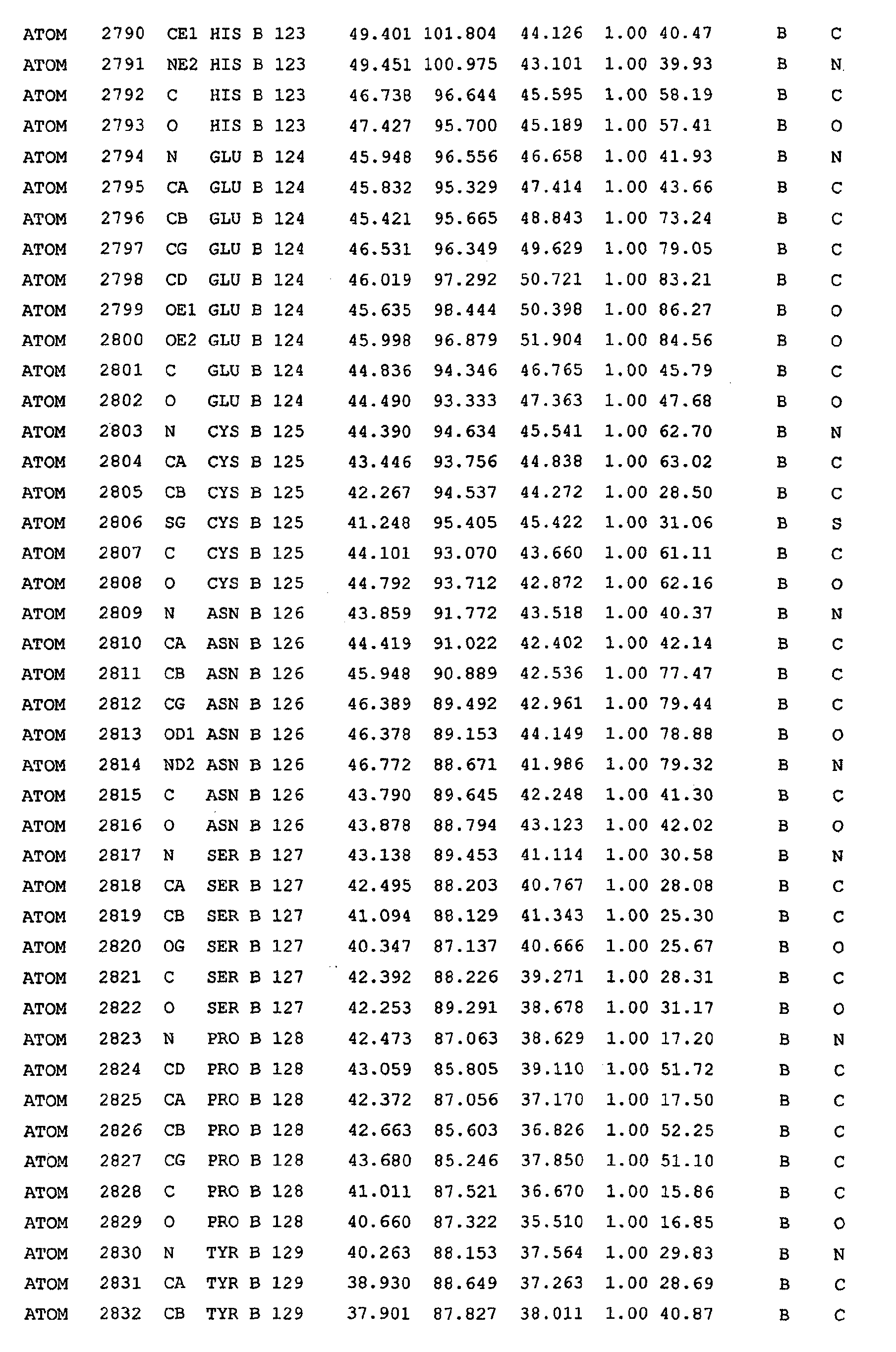

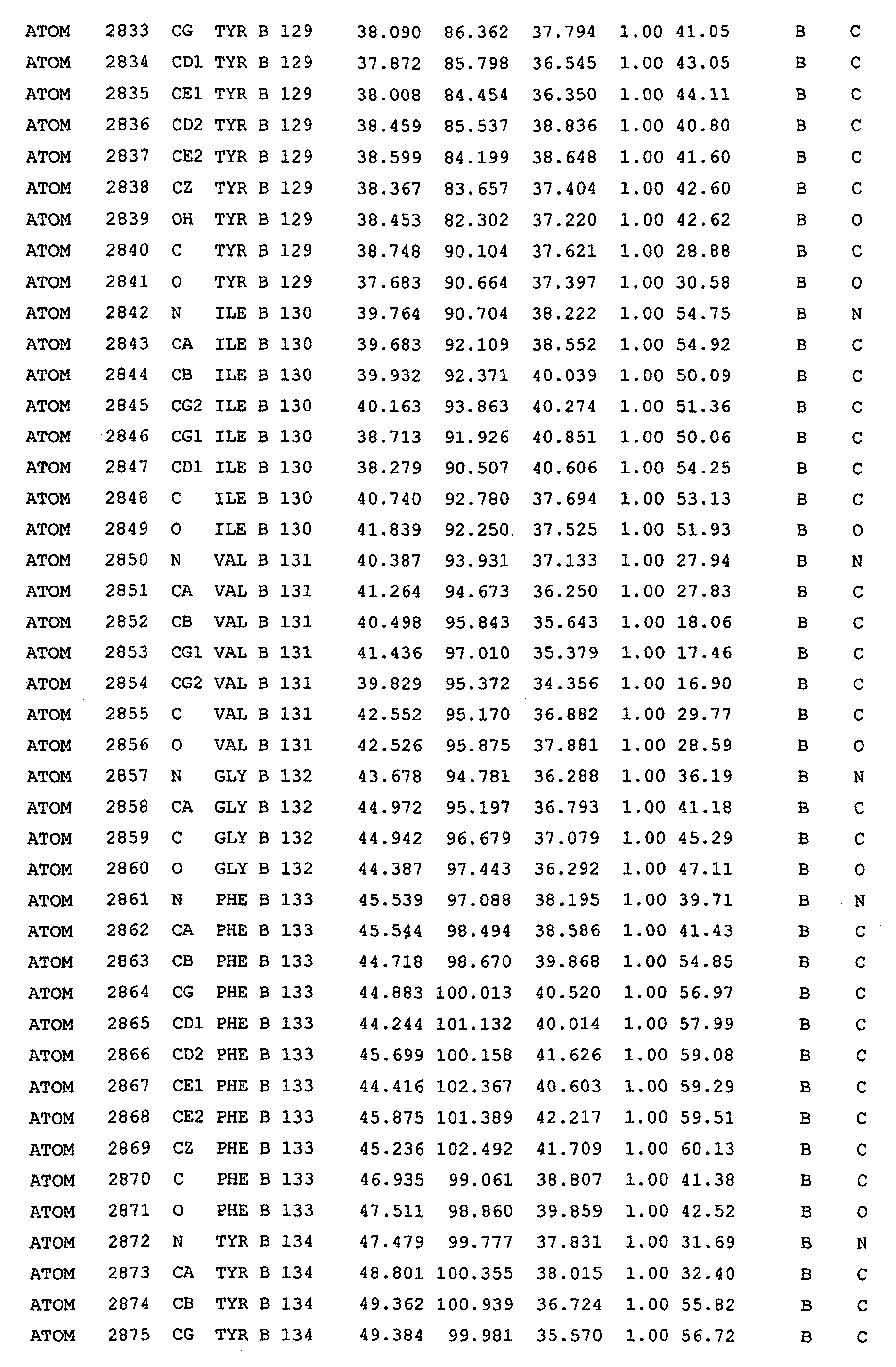

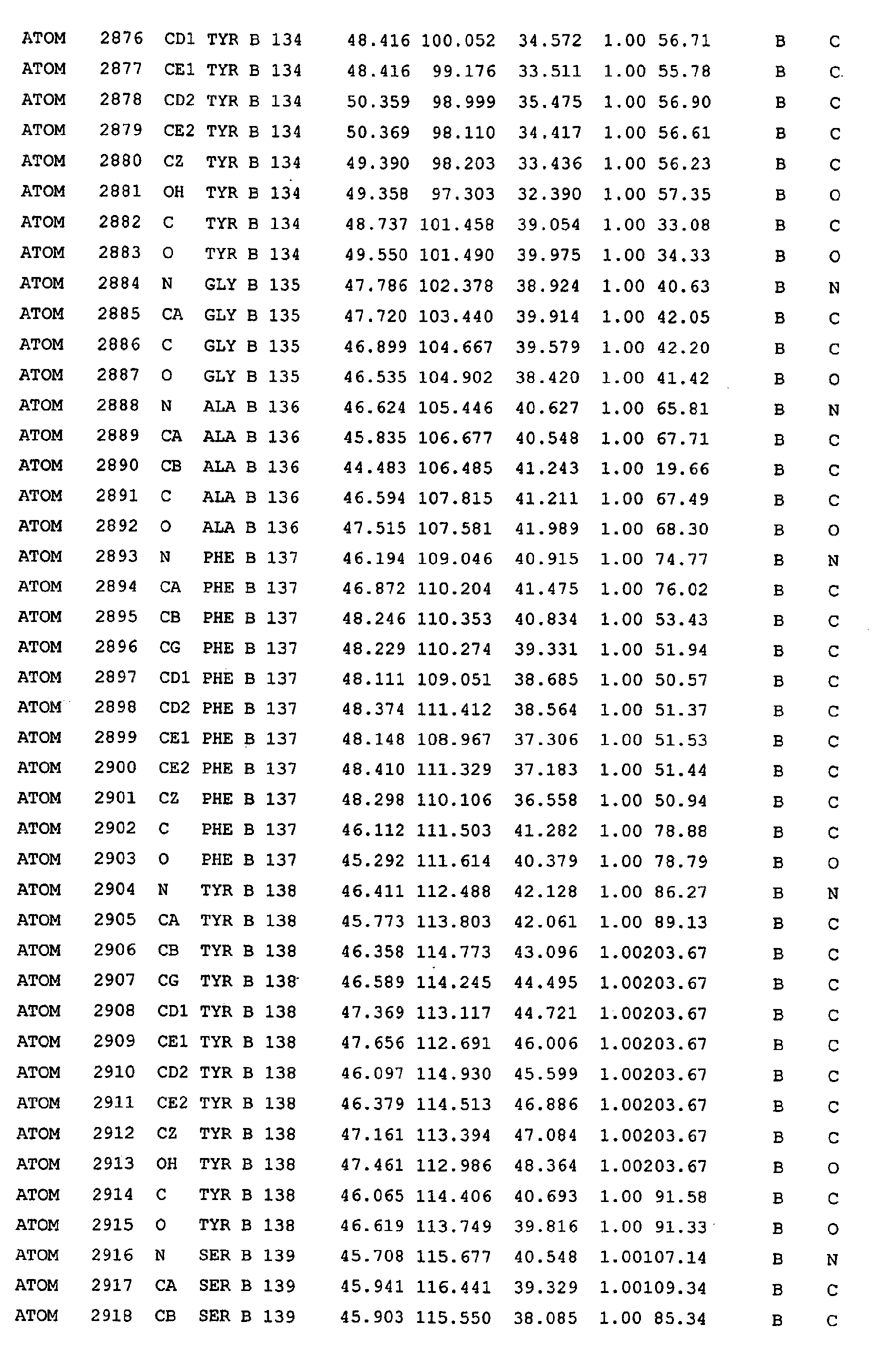

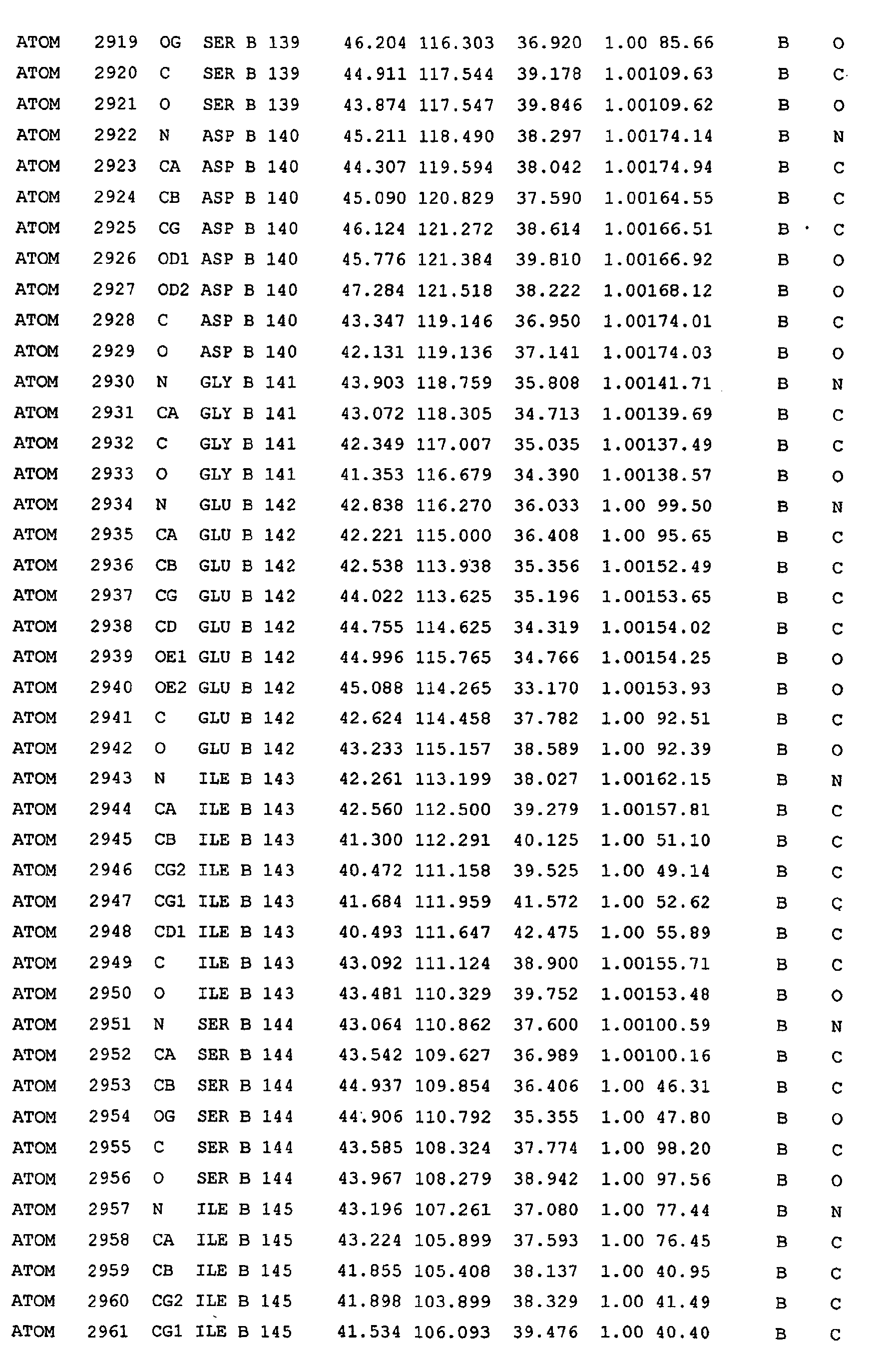

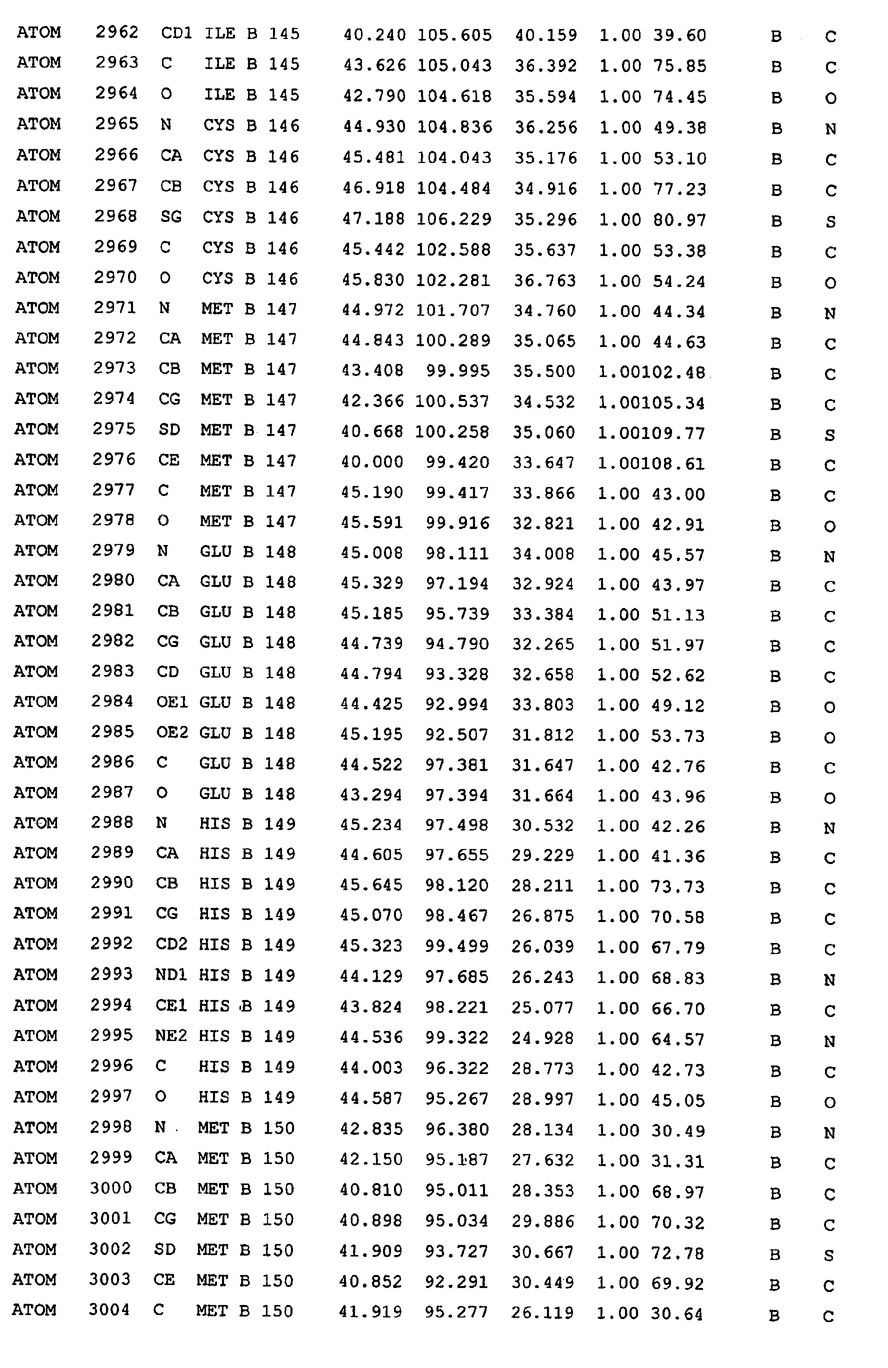

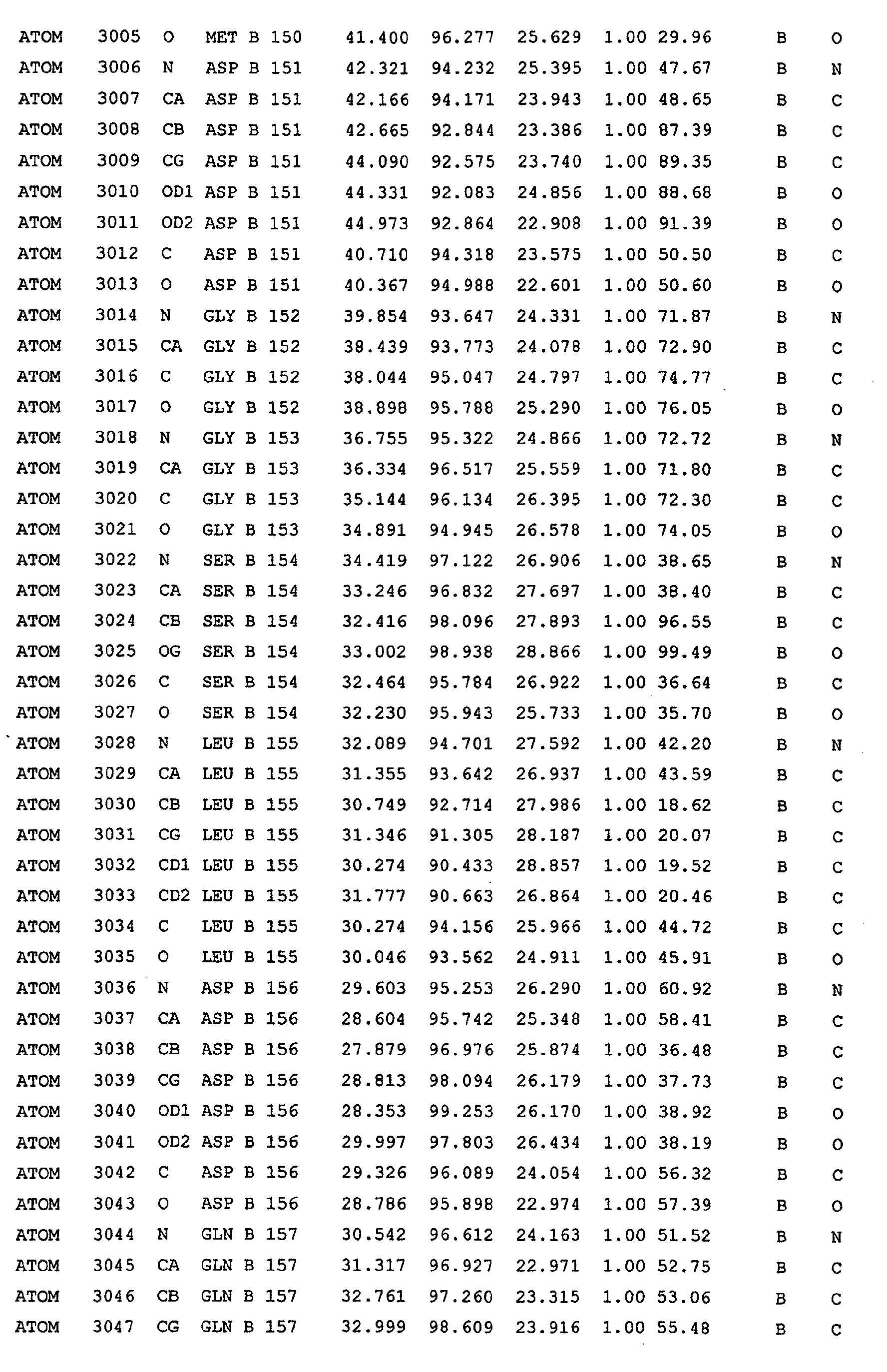

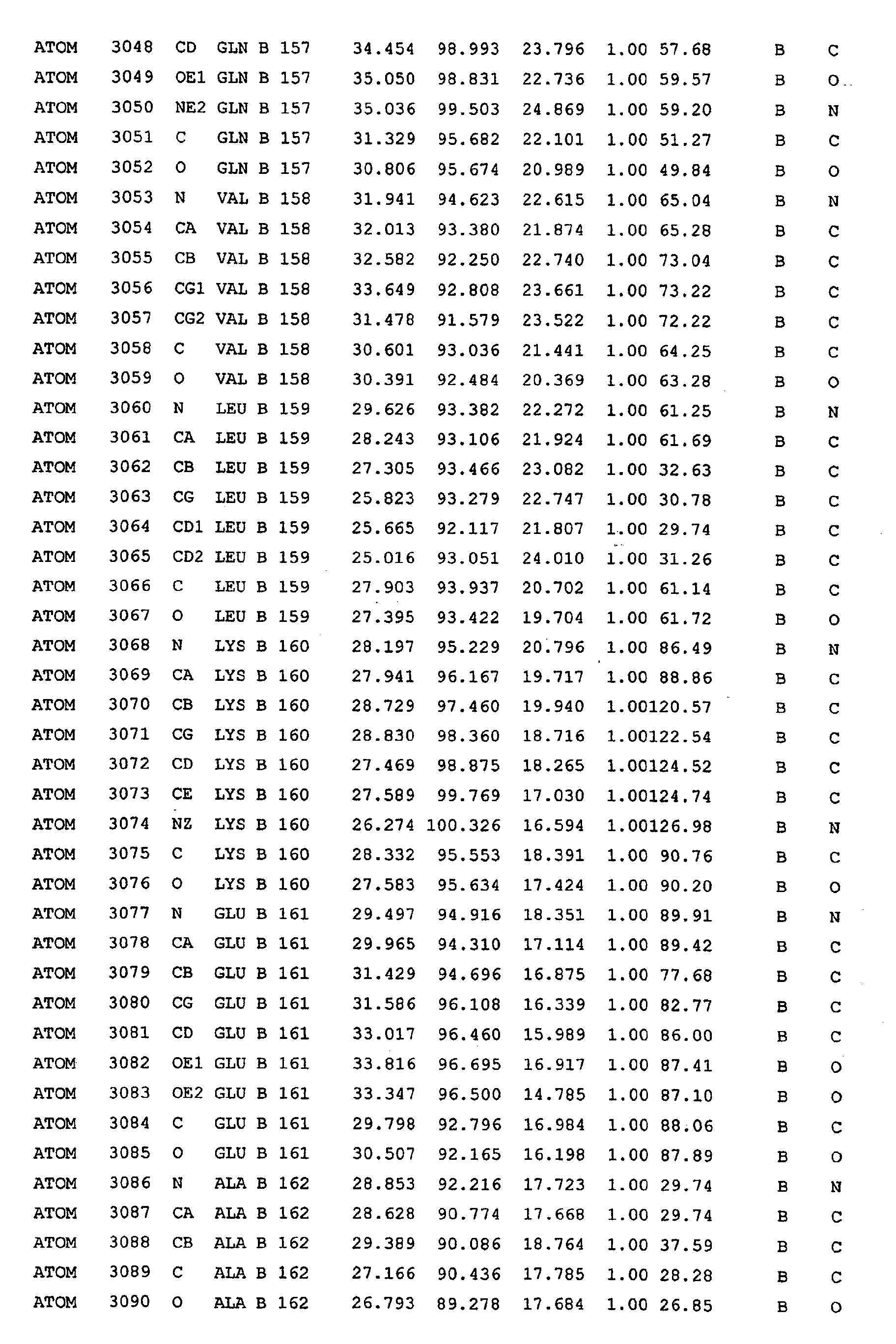

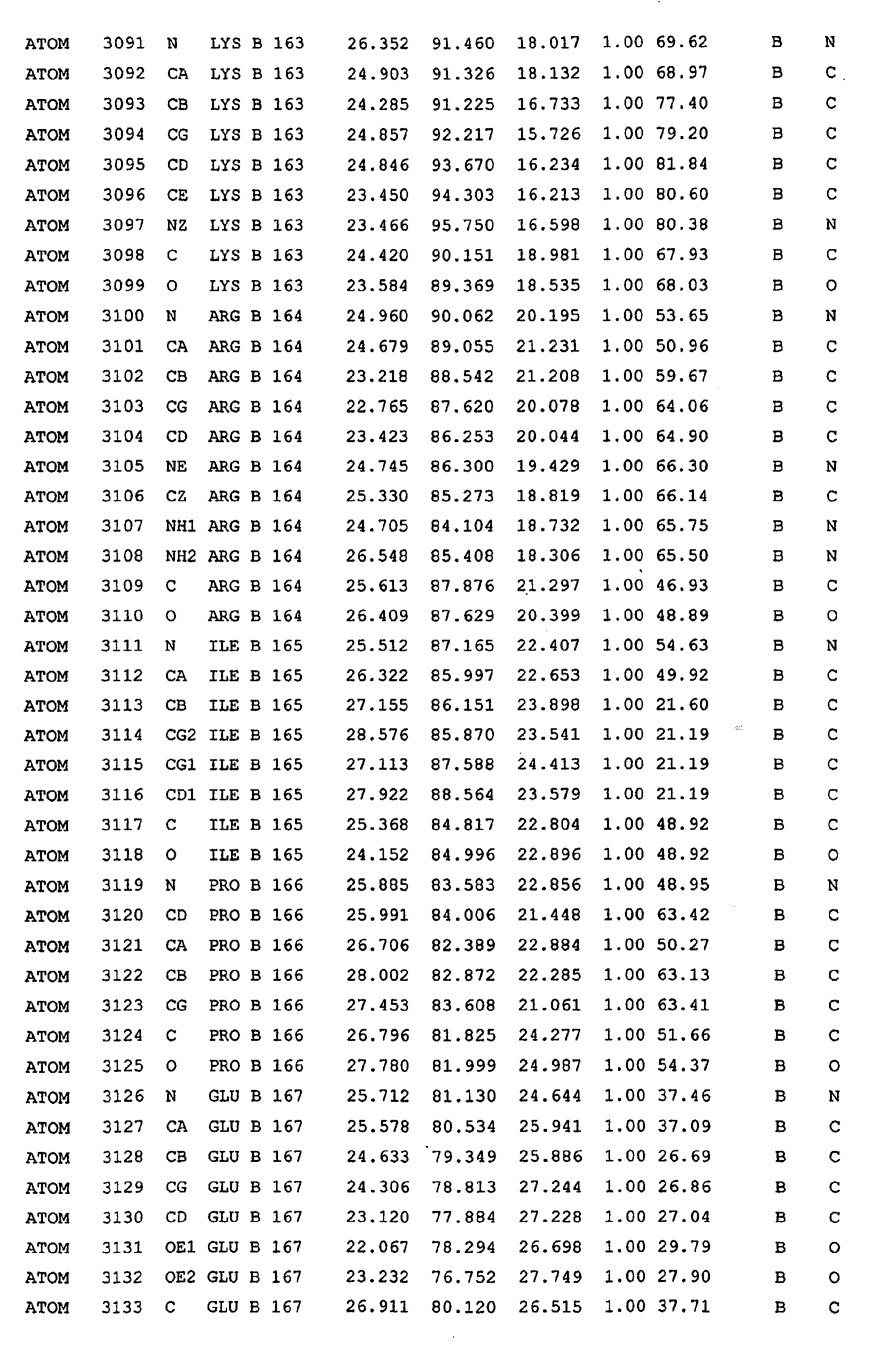

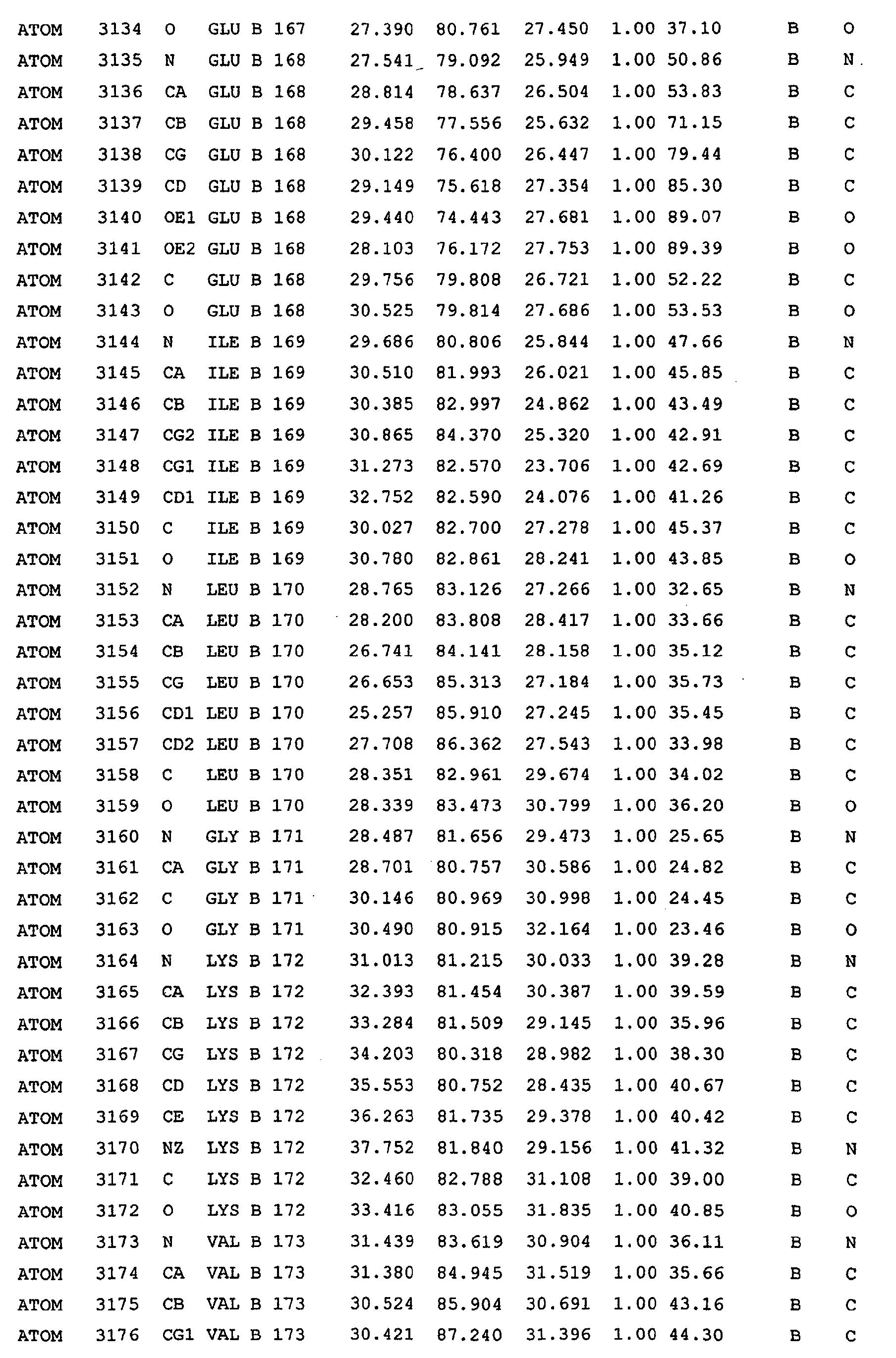

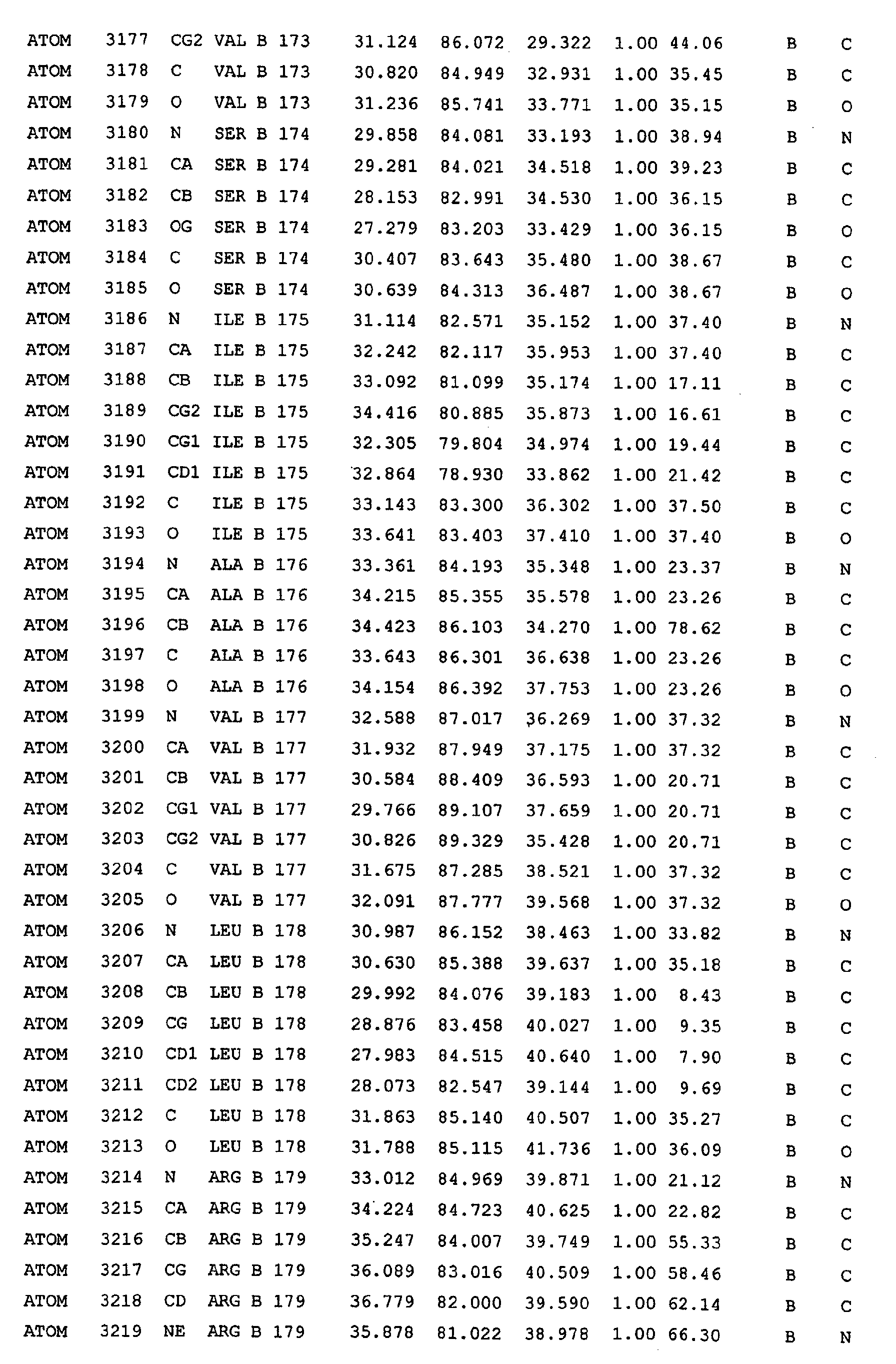

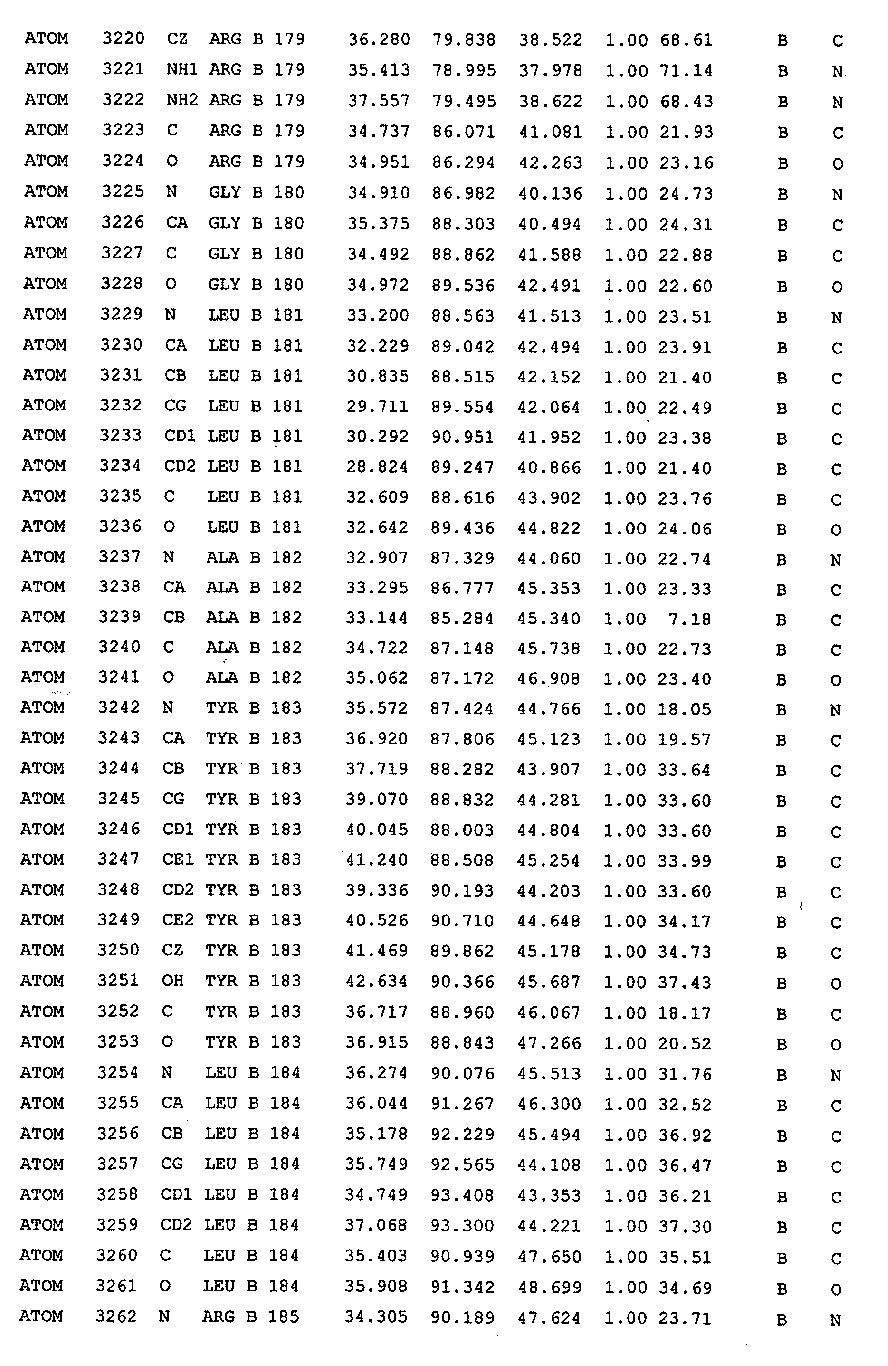

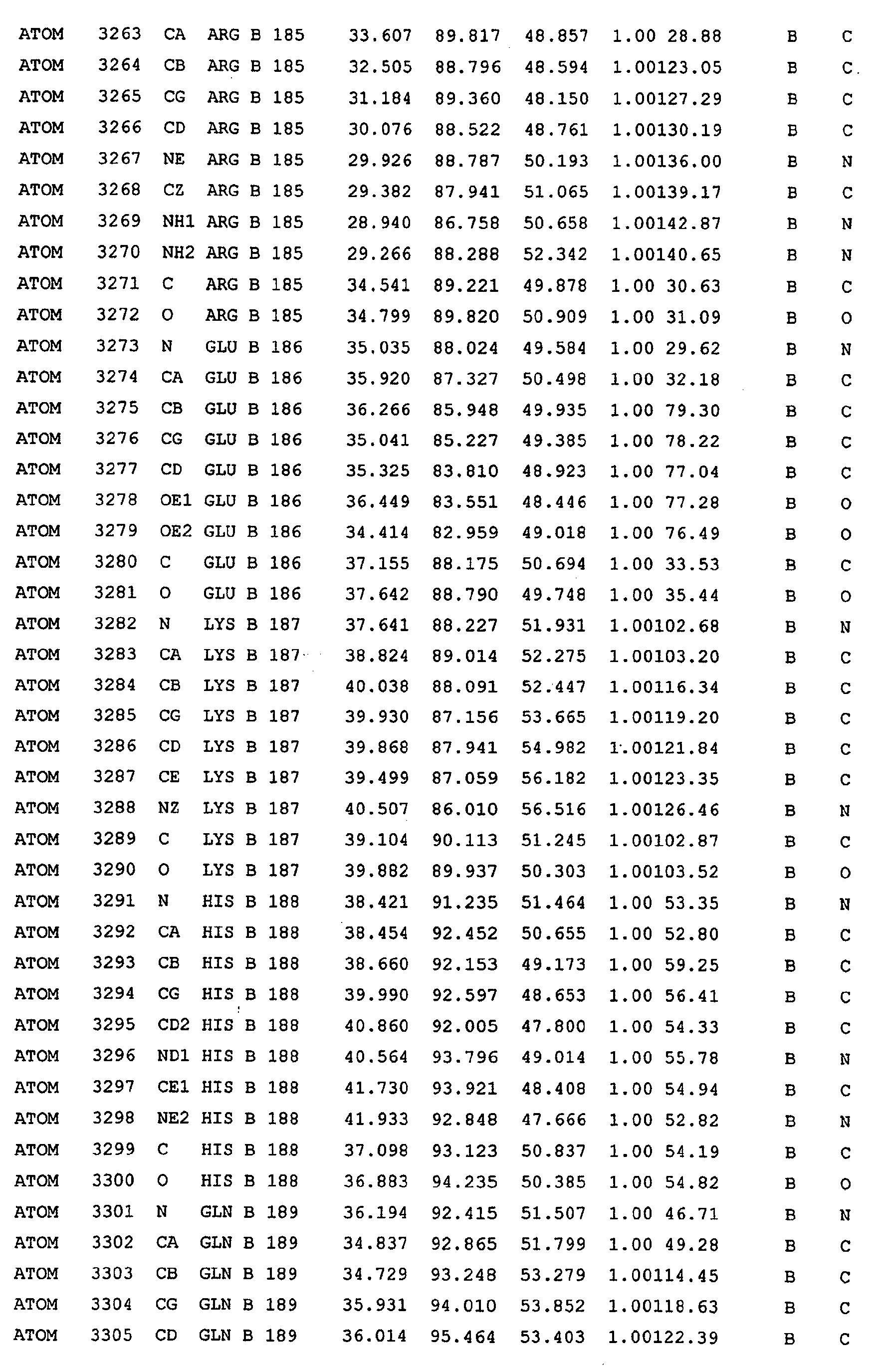

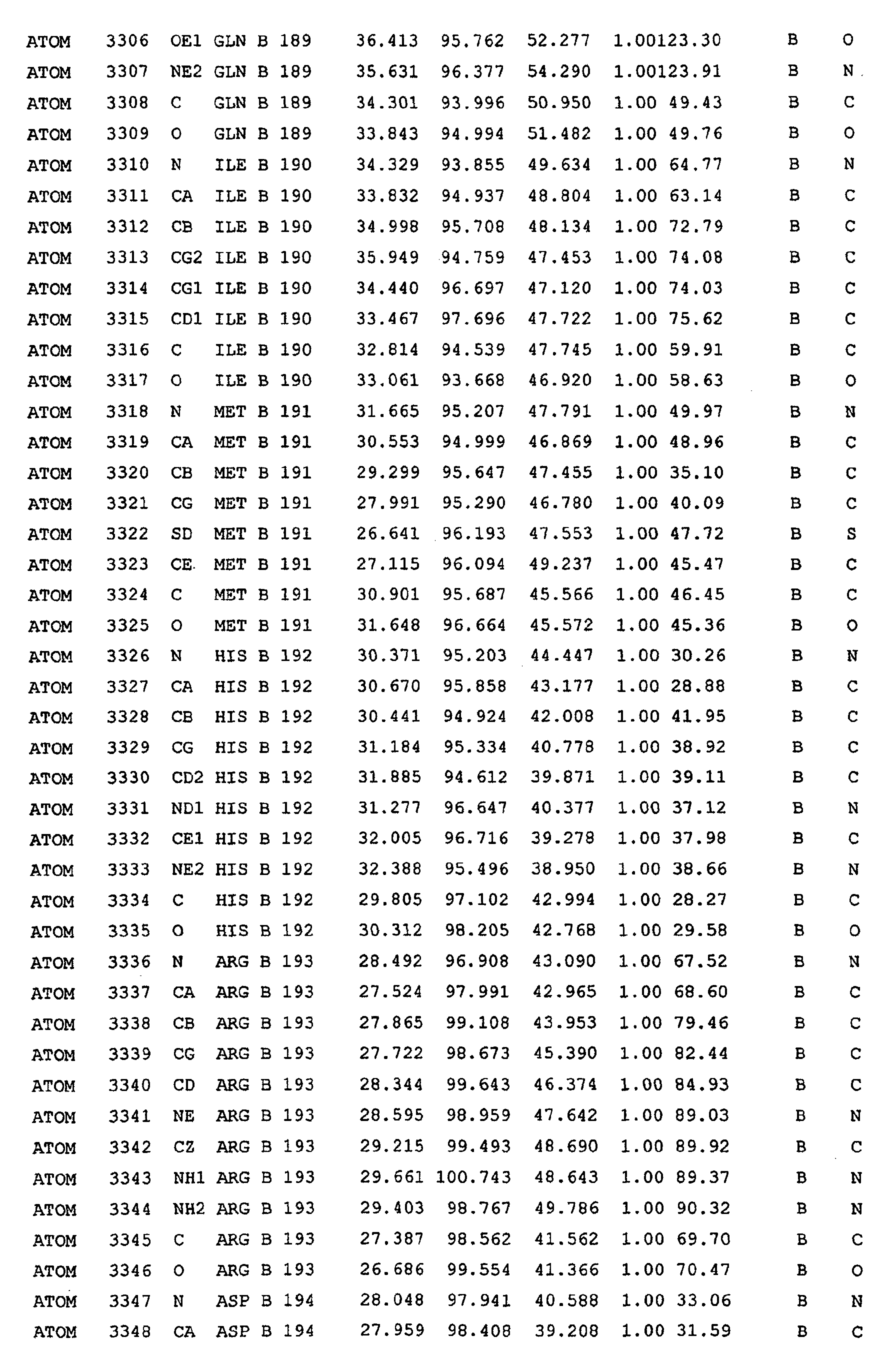

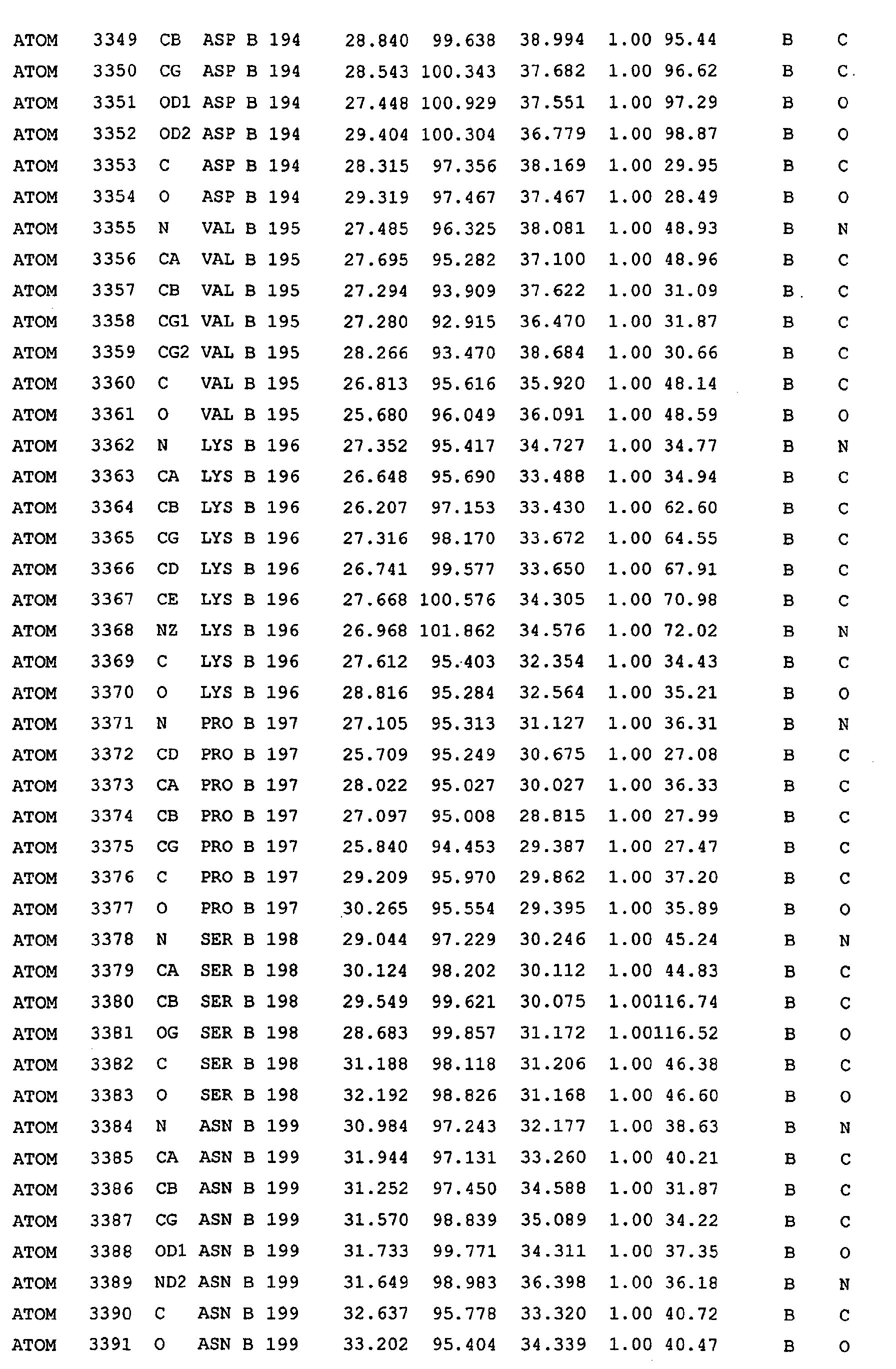

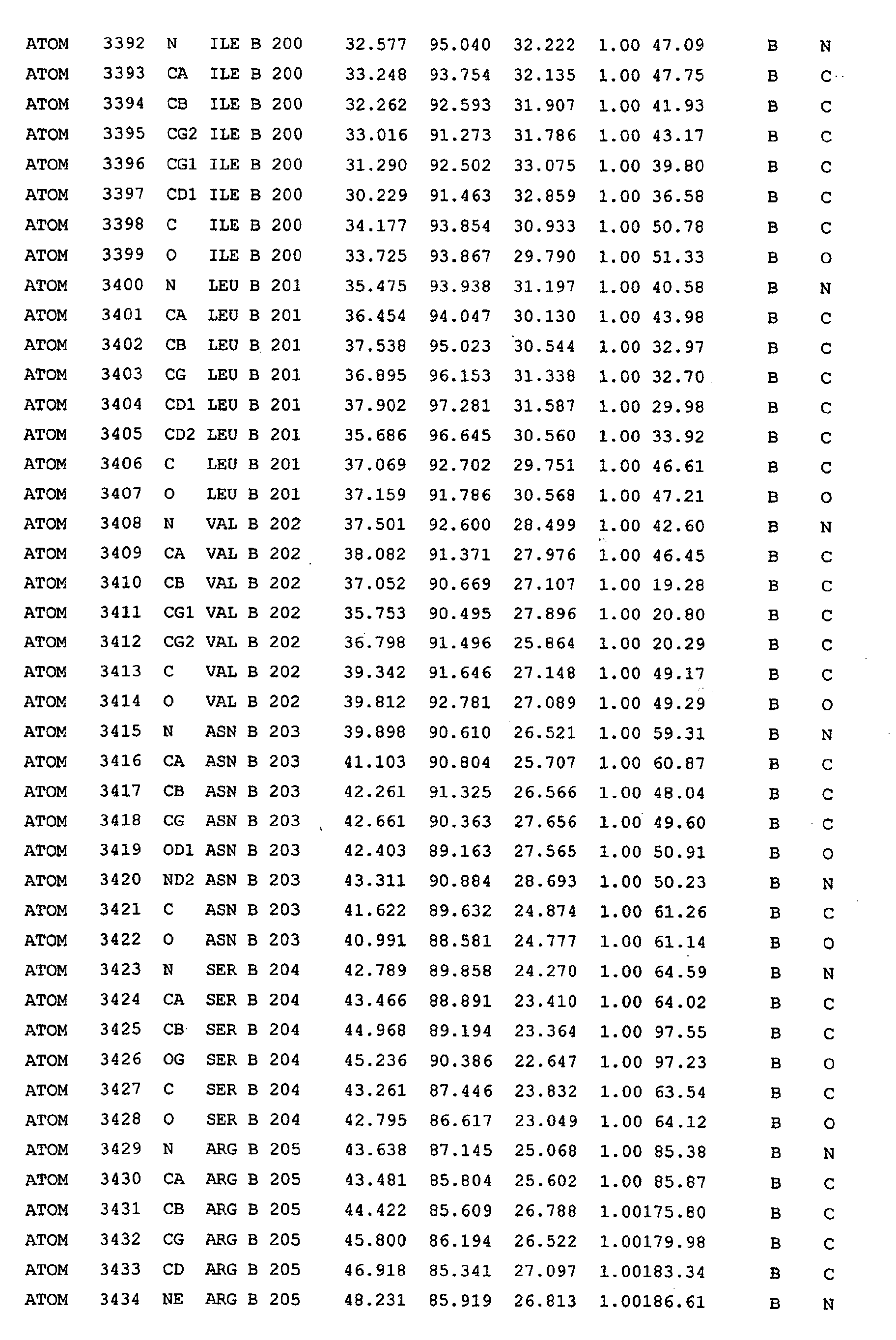

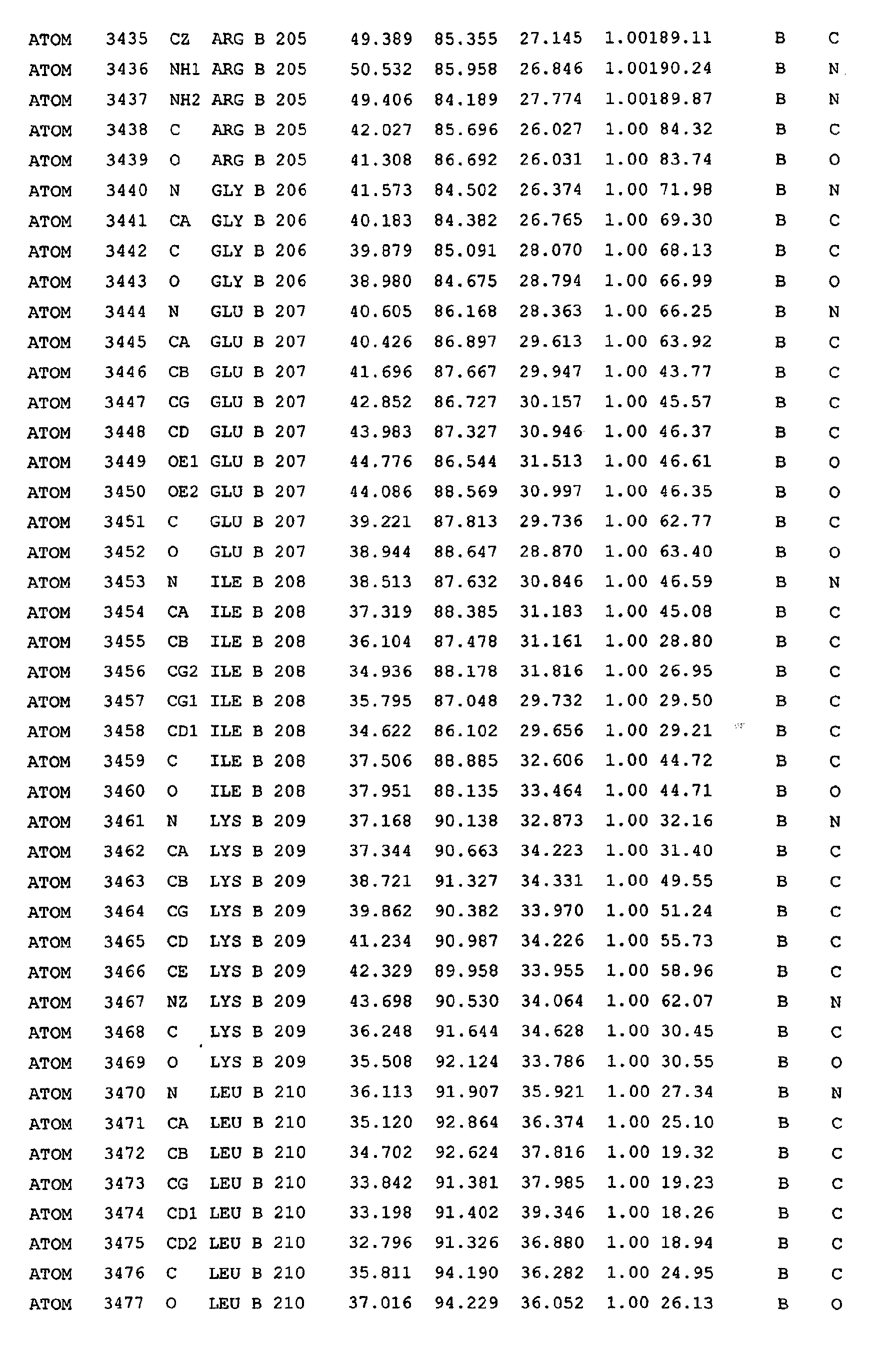

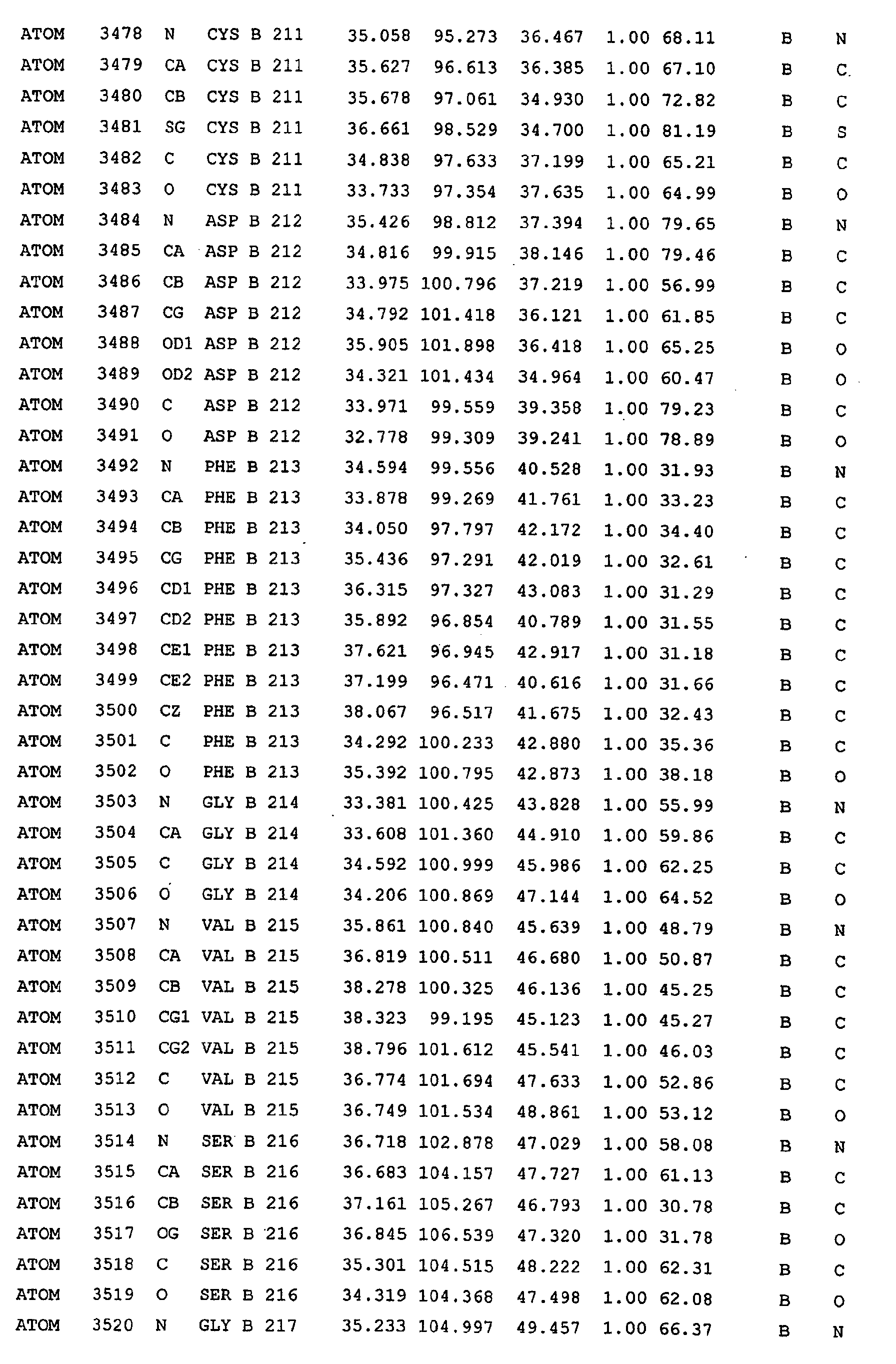

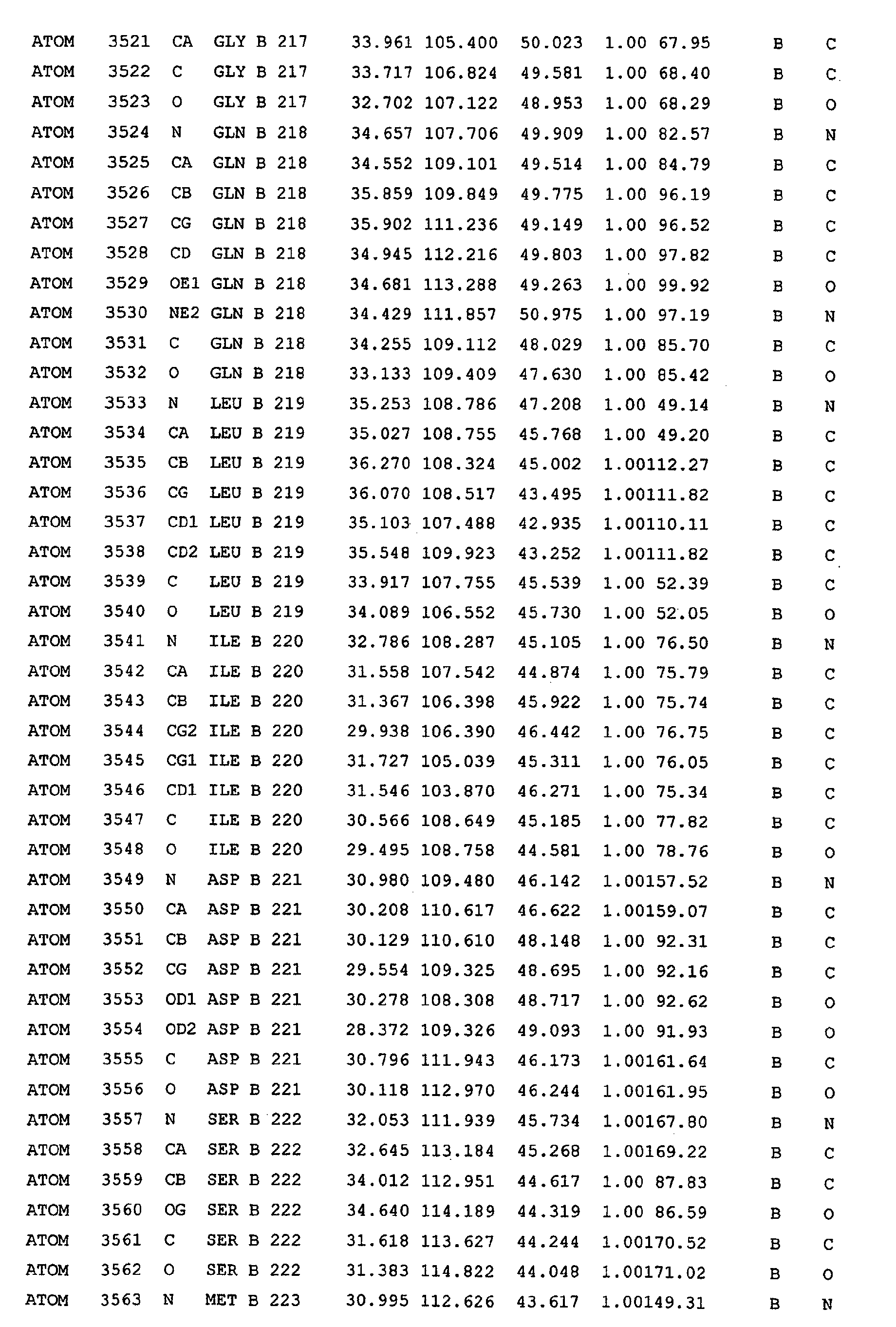

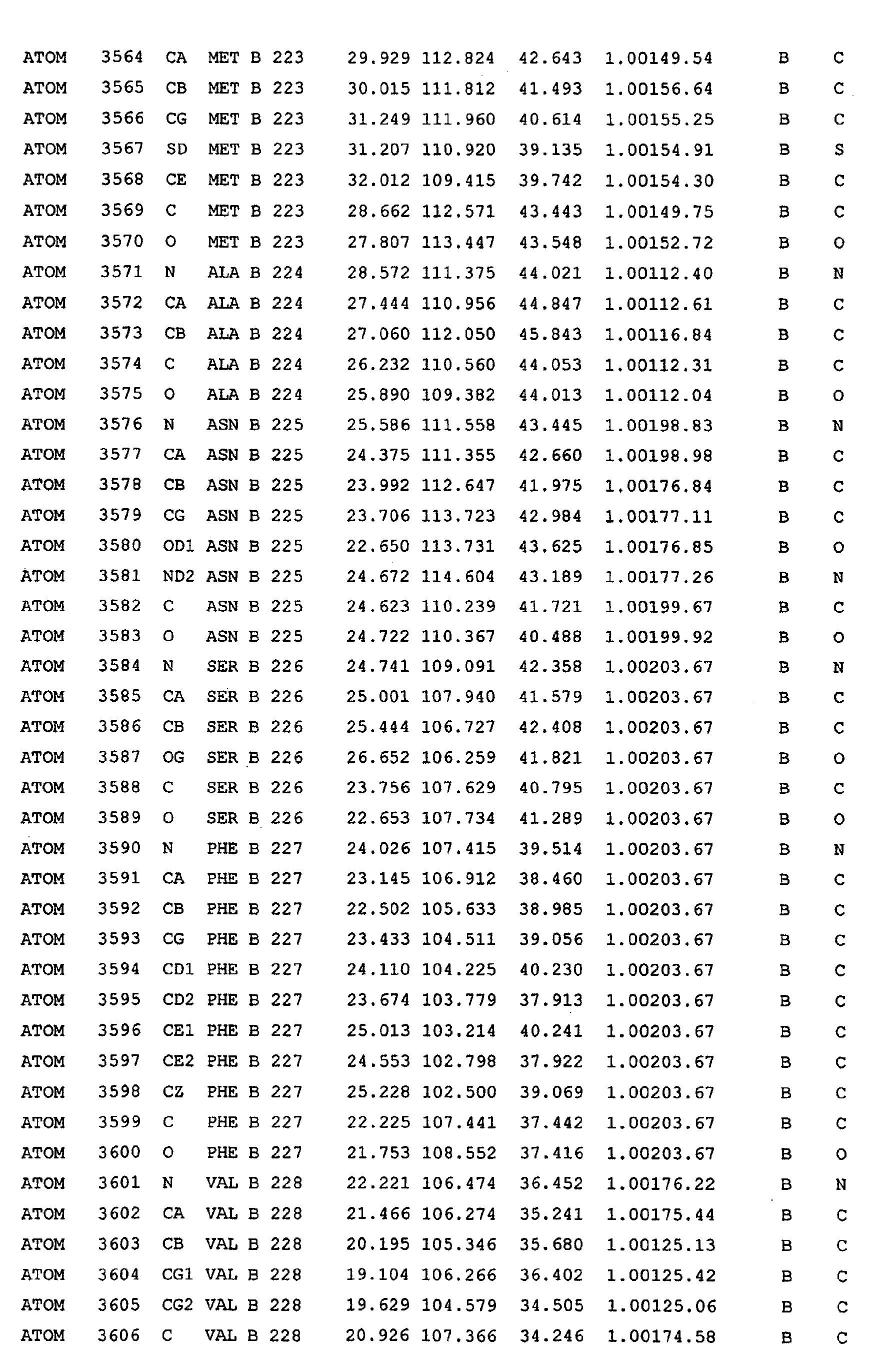

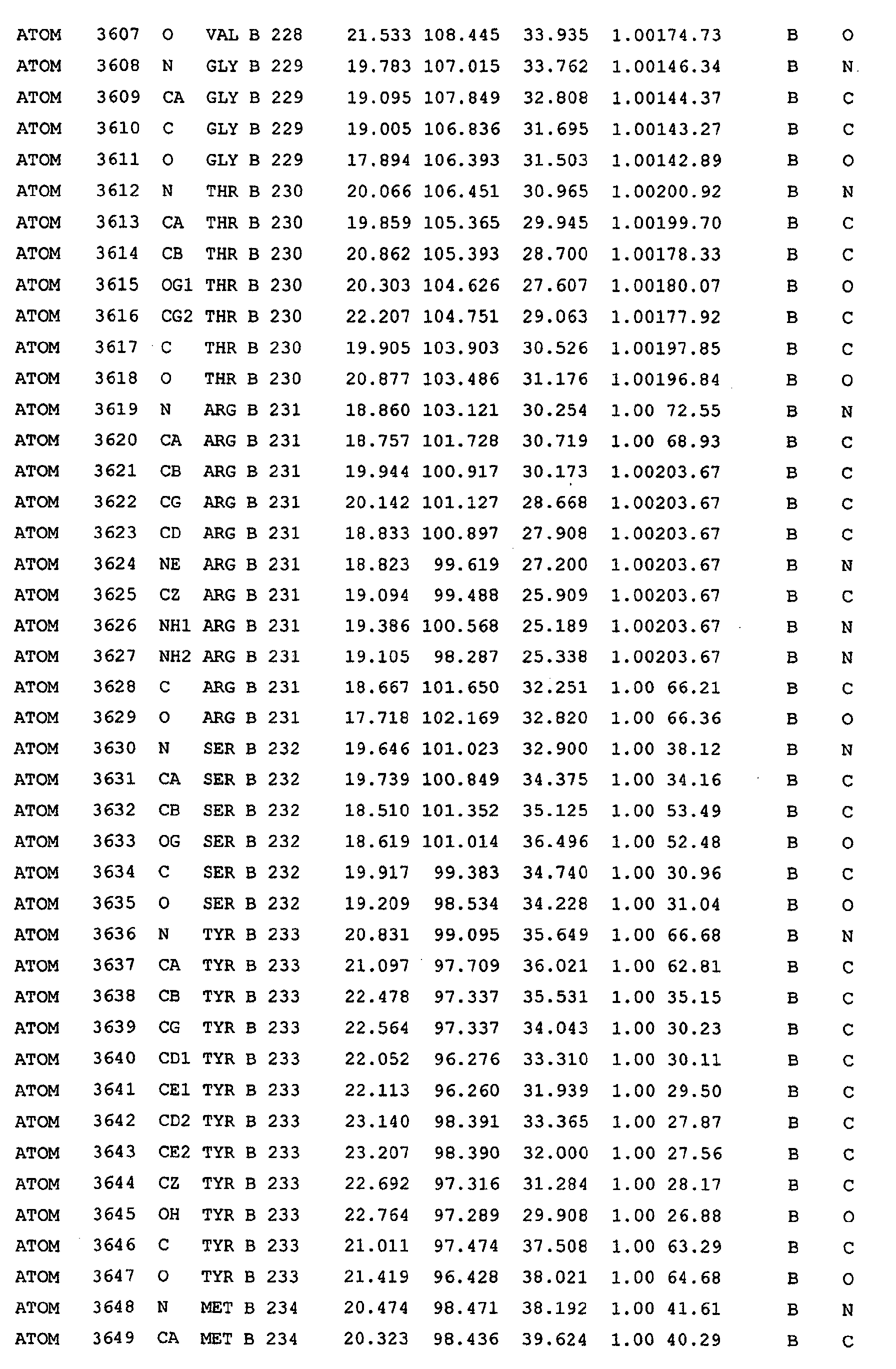

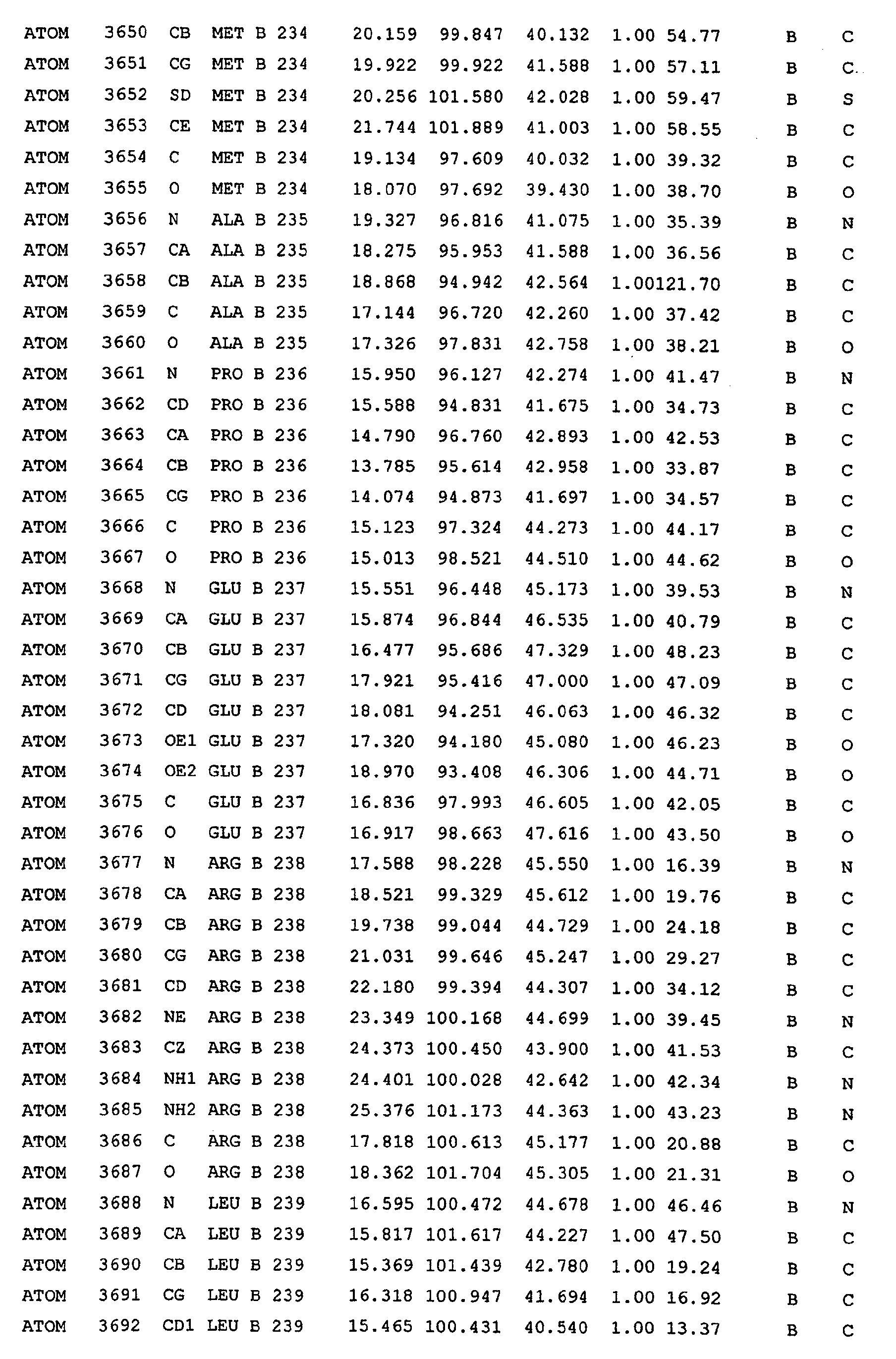

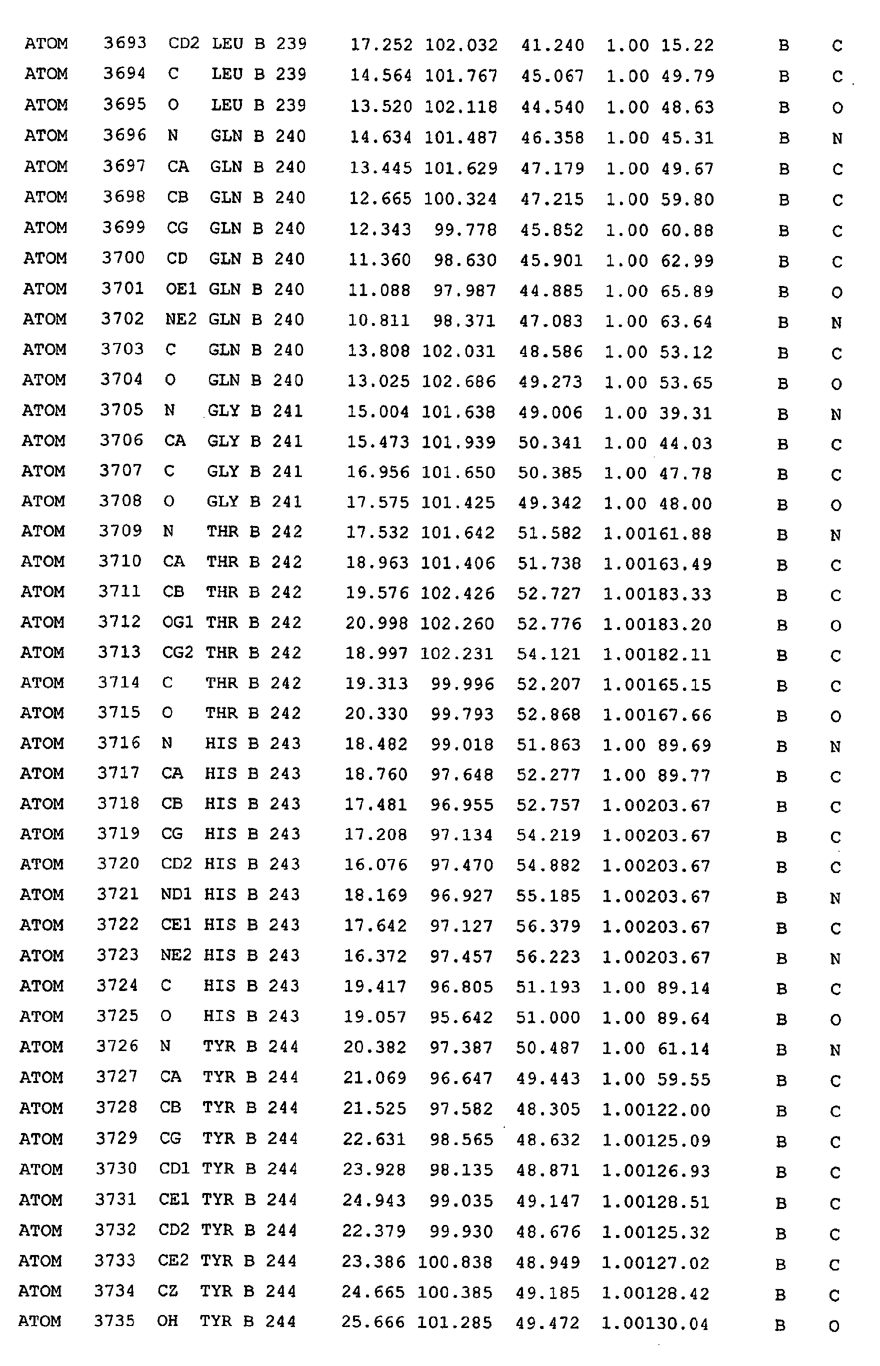

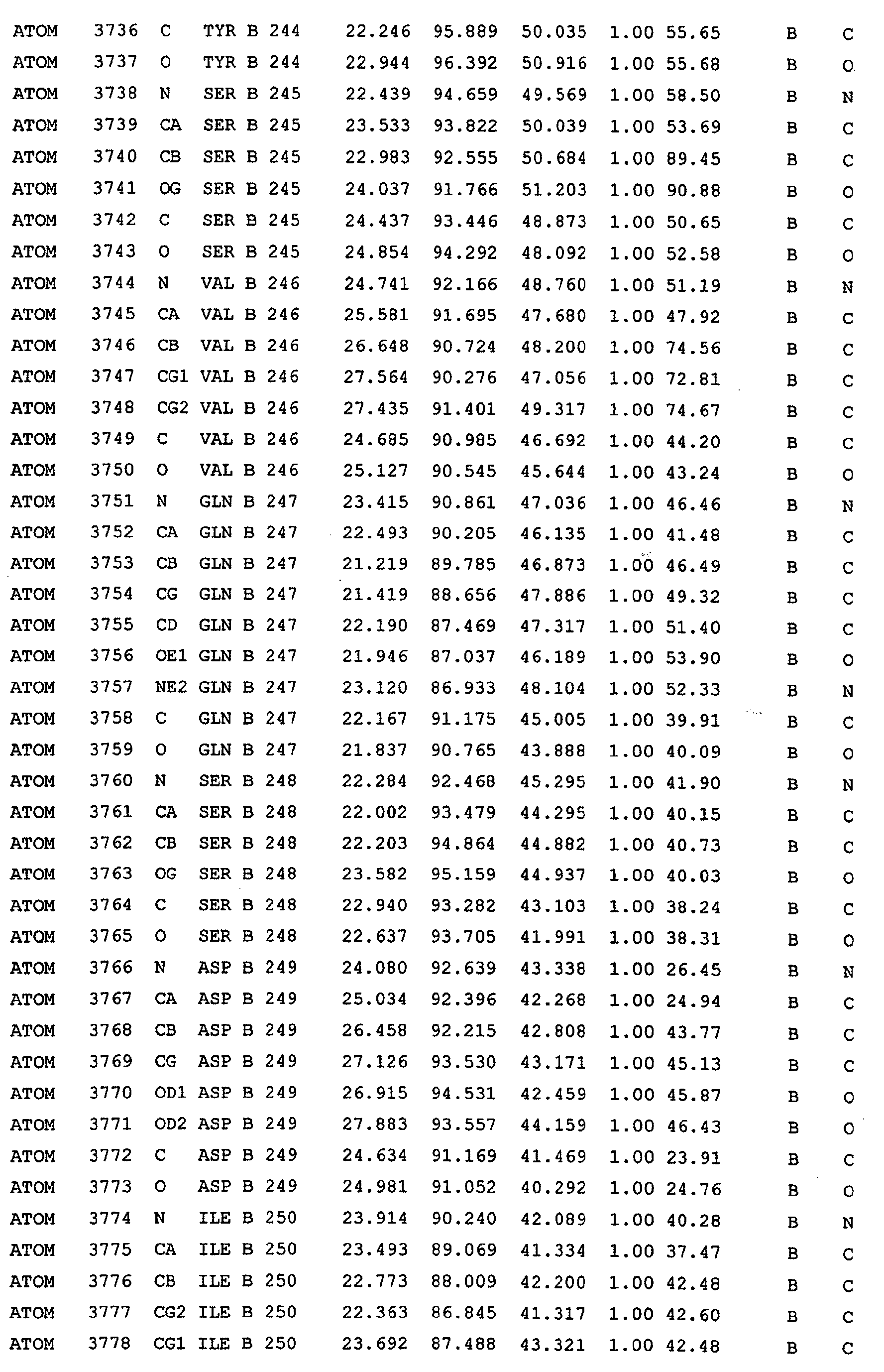

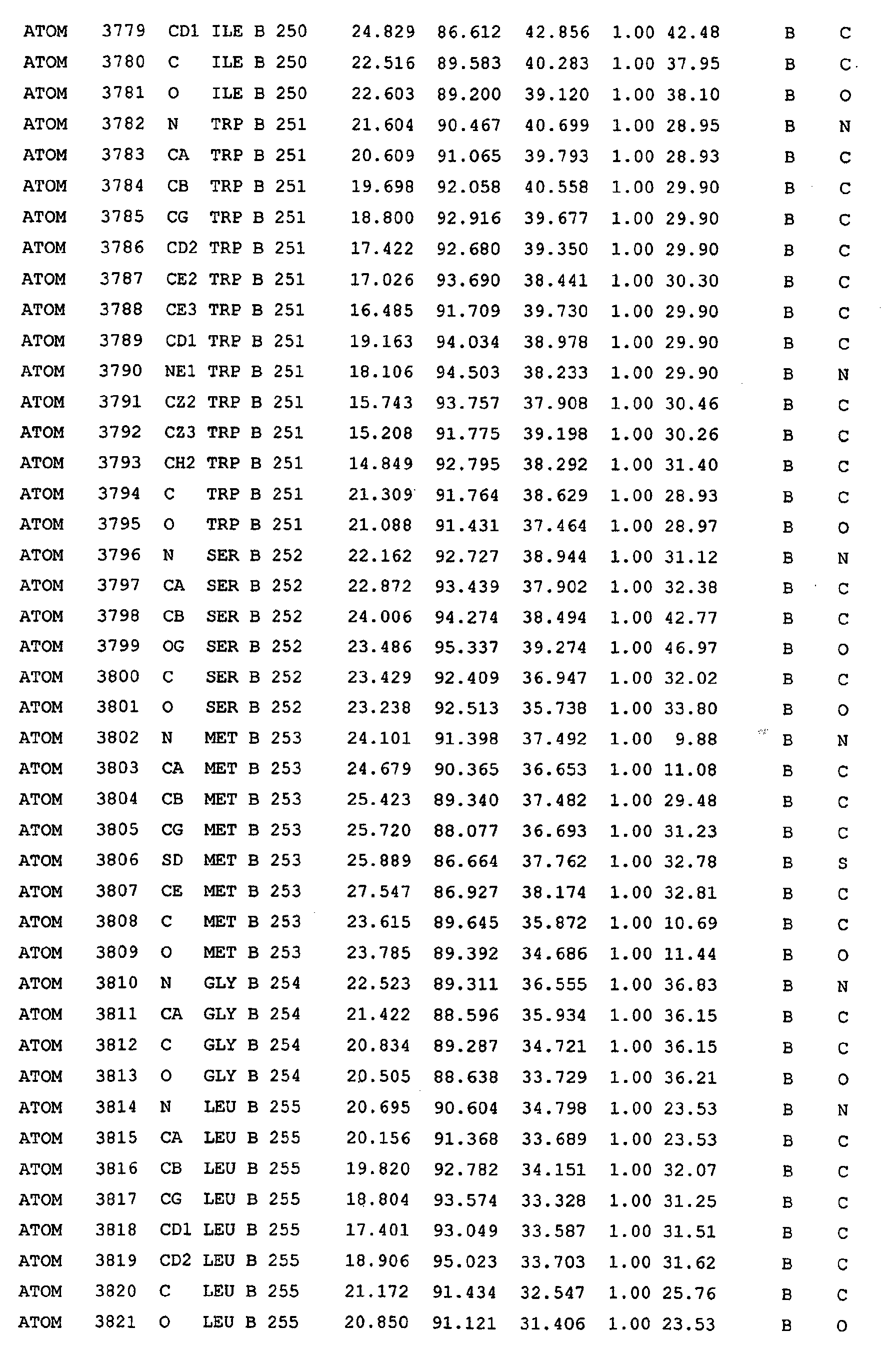

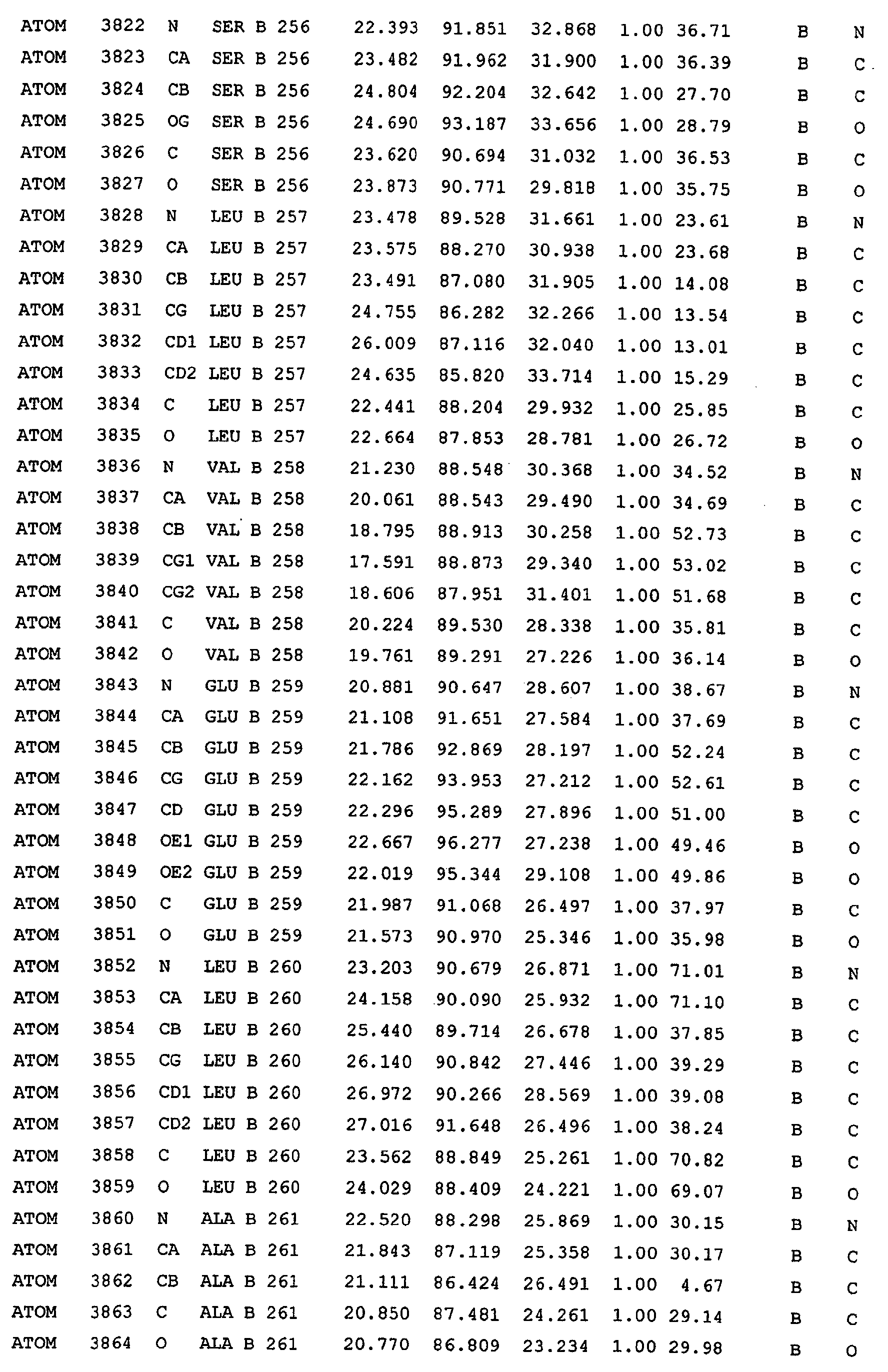

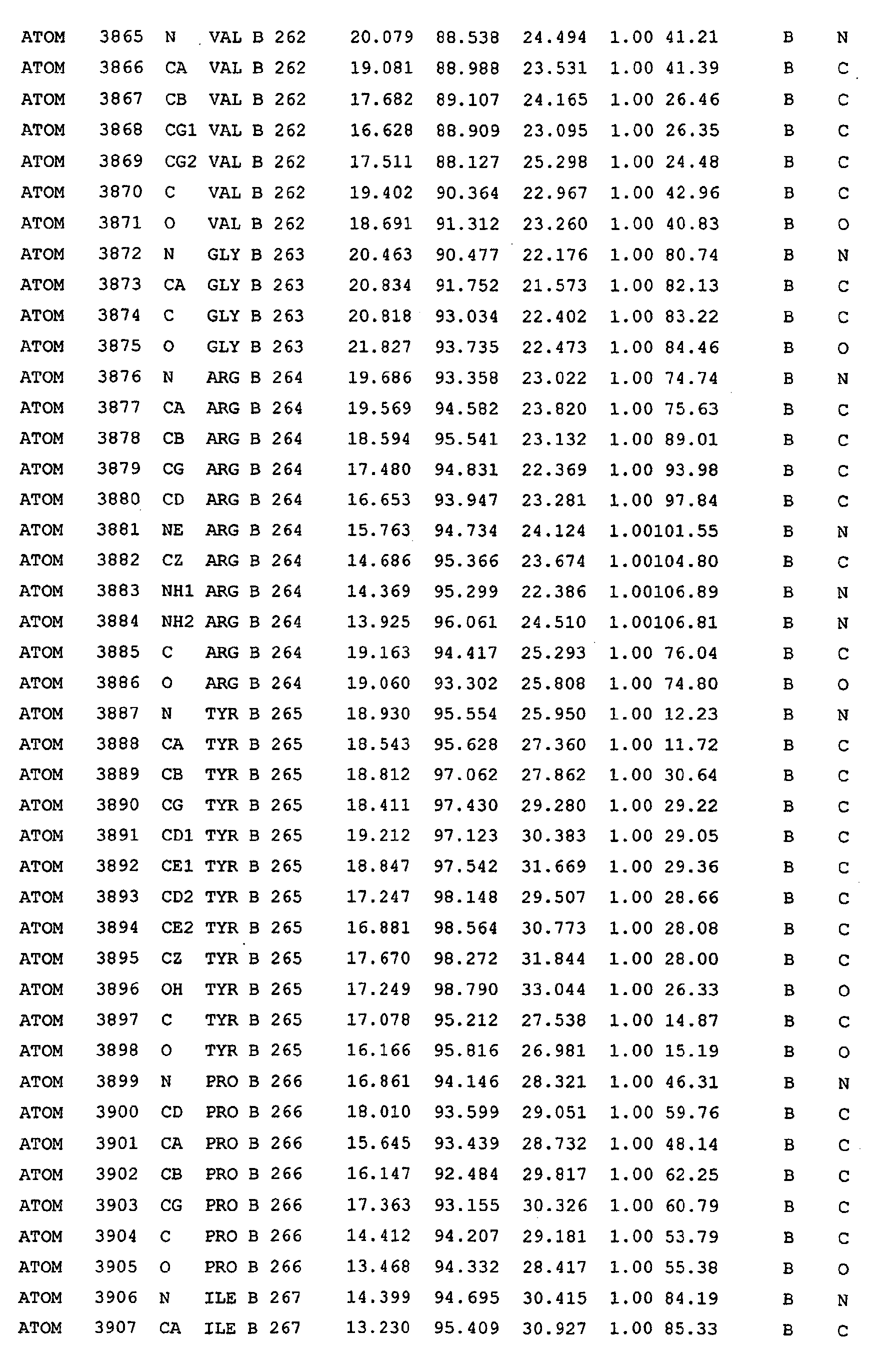

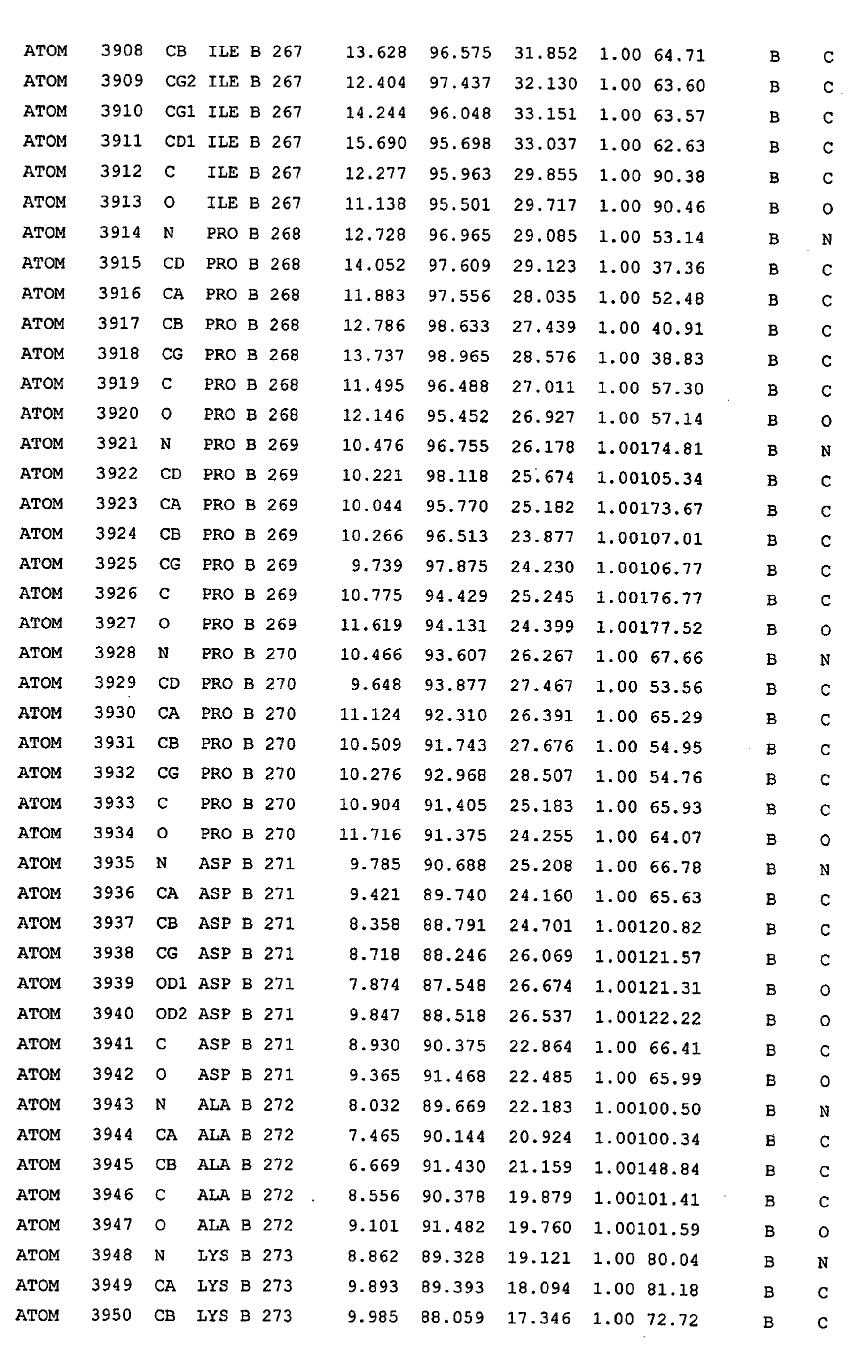

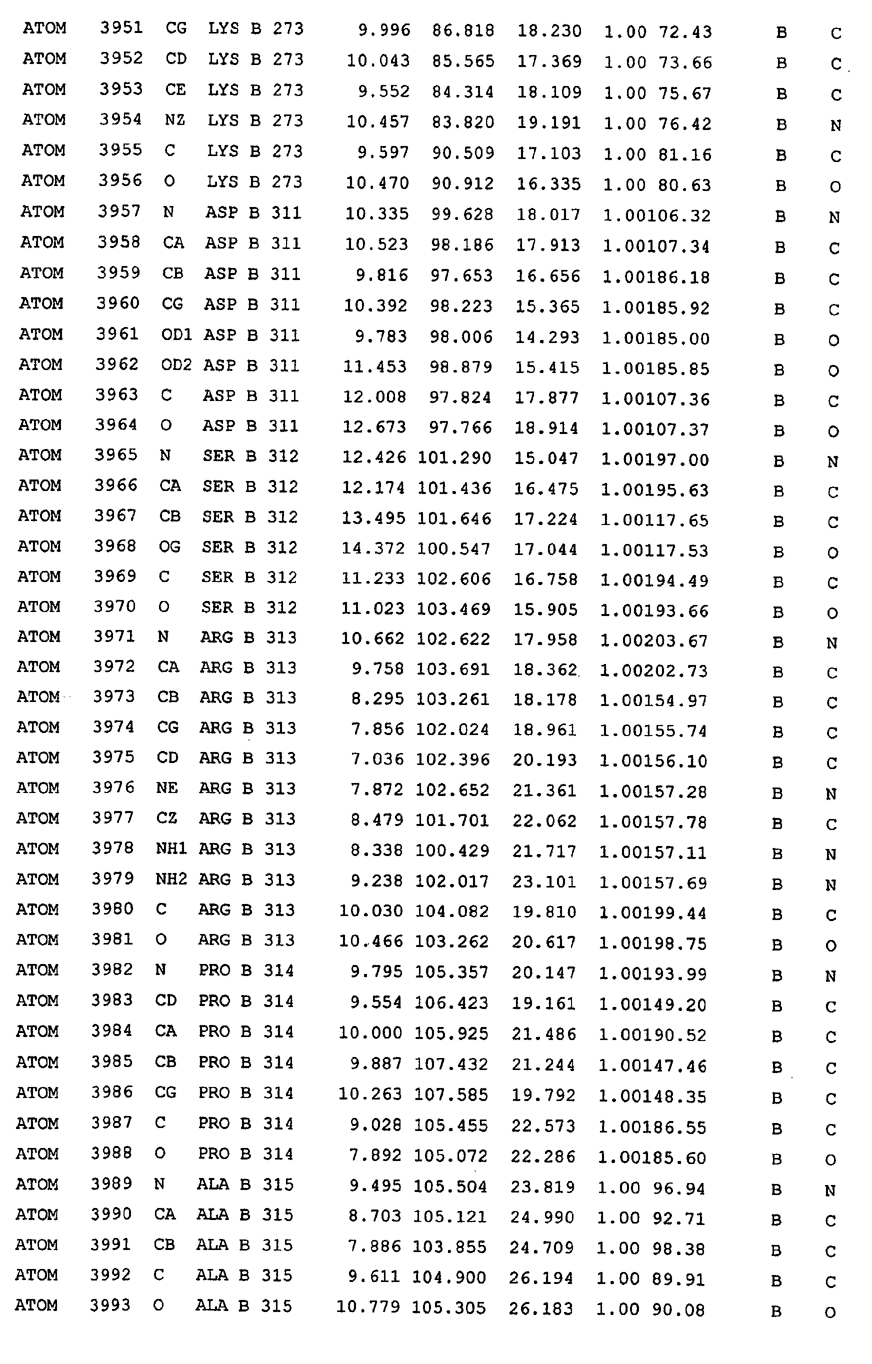

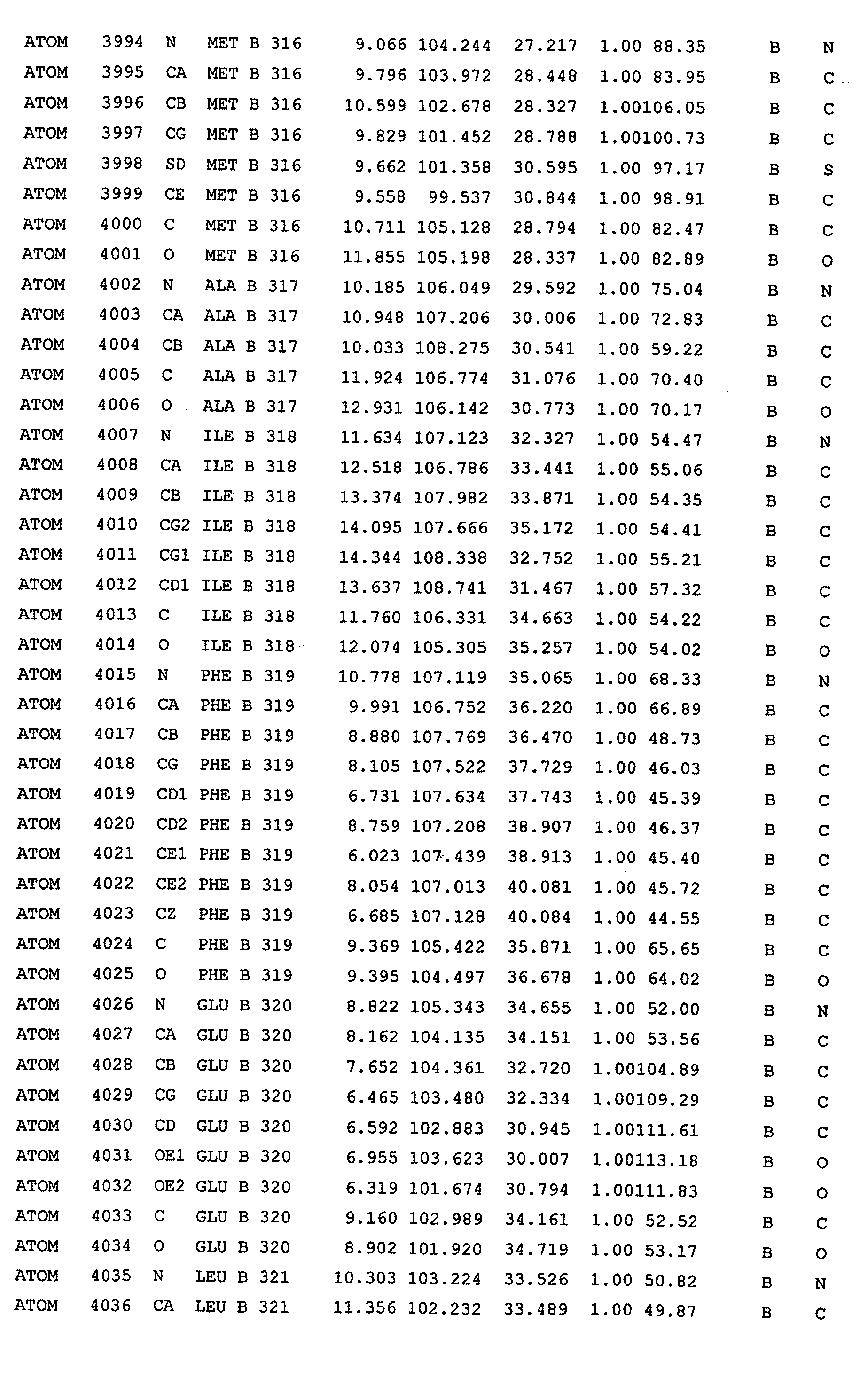

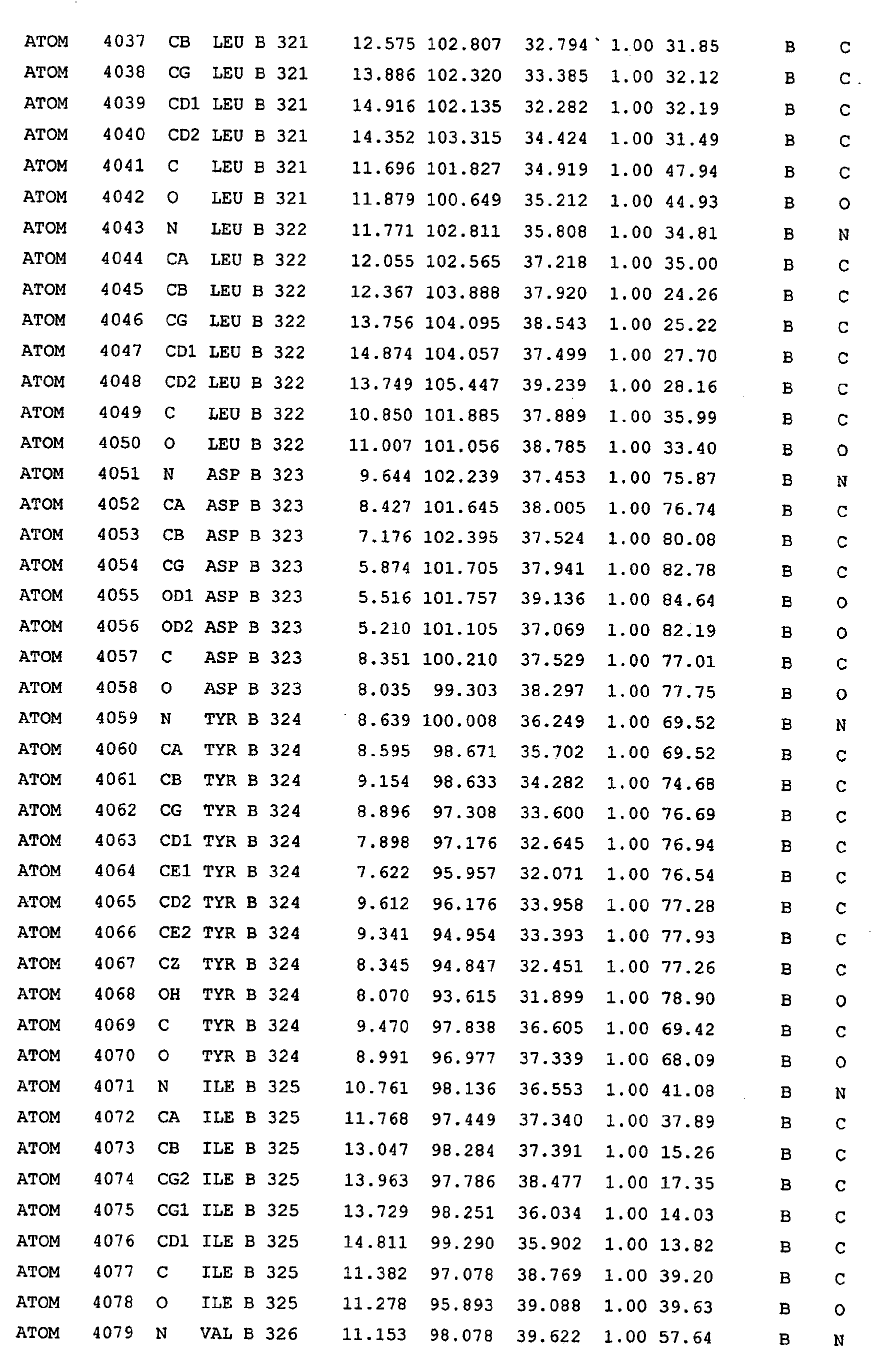

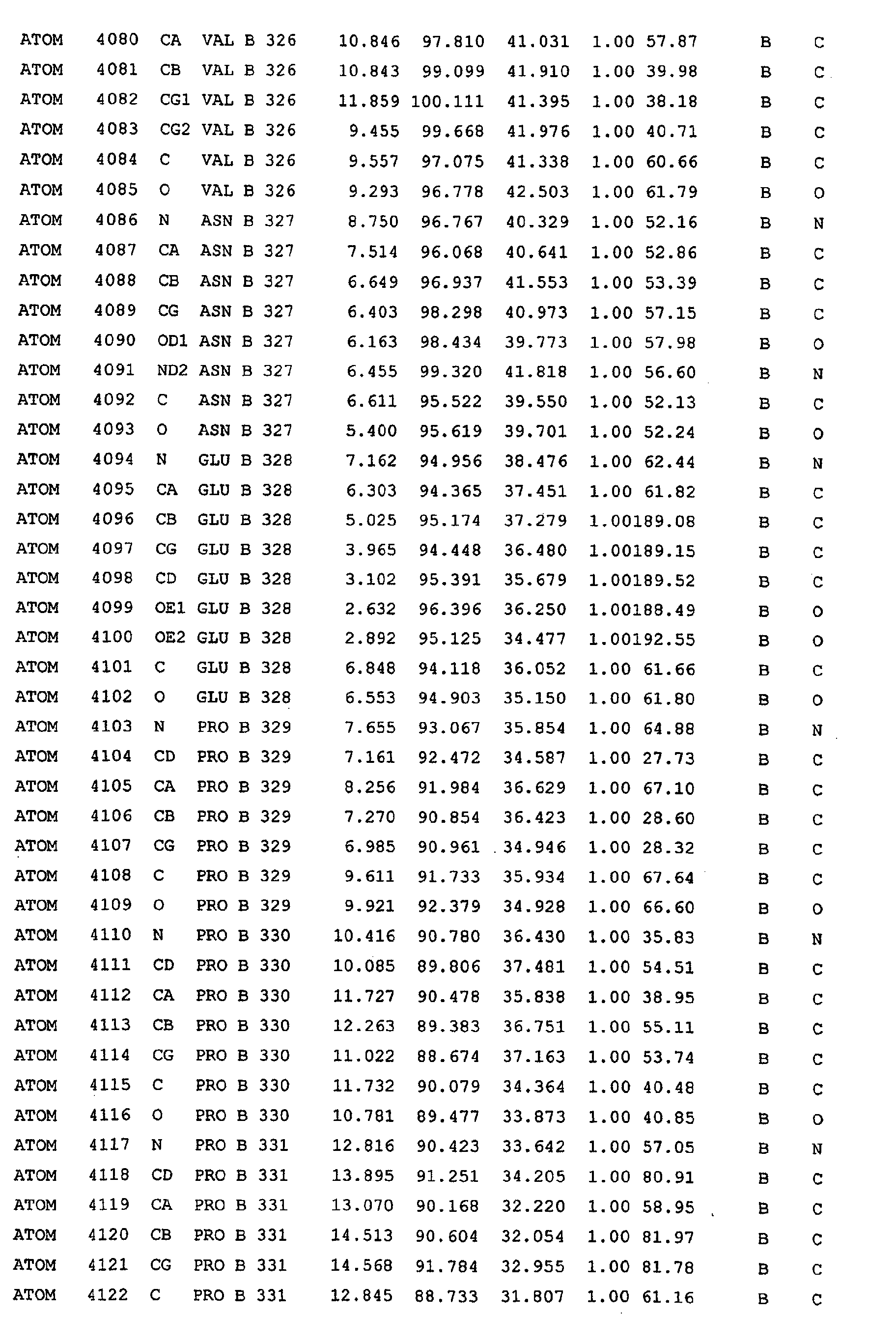

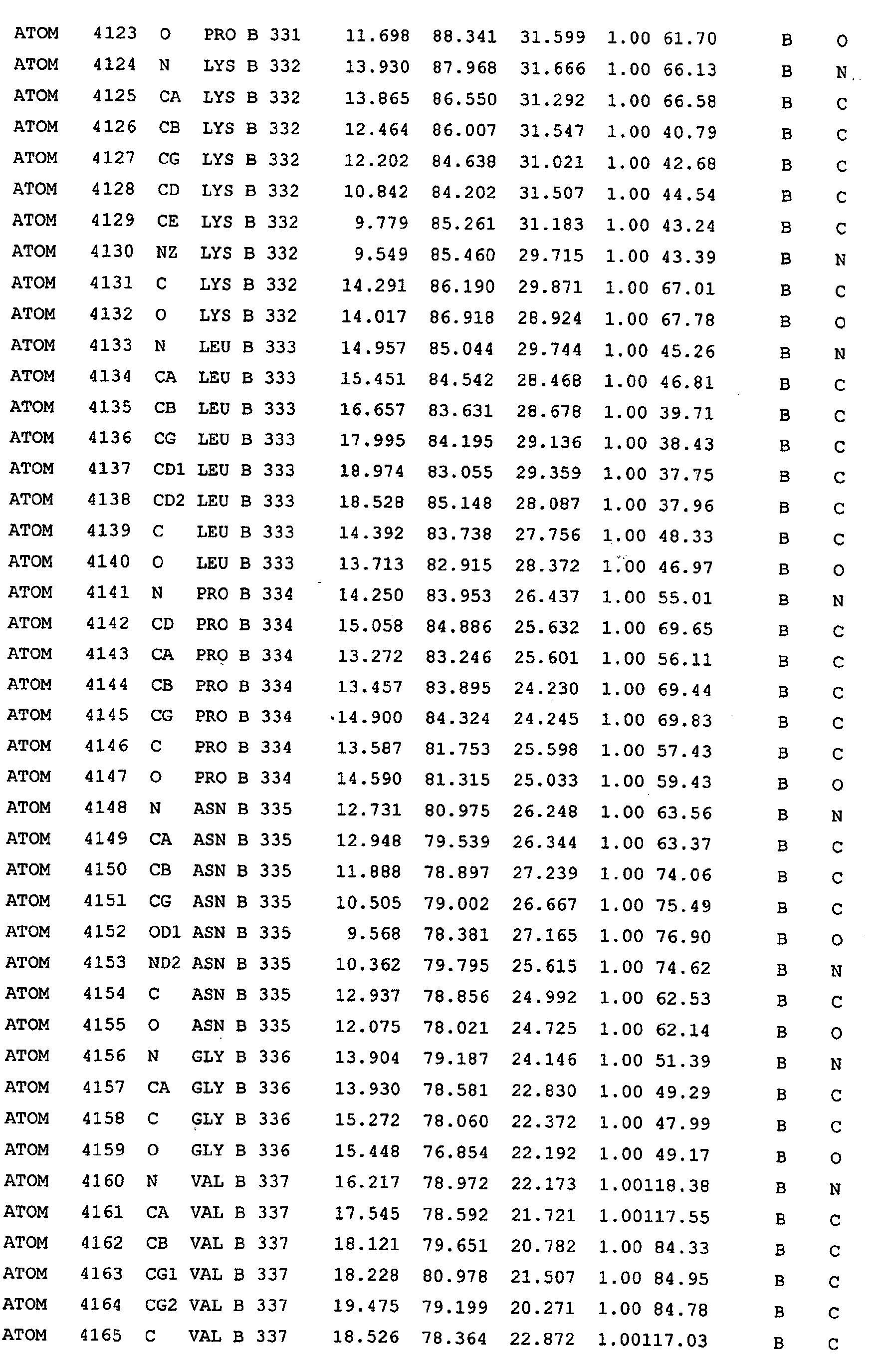

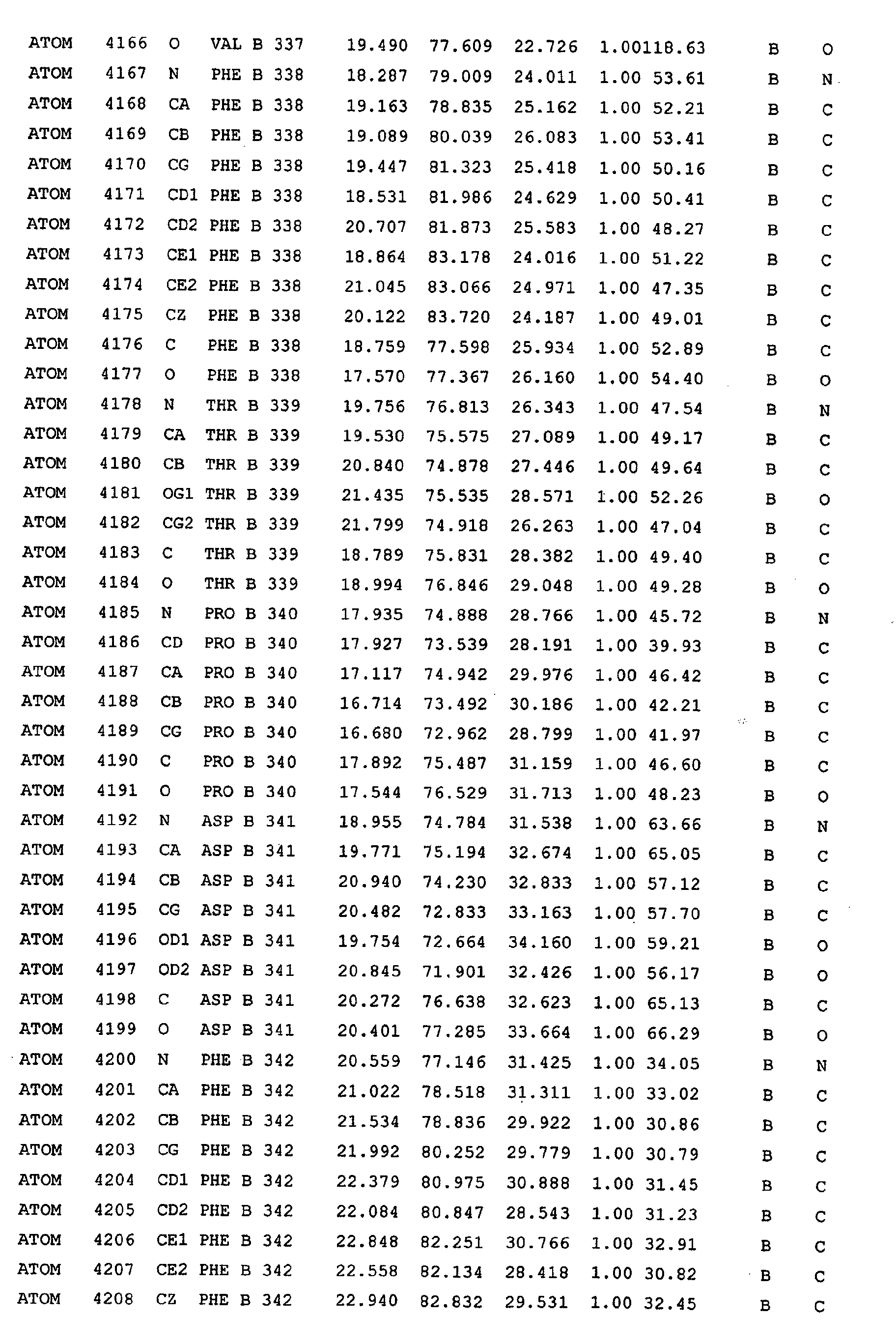

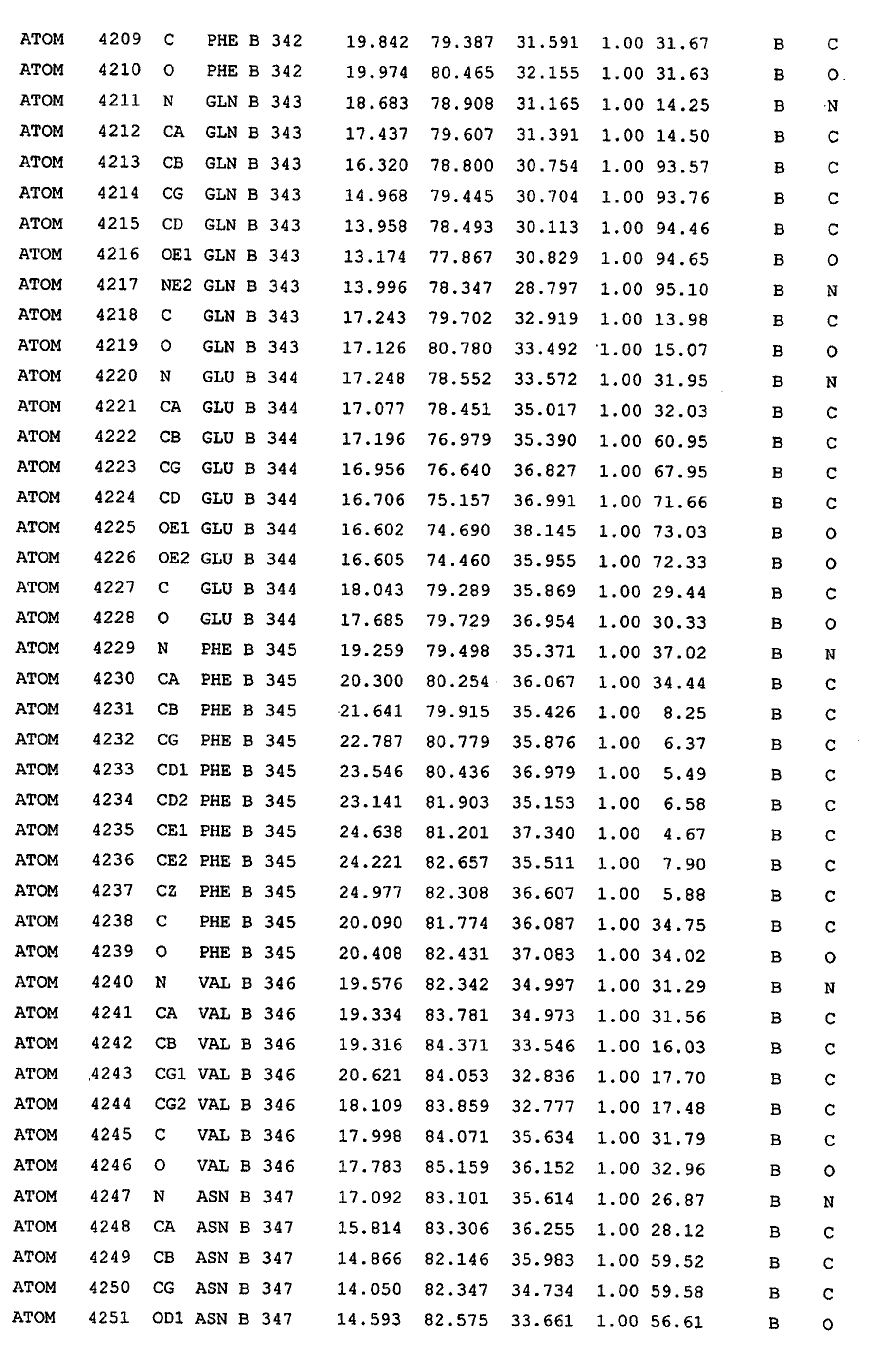

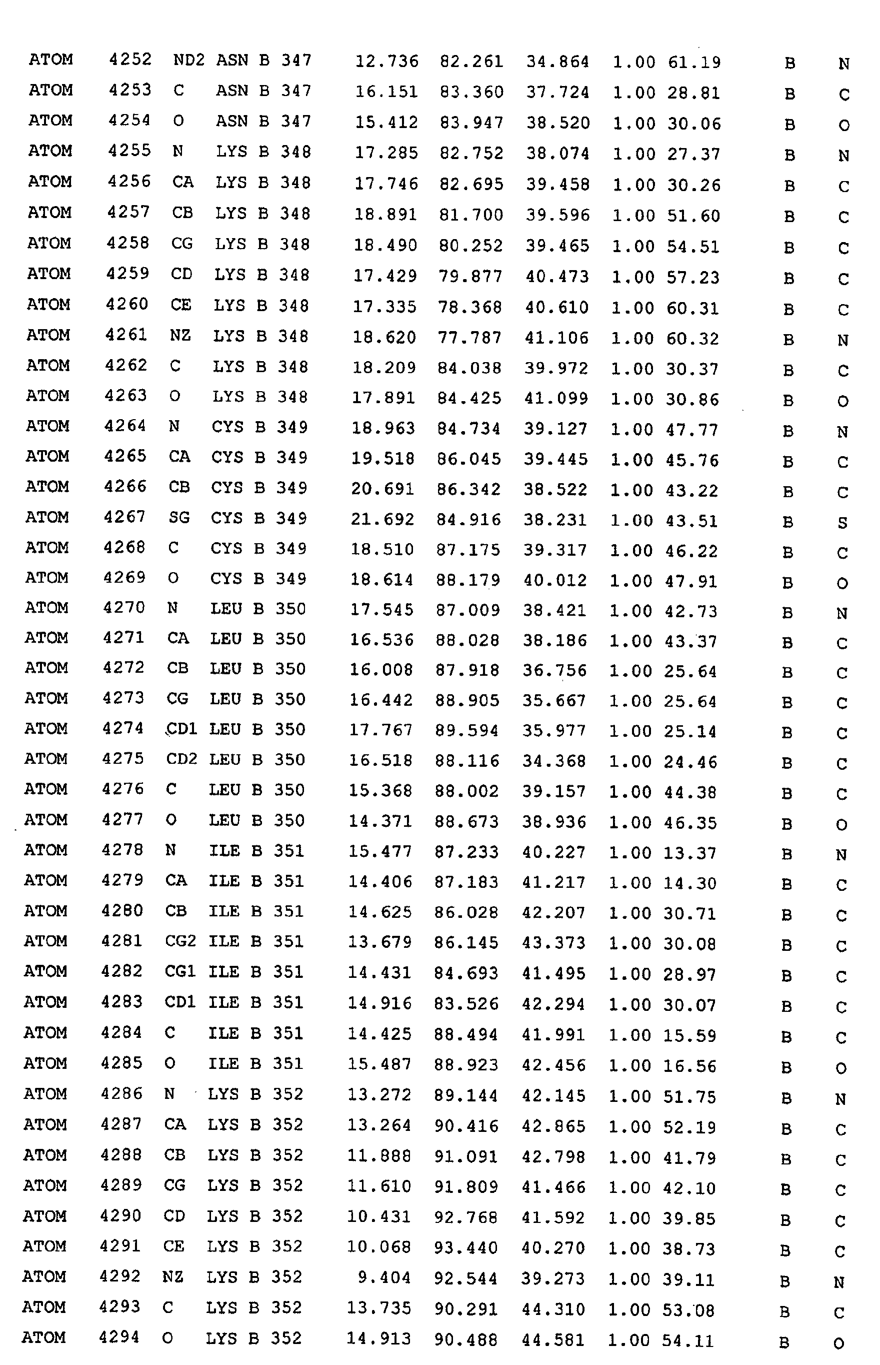

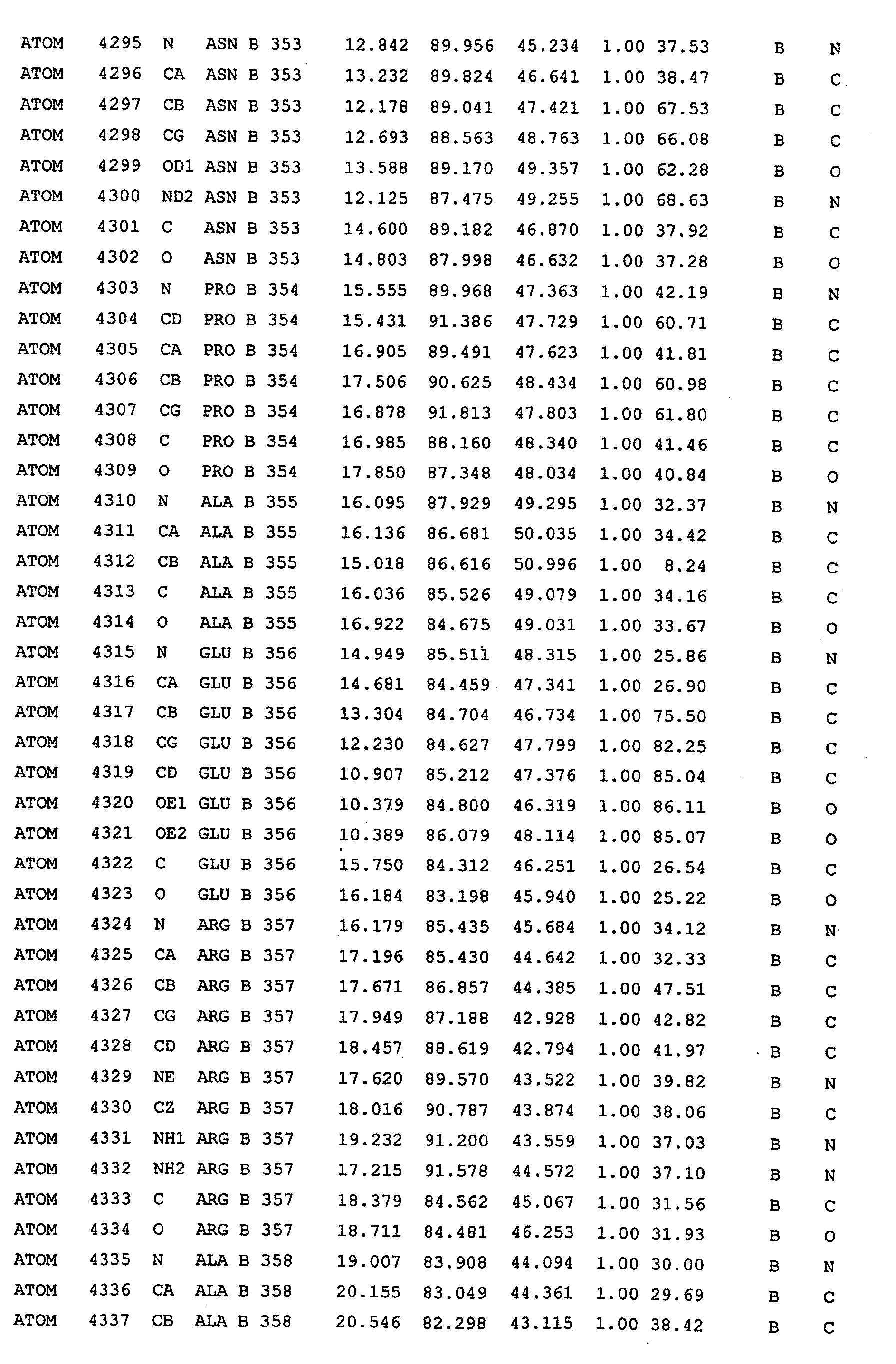

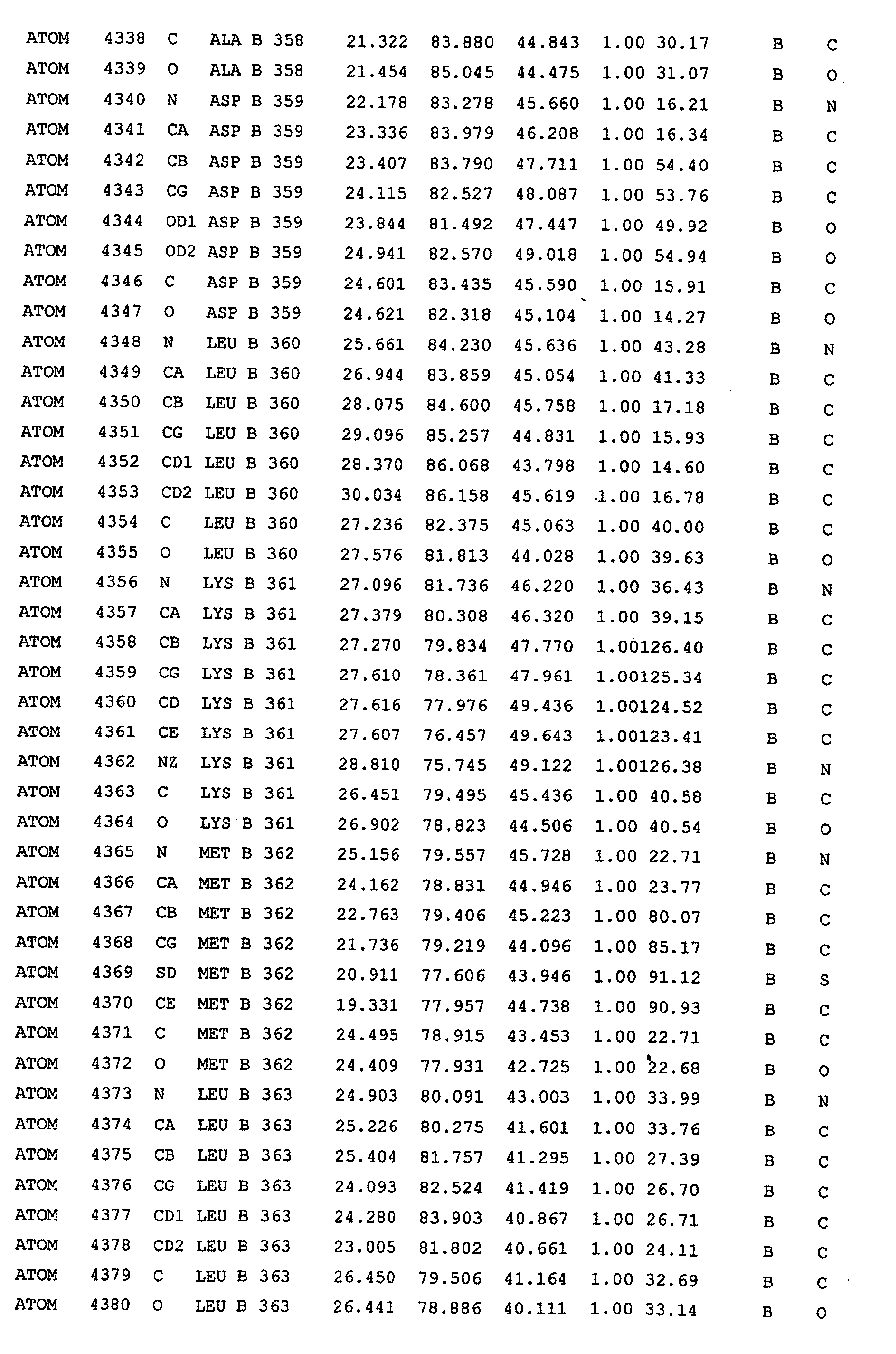

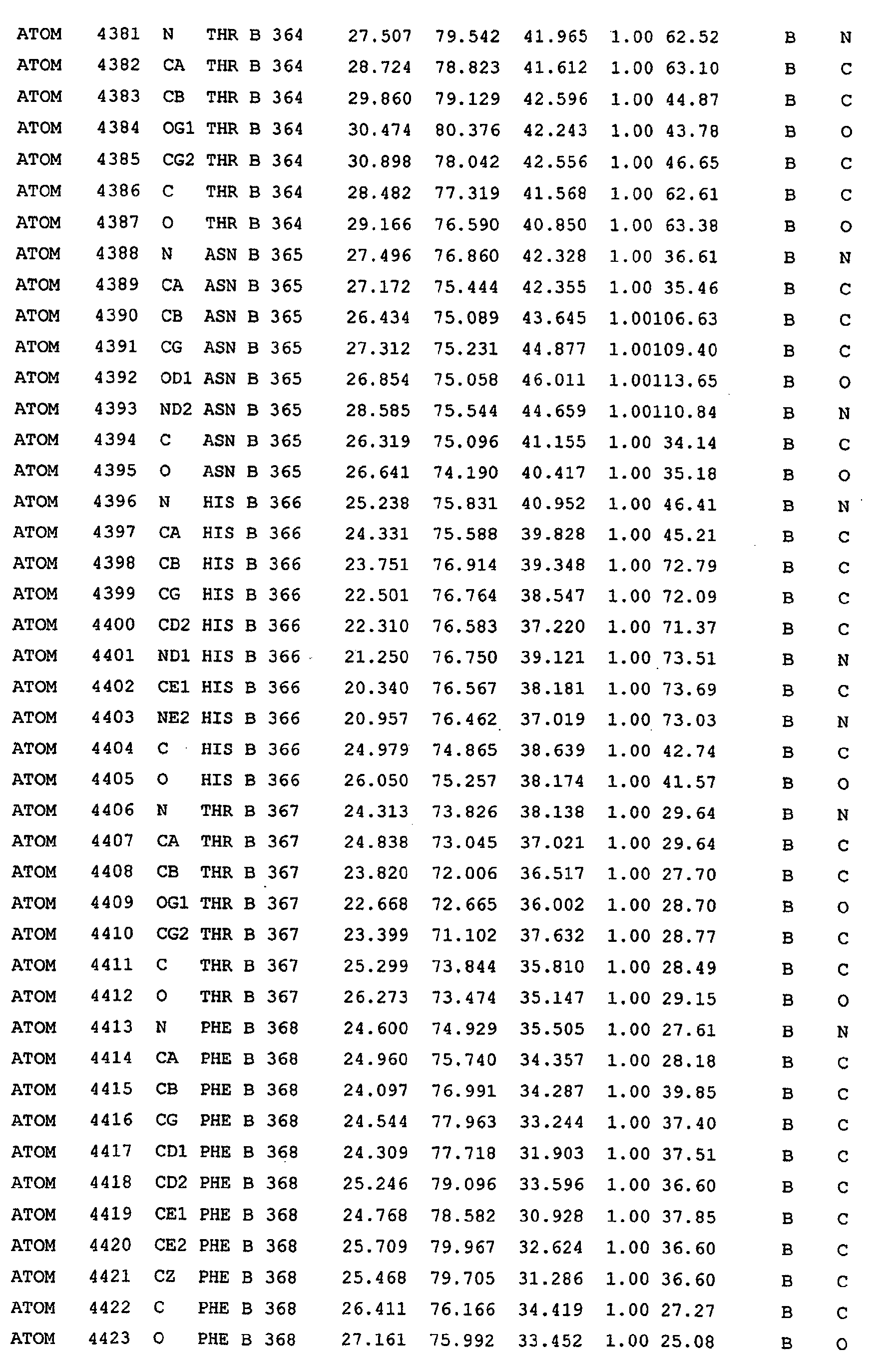

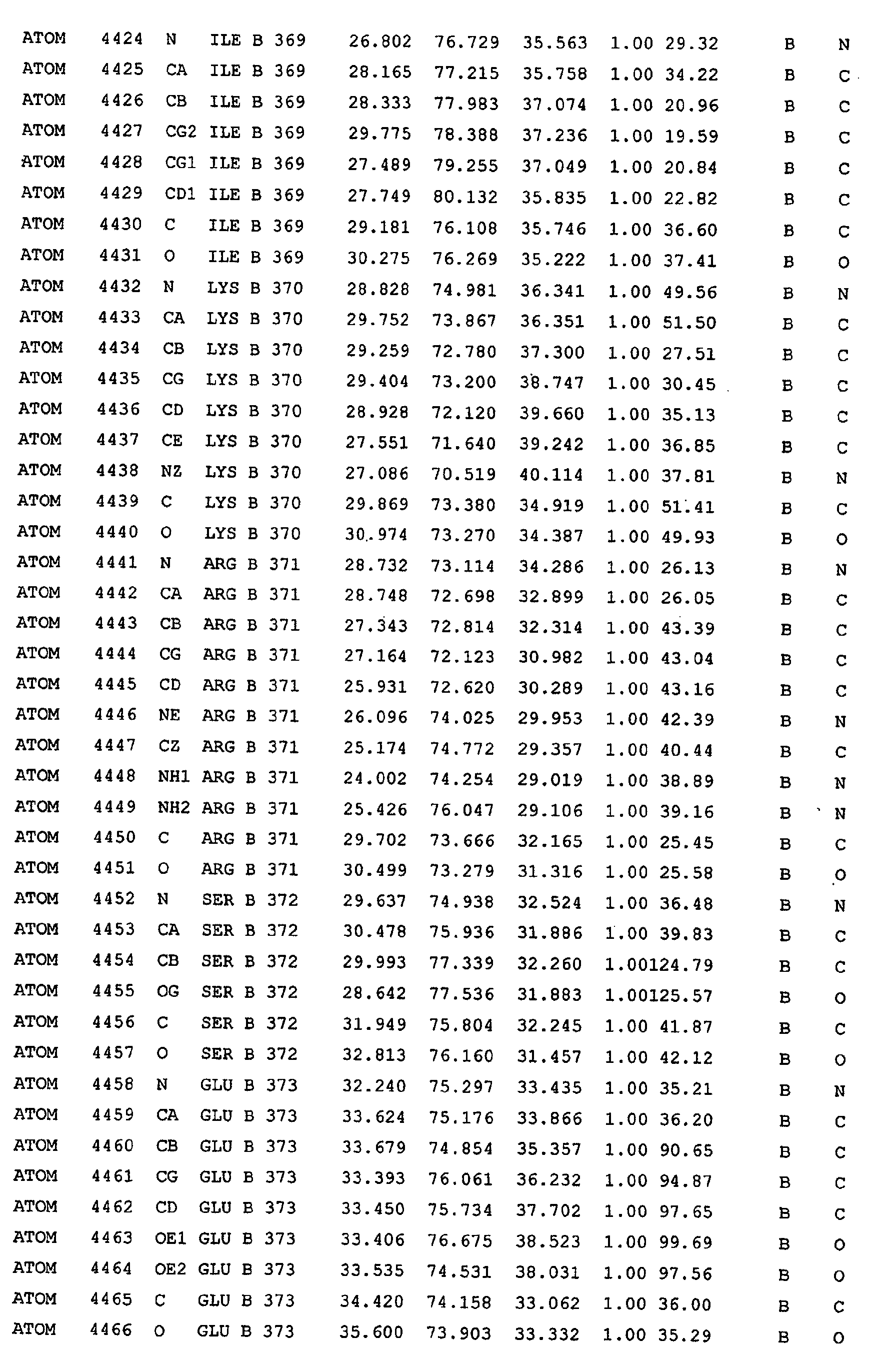

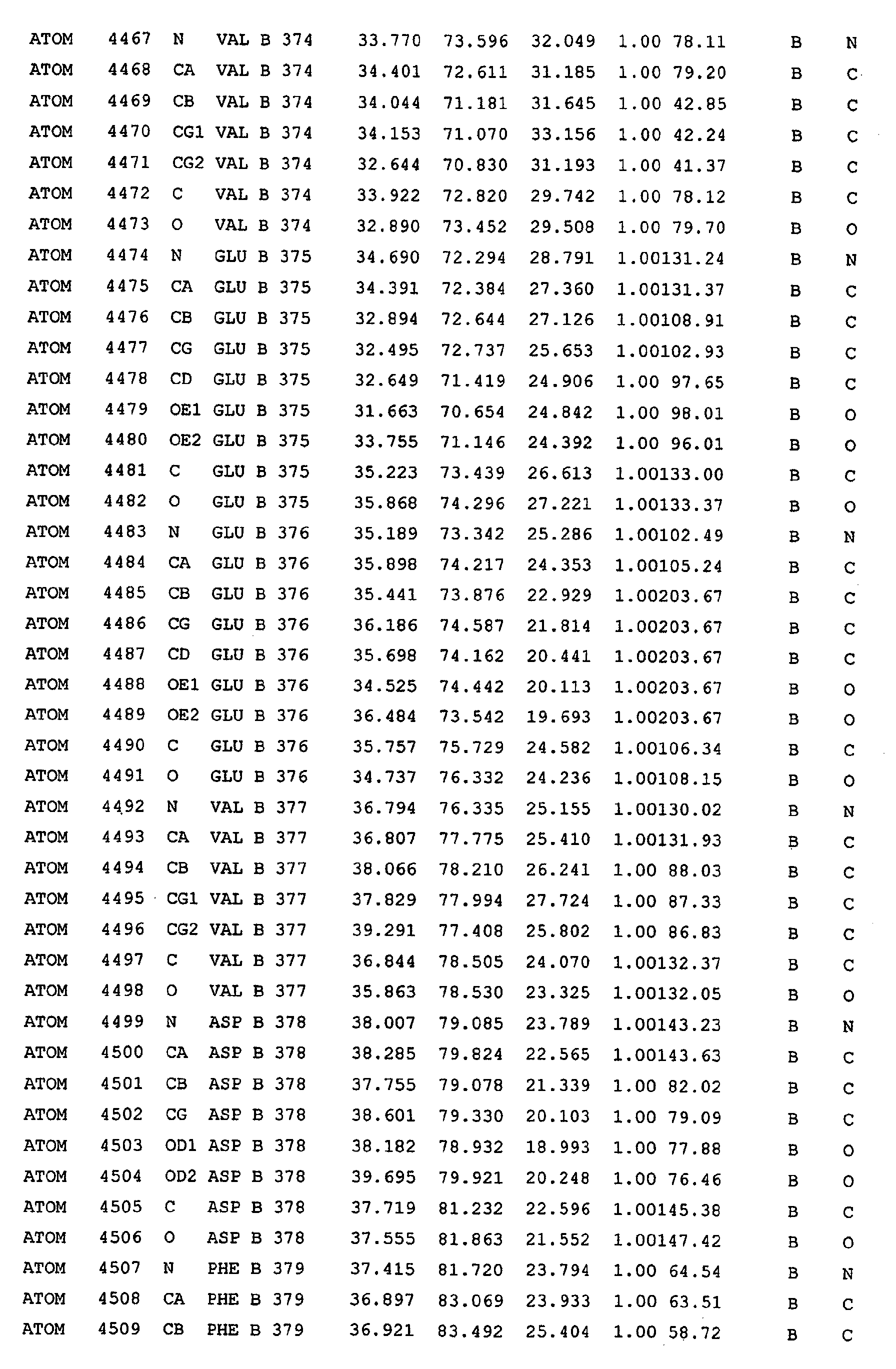

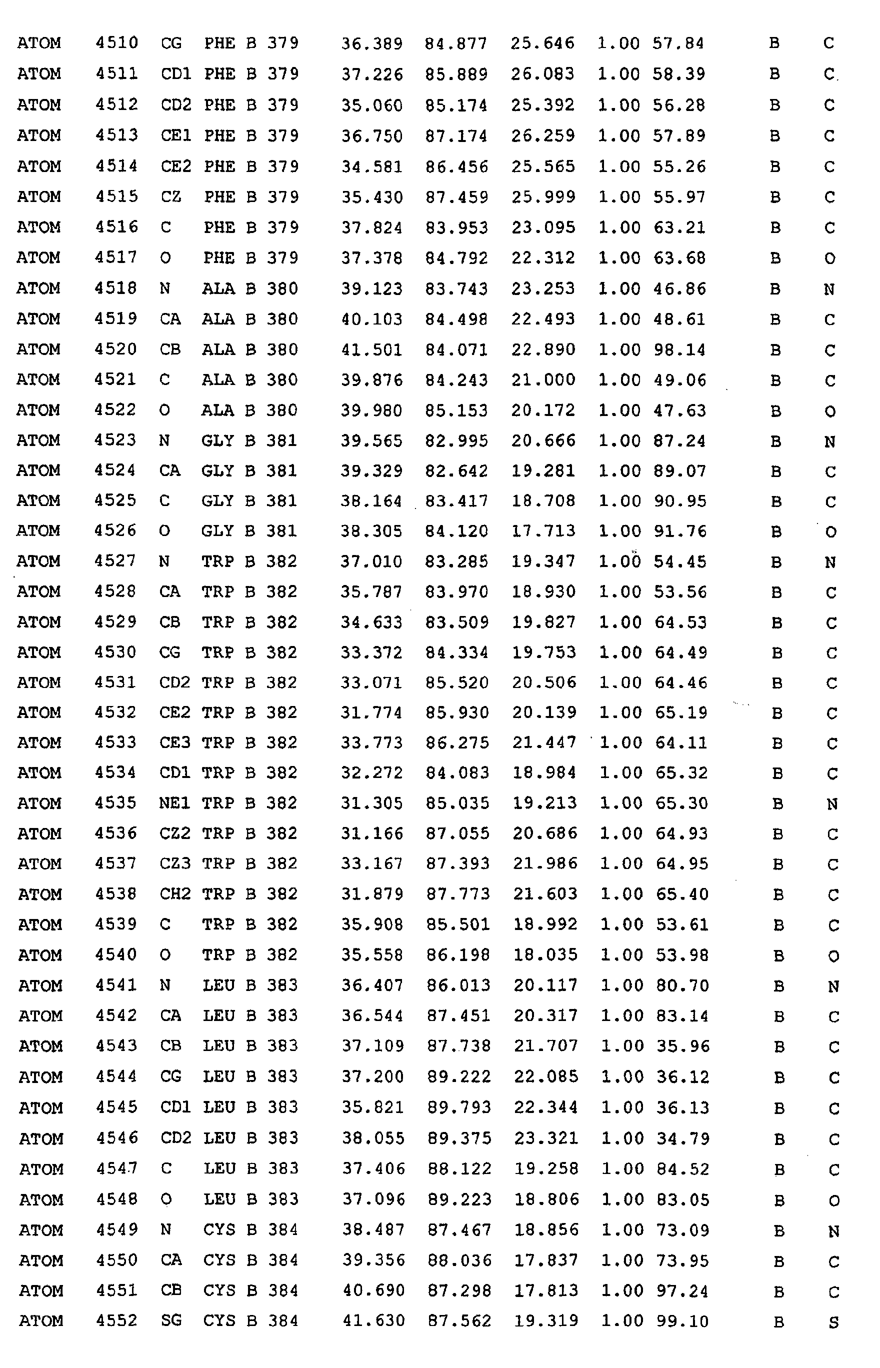

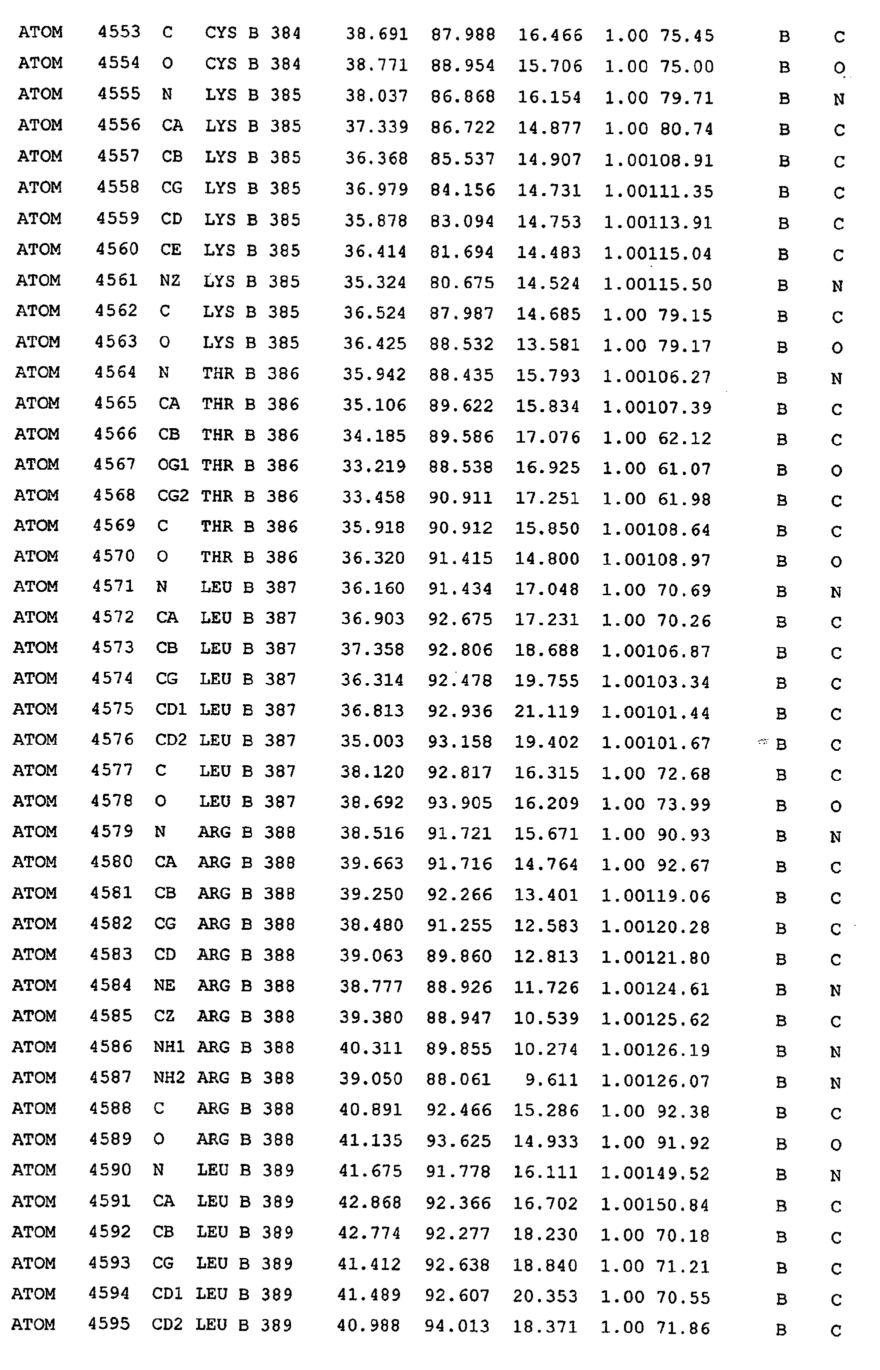

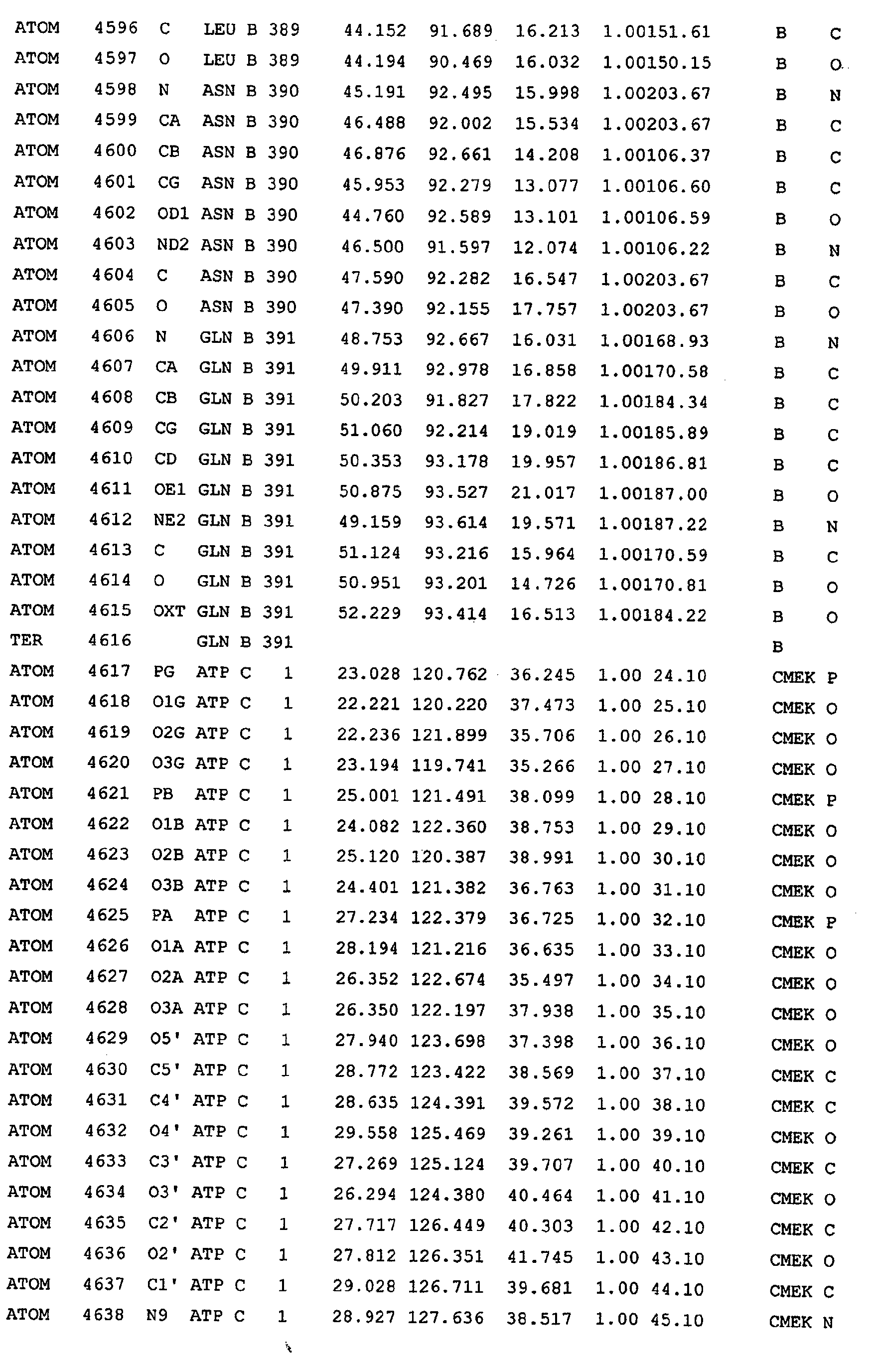

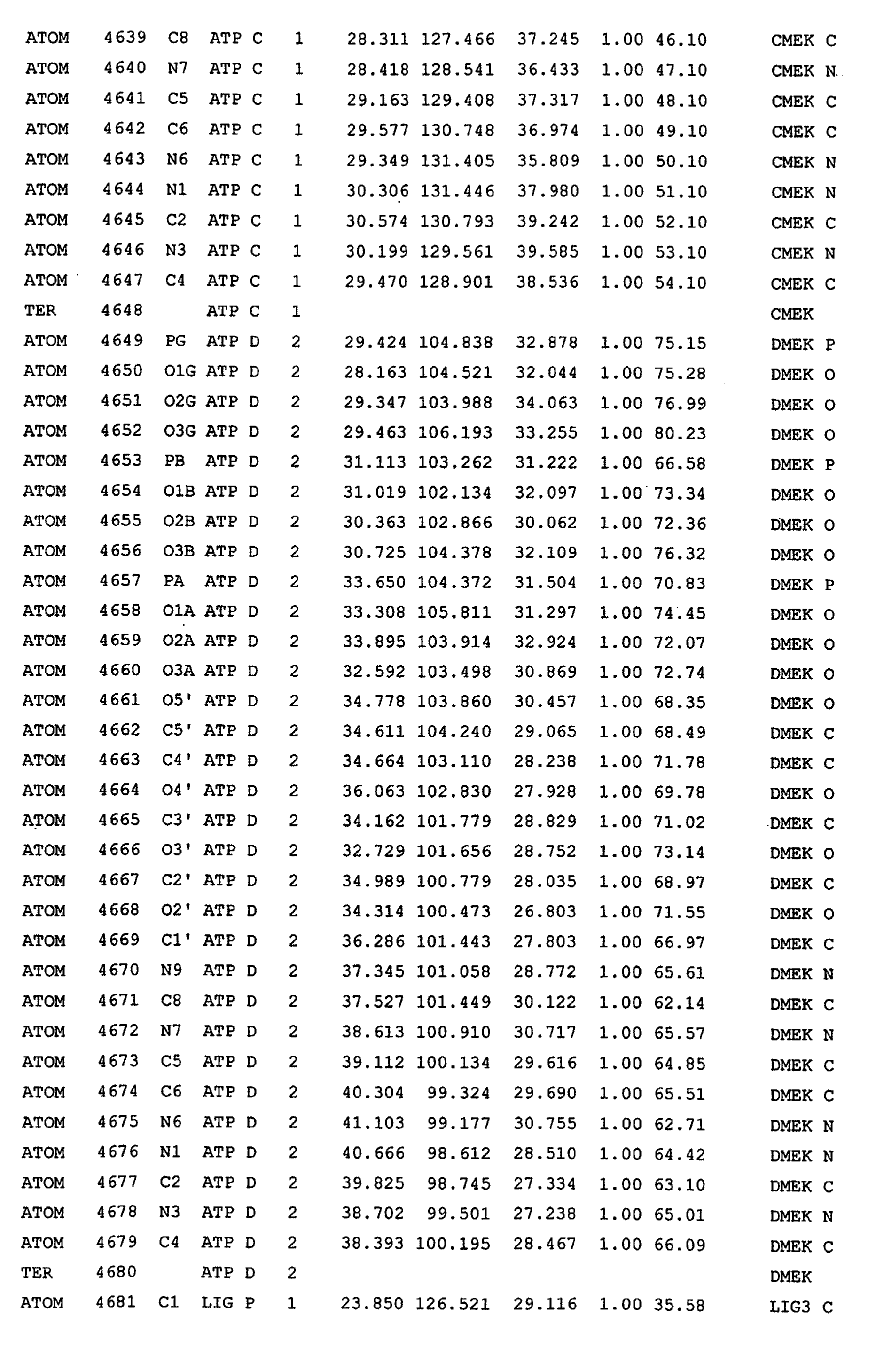

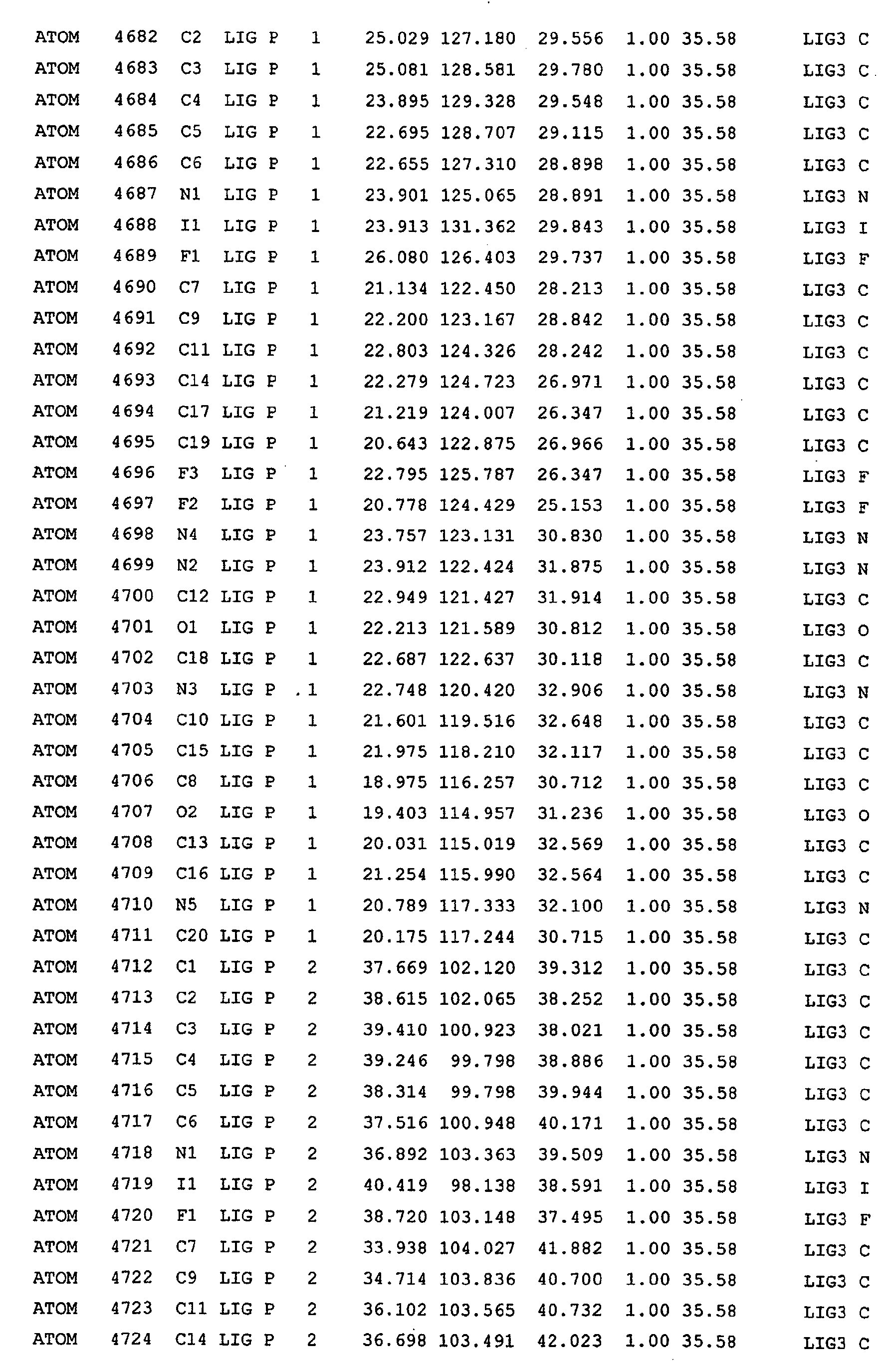

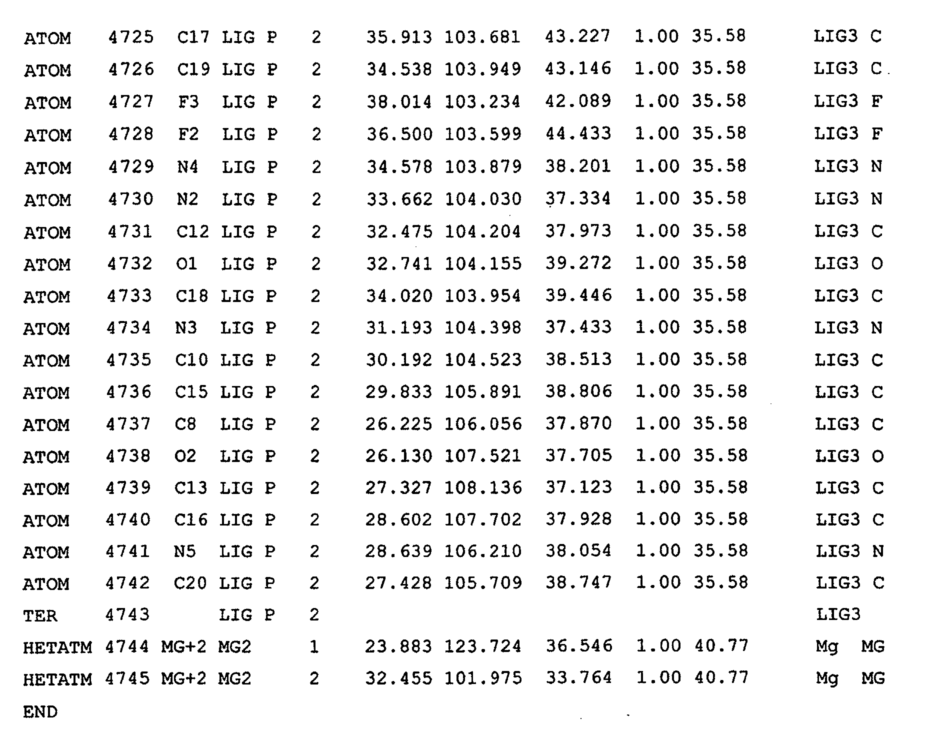

**Table 3**

| **Multiple Sequence Alignment of Inhibitor-Binding Site Residues of MEK1 and MEK2 That are Within Either 4 Å or 5 Å of the MEK1 Inhibitor-Binding Site** | | | |
|---|---|---|---|
| Residues that are within 4 Å of the ligand | | Residues that are within 5 Å of the ligand | |
| MEK1 | Corresponding residues in MEK2 | MEK1 | Corresponding residues in MEK2 |
| G77 | G81 | G77 | G81 |
| N78 | N82 | N78 | N82 |
| G79 | G83 | G79 | G83 |
| G80 | G84 | G80 | G84 |
| K97 | K101 | K97 | K101 |
| I99 | I103 | I99 | I103 |
| L115 | L119 | L115 | L119 |
| L118 | L122 | L118 | L122 |
| V127 | V131 | I126 | I130 |
| F129 | F133 | V127 | V131 |
| I141 | I145 | G128 | G132 |
| M143 | M147 | F129 | F133 |
| C207 | C211 | I141 | I145 |
| D208 | D212 | M143 | M147 |
| F209 | F213 | D190 | D194 |
| G210 | G214 | N195 | N199 |
| V211 | V215 | L206 | L210 |
| S212 | S216 | C207 | C211 |
| L215 | L219 | D208 | D212 |
| I216 | I220 | F209 | F213 |
| M219 | M223 | G210 | G214 |
| | | V211 | V215 |
| | | S212 | S216 |
| | | L215 | L219 |
| | | I216 | I220 |
| | | M219 | M223 |
| | | F223 | F227 |

**Table 4**

| **Multiple Sequence Alignment of Inibitor-Binding Site Residues of MEK1 and MEK2 That are Within Either 4 Å or 5 Å of the MEK1 Cofactor-Binding Site** | | | |
|---|---|---|---|
| Residues that are within 4 Å of the ATP co-factor | | **Residues that are within 5 Å of the ATP co-factor** | |
| MEK1 | Corresponding residues in MEK2 | MEK1 | Corresponding residues in MEK2 |
| L74 | L78 | L74 | L78 |
| G75 | G79 | G75 | G79 |
| A76 | A80 | A76 | A80 |
| G77 | G81 | G77 | G81 |
| N78 | N82 | N78 | N82 |
| G80 | G84 | G79 | G83 |
| V81 | V85 | G80 | G84 |
| V82 | V86 | V81 | V85 |
| A95 | A99 | V82 | V86 |
| K97 | K101 | A95 | A99 |
| V127 | V131 | K97 | K101 |
| M143 | M147 | V127 | V131 |
| E144 | E148 | M143 | M147 |
| H145 | H149 | E144 | E148 |
| M146 | M150 | H145 | H149 |
| G149 | G153 | M146 | M150 |
| S150 | S154 | D147 | D151 |
| D152 | D156 | G149 | G153 |
| Q153 | Q157 | S150 | S154 |
| K192 | K196 | D152 | D156 |
| S194 | S198 | Q153 | Q157 |
| N195 | N199 | D190 | D194 |
| L197 | L201 | K192 | K196 |
| D208 | D212 | S194 | S198 |
| V224 | V228 | N195 | N199 |
| | | L197 | L201 |
| | | C207 | C211 |
| | | D208 | D212 |
| | | V224 | V228 |
| | | G225 | G229 |

**Table 5**

| **Sequence Identities Within the Kinase Domain and the Inhibitor-Binding Site for Human MEK1 Versus Other Members of the MEK Family** | | | | | | |
|---|---|---|---|---|---|---|
| Sequence Name (Human) | Kinase Domain Sequence Identity to MEK1 | | Ligand-Binding Site Sequence Identity (within 4 Å) to MEK1 | | Ligand-Binding Site Sequence Identity (within 5 Å) to MEK1 | |
| | Counting gaps | Not counting gaps | Counting gaps | Not counting gaps | Counting gaps | Not counting gaps |
| MPK2 | 84.83 | 85.89 | 100 | 100 | 100 | 100 |
| MPK3 | 35.26 | 41.88 | 71.43 | 71.43 | 62.96 | 62.96 |
| MPK4 | 35.74 | 42.96 | 71.43 | 71.43 | 70.37 | 70.37 |
| MPK5 | 38.11 | 46.82 | 80.95 | 80.95 | 81.48 | 81.48 |
| MPK6 | 35.56 | 42.55 | 71.43 | 71.43 | 62.96 | 62.96 |
| MPK7 | 34.45 | 40.79 | 61.90 | 61.90 | 62.96 | 62.96 |

**Table 6**

| **Sequence Identities Within the Kinase Domain and the ATP-Binding Site for Human MEK1 Versus Other Members of the MEK Family** | | | | | | |
|---|---|---|---|---|---|---|
| Sequence Name (Human) | Kinase Domain Sequence Identity to MEK1 | | ATP-Binding Site Sequence Identity (within 4 Å) to MEK1 | | ATP-Binding Site Sequence Identity (within 5 Å) to MEK1 | |
| | Counting gaps | Not counting gaps | Counting gaps | Not counting gaps | Counting gaps | Not counting gaps |
| MPK2 | 84.83 | 85.89 | 100 | 100 | 100 | 100 |
| MPK3 | 35.26 | 41.88 | 76.00 | 76.00 | 76.67 | 76.67 |
| MPK4 | 35.74 | 42.96 | 68.00 | 68.00 | 66.67 | 66.67 |
| MPK5 | 38.11 | 46.82 | 76.00 | 76.00 | 80.00 | 80.00 |
| MPK6 | 35.56 | 42.55 | 76.00 | 76.00 | 76.67 | 76.67 |
| MPK7 | 34.45 | 40.79 | 60.00 | 60.00 | 60.00 | 60.00 |

**Table 7**

| **A Detailed Description of the Secondary Structure of MEK1 as Determined by the X-ray Crystallographic Structural Analysis** | |
|---|---|
| Secondary Structure | Amino Acid Residues |
| Unstructured* | 62:67 |
| Beta Sheet | 68:76 |
| Beta Sheet | 81:87 |
| Beta Sheet | 92:98 |
| Alpha Helix | 105:120 |
| Beta Sheet | 129:134 |
| Beta sheet | 139:143 |
| Beta Sheet | 149:150 |
| Alpha Helix | 151:156 |
| Alpha Helix | 163:184 |
| Beta Sheet | 196:198 |
| Beta Sheet | 204:206 |
| Alpha Helix | 213:218 |
| Flexible Loop* | 219:231 |
| Alpha Helix | 232:236 |
| Alpha Helix | 243:258 |
| Flexible Loop* | 259:309 |
| Alpha Helix | 310:318 |
| Alpha Helix | 332:341 |
| Alpha Helix | 352:357 |
| Alpha Helix | 359:362 |
| Alpha Helix | 372:379 |
| Unstructured* | 380:383 |

| | |
|---|---|
| * These features were not included in the structural model due to the lack of interpretable electron density. | |

**Table 8**

| **RMS Deviation Values for Selected C alpha and Backbone Atoms of MEK1 and MEK2 Structures*** | | | | | | | |
|---|---|---|---|---|---|---|---|
| RMSD between overall MEK1 and MEK2 structures | | RMSD between the kinase domain of MEK1 and MEK2 structures | | RMSD between the 4 Å inhibitor binding site residues of MEK1 and MEK2 | | RMSD between the 5 Å inhibitor binding site residues of MEK1 and MEK2 | |
| Using C alpha only | Using Backbone atoms | Using C alpha only | Using Backbone atoms | Using C alpha only | Using Backbone atoms | Using C alpha only | Using Backbone atoms |
| 1.02 | 1.11 | 0.95 | 1.02 | 1.08 | 1.22 | 0.99 | 1.12 |

| | | | | | | | |
|---|---|---|---|---|---|---|---|
| * The residues used in the RMS deviation calculations can be found in the embodiment. | | | | | | | |

## Claims

1. An isolated peptide selected from the group consisting of:
a Mitogen Activated Protein Kinase 1 /ERK kinase 1 (MEK1) peptide having an NH₂-terminal truncation lacking from at least 30 to at most 70 amino acid residues from the NH₂-terminal region of the full-length MEK1 peptide set forth in SEQ ID NO: 2, or a conservatively substituted variant thereof;
a MEK1 peptide having a deletion of insertion loop-forming amino acid residues selected from amino acid 280 to amino acid 323 of SEQ ID NO: 2 or at least 40 amino acids from between amino acid residue 264 and amino acid 310 of SEQ ID NO: 2, or a conservatively substituted variant thereof;
a peptide comprising (1) an NH₂-terminal truncation lacking from at least 30 to at most 70 amino acid residues from the NH₂-terminal region of the full-length MEK1 peptide set forth in SEQ ID NO: 2; and (2) a deletion of insertion loop-forming amino acid residues selected from amino acid 280 to amino acid 323 of SEQ ID NO: 2 or at least 40 amino acids from between amino acid residue 264 and amino acid 310 of SEQ ID NO: 2, or a conservatively substituted variant thereof;
a Mitogen Activated Protein Kinase 2/ERK kinase 2 (MEK2) peptide having an NH₂-terminal truncation lacking from at least 34 to at most 74 amino acid residues from the NH₂-terminal region of the full-length MEK2 peptide set forth in SEQ ID NO: 4, or a conservatively substituted variant thereof; and
a peptide that is defined by the structural coordinates of the MEK1 or MEK2 peptide as set forth in Table 1 or Table 2 or a related set of structural coordinates having a root mean square deviation of not more than about 1.25 Å away from the core C alpha atoms of the MEK1 or MEK2 structural coordinates as set forth in Table 1 or Table 2.

2. A peptide comprising a binding pocket selected from the group consisting of :
(a) a Mitogen Activated Protein Kinase 1 /ERK kinase 1 (MEK1) peptide ligand-binding pocket that is defined by the structural coordinates of the following amino acid residues within about 4 Å of a MEK1 inhibitor located in the ligand-binding site: G77, N78, G79, G80, K97, I99, L115, L118, V127, F129, I141, M143, C207, D208, F209, G210, V211, S212, L215, I216 and M219 of SEQ ID NO: 2, or a conservatively substituted variant thereof;
(b) a MEK1 peptide ligand-binding pocket that is defined by the structural coordinates of the following amino acid residues within about 5 Å of a MEK1 inhibitor located in the ligand-binding site: G77, N78, G79, G80, K97, 199, L115, L118, I126, V127, G128, F129, I141, M143, D190, N195, L206, C207, D208, F209, G210, V211, S212, L215, I216, M219 and F223 of SEQ ID NO: 2, or a conservatively substituted variant thereof;
(c) a Mitogen Activated Protein Kinase 2/ERK kinase 2 (MEK2) MEK2 peptide ligand-binding pocket that is defined by the structural coordinates of the following amino acid residues within about 4 Å of a MEK2 inhibitor located in the ligand-binding site: G81, N82, G83, G84, K101, I103, L119, L122, V131, F133, I145, M147, C211, D212, F213, G214, V215, S216, L219, I220, M223 of SEQ ID NO: 4, or a conservatively substituted variant thereof;
(d) a MEK2 peptide ligand-binding pocket that is defined by structural coordinates of the following amino acid residues within about 5 Å of a MEK2 inhibitor located in the ligand-binding site: G81, N82, G83, G84, K101, I103, L119, L122, I130, V131, G132, F133, I145, M147, D194, N199, L210, C211, D212, F213, G214, V215, S216, L219, I220, M223, F227.of SEQ ID NO: 4, or a conservatively substituted variant thereof;
(e) a MEK1 peptide cofactor-binding pocket that is defined by the structural coordinates of the following residues within about 4 Å of a cofactor located in the cofactor-binding site: L74, G75, A76, G77, N78, G80, V81, V82, A95, K97, V127, M143, E144, H145, M146, G149, S150, D152, Q153, K192, S194, N195, L197, D208 and V224 of SEQ ID NO: 2, or a conservatively substituted variant thereof;
(f) a MEK1 peptide cofactor-binding pocket that is defined by the structural coordinates of the following residues within about 5 Å of a cofactor located in the cofactor-binding site: L74, G75, A76, G77, N78, G79, 80, V81, V82, A95, K97, V127, M143, E144, H145, M146, D147, G149, S150, D152, Q153, D190, K192, S194, N195, L197, C207, D208, V224 and G225 of SEQ ID NO:2, or a conservatively substituted variant thereof;
(g) a MEK2 peptide cofactor-binding pocket that is defined by the structural coordinates of the following residues within about 4 Å of a cofactor located in the cofactor-binding site: L78, G79, A80, G81, N82, G84, V85, V86, A99, K101, V131, M147, E148, H149, M150, G153, S154, D156, Q157, K196, S198, N199, L201, D212, V228 of SEQ ID NO: 4, or a conservatively substituted variant thereof;
(h) a MEK2 peptide cofactor-binding pocket that is defined by the structural coordinates of the following residues within about 5 Å of a cofactor in the cofactor-binding site: L78, G79, A80, G81, N82, G83, G84, V85, V86, A99, K101, V131, M147, E148, H149, M150, D151, G153, S154, D156, Q157, D194, K196, S198, N199, L201, C211, D212, V228, G229 of SEQ ID NO: 4, or a conservatively substituted variant thereof; and
(i) a binding pocket that is defined by the atoms found in the structural coordinates of the MEK1 or MEK2 peptide as set forth in Table 1 or Table 2, or in a related set of structural coordinates having a root mean square deviation of not more than about 1.25 Å away from the binding pocket C alpha atoms of the MEK1 or MEK2 binding pockets according to (a) - (h), or a conservatively substituted variant thereof.

3. A crystalline structure of a peptide:ligand:cofactor complex comprising a peptide according to claim 1 or claim 2.

4. Three-dimensional structural coordinates of a peptide:ligand:cofactor complex, comprising:
a peptide;
a cofactor; and
a ligand,
wherein the complex has the atomic coordinates set forth in Table 1 or Table 2, or a related set of structural coordinates having a root mean square deviation of not more than about 1.25 Å away from the core C alpha atoms of the MEK1 or MEK2 structural coordinates as set forth in Table 1 or Table 2.

5. An expression vector for producing the peptide according to claim 1 or claim 2 in a host cell comprising a polynucleotide encoding the modified peptide, and transcriptional and translational regulatory sequences functional in the host cell operably linked to the modified peptide.

6. A host cell stably transformed and transfected with a polynucleotide selected from the group consisting of a polynucleotide encoding the peptide according to claim 1 or claim 2, or a conservatively substituted variant thereof.

7. A method of utilizing molecular replacement to obtain structural information about a molecule or a molecular complex of unknown structure comprising:
crystallizing said molecule or molecular complex;
generating an X-ray diffraction pattern from said crystallized molecule or molecular complex; and
applying at least a portion of the structural coordinates set forth in Table 1 or Table 2, or a related set of structural coordinates having a root mean square deviation of not more than about 1.25 Å away from the core C alpha atoms of the MEK1 or MEK2 structural coordinates as set forth in Table 1 or Table 2, to the X-ray diffraction pattern to generate a three-dimensional electron density map of at least a portion of the molecule or molecular complex whose structure is unknown.

8. A method for generating a three-dimensional computer representation of a peptide according to claim 1 or claim 2, or a binding pocket thereof, comprising applying the atomic coordinates set forth in Table 1 or Table 2, or a related set of structural coordinates having a root mean square deviation of not more than about 1.25 Å away from the core C alpha atoms of the MEK1 or MEK2 structural coordinates as set forth in Table 1 or Table 2, to a computer algorithm to generate a three-dimensional representation of the peptide or peptide binding pocket.

9. A machine-readable medium having stored thereon data comprising the atomic coordinates as set forth in Table 1 or Table 2, or a related set of structural coordinates having a root mean square deviation of not more than about 1.25 Å away from the core C alpha atoms of the MEK1 or MEK2 structural coordinates as set forth in Table 1 or Table 2.

10. A method for modifying or designing a chemical entity having the potential to associate with a peptide according to claim 1 or claim 2, comprising,
(a) generating a three-dimensional computer representation of the peptide or a binding pocket of the peptide; and
(b) generating a chemical entity that spatially conforms to the three-dimensional representation of the peptide or the peptide binding pocket, wherein the chemical entity is generated by a method comprising (i) assembling molecular fragments into the chemical entity; (ii) de novo design of the chemical entity or a fragment thereof; (iii) selecting the chemical entity from a small molecule database; or (iv) modifying a known inhibitor, or portion thereof, of MEK activity.

11. A method for screening and identifying a potential inhibitor or enhancer of the activity of a peptide according to claim 1 or claim 2, comprising:
(a) generating a three-dimensional computer representation of the peptide or a binding pocket of the peptide;
(b) applying an iterative process whereby a chemical entity is applied to the three-dimensional representation to determine whether the chemical entity associates with the peptide or peptide binding pocket; and
(c) evaluating the effect(s) of the chemical entity on peptide activity to determine whether the chemical entity functions as an activity inhibitor or enhancer.

12. A method for evaluating the potential of a chemical entity to associate with a peptide according to claim 1 or claim 2, comprising
(a) generating a three-dimensional representation of the peptide or a binding pocket of the peptide;
(b) applying a three-dimensional representation of a chemical entity to the three-dimensional representation; and
(c) quantifying the association between the chemical entity and the binding pocket.

13. The method according to any one of claims 8, 10, 11, or 12, wherein the peptide binding pocket is selected from the group consisting of:
(a) a Mitogen Activated Protein Kinase 1 /ERK kinase 1 (MEK1) peptide ligand-binding pocket that is defined by the structural coordinates of the following amino acid residues within about 4 Å of a MEK1 inhibitor located in the ligand-binding site: G77, N78, G79, G80, K97, I99, L115, L118, V127, F129, I141, M143, C207, D208, F209, G210, V211, S212, L215, I216 and M219 of SEQ ID NO: 2, or a conservatively substituted variant thereof;
(b) a MEK1 peptide ligand-binding pocket that is defined by the structural coordinates of the following amino acid residues within about 5 Å of a MEK1 inhibitor located in the ligand-binding site: G77, N78, G79, G80, K97, 199, L115, L118, I126, V127, G128, F129, I141, M143, D190, N195, L206, C207, D208, F209, G210, V211, S212, L215, I216, M219 and F223 of SEQ ID NO: 2, or a conservatively substituted variant thereof;
(c) a Mitogen Activated Protein Kinase 2/ERK kinase 2 (MEK2) MEK2 peptide ligand-binding pocket that is defined by the structural coordinates of the following amino acid residues within about 4 Å of a MEK2 inhibitor located in the ligand-binding site: G81, N82, G83, G84, K101, I103, L119, L122, V131, F133, I145, M147, C211, D212, F213, G214, V215, S216, L219, I220, M223 of SEQ ID NO: 4, or a conservatively substituted variant thereof;
(d) a MEK2 peptide ligand-binding pocket that is defined by structural coordinates of the following amino acid residues within about 5 Å of a MEK2 inhibitor located in the ligand-binding site: G81, N82, G83, G84, K101, I103, L119, L122, I130, V131, G132, F133, I145, M147, D194, N199, L210, C211, D212, F213, G214, V215, S216, L219, I220, M223, F227 of SEQ ID NO: 4, or a conservatively substituted variant thereof;
(e) a MEK1 peptide cofactor-binding pocket that is defined by the structural coordinates of the following residues within about 4 Å of a cofactor located in the cofactor-binding site: L74, G75, A76, G77, N78, G80, V81, V82, A95, K97, V127, M143, E144, H145, M146, G149, S150, D152, Q153, K192, S194, N195, L197, D208 and V224 of SEQ ID NO: 2, or a conservatively substituted variant thereof;
(f) a MEK1 peptide cofactor-binding pocket that is defined by the structural coordinates of the following residues within about 5 Å of a cofactor in the cofactor-binding site: L74, G75, A76, G77, N78, G79, 80, V81, V82, A95, K97, V127, M143, E144, H145, M146, D147, G149, S150, D152, Q153, D190, K192, S194, N195, L197, C207, D208, V224 and G225 of SEQ ID NO:2, or a conservatively substituted variant thereof;
(g) a MEK2 peptide cofactor-binding pocket that is defined by the structural coordinates of the following residues within about 4 Å of a cofactor located in the cofactor-binding site: L78, G79, A80, G81, N82, G84, V85, V86, A99, K101, V131, M147, E148, H149, M150, G153, S154, D156, Q157, K196, S198, N199, L201, D212, V228 of SEQ ID NO: 4, or a conservatively substituted variant thereof;
(h) a MEK2 peptide cofactor-binding pocket that is defined by the structural coordinates of the following residues within about 5 Å of a cofactor located in the cofactor-binding site: L78, G79, A80, G81, N82, G83, G84, V85, V86, A99, K101, V131, M147, E148, H149, M150, D151, G153, S154, D156, Q157, D194, K196, S198, N199, L201, C211, D212, V228, G229 of SEQ ID NO: 4, or a conservatively substituted variant thereof; and
(i) a binding pocket that is defined by the atoms found in the structural coordinates of the MEK1 or MEK2 peptide as set forth in Table 1 or Table 2, or in a related set of structural coordinates having a root mean square deviation of not more than about 1.25 Å away from the binding pocket C alpha atoms of the MEK1 or MEK2 binding pockets according to (a) - (h), or a conservatively substituted variant thereof.

14. A method of purifying a peptide according to claim 1 or claim 2 from a fermentation broth containing the peptide and contaminant proteins other than the peptide, comprising subjecting the fermentation broth to immobilized metal chelate chromatography comprising pyrrole-2-carboxylate, zinc chloride, and an immobilized metal selected from the group consisting of nickel, zinc, copper and cobalt.

15. A method of growing the crystalline structure according to claim 3, comprising:
providing a peptide solution comprising the peptide, a ligand, a cofactor, a buffering agent, a reducing agent, and a source of ionic strength;
providing a precipitant solution comprising
(a) if the peptide is MEK1 peptide, polyethylene glycol (PEG), a source of ionic strength, a buffering agent, and a reducing agent; or
(b) if the peptide is MEK2 peptide, a source of ionic strength, a buffering agent, and a reducing agent;
mixing a droplet of said peptide solution with a droplet of said precipitant solution;
suspending the resulting mixed droplet over a well of said precipitant solution at a vapor pressure of the solution in said well being lower than in the resulting solution in the mixed droplet; and
allowing the suspended mixed droplet to stand for a prolonged period until a peptide:ligand:cofactor ternary complex crystal grows to a size suitable for X-ray diffraction.
